# EUROPEAN PATENT SPECIFICATION

(11) **EP 2 341 915 B1**
(45) Date of publication and mention of the grant of the patent: **20.03.2019**
(21) Application number: 08807185.7
(22) Date of filing: 01.08.2008
(51) Int. Cl.: A61K 31/80, A61P 19/02, A61P 17/06, A61P 29/00, C07F 9/6593, C07F 9/26, C07F 9/38, C07F 9/40, C07C 211/14, C07C 237/10, C07F 9/6581

(54) **PHOSPHORYLATED DENDRIMERS AS ANTIINFLAMMATORY DRUGS**
PHOSPHORYLIERTE DENDRIMERE ALS ENTZÜNDUNGSHEMMENDE ARZNEIMITTEL
DENDRIMÈRES PHOSPHORYLÉS EN TANT QUE MÉDICAMENTS ANTI-INFLAMMATOIRES

(43) Date of publication of application: 13.07.2011
(73) Proprietor: Centre National de la Recherche Scientifique, 75794 Paris Cedex 16 (FR); INSERM (Institut National de la Santé et de la Recherche Médicale), 75654 Paris Cedex 13 (FR); Université Paul Sabatier (Toulouse III), 31062 Toulouse Cedex 9 (FR)
(72) Inventor: POUPOT, Mary, F-31280 Aigrefeuille (FR); POUPOT, Rémy, F-31280 Aigrefeuille (FR); FOURNIE, Jean-Jacques, F-31450 Corronsac (FR); PORTEVIN, Damien, London NW 42NY (GB); FRUCHON, Séverine, F-31400 Toulouse (FR); DAVIGNON, Jean-Luc, F-31700 Beauzelle (FR); TURRIN, Cédric-Olivier, F-31400 Toulouse (FR); CAMINADE, Anne-Marie, F-31400 Toulouse (FR); MAJORAL, Jean-Pierre, F-31520 Ramonville Saint-Agne (FR); ROLLAND, Olivier, F-75018 Paris (FR)
(74) Representative: Grosset-Fournier, Chantal Catherine
(86) International application number: PCT/IB2008/002547
(87) International publication number: WO 2010/013086

(56) References cited:
- WO-A-98/03573
- WO-A-03/080121
- WO-A-03/089010
- WO-A-2006/024769
- WO-A-2006/040579
- WO-A-2007/106437
- WO-A2-2004/047866
- US-A1- 2008 317 699
- POUPOT M ET AL: "Design of phosphorylated dendritic architectures to promote human monocyte activation" THE FASEB JOURNAL, vol. 20, no. 13, 1 November 2006 (2006-11-01), pages 2339-2351, XP009116041 ISSN: 0892-6638 cited in the application
- ROLLAND O ET AL: "Tailored control and optimisation of the number of phosphonic acid termini on phosphorus-containing dendrimers for the ex-vivo activation of human monocytes" CHEMISTRY - A EUROPEAN JOURNAL, vol. 14, no. 16, 29 May 2008 (2008-05-29), pages 4836-4850, XP009116043 ISSN: 0947-6539 cited in the application
- FRUCHON S ET AL: "Anti-inflammatory and immunosuppressive activation of human monocytes by a bioactive dendrimer" JOURNAL OF LEUKOCYTE BIOLOGY, vol. 85, no. 3, March 2009 (2009-03), pages 553-562, XP009115979 ISSN: 0741-5400

## Description

The present invention relates to phosphorylated dendrimers for use in the treatment of chronic inflammatory processes.

Dendrimers are macromolecules constituted by monomers which combine according to a tree-like process around a plurifunctional central core.

Dendrimers, also called "cascade molecules", are highly branched functional polymers with a defined structure. These macromolecules are effectively polymers since they are based on the combination of repeating units. However, dendrimers differ fundamentally from standard polymers to the extent that they have specific properties due to their tree-like construction. The molecular weight and the form of the dendrimers can be precisely controlled (Fréchet, J. M. J. et al. (2001) Dendrimers and other dendritic polymers, John Wiley and Sons, New York, NY; Newkome, G. R. et al. (2001) Dendrimers and dendrons: concepts, syntheses, applications 2nd ed Ed., Wiley-VCH, Weinheim).

The dendrimers are constructed stepwise, by the repetition of a sequence of reactions allowing the multiplication of each repeating unit and terminal functions. Each sequence of reactions forms what is called a "new generation". The tree-like construction is carried out by the repetition of a sequence of reactions which makes it possible to obtain a new generation and a growing number of identical branches at the end of each reaction cycle. After a few generations, the dendrimer generally assumes a highly branched and plurifunctionalized globular form due to the numerous terminal functions present at the periphery. These structural features, together with the high density of chemical reactive functions on the outer shell of these molecules enhanced their use in biology and medicine (Cloninger, M. J. (2002) Curr Opin Chem Biol 6(6), 742-748). Dendrimers can be used as drug carriers that are able to enhance aqueous solubility, improve drug transit across biological barriers and target the drug delivery to injured tissues (Lee, C. C. et al. (2005) Nat Biotechnol 23(12), 1517-1526; Cheng, Y. et al. (2008) Front Biosci 13, 1447-1471). Dendrimers also act as drugs themselves. Among others, dendrimers glucosamine conjugates were safely used to prevent scar tissue formation *via* immuno-modulatory and anti-angiogenic effects (Shaunak, S. et al. (2004) Nat Biotechnol 22(8), 977-984). Multiple antigenic peptide dendrimers have proved to be promising compounds for immune response modification or immuno-diagnosis (Crespo, L. et al. (2005) Chem Rev 105(5), 1663-1681; Singh, P. (2007) Biotechnol Appl Biochem 48(Pt 1), 1-9). Polyanionic (with sulfonate groups) dendrimers were shown to provide an efficient microbicide activity against different viruses (McCarthy, T. D. et al. (2005) Mol Pharm 2(4), 312-318). Cationic phosphorus-containing dendrimers reduce the replication of the abnormal scrapie isoform of the prion protein in mice (Solassol, J. et al. (2004) J Gen Virol 85(Pt 6), 1791-1799). Recently the inventors reported the effects of synthetic molecules belonging to the dendrimer family on the innate immune system (Poupot, M. et al. (2006) Faseb J 20(13), 2339-2351; Griffe, L. et al. (2007) Angew Chem Int Ed Engl 46(14), 2523-2526; Rolland, O. et al. (2008) Chem. Eur. J., 14(16), 4836-4850).

Mononuclear phagocytes, including monocytes and macrophages (MΦ, the tissue-counterpart of the former), are essential in innate immunity as a first line of defence against bacterial and parasitic infections. They also ensure the engaging of the delayed adaptive immune response. Monocytes/MΦ are a heterogeneous population encompassing a large spectrum of phenotypes from pro-inflammatory to anti-inflammatory responses, depending on the stimulus they receive. Indeed, MΦ activation can take several aspects. Besides the classical activation pathway, a so-called alternative activation mechanism emerged. The classical activation pathway of MΦ is mediated by interferon (IFN)-γ as primer and then triggering by tumor necrosis factor (TNF) or bacterial lipopolysaccharides (LPS). These MΦ produce mediators making them effector cells in type Th-1 cellular immune responses and are cytotoxic effectors against intracellular pathogens. The "alternative activation" of MΦ describes closely related responses induced either by interleukin (IL)-4 or IL-13 or by IL-10 or glucocorticoids. These MΦ appear to be involved in immuno-suppression and tissue repair. This dual classification with pro-inflammatory classically-activated MΦ and anti-inflammatory alternatively-activated MΦ has been recently broadened with the proposition of type 2-activated MΦ. Although they are activated, after IFN-γ priming or not, by ligation of FcγR and Toll-like receptors (TLR) triggering, type 2 MΦ rather display anti-inflammatory responses. The closely interlinked pathways of MΦ activation and the intricate responses displayed by these cells explain the still confusing classification of polarized mononuclear phagocytes. During infection, MΦ are critical mediators of inflammatory processes aimed at the removal of pathogens. However, inflammation is also associated with deleterious effects for the tissues and must be repressed to allow complete healing. Due to their dual pattern of activation, MΦ play a pivotal role both in triggering and resolving inflammation.

The inventors surprisingly discovered that some phosphorylated dendrimers lead to an anti-inflammatory type activation of the monocytes.

Thus one of the purposes of the present invention is to propose a novel anti-inflammatory treatment using synthetic and essentially non-toxic compounds with low production costs.

Thus, the present invention relates to dendrimers with monophosphonic or bisphosphonic terminations for the treatment of chronic inflammatory diseases, in particular auto-immune diseases.

Said treatment is mediated by an anti-inflammatory type activation of the monocytes.

Some dendrimers used according to the present invention and their preparation are disclosed in the international application WO 2006/024769.

Dendrimers of structure PAMAM are in particular described by Tomalia, D. A. et al. (1985) Polym. J. (Tokyo) 17, 117; Tomalia, D. A. et al. (1986) Macromolecules 19, 2466.

Dendrimers of structure DAB are in particular described by de Brabander-van den Berg, E. M. M. et al. (1993) Angew. Chem. Int. Ed. Engl. 32, 1308.

The dendrimers of structure PMMH are in particular described in "A general synthetic strategy for neutral phosphorus containing dendrimers" by Launay, N. et al. (1994) Angew. Chem. 106, 1682 / Angew. Chem. Int. Ed. Engl. 33, 1589 and in "Synthesis of bowl-shaped dendrimers from generation 1 to generation 8" Launay, N. et al. (1997) J. Organomet. Chem. 529, 51.

An example of a dendrimer of type PAMAM for which n = 4 and m = 4 is represented below:

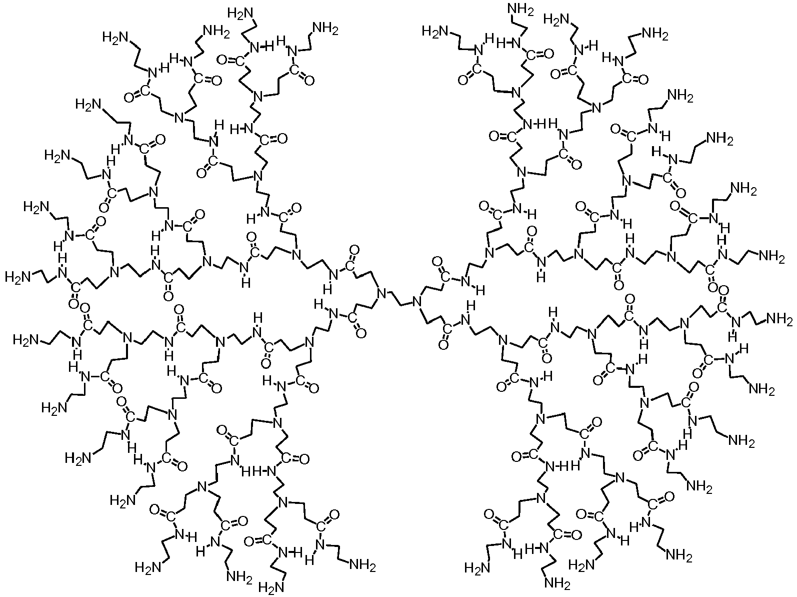

An example of a dendrimer of type DAB, for which n = 5 and m = 4 is represented below:

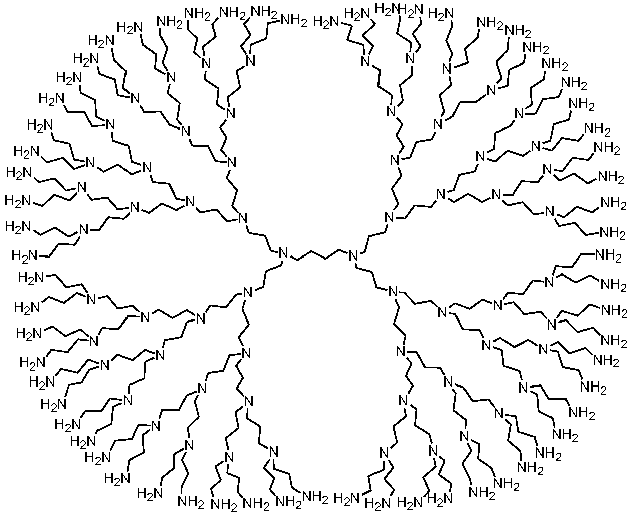

An example of a dendrimer of type PMMH with a thiophosphoryl core, for which n = 4 and m = 3 is represented below:

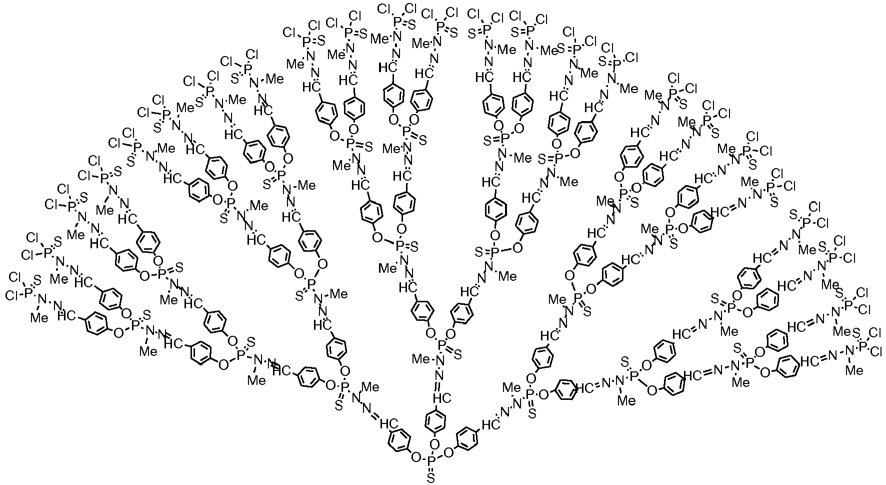

An example of a dendrimer of type PMMH with a cyclotriphosphazene core, for which n = 2 and m = 6, without an intermediate chain, is represented below:

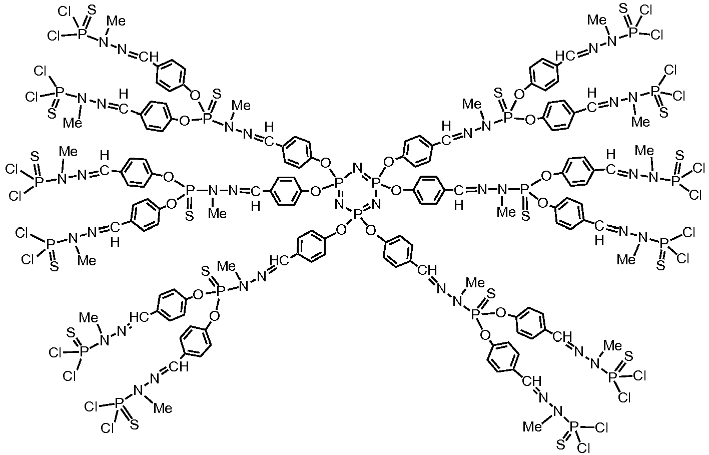

Another example of a dendrimer of type PMMH with a cyclotriphosphazene core, for which n = 2 and m = 6, with an intermediate chain, is represented below:

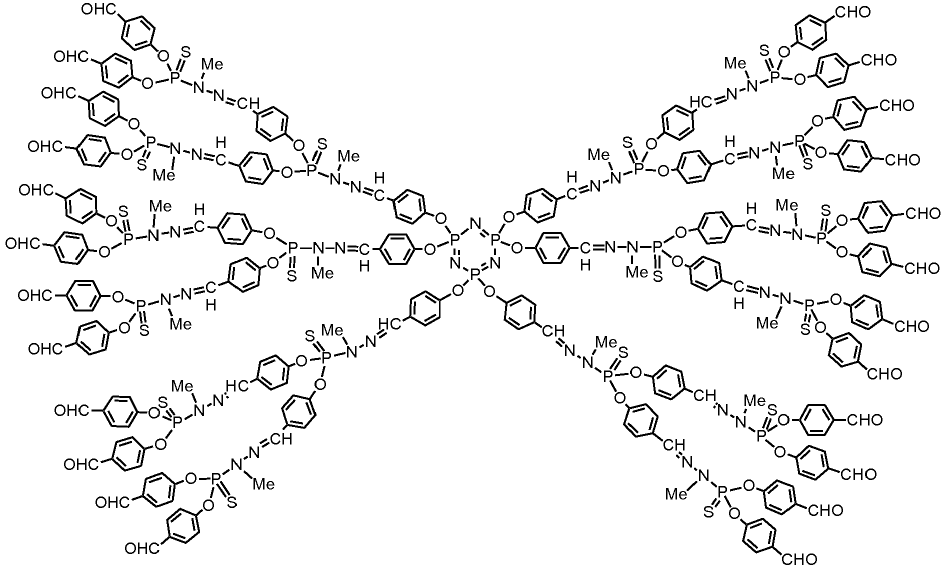

The invention relates to dendrimers with monophosphonic or bisphosphonic terminations corresponding to the following general formula (1a): 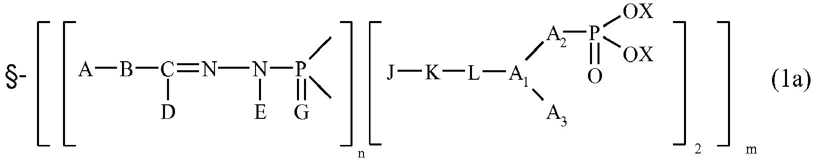 where n represents an integer from 0 to 3, namely:
- when n = 0, formula (1a) corresponds to the following formula (2a), 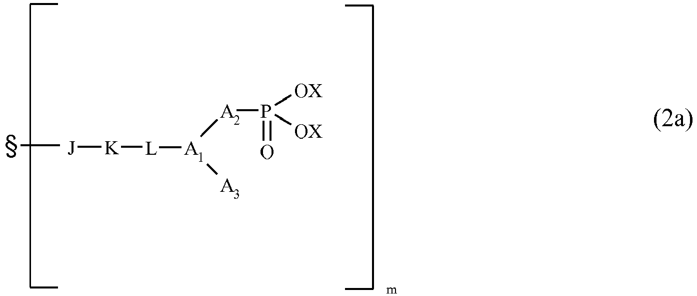
- when n = 1, formula (1a) corresponds to the following formula (3a), 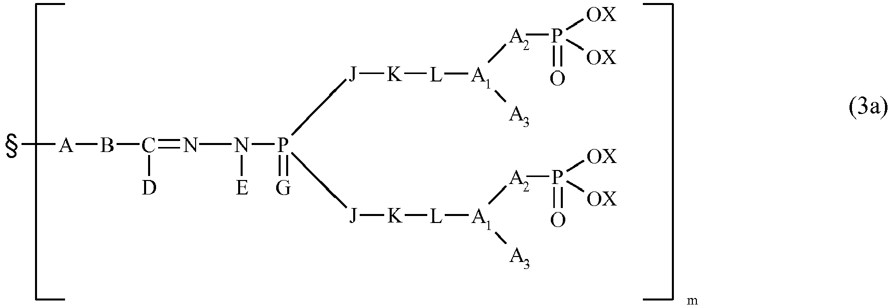
- when n = 2, formula (1a) corresponds to the following formula (4a), 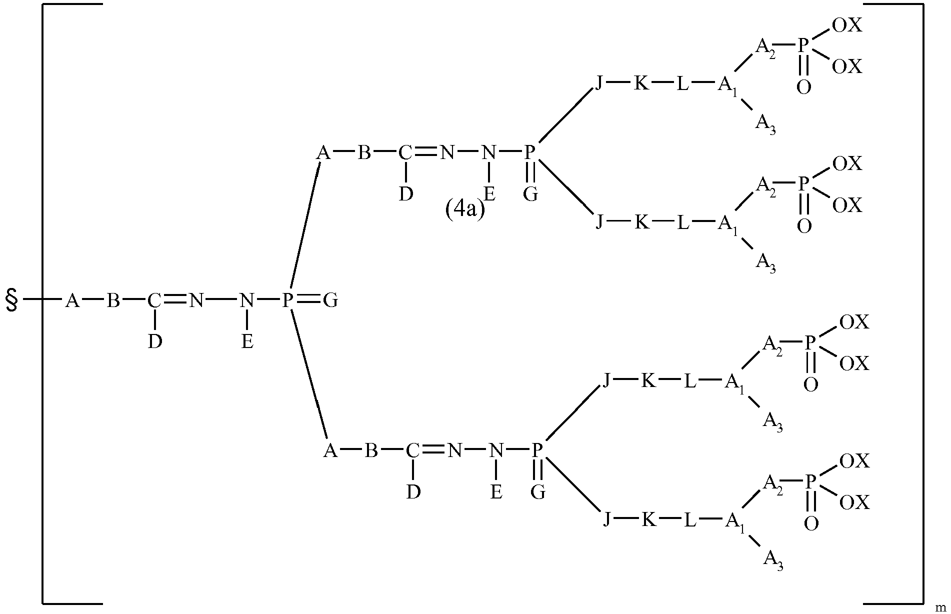
- and when n = 3, formula (1a) corresponds to the following formula (5a), 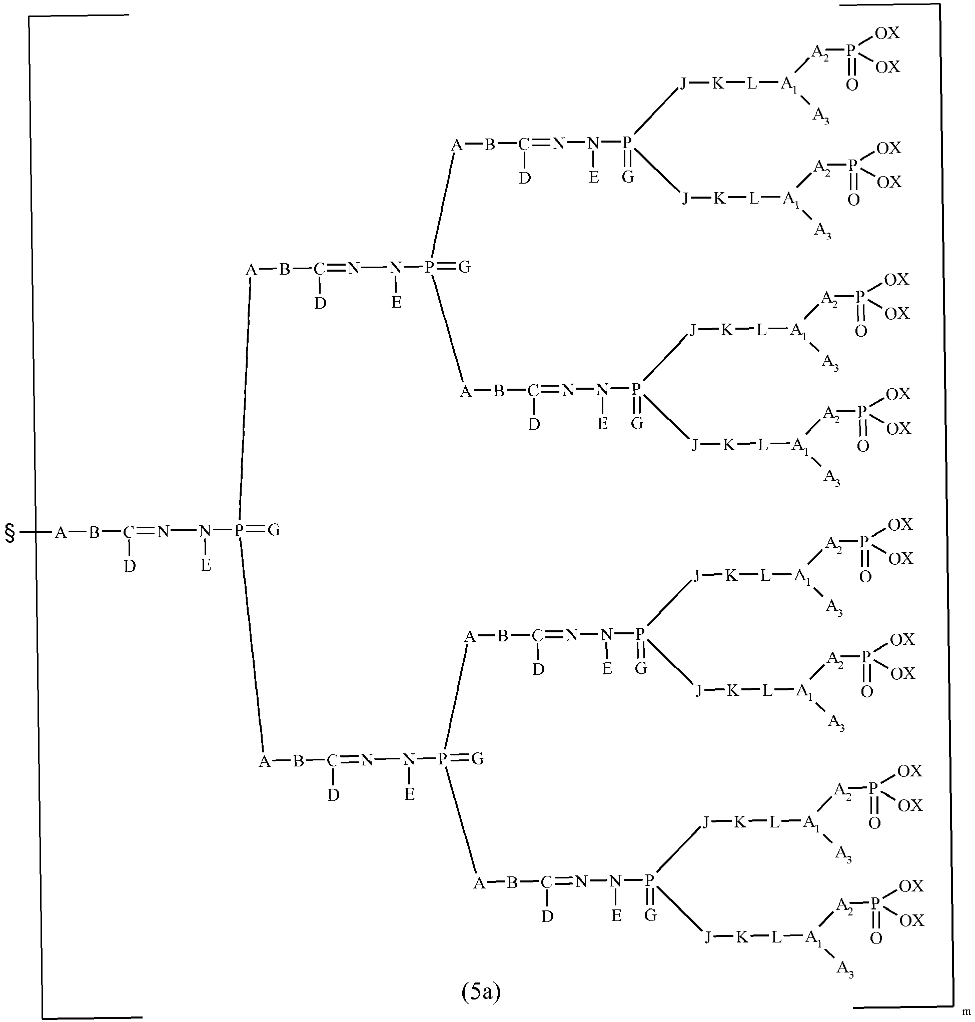 and in which formulae:
   - the central core § is 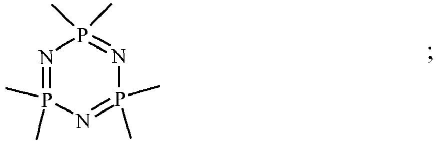
   - m represents 6;
   - the generation chain corresponds to the formula: where 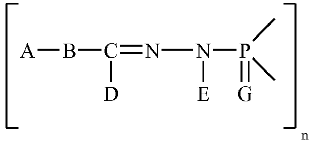
      - A represents an oxygen, sulphur, phosphorus atom or a NR- group;
      - B represents an aryl group of 6 to 24 carbon atoms, a heteroaryl group of 1 to 24 carbon atoms, an alkyl group of 1 to 16 carbon atoms, an oligoethyleneglycol group, a polyethyleneglycol group, two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹; according to the invention, when B represents two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group selected from the groups comprising C₆H₄-O-C₆H₄ and C₆H₄-(CH₂CH₂O)q-C₆H₄ with q = 1 to 12.
      - D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a -NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the intermediate chain corresponds to the formula:

      -J-K-L-

      where
      - J represents an oxygen atom, a sulphur atom, or a -NR- group;
      - K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, - OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - L represents a linear, branched or cyclic hydrocarbon chain with 0 to 10 members, optionally containing one or more double or triple bonds, each of said members being able to be optionally a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, -NO₂, -NRR', -CN, -CF₃, -OH, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      R and R' representing independently of each other H or an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the terminal group corresponds to the formula: 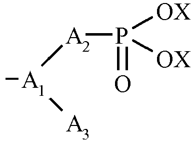 where A₁, A₂, A₃ and X have been defined previously, each X being identical or different,
   for use in the treatment of chronic inflammatory diseases and chonic inflammatory diseases of auto-immune origin.

In a more particular embodiment, the invention relates to a dendrimer of general
formula (1a) in which A₃ represents: 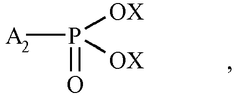 said general formula (1a) then corresponding to the following general formula (1): 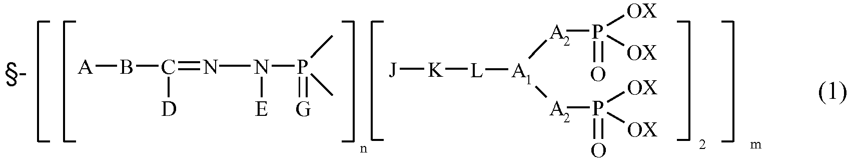 §, A, B, C, D, E, G, J, K, L, A₁, A₂, X, m and n being as defined above, for use in the treatment of chronic inflammatory diseases and chonic inflammatory diseases of auto-immune origin.

In a particular embodiment of the invention, the dendrimers with bisphosphonic terminations correspond to the following general formula (1) where n represents an integer from 0 to 3, namely:
- when n = 0, formula (1) corresponds to the following formula (2), 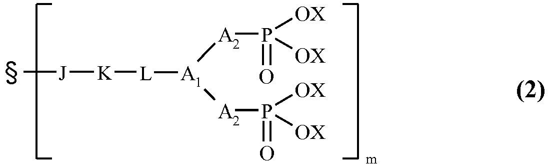
- when n = 1, formula (1) corresponds to the following formula (3), 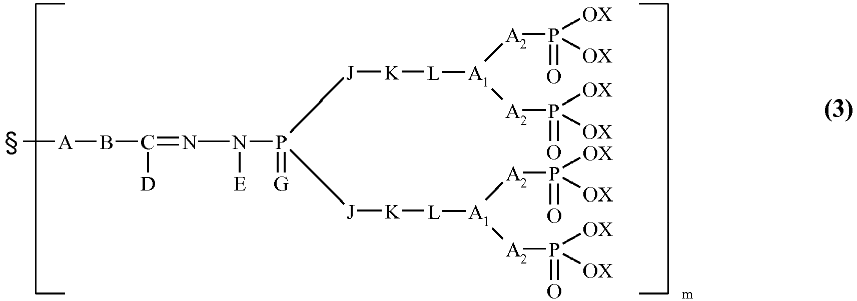
- when n = 2, formula (1) corresponds to the following formula (4), 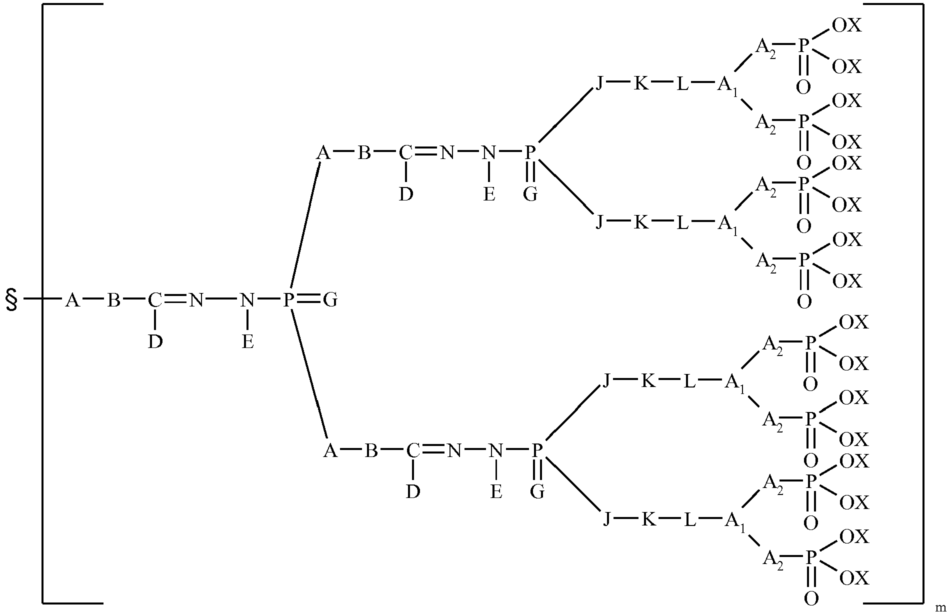 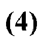
- and when n = 3, formula (1) corresponds to the following formula (5), 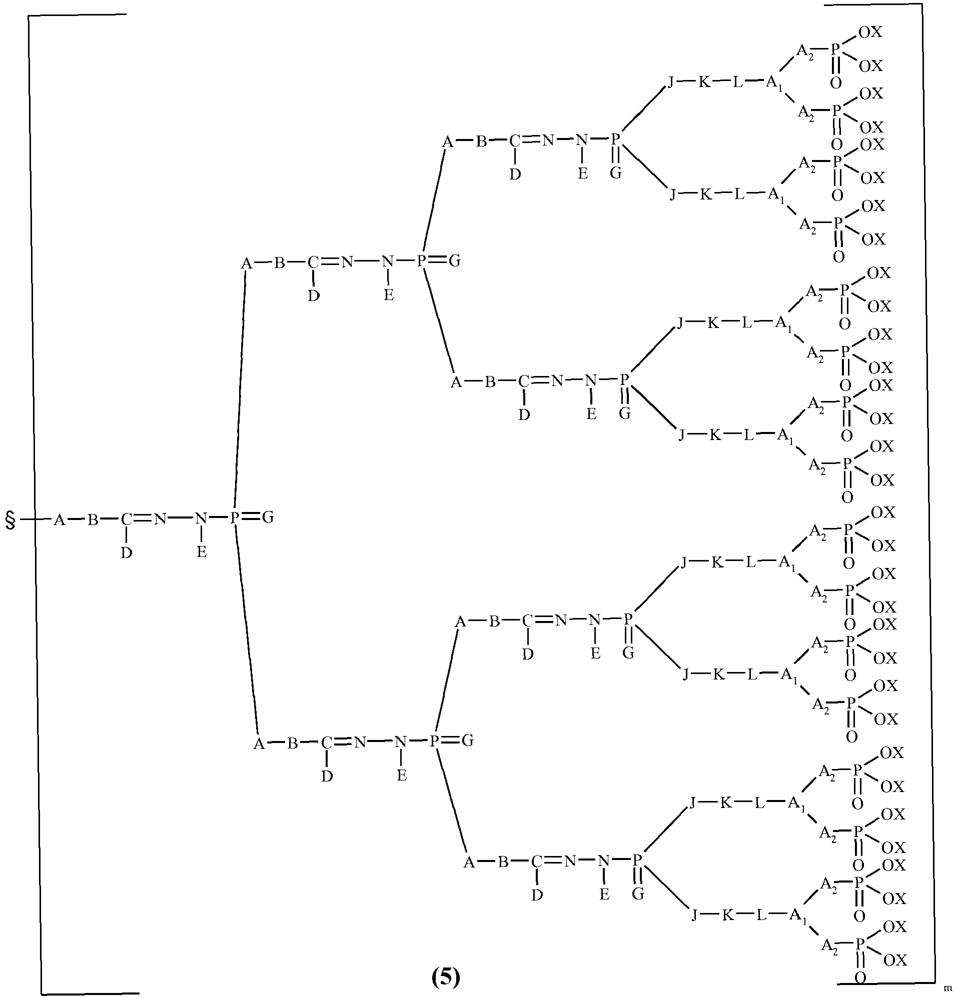 and in which formulae:
   - the central core § is: 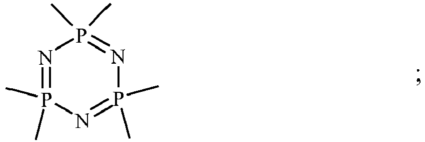
   - m represents 6;
      the generation chain corresponds to the formula: where 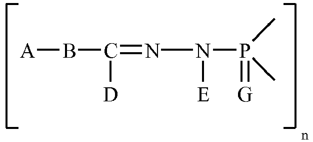
      - A represents an oxygen, sulphur, phosphorus atom or a NR- group;
      - B represents an aryl group of 6 to 24 carbon atoms, a heteroaryl group of 1 to 24 carbon atoms, an alkyl group of 1 to 16 carbon atoms, an oligoethyleneglycol group, a polyethyleneglycol group, two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹; According to the invention, when B represents two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group selected from the groups comprising C₆H₄-O-C₆H₄ and C₆H₄-(CH₂CH₂O)q-C₆H₄ with q = 1 to 12.
      - D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹; each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the intermediate chain corresponds to the formula:

      -J-K-L-

      where
      - J represents an oxygen atom, a sulphur atom, or a -NR- group;
      - K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, - OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - L represents a linear, branched or cyclic hydrocarbon chain with 0 to 10 members, in particular with 0 to 6 members, optionally containing one or more double or triple bonds, each of said members optionally being able to be a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, -NO₂, -NRR', -CN, -CF₃, -OH, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the terminal group corresponds to the formula: 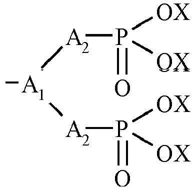 where A₁, A₂ and X have been defined previously, each X being identical or different.
According to a preferred embodiment, the invention relates to the use as defined above of a dendrimer of general formula (1), in which § represents 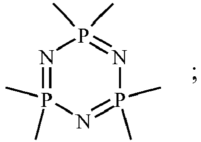 m represents 6;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non-substituted linear saturated hydrocarbon chain with two carbon atoms;
A₁ represents a nitrogen atom;
A₂ represents a CH₂ group;
X represents a methyl group, or a hydrogen or sodium atom;
said dendrimer being designated GCn, n being defined above.

According to a particularly preferred embodiment, the invention relates to the use as defined above of compounds of the following formulae: 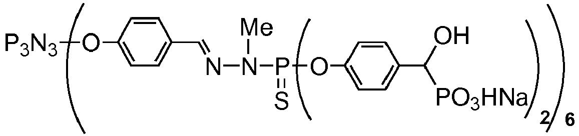 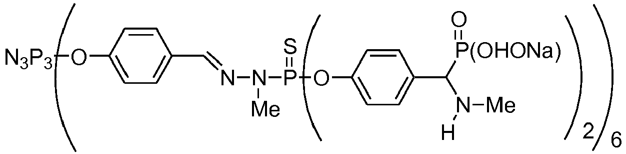 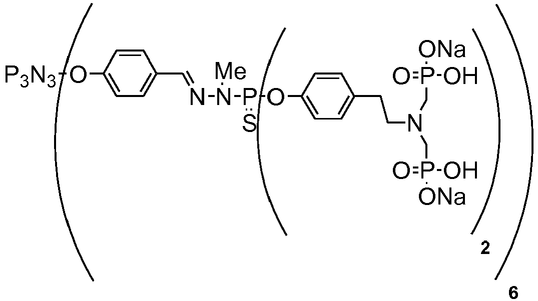 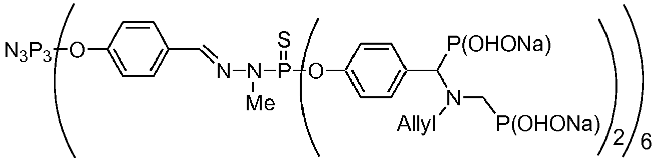 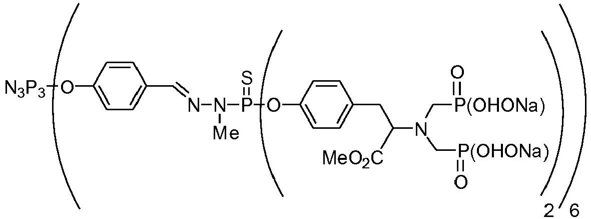 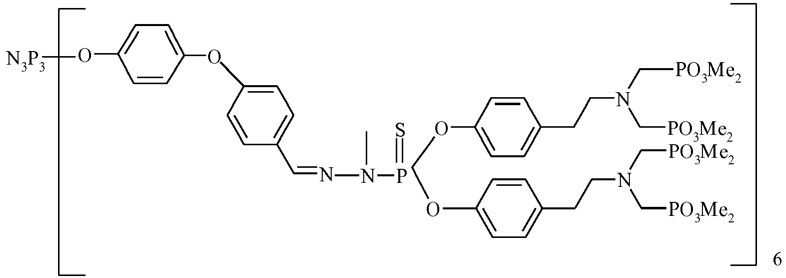 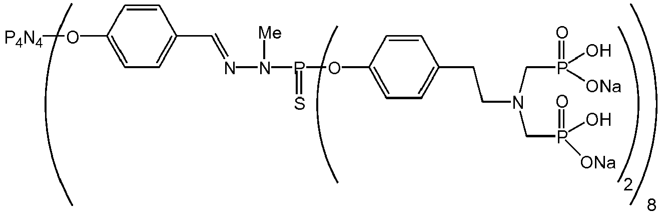 or of GC1 compounds of the following formulae:

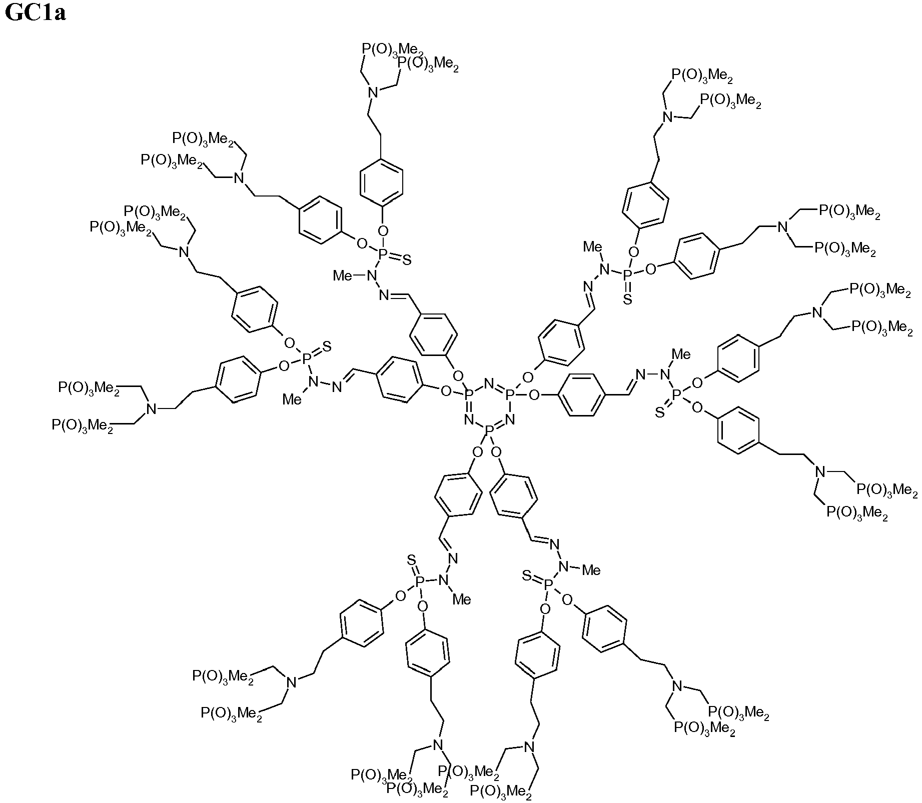

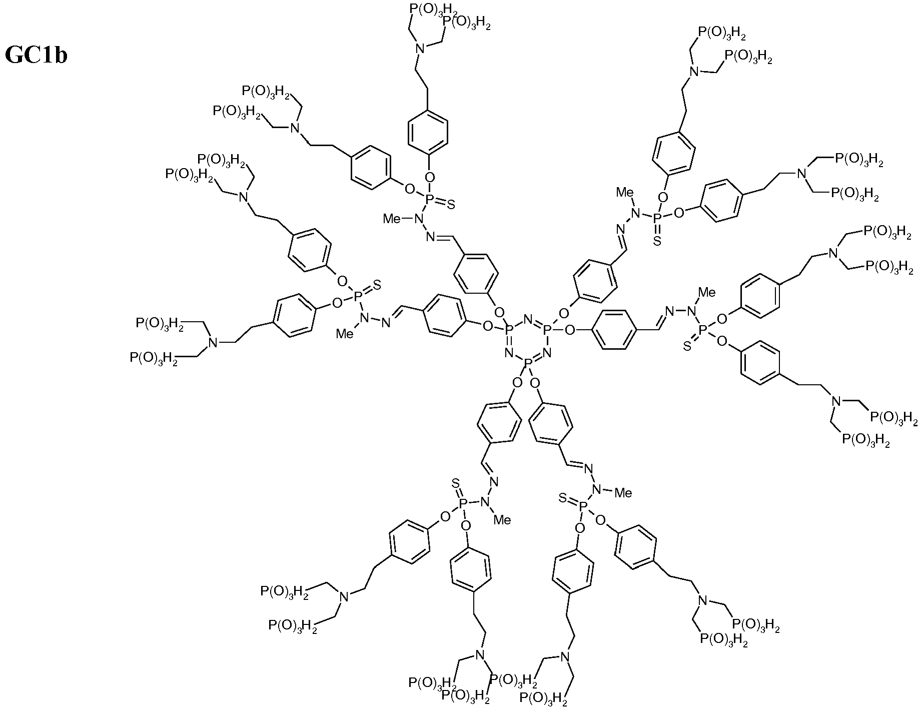

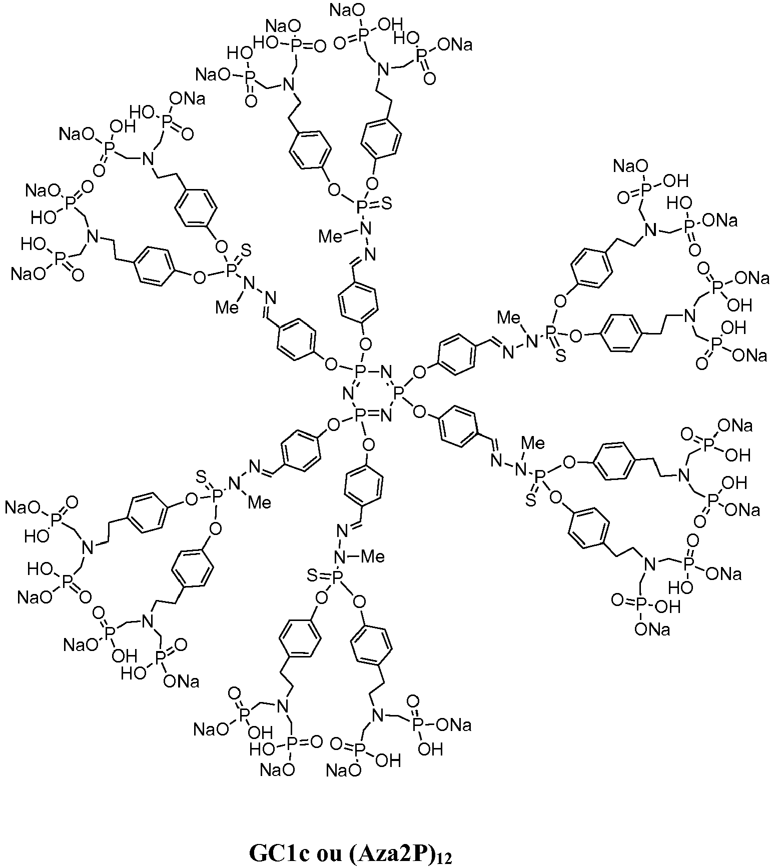

The present invention also relates to the use of dendrimers with bisphosphonic terminations corresponding to the following general formula (7): 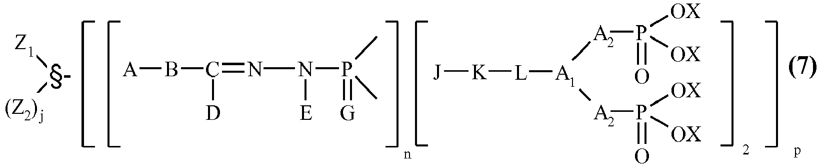 where n represents an integer from 0 to 3, m represents 3, 6 or 8, p represents m - 1 or m - 2, and j represents 0 when p represents m - 1 and 1 when p represents m - 2, namely:
- when p = m - 1, formula (7) corresponds to the following formula (8): 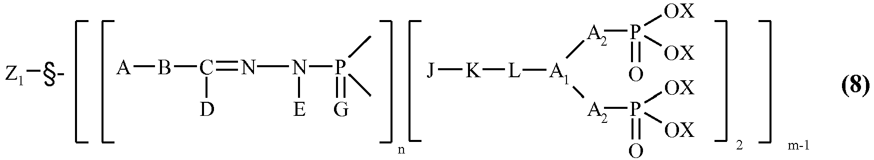
- when p = m - 2, formula (7) corresponds to the following formula (9): 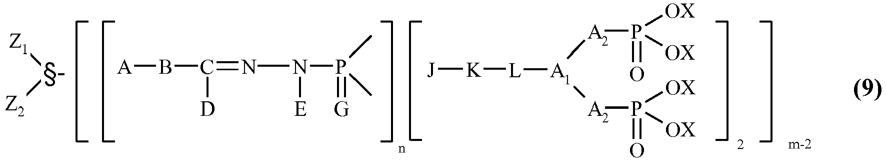 and in which formulae:
   - the central core § is: 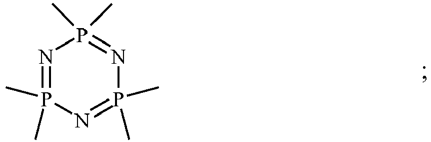
   - the generation chain corresponds to the formula: 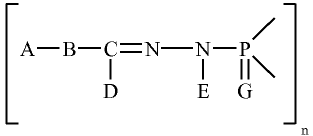 where
      - A represents an oxygen, sulphur, phosphorus atom or a NR- group;
      - B represents an aryl group of 6 to 24 carbon atoms, a heteroaryl group of 1 to 24 carbon atoms, an alkyl group of 1 to 16 carbon atoms, an oligoethyleneglycol group, a polyethyleneglycol group, two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹. According to the invention, when B represents two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group selected from the groups comprising C₆H₄-O-C₆H₄ and C₆H₄-(CH₂CH₂O)q-C₆H₄ with q = 1 to 12.
      - D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the intermediate chain corresponds to the formula:

      -J-K-L-

      where
      - J represents an oxygen atom, a sulphur atom, or a -NR- group;
      - K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, - OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
      - L represents a linear, branched or cyclic hydrocarbon chain with 0 to 10 members, in particular with 0 to 6 members, optionally containing one or more double or triple bonds, each of said members optionally being able to be a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, -NO₂, -NRR', -CN,
      - CF₃, -OH, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
   - the terminal group corresponds to the formula: 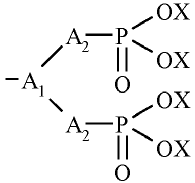 where A1, A2 and X have been defined previously, each X being identical or different; Z₁ and Z₂ being identical or different, optionally linked together, in particular by means of a covalent bond, and representing H or a linear, branched or cyclic hydrocarbon chain with 1 to 16 members, optionally containing one or more double or triple bonds, each of said members being optionally chosen from a heteroatom, said heteroatom being preferably chosen from a nitrogen, oxygen, phosphorus, silicon or sulphur atom, an aryl group of 6 to 24 carbon atoms, a heteroaryl group of 1 to 24 carbon atoms, a carboxyl group, a >C=NR group, each member being able to be optionally substituted by at least one substituent chosen from a hydroxyl group, a NR"R"' group, an alkoxy group of 1 to 16 carbon atoms, an alkyl group of 1 to 16 carbon atoms, a halogen atom, a NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R" and R'" representing independently of each other H or an alkyl group of 1 to 16 carbon atoms, an aryl group of 6 to 24 carbon atoms or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, the first member of said hydrocarbon chain preferably being oxygen or nitrogen,
      for use in the treatment of chronic inflammatory diseases and chonic inflammatory diseases of auto-immune origin.

The invention relates more particularly to the use as defined above, of a dendrimer of general formula (8), in which § represents 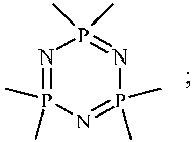 m represents 6;
p represents 5;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non-substituted linear saturated hydrocarbon chain with two carbon atoms;
A₁ represents a nitrogen atom;
A₂ represents a CH₂ group;
X represents a methyl group, or a hydrogen or sodium atom;
Z₁ represents a phenyloxy group;
said dendrimer being designated GCn', n being defined above.

Preferably, the present invention relates in particular to the use as defined above:
- of the compounds of the following formulae: 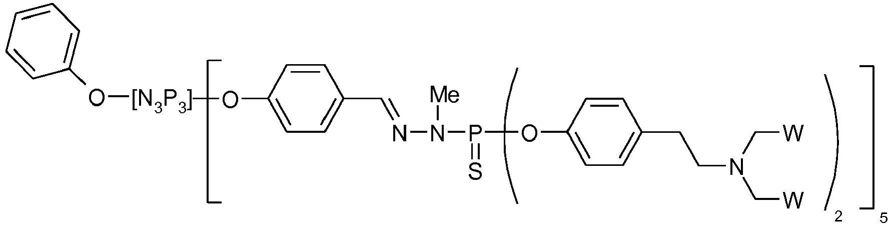 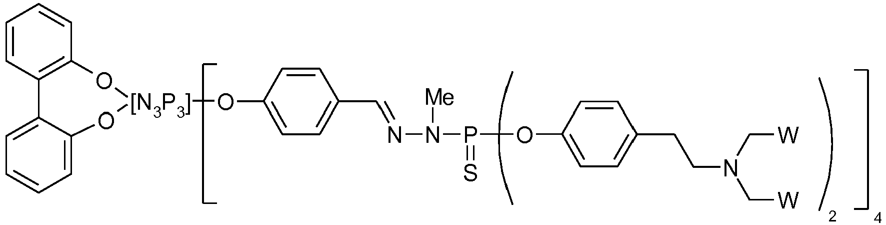 in which W represents PO₃Me₂, PO₃HNa, PO₃H₂, said compounds corresponding, in particular, to compound GC1' of the following formula (10): 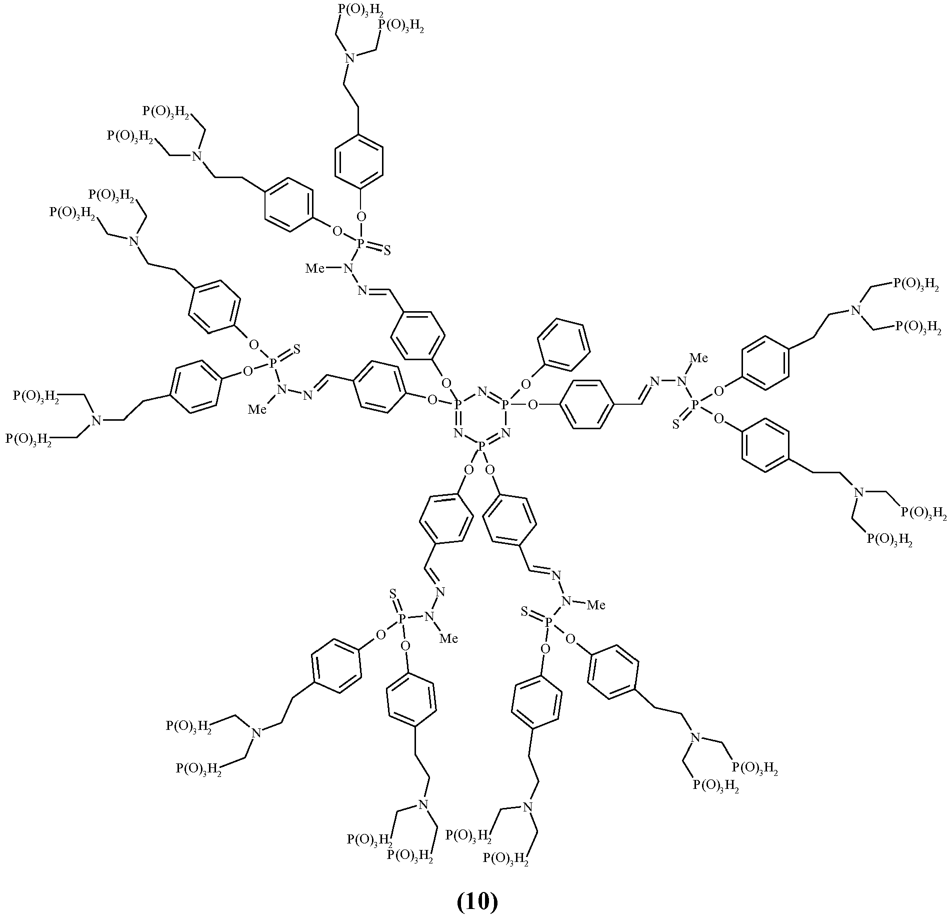 or of the formula (**10a**) 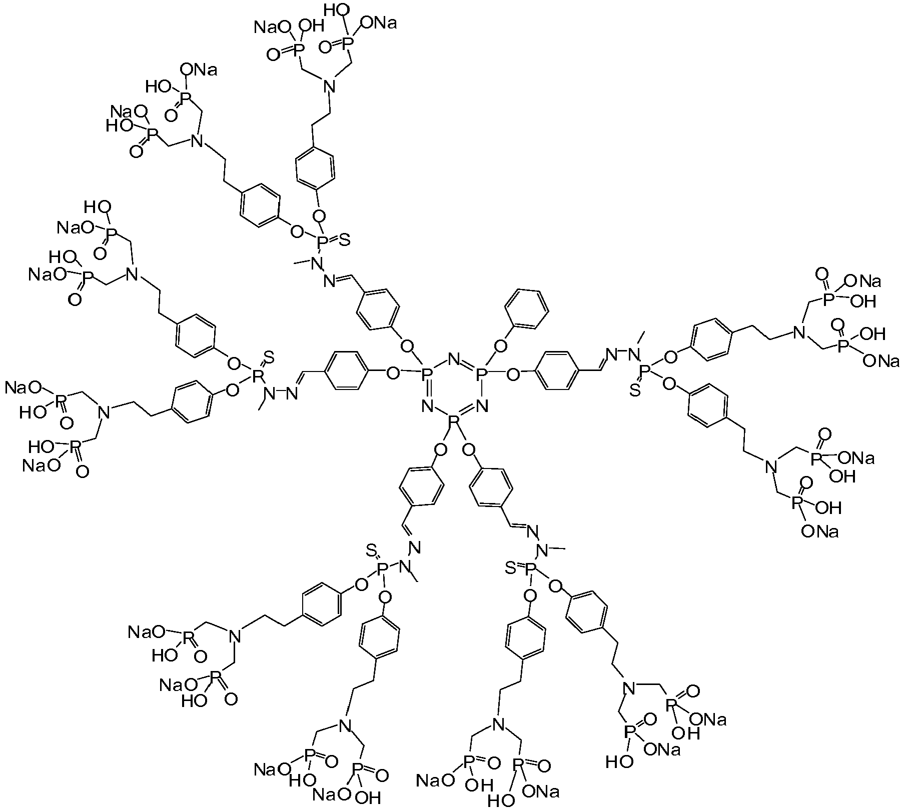
- or compounds of the following formula: 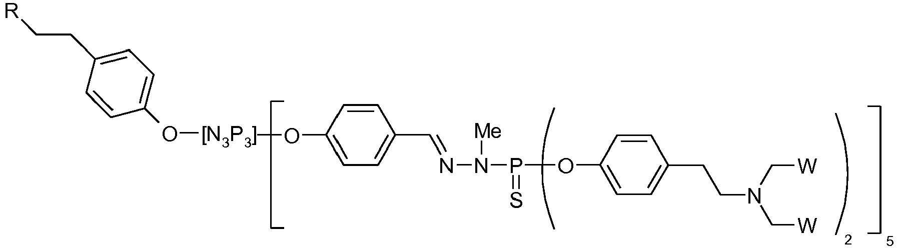 in which W represents PO₃Me₂, PO₃HNa, or PO₃H₂ and R is selected from the group comprising (a) fluorescent groups chosen from: 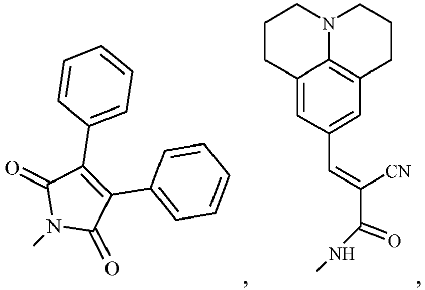 and (b) a biotinyl group of formula 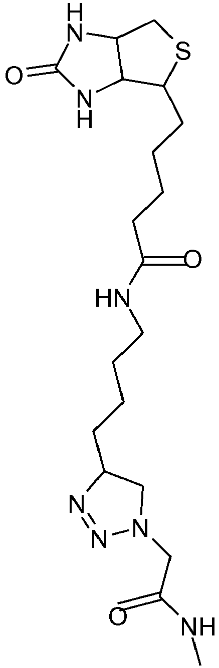 said compounds corresponding in particular to the compounds of the following formulae :

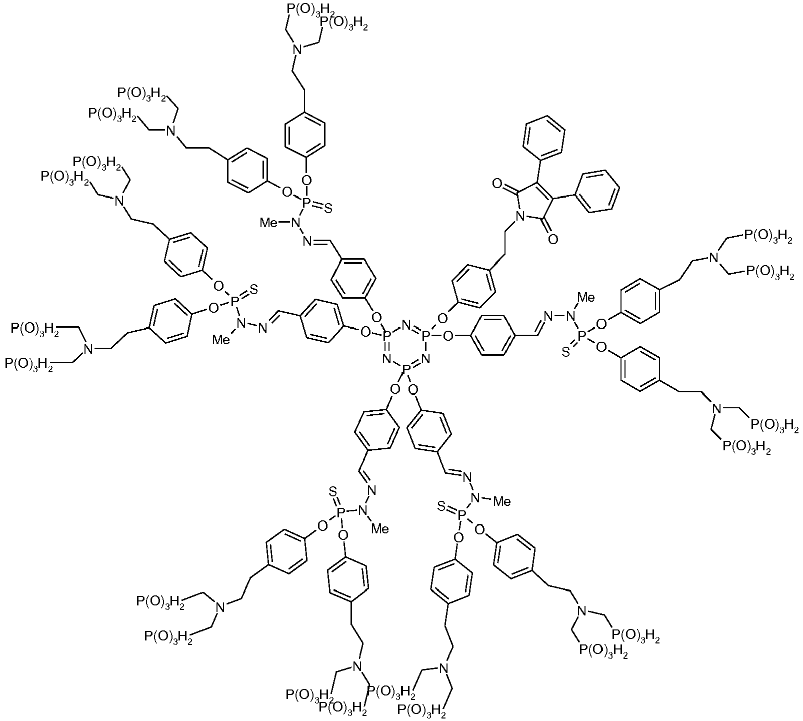

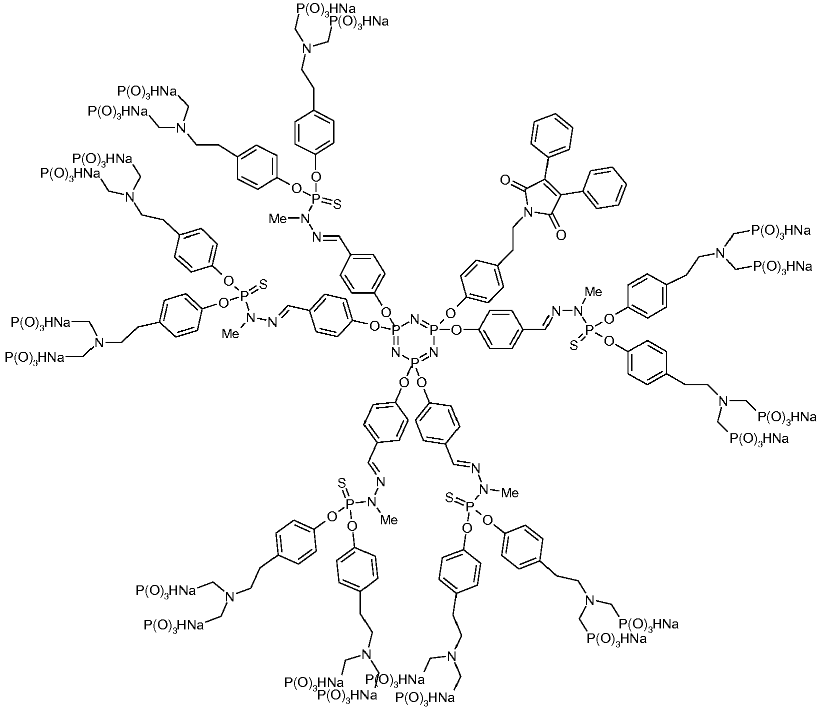

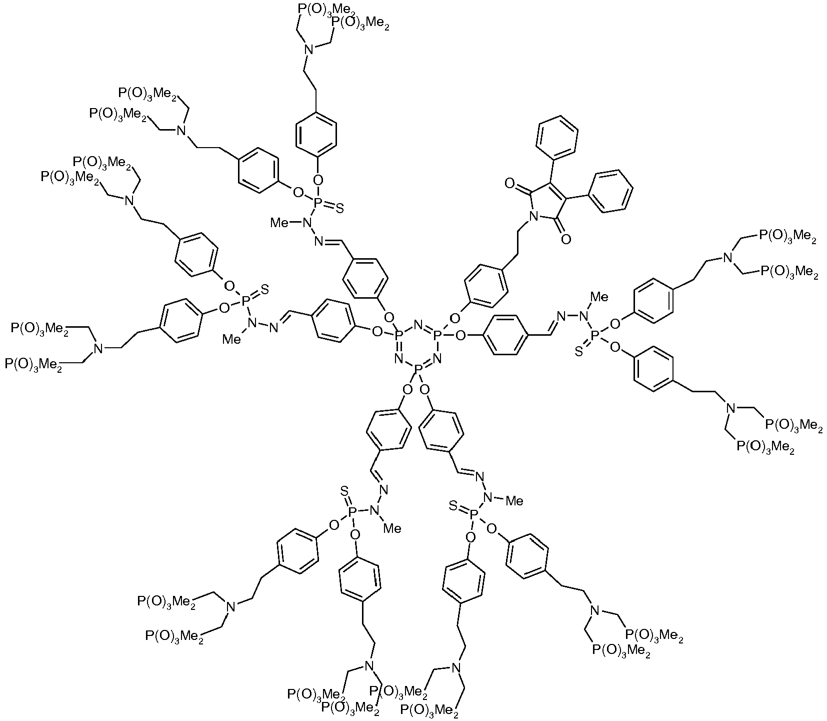

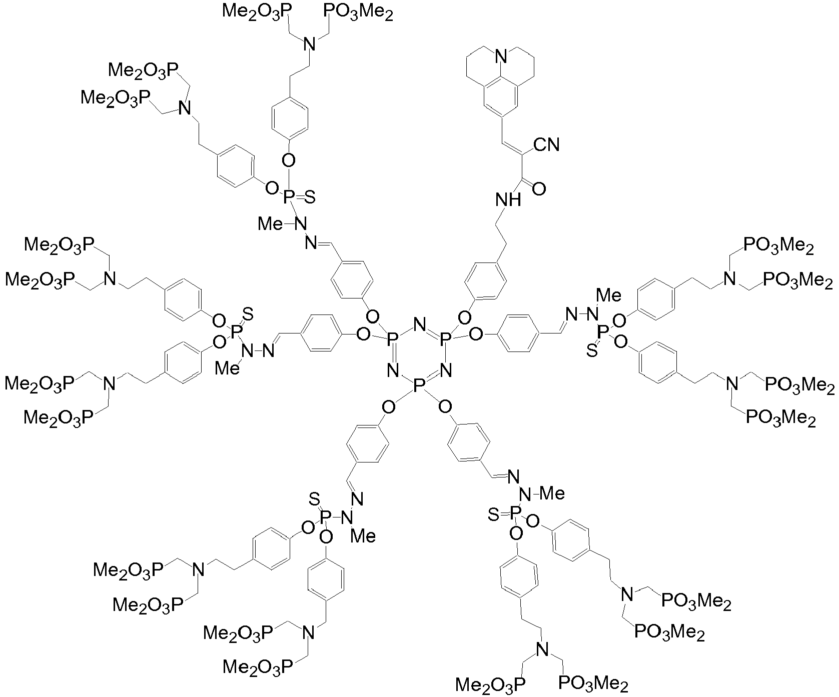

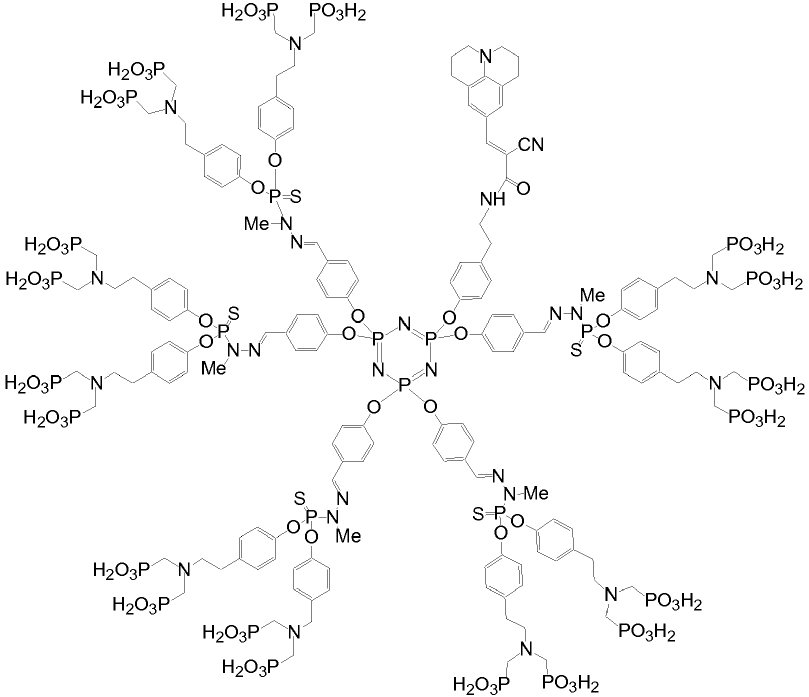

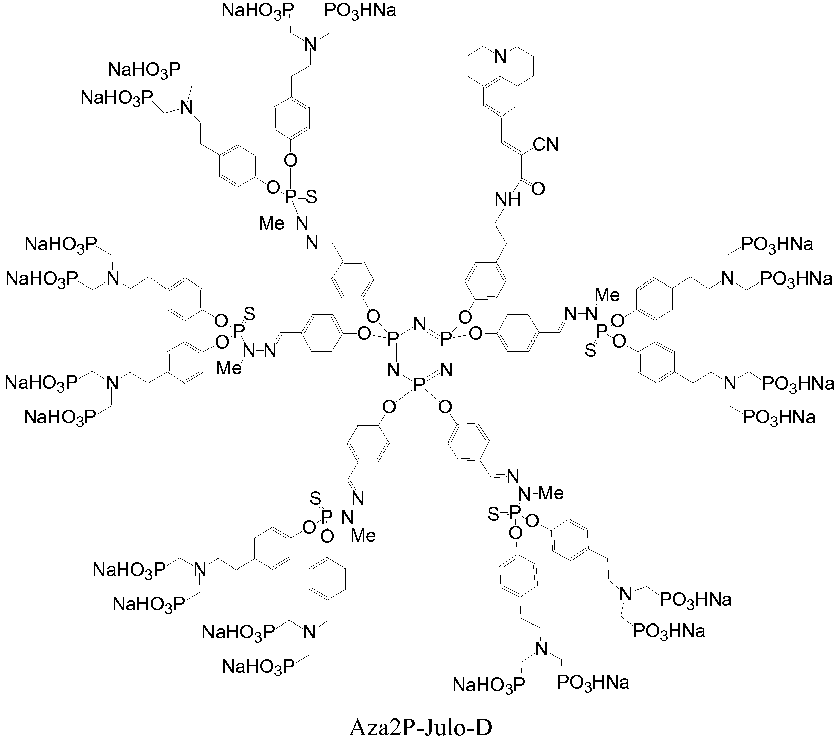

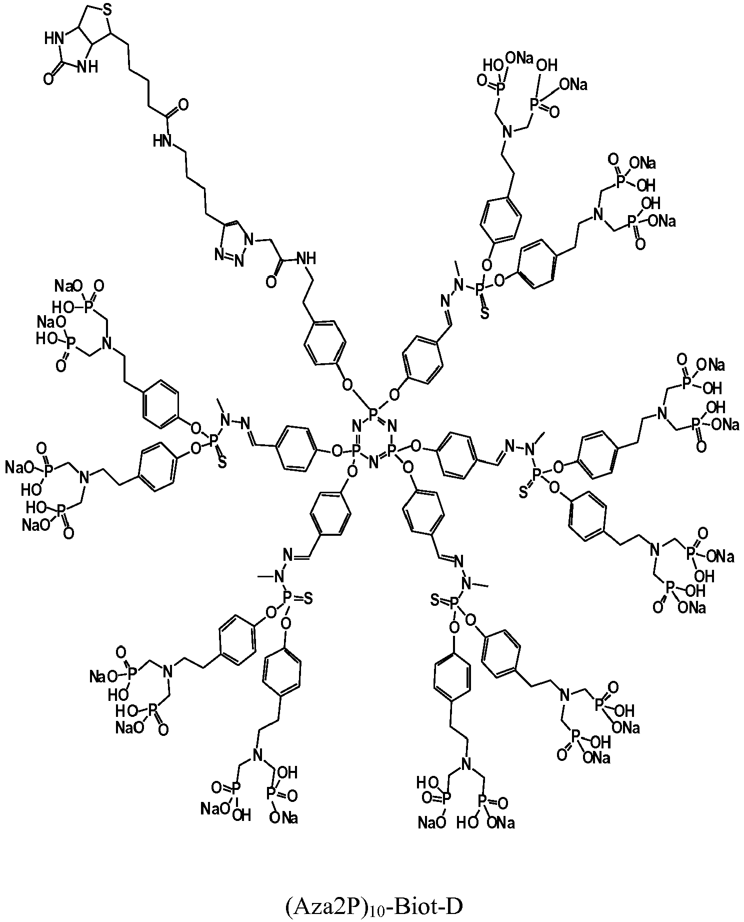

The present invention also relates to the use of dendrimers with bisphophonic terminations of the following formula: 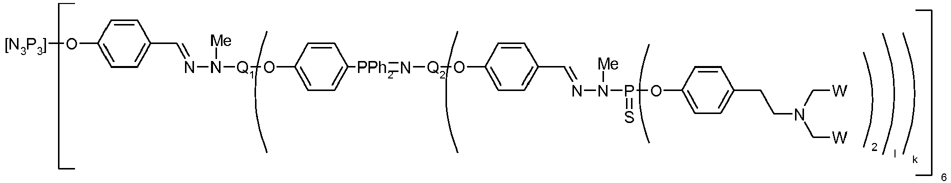 in which W represents PO₃Me₂, PO₃HNa, or PO₃H₂, Q₁ and Q₂, identical or different, represent P=S or cyclotriphosphazene (N₃P₃), 1 represents 2 when Q₂ represents P=S or 5 when Q₂ represents N₃P₃ and k represents 2 when Q₁ represents P=S or 5 when Q₁ represents N₃P₃, said dendrimers being in particular represented by the following formulae:

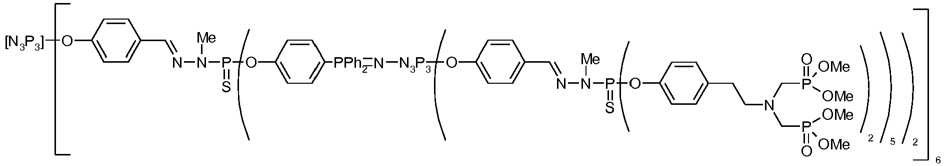

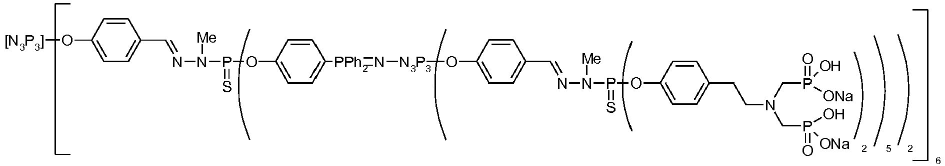

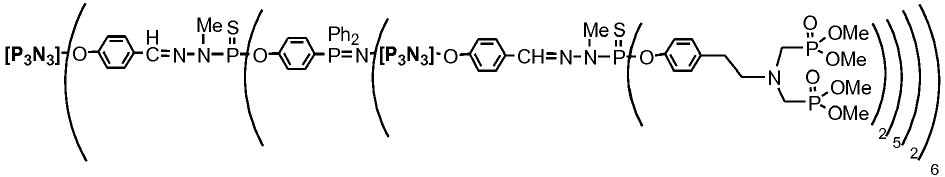

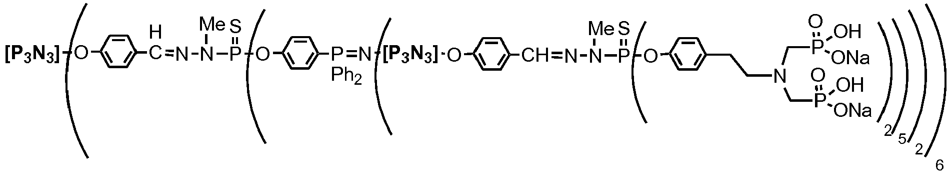

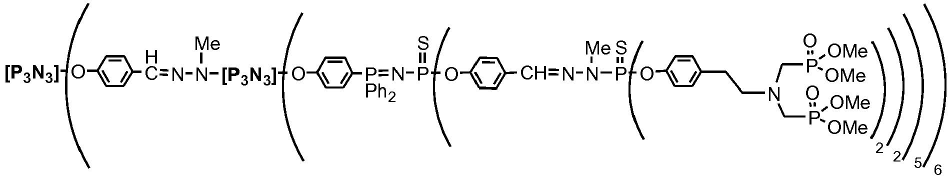

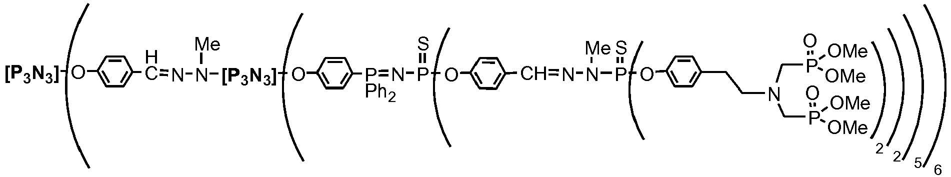

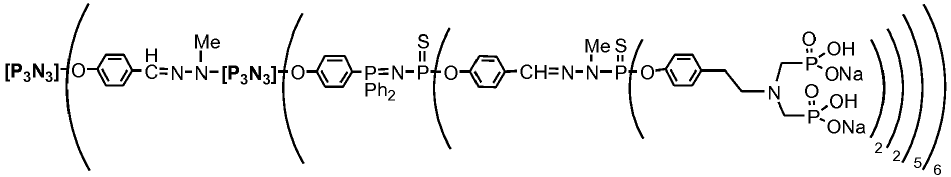

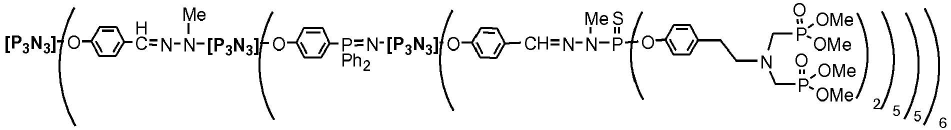

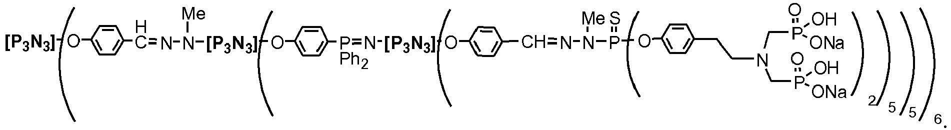

The present invention also relates to the use as defined above of dendrimers with monophosphonic or bisphosphonic terminations of the following formula: 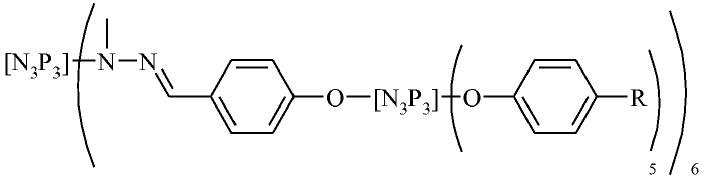 in which R represents a group chosen from 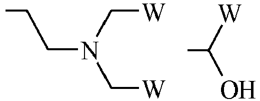 where W represents PO₃Me₂, PO₃HNa, or PO₃H₂.

The present invention also relates to the use as defined above of dendrimers with bisphosphonic terminations of the following formula: 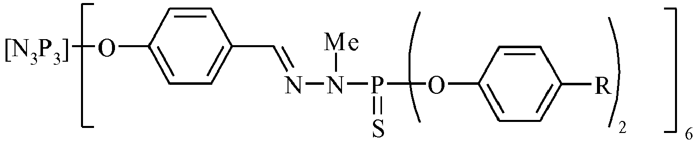 in which R represents a group chosen from: 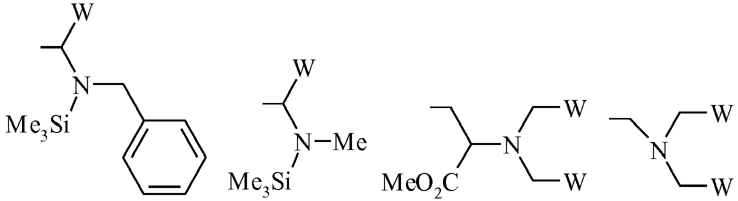 where W represents PO₃Si₂Me₆, PO₃Me₂, PO₃HNa, or PO₃H₂.

The present invention also relates to the use as defined above of dendrimers with monophosphonic terminations of the following formula: 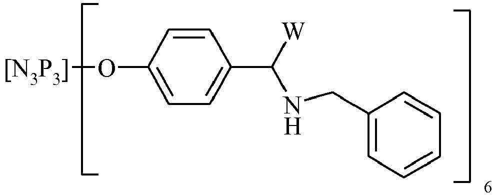 in which W represents PO₃Me₂, PO₃HNa, or PO₃H₂.

In a particular embodiment, the present invention relates to the use of the compounds of the following formulae:

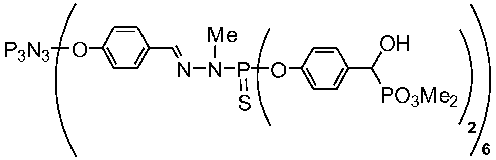

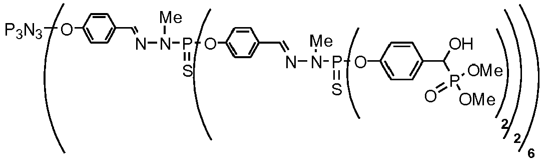

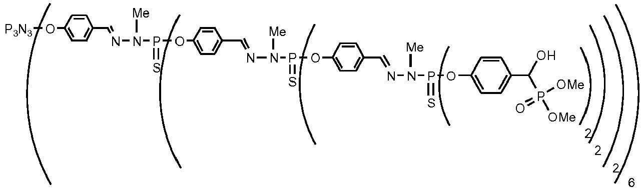

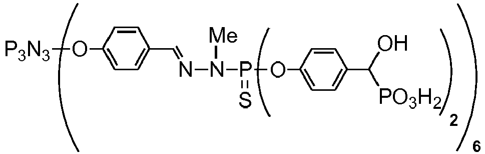

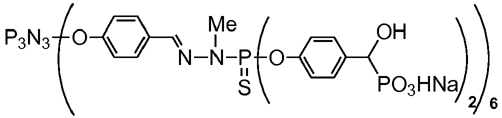

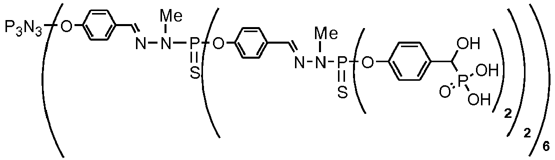

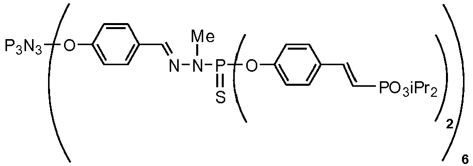

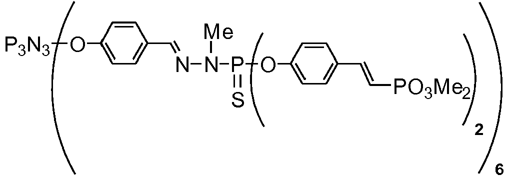

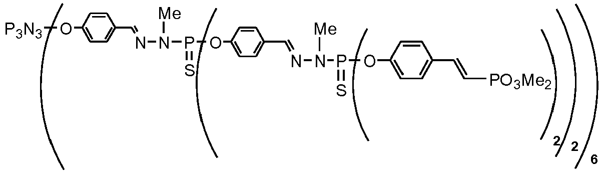

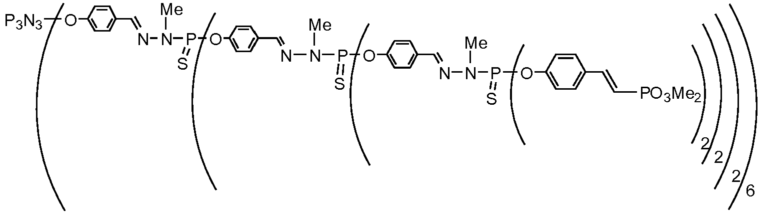

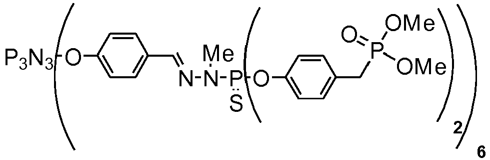

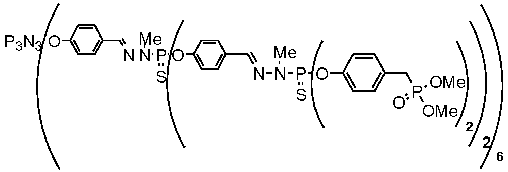

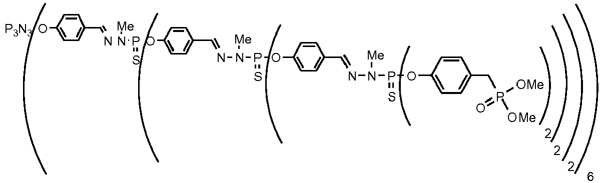

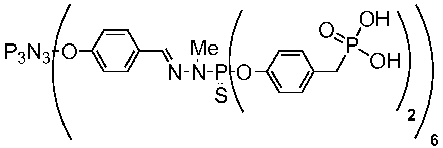

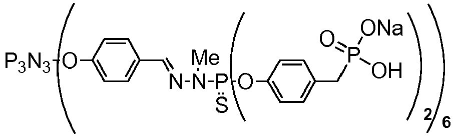

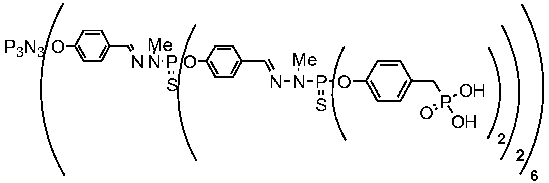

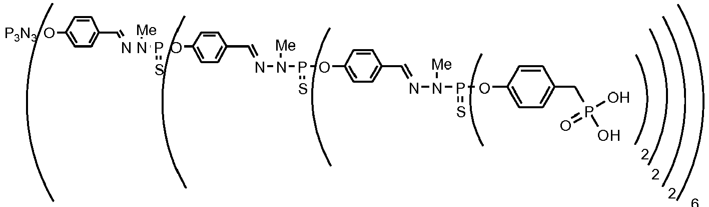

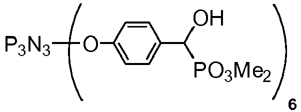

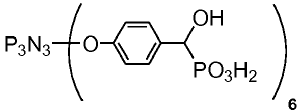

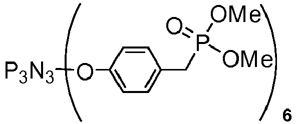

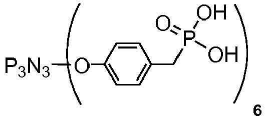

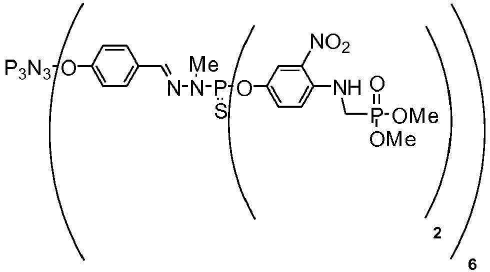

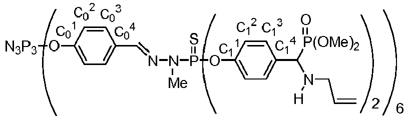

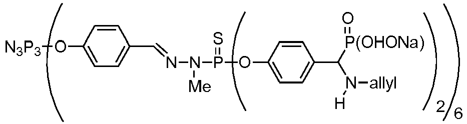

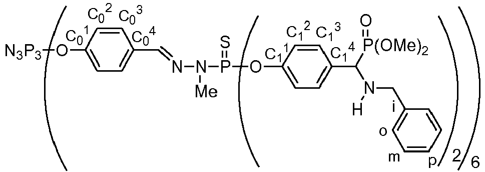

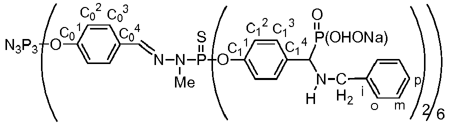

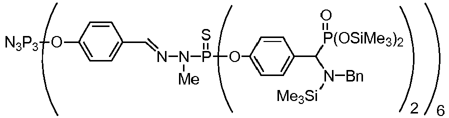

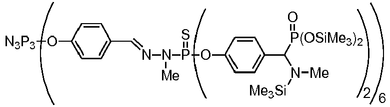

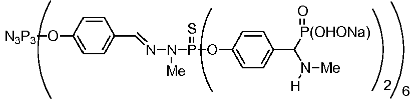

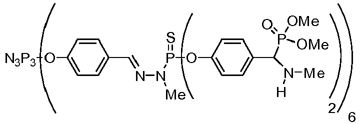

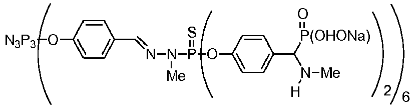

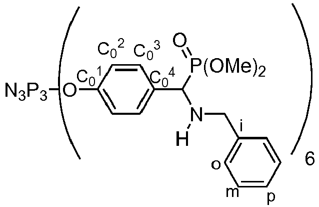

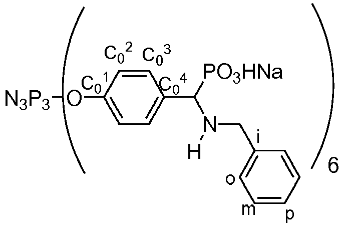

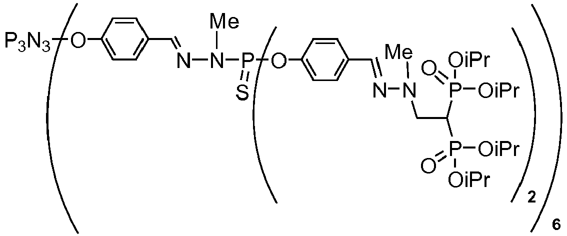

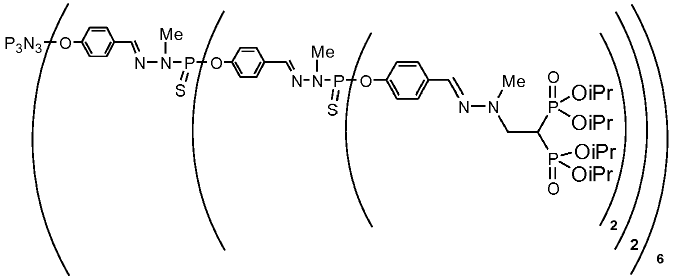

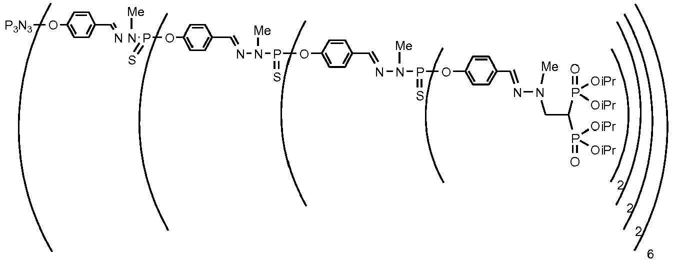

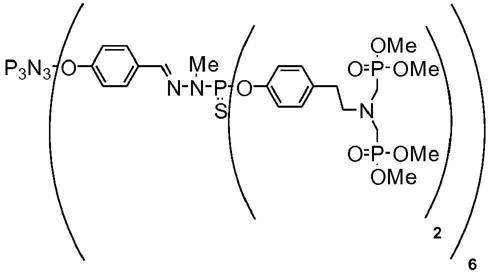

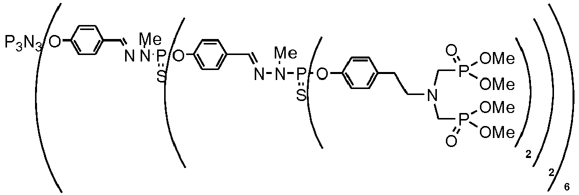

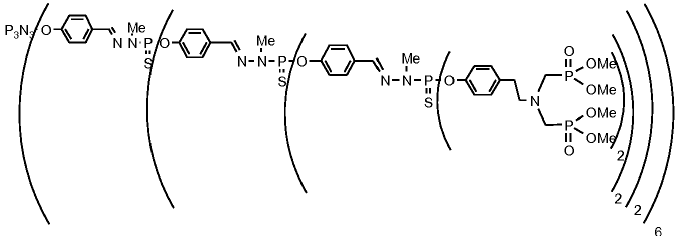

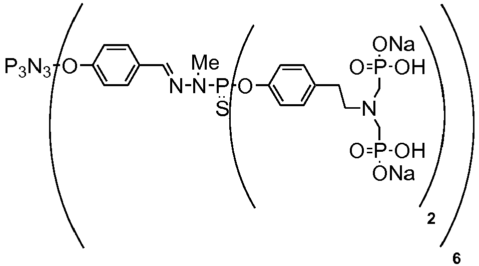

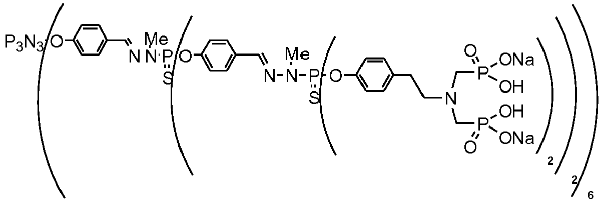

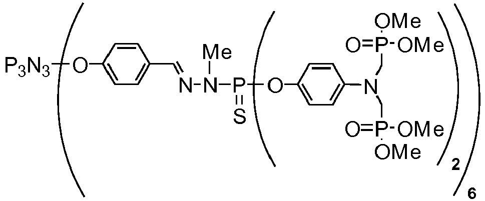

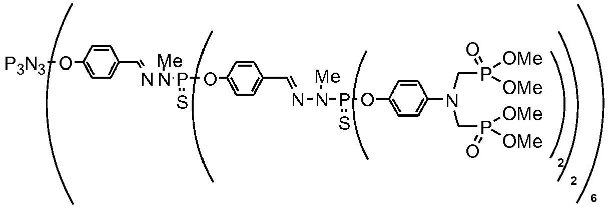

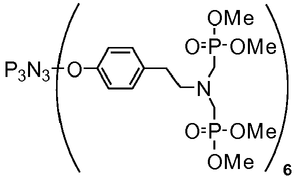

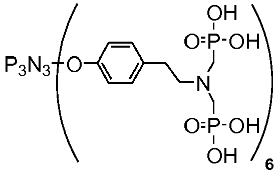

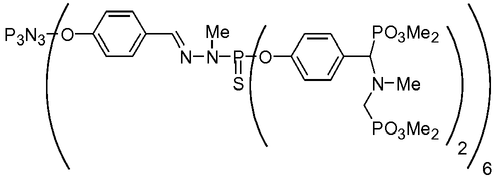

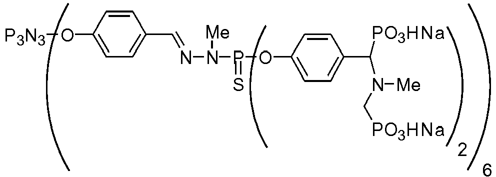

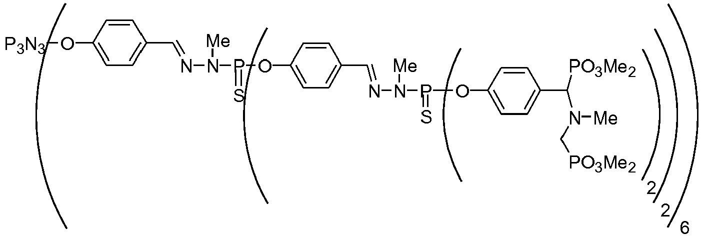

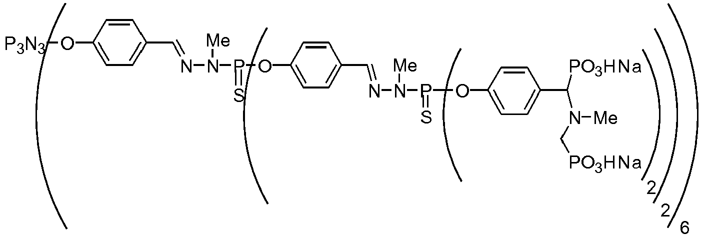

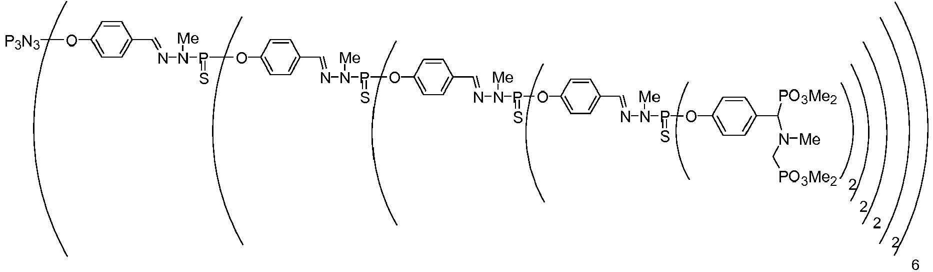

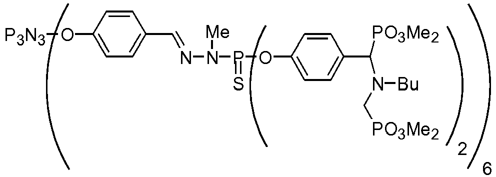

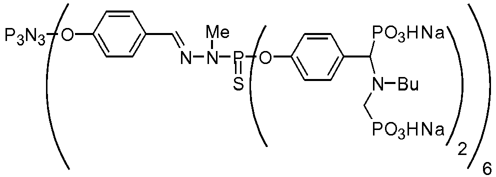

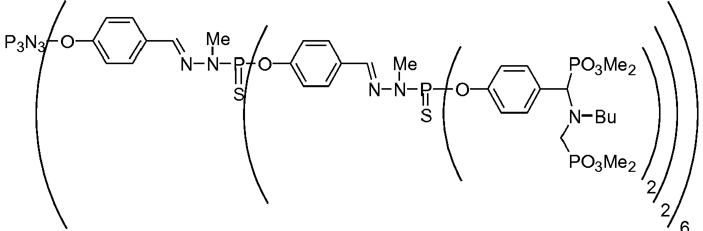

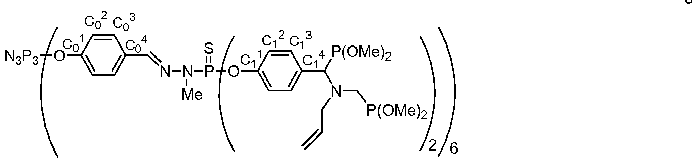

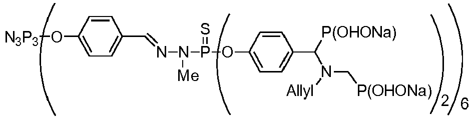

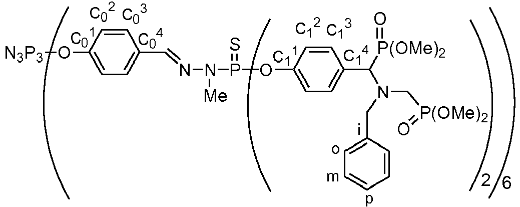

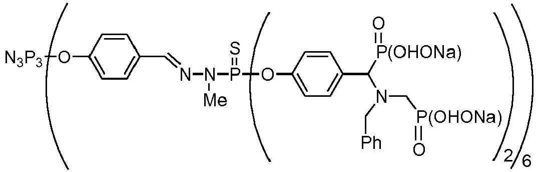

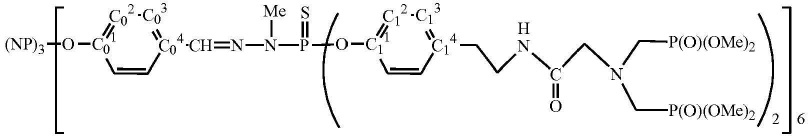

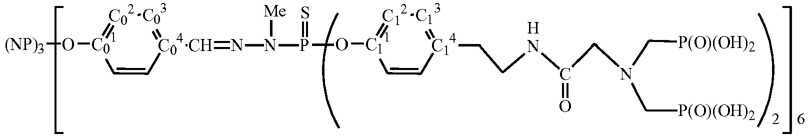

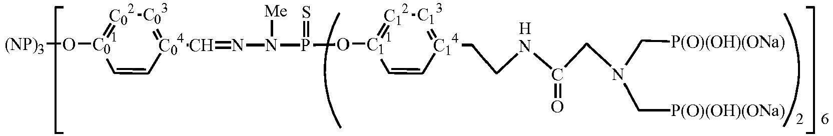

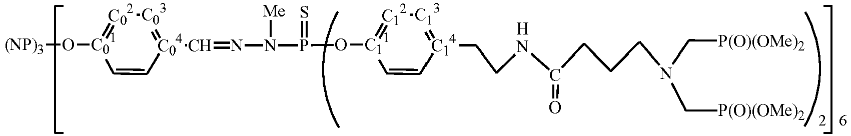

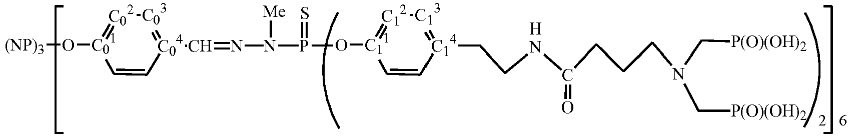

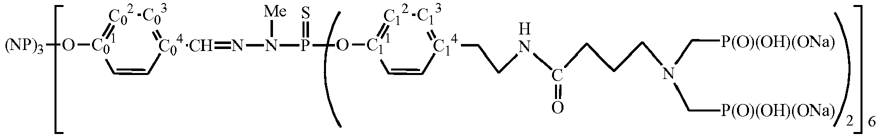

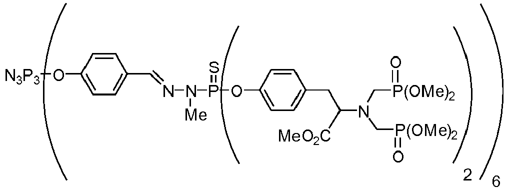

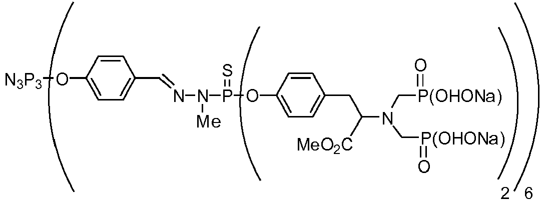

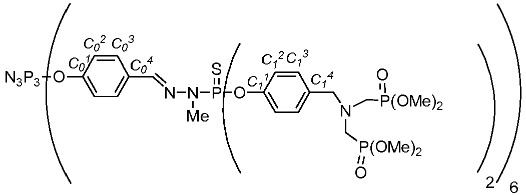

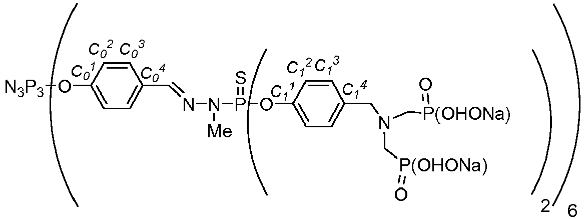

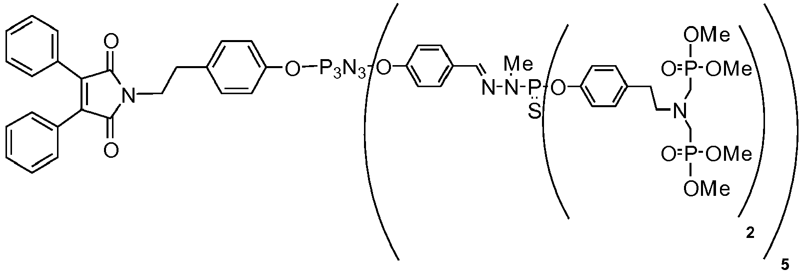

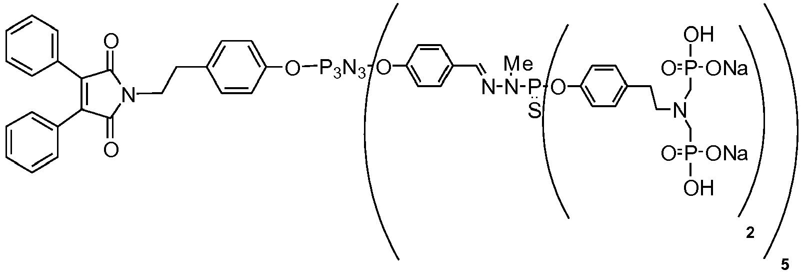

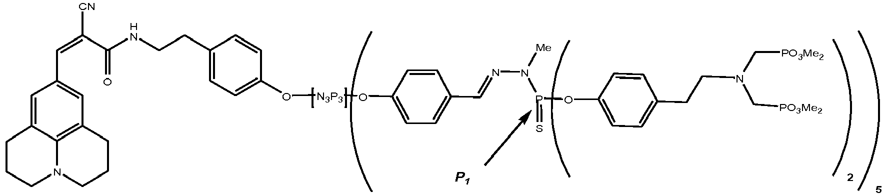

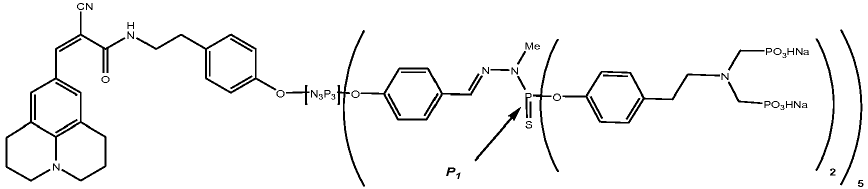

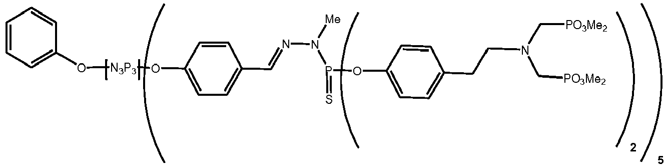

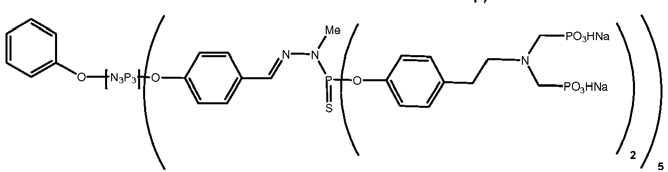

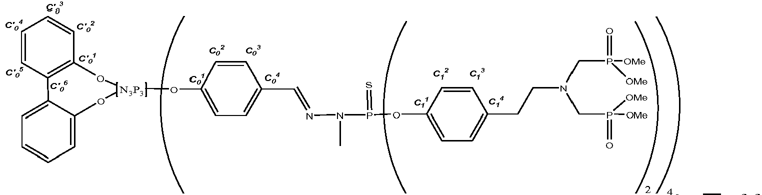

The instant invention also concerns the use of dendrimers of formula (1a) or formula (7) with mono- or biphosphonic terminations for the preparation of drugs useful for the treatment of chronic inflammatory diseases, said drugs acting through an anti-inflammatory type activation of monocytes.

The present invention also concerns the use of at least one dendrimer of formula (1a) or formula (7) with mono- or biphosphonic terminations for the preparation of drugs useful for the treatment of chronic inflammatory diseases, in particular from auto-immune origin.

According to the present invention, inflammatory diseases are selected from the group comprising chronic inflammatory diseases and chronic inflammatory diseases of auto-immune origin.

In an advantageous embodiment of the invention, the chronic inflammatory disease is selected from the group comprising rheumatoid arthritis, psoriasis and juvenile idiotypical arthritis.

The present invention also concerns pharmaceutical compositions containing as active substance at least one dendrimer of formula (1a) or formula (7) with monophosphonic or bisphosphonic terminations associated with a pharmaceutically acceptable carrier for use in the treatment of chronic inflammatory diseases, particularly those of auto-immune origin.

In another advantageous embodiment of the instant invention, the dendrimers of formula (1a) or formula (7) with monophosphonic or bisphosphonic terminations may be associated with other active substances, in particular with other classical steroid or non-steroid anti-inflammatory compounds as a combined preparation for simultaneous, separate or sequential use in the treatment of inflammatory diseases.

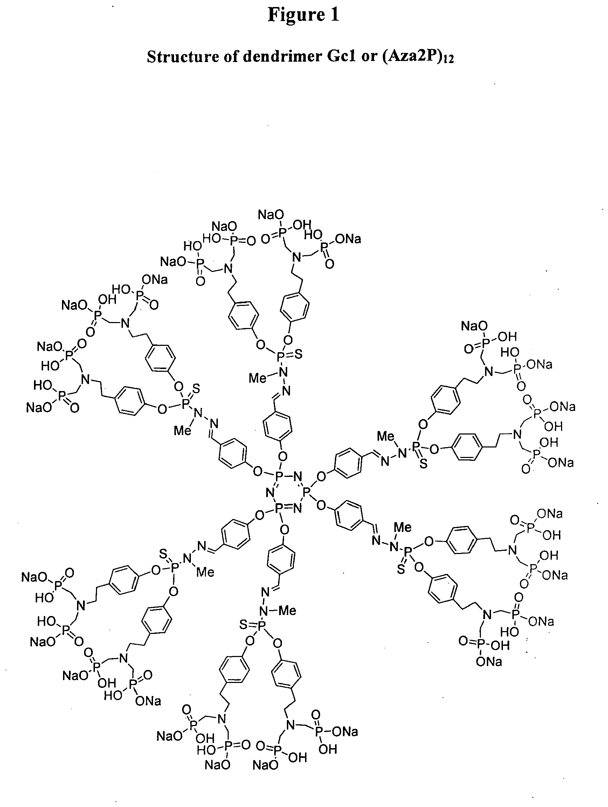

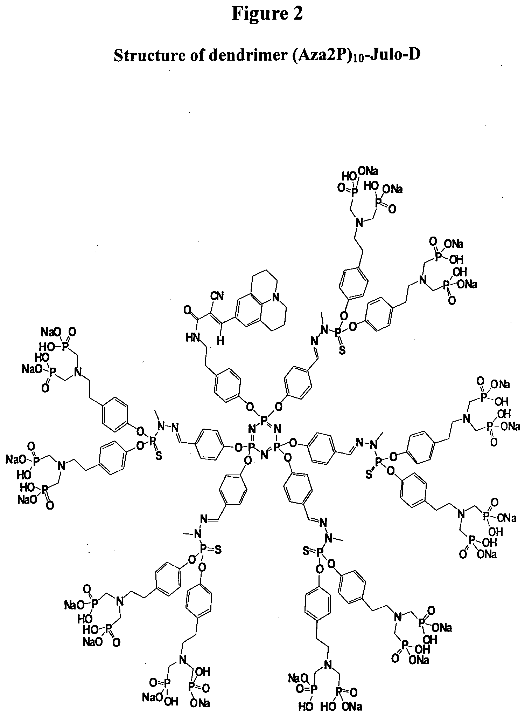

Examples 1 to 9 and figures 1 to 15 which follow illustrate the invention.
**Figures 1 to 3** show the structure of the bisphosphonic-capped dendrimers tested in examples 5 to 7.

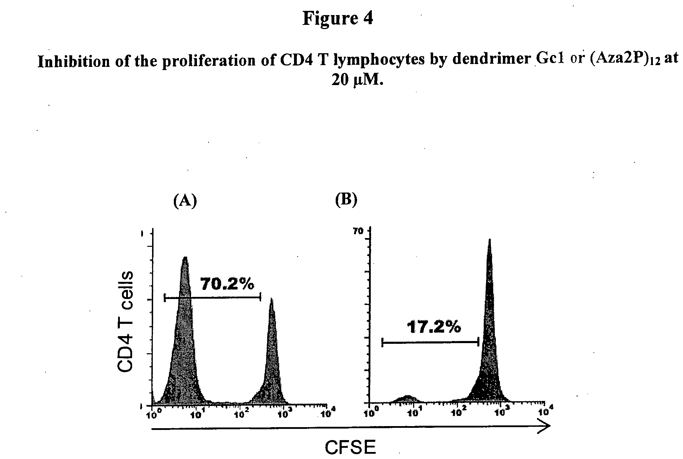

**Figure 4** shows the inhibitory properties of dendrimer Gc1 (or (Aza2P)₁₂) at 20 µM toward the proliferation of CD4 T lymphocytes in PBMC induced by IL2; (A) proliferation with IL2 alone (positive control); (B) proliferation with IL2 and Gc1 at 20 µM.

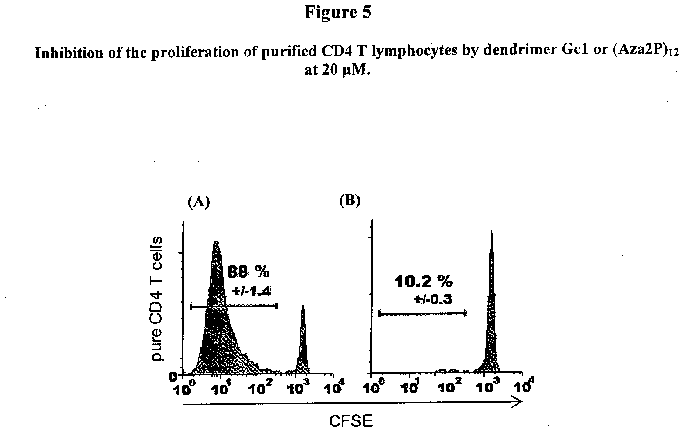

**Figure 5** shows the inhibitory properties of dendrimer Gc1 (or (Aza2P)₁₂) at 20 µM toward the proliferation of purified CD4 T lymphocytes stimulated by anti-CD3/anti-CD28 monoclonal antibodies (mAbs) and IL2; (A) proliferation with mAbs and IL2; (B) proliferation with mAbs and IL2 in presence of Gc1 at 20 µM.

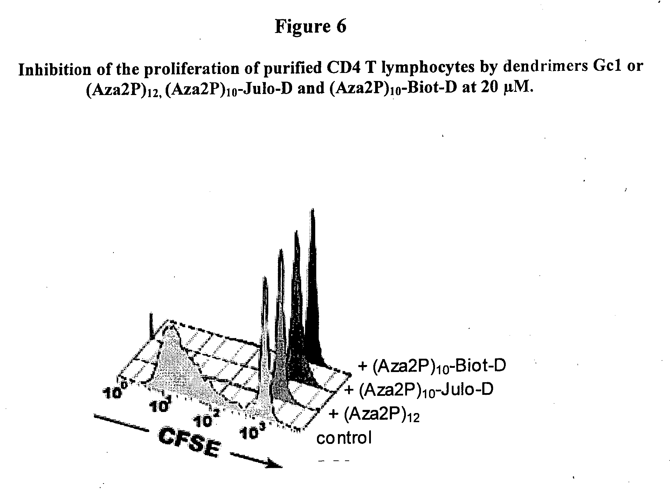

**Figure 6** shows the inhibitory properties of dendrimer Gc1 (or (Aza2P)₁₂) and of its analogs (Aza2P)₁₁-Julo-D and (Aza2P)₁₀-Biot-D at 20 µM toward the proliferation of purified CD4 T lymphocytes stimulated by anti-CD3/anti-CD28 mAbs and IL2.

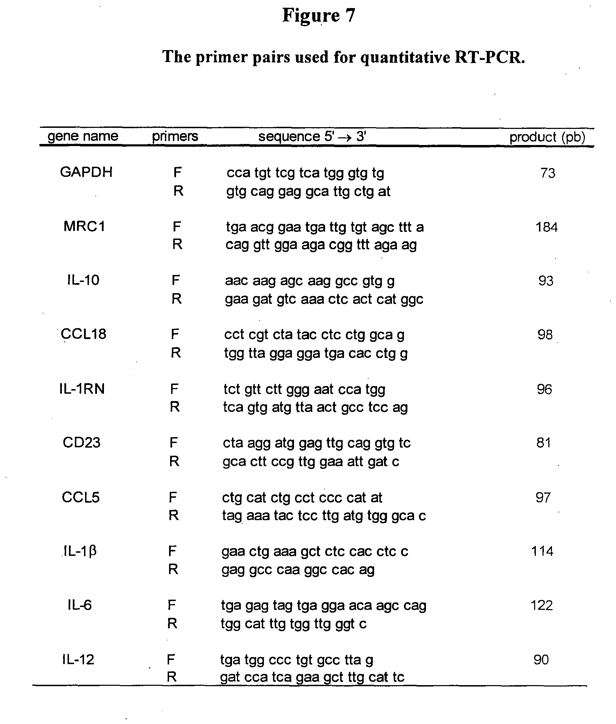

**Figure 7** shows the primer pairs used for quantitative RT-PCR. F: forward primer, R: reverse primer. Products (pb) correspond to the expected RT-PCR product size.

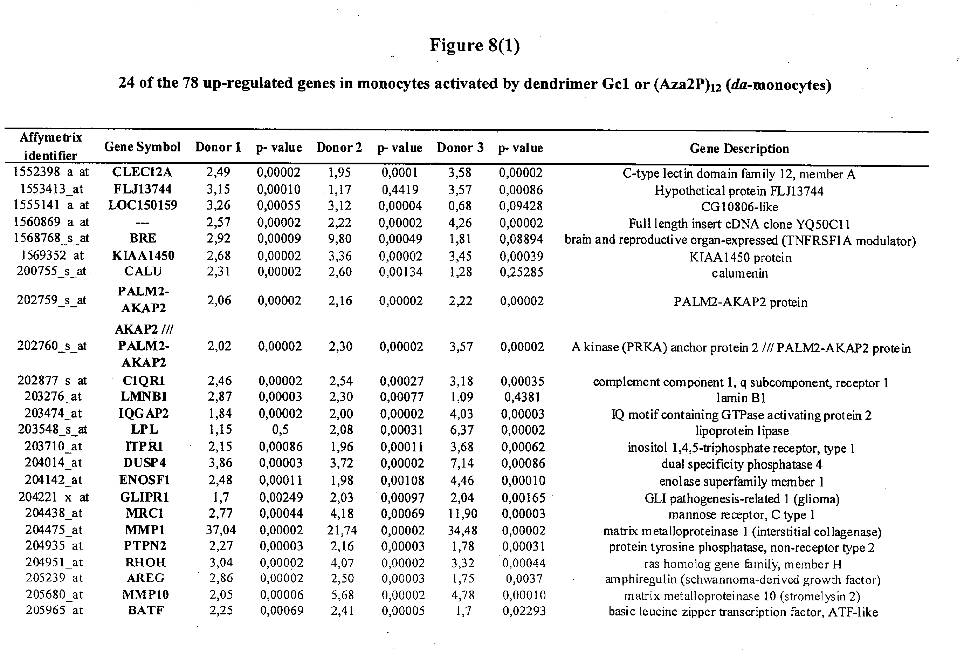

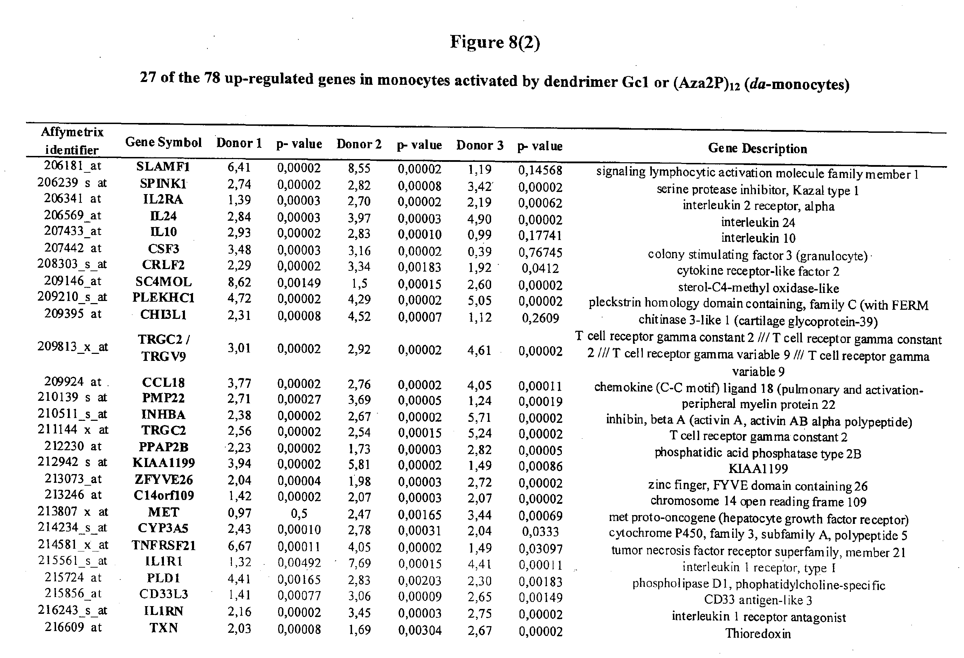

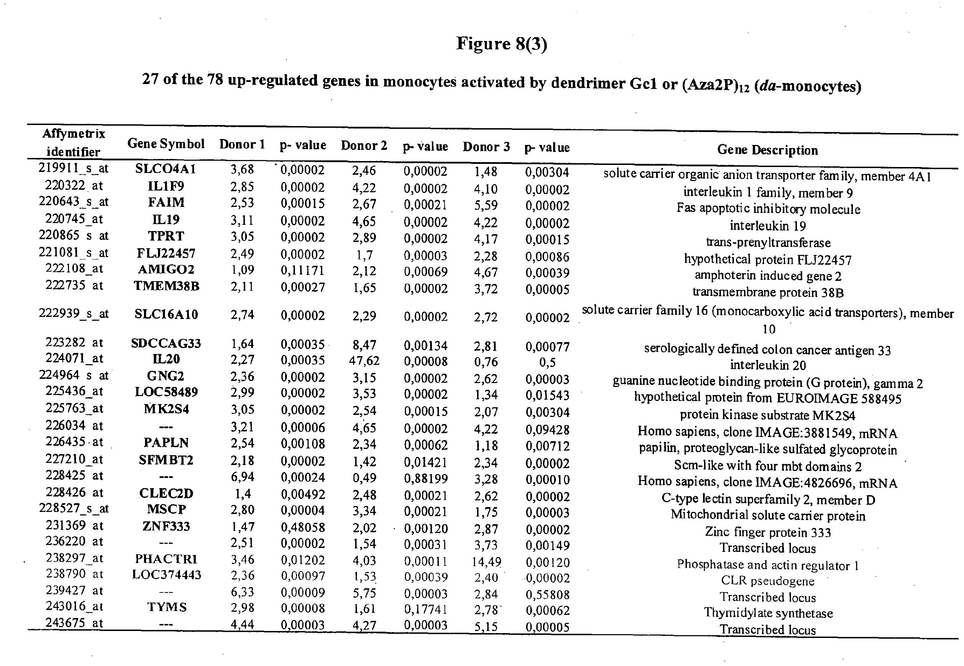

**Figures 8(1)****,** **8(2)** **and** **8(3)** show the 78 up-regulated genes in monocytes activated by dendrimer Gc1 (or (Aza2P)₁₂) (*da*-monocytes).

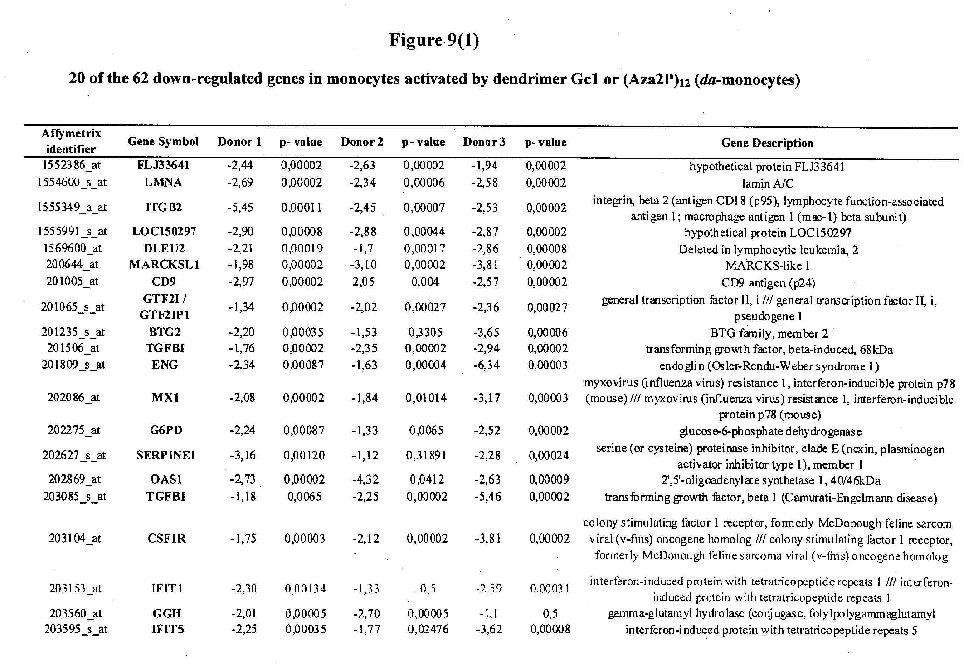

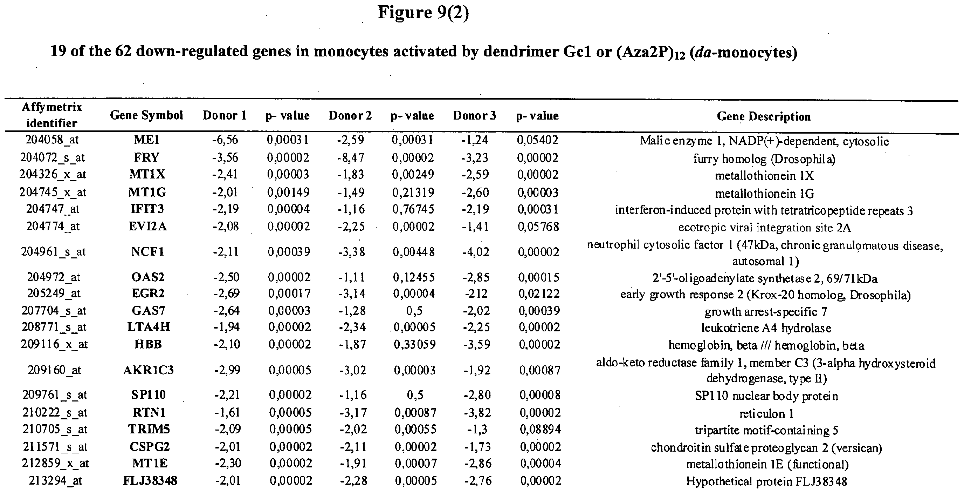

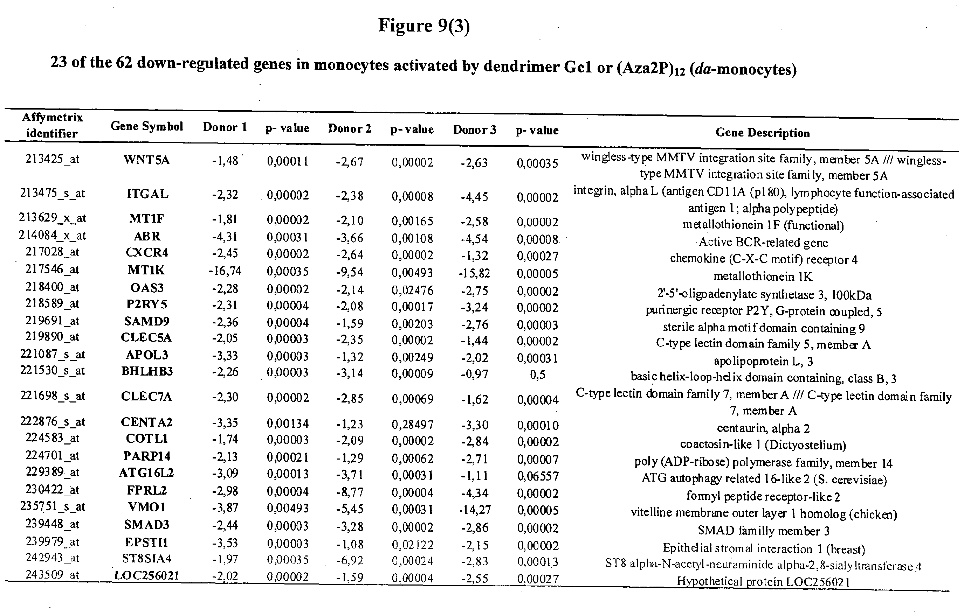

**Figures 9(1)****,** **9(2)** **and** **9(3)** show the 62 down-regulated genes in *da*-monocytes.

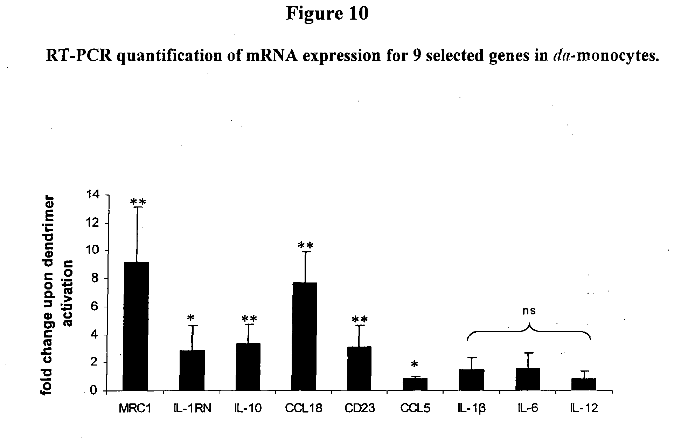

**Figure 10** shows the RT-PCR quantification of mRNA expression for 9 selected genes in *da*-monocytes. Expression levels are normalized to the GAPDH mRNA. Relative expressions are calculated using the ΔΔCt method and results represent the relative fold change induced by the dendrimer in comparison to non-stimulated monocytes. Data are expressed as mean ± SD from 6 independent experiments. * p<0.05, ** p<0.005, one tailed t-test.

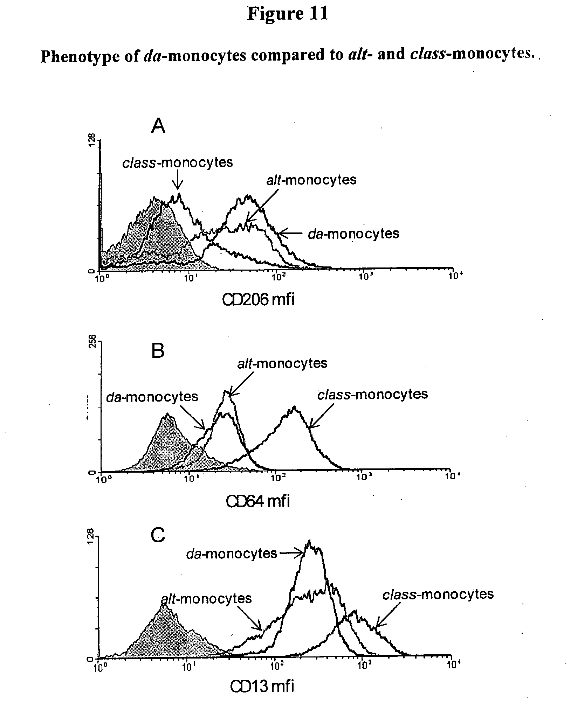

**Figure 11** shows the phenotype of *da*-monocytes compared to *alt-* and *class-*monocytes. Cell surface expression levels for (A) CD206 (the mannose receptor MRC1), (B) CD64 (a F_{c}γRI) and (C) CD13 (the aminopeptidase N) are analyzed by flow cytometry. Mfi detected on class-monocytes labelled by isotype-matched mAbs are represented in grey as negative control. Data shown are representative of 3 independent experiments.

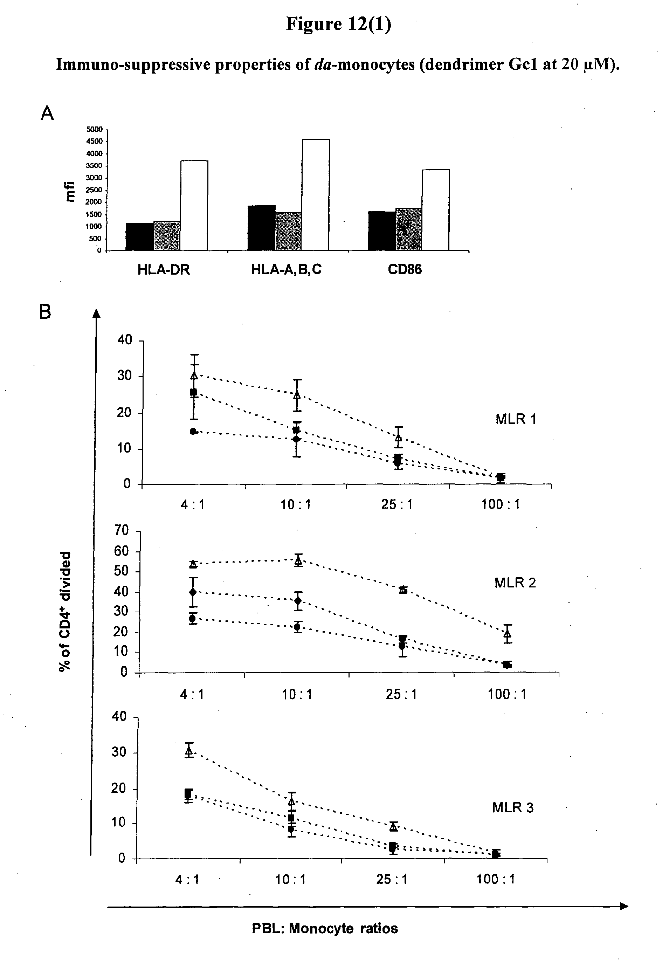

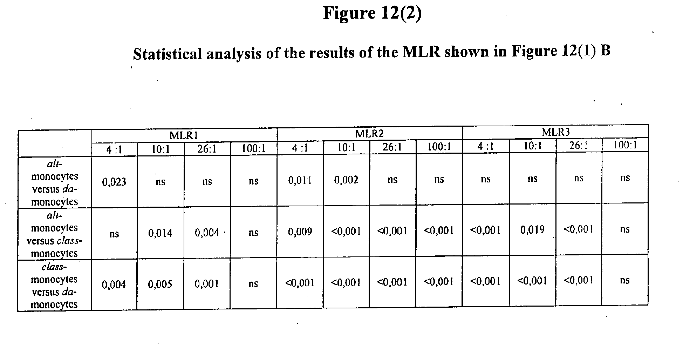

**Figures 12(1)** **and** **12(2)** show the immuno-suppressive properties of *da*-monocytes (dendrimer Gc1 at 20 µM). Figure 12(1)A shows flow cytometry analysis (showed as mfi) of the cell surface expression levels of antigen-presenting molecules (HLA-DR and HLA-A,B,C) and the co-stimulatory molecule CD86 on *da-* (black bars), *alt-* (grey bars) and *class-*monocytes (white bars). Data shown are representative of 3 independent experiments. In Figure 12(1)B, CD4⁺ T lymphocytes are gated to quantify CFSE dilution by flow cytometry after MLR. For the different PBL:monocyte (● *da*-monocytes, ■ *alt*-monocytes and Δ *class-*monocytes) ratios, the percentages of divided CD4⁺ T cells represent cells having undergone at least one division. Each point is the mean of percentages of divided CD4⁺ T cells ± SD from triplicates. The results for 3 different MLR are shown. Figure 12(2) gives the statistical analysis of the results shown in Figure 12(1)B.

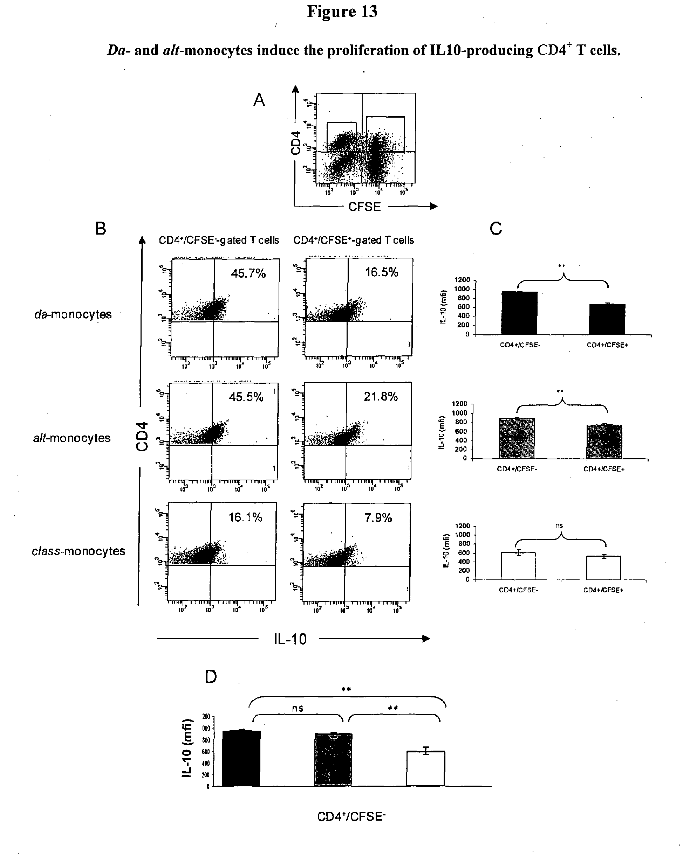

**Figure 13** shows that *da-* and *alt*-monocytes, but not *class*-monocytes, induce the proliferation of IL10-producing CD4⁺ T cells. A) After MLR and a 5 hour re-stimulation, divided CD4⁺/CFSE⁻ T lymphocytes and non-divided CD4⁺/CFSE⁺ T lymphocytes are gated to quantify intracellular IL10 by flow cytometry. B) Flow cytometry analysis of intracellular IL10 (% of IL10⁺ CD4⁺ T cells) in divided (CD4⁺/CFSE⁻-gated, left column) and non-divided (CD4⁺/CFSE⁺-gated, right column) T cells. Presented results are representative of 3 independent MLR. C) Comparison (mean of mfi ± SD from triplicates) of intracellular IL10 in divided and non-divided CD4⁺ T cells in MLR with *da-* (upper black bars), *alt-* (middle grey bars) and *class*-monocytes (lower white bars). ** p<0.005, Student's t-test. D) Comparison (mean of mfi ± SD from triplicates) of intracellular IL10 in divided CD4⁺ T cells in MLR with *da-* (left black bar), *alt-* (middle grey bar) and *class*-monocytes (right white bar). ** p<0.001, one-way ANOVA. In flow cytometry dot plots, all quadrants are set using appropriate isotype controls.

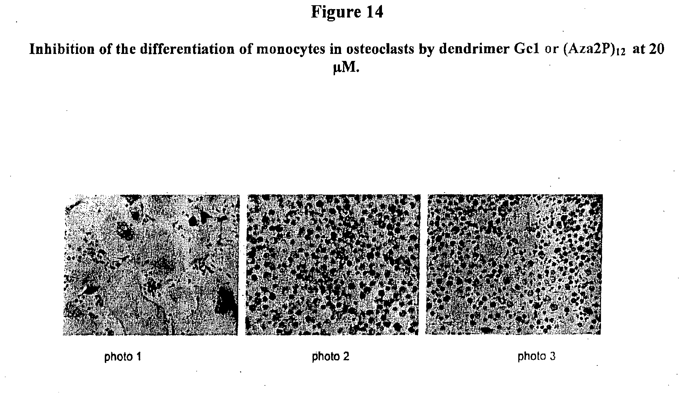

**Figure 14** shows the inhibition of the differentiation of human monocytes in osteoclasts by dendrimer Gc1 or (Aza2P)₁₂ at 20 µM, as in example 3. Picture 1: differentiation in control medium (without dendrimer Gc1); picture 2: differentiation in the presence of dendrimer Gc1 or (Aza2P)₁₂ at 20 µM; picture 3: differentiation after pre-incubation (6 hours) with dendrimer Gc1 or (Aza2P)₁₂ at 20 µM.

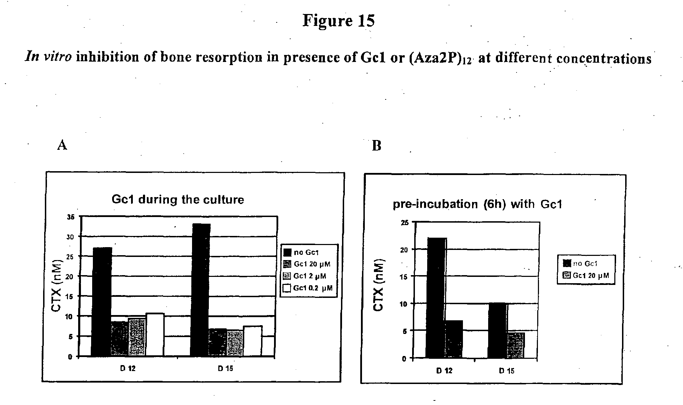

**Figure 15** shows the *in vitro* inhibition of bone resorption in presence of dendrimer Gc1 or (Aza2P)₁₂ at different concentrations as explained in example 4; A) Gc1 or (Aza2P)₁₂ added in the culture medium at different concentrations; B) after pre-incubation (6 hours) in Gc1 or (Aza2P)₁₂ at 20 µM.

Examples 1 to 4 illustrate the synthesis of some dendrimers.

### GENERAL POINTS

The reactions were carried out under a dry argon atmosphere (argon U, Air Liquide). The following solvents were dried and distilled under argon immediately before use according to the techniques described by Perrin et al, Purification of Laboratory Chemicals, Third Edition; Press, P., Ed.: Oxford, 1988**:** tetrahydrofuran, dichloromethane, acetonitrile, pentane, toluene, diethyl ether, chloroform, triethylamine, pyridine.

Thin layer chromatography analyses were carried out on aluminium plates coated with silica of the Merck Kieselgel 60F₂₅₄ type.

The NMR spectra were recorded on Brüker devices (AC200, AM250, DPX 300). The chemical shifts are expressed in parts per million (ppm) relative to phosphoric acid at 85 % in water for the ³¹P NMR and relative to tetramethylsilane for the ¹H and ¹³C NMR. The following abbreviations were used in order to express the multiplicity of signals: s (singlet), d (doublet), bd (broad doublet), dd (doublet of doublets), AB syst. (AB system), t (triplet), dt (doublet of triplets), q (quadruplet), hept (heptuplet), m (unresolved multiplet).

Infrared vibrational spectroscopy was carried out on a Perkin Elmer FT 1725x spectrometer. The UV-visible spectroscopy was carried out on an HP 4852A device. The thermogravimetric measurements were carried out on a Netzch DSC 204 or Setaram TGA 92-16.18 device.

### Numbering used for the NMR attribution:

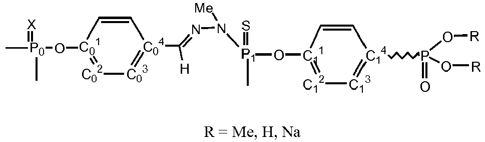

### Example of numbering for a first-generation dendrimer

### Example 1: Synthesis of a dendrimer of polyether type of first generation with azabis phosphonic acid ends derived from tyramine

### 1.1. Synthesis of phenol aza-bis-dimethyl-phosphonate derived from tyramine

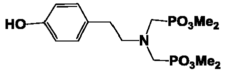

Tyramine (6 g, 43.7 mmol) and dimethyl-phosphite (10.32 ml, 112.5 mmol) are mixed at 0°, then a 37% solution of formaldehyde in water (12.6 ml) is slowly added, still at 0°C. The mixture is taken to ambient temperature for 30 minutes and refluxed for 1 hour with magnetic stirring. Finally the crude reaction product is placed under reduced pressure in order to evaporate the excess of formaldehyde. The product is extracted with a chloroform/water mixture (4/1) (3x100 ml of chloroform). The organic phase is recovered then subjected to chromatography on silica using acetone as eluent. The final product is isolated with a yield of 65%.
³¹P-{¹H} NMR (CDCl₃): δ = 30.2 (s, P(O)(OMe)₂) ppm.
¹H NMR (CDCl₃): δ = 2.68 (deformed t, ³*J*_{HH} = 7.2 Hz, 2H, -CH₂-CH₂-N); 3.05 (deformed t, ³*J*_{HH} = 7.2 Hz, 2H, -CH₂-CH₂-N-); 3.20 (d, ²*J*_{HP} = 8.9 Hz, 4H, N-CH₂-P); 3.75 (d, ³*J*_{HP} = 10.7 Hz, 12H, -OMe); 6.6-7.1 (m, 4H, CHₐᵣₒₘ); 8.16 (broad s, 1H, -OH) ppm.
¹³C-{¹H} NMR (CDCl₃): δ = 32.7 (s, C₅); 49.4 (dd, ³*J*_{CP} = 6.8 Hz, ¹*J*_{CP} = 158.5 Hz, C₇); 52.8 (d, ²*J*_{CP} = 3 Hz, C₈); 58.8 (t, ³*J*_{CP} = 7.5 Hz, C₆); 115.4 (s, C₃); 129.8 (s, C₂); 129.8 (s, C₄); 155.9 (s, C₁) ppm.

### 1.2. Synthesis of a dendrimer with azabis phosphonate terminations derived from tyramine

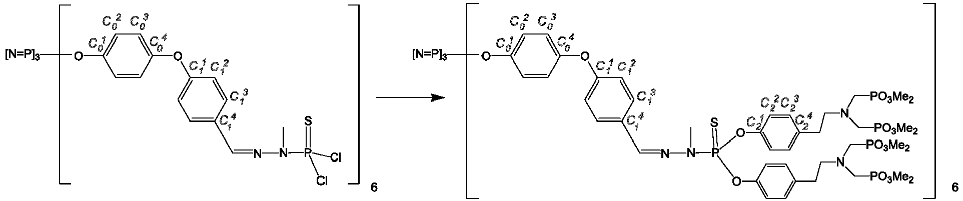

Tyramine aza-bisphosphonate of **Exemple 1.1** (388 mg, 1,020 mmol) and caesium carbonate (565 mg, 1,734 mmol) are added to a solution of a dendrimer of polyether type of first generation with PSCl₂ terminations (177 mg, 0,074 mmol) in a mixture of aceton / THF (10 mL / 3 mL). The reaction mixture is stirred at ambient temperature during 12 h, centrifuged and the resulting clear solution is evaporated to dryness under reduced pressure. The obtained oil is purified by chromatography on silica gel (gradient acetone / triethylamine (100:0 a 90:10), Rf = 0.97 in aceton / triethylamine (90:10)) to afford the dendrimer with dimethylphosphonate ends as a pale yellow solid (yield: 91%).
³¹P-{¹H} NMR (CDCl₃, 121.5 MHz): *δ* = 9.27 (s, N₃P₃); 26.78 (s, PO₃Me₂); 63.18 (s, P₁); ¹H NMR (CDCl₃, 300.13 MHz): *δ* = 2.74 (t, ³*J*_{HH} = 7.2 Hz, 24H, CH₂-CH₂-N); 3.05 (t, ³*J*_{HH} = 7.2 Hz, 24H, CH₂-CH₂-N); 3.17 (d, ²*J*_{HP} = 9.3 Hz, 48H, N-CH₂-P); 3.29 (d, ³*J*_{HP} = 10.2 Hz, 18H, CH₃-N-P₁); 3.71 (dd, ³*J*_{HP} = 10.5 Hz, ⁷*J*_{HP} = 1.5 Hz, 144H, OMe); 6.93 (m, 12H, C₀³-H); 6.94 (m, 12H, C₁²-H); 7.00 (m, 12H, C₀²-H); 7.10 (m, 24H, C₂²-H); 7.16 (m, 24H, C₂³-H); 7.60 (br s, 6H, CH=N); 7.67 (m, 12H, C₁³-H); ¹³C-{¹H} NMR (CDCl₃, 75.6 MHz): *δ* = 32.87 (br s, CH₃-N-P₁); 33.04 (s, CH₂-CH₂-N); 49.48 (dd, ¹*J*_{CP} = 157.5 Hz, ³*J*_{CP} = 7.3 Hz, N-CH₂-P); 52.62 (d, ²*J*_{CP} = 3.4 Hz, OMe); 52.66 (d, ²*J*_{CP} = 3.4 Hz, OMe) ; 58.08 (t, ³*J*_{CP} = 7.5 Hz, CH₂-CH₂-N); 118.50 (s, C₁²); 120.40 (s, C₀³); 121.27 (d, ³*J*_{CP} = 4.5 Hz, C₂²); 122.23 (s, C₀²); 128.61 (s, C₁³); 129.87 (s, C₂³); 130.21 (s, C₁⁴); 136.47 (d, ⁵*J*_{CP} = 1.8 Hz, C₂⁴); 138.89 (d, ³*J*_{CP} = 13.8 Hz, CH=N); 146.37 (td, ²*J*_{CP} = 5.2 Hz, ⁴*J*_{CP} = 2.5 Hz, C₀¹); 148.96 (d, ²*J*_{CP} = 7.0 Hz, C₂¹); 153.58 (s, C₀⁴); 158.32 (s, C₁¹) ppm.

### 1.3. Synthesis of the dendrimer with sodium salt of azabisphosphonic acid ends derived from tyramine

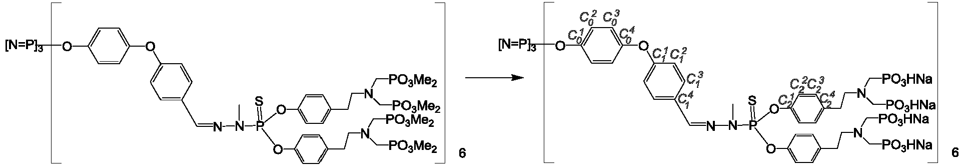

Trimethylsilyl bromide (535 µL, 4.00 mmol) is added to a solution of dendrimer dimethylphosphonate ends (440 mg, 5.75 10⁻² mmol) (obtained in example 1.2.) in acetonitrile (10 mL) at 0°C. The mixture is stirred at 25 °C during 12 h then evaporated to dryness under reduced pressure. The obtained residue is treated with methanol (2 x 15 mL), washed with ether (20 mL) and suspendedin water (1 mL/100 mg) in the presence of one equivalent of NaOH for one phosphonic end. The solution is lyophilised to afford the dendrimer with sodium salt of phosphonic acid ends as a white solid (yield: 85%).
³¹P-{¹H} NMR (D₂O/CD₃CN 7:3, 121.5 MHz): *δ* = 6.82 (s, PO₃HNa); 10.43 (s, N₃P₃); 13.82 (s, PO₃Na₂); 64.56 (s, P₁); ¹H NMR (H₂O/CD₃CN 7:3; 300.13 MHz): *δ* = 3.25 (AA' part of a AA'BB' system, m, 24H, CH₂-CH₂-N); 3.51 (d, ²*J*_{HP} = 11.7 Hz, 48H, N-CH₂-P and 18H, CH₃-N-P₁); 3.82 (BB' part of a AA'BB' system, m, 24H, CH₂-CH₂-N); 7.06 (m, 36 H, C₀²-H, C₀³-H, C₁²-H); 7.30 (m, 24H, C₂²-H); 7.52 (m, 24H, C₂³-H); 7.79 (br s, 6H, CH=N); 7.98 (m, 12H, C₁³-H); ¹³C-{¹H} NMR (D₂O/CD₃CN 7:3, 75.6 MHz): *δ* = 29.01 (s, CH₂-CH₂-N); 32.74 (br s, CH₃-N-P₁); 52.74 (br s, N-CH₂-P); 57.78 (s, CH₂-CH₂-N); 120.45 (s, C₁²); 121.35 (s, C₀³) ; 121.27 (d, ³*J*_{CP} = 4.5 Hz, C₂²); 122.53 (s, C₀²); 128.94 (s, C₁³); 130.50 (s, C₁⁴); 130.85 (s, C₂³); 134.55 (s, C₂⁴); 141.00 (br s, CH=N); 146.01 (m, C₀¹); 149.36 (d, ²*J*_{CP} = 6.1 Hz, C₂¹); 153.79 (s, C₀⁴); 158.17 (s, C₁¹) ppm.

### Reference example 2: Synthesis of a dendrimer of carbosilane type of first generation with azabis phosphonic acid ends derived from tyramine

### 2.1: Synthesis of the dendrimer with azabisphosphonate ends derived from tyramine

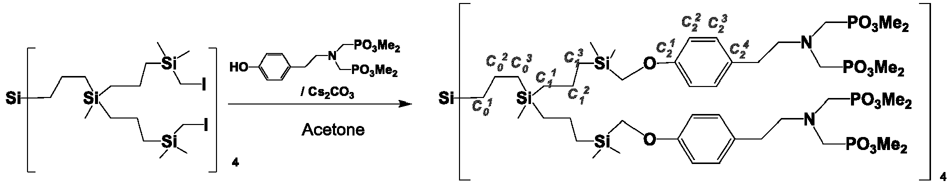

Tyramine aza-bisphosphonate obtained according to **example 1.1** (320 mg, 0.830 mmol) and caesium carbonate (540 mg, 1.66 mmol) are added to a solution of carbosilane type dendrimer of first generation with SiCH₂I ends (200 mg, 8.7.10⁻⁵ mmol) in acetone (2 mL). The mixture is stirred at 40 °C during 20 h, centrifuged and the resulting clear solution is evaporated to dryness under reduced pressure. The obtained oil is purified by chromatography on silica gel (gradient acetone / methanol (100:0 to 0:100), Rf = 0.38 in acetone / methanol (90:10)) to afford the dendrimer with dimethylphosphonate ends as a pale yellow solid (yield: 65%).
³¹P-{¹H} NMR (acetone-*d6*, 162.0 MHz): *δ* = 26.44 (s, PO₃Me₂); ¹H NMR (CDCl₃, 400.13 MHz): *δ* = 0.00 (s, 12H, Si-CH₃); 0.14 (s, 48H, Si-(CH₃)₂); 0.67 (br s, 32H, C₀¹-H, C₀³-H, C₁¹-H); 0.77-0.80 (m, 16H, C₁³-H); 1.45-1.52 (m, 24H, C₀²-H, C₁²-H); 2,75 (AA' part of a AA'BB' system, m, 16H, CH₂-CH₂-N); 3.03 (BB' part of a AA'BB' system, m, 16H, CH₂-CH₂-N); 3.17 (d, ²*J*_{HP} = 9.3 Hz, 32H, N-CH₂-P); 3.61 (s, 16H, Si-CH₂-O); 3.72 (d, ³*J*_{HP} = 10.4 Hz, 96H, OMe); 6.89 (m, 16H, C₂²-H); 7.18 (m, 16H, C₂³-H); ¹³C-{¹H} NMR (CDCl₃, 100.6 MHz): *δ* = -5.25 (s, Si-CH₃); -5.12 (s, Si-(CH₃)₂); 17.60 (s, C₀²); 18.32 (s, C₁²); 18.37 (C₁³); 18.37 (C₁¹); 18.71 (s, C₀¹); 18.97 (s, C₀³); 32.23 (s, CH₂-CH₂-N); 49.98 (dd, ¹*J*_{CP} = 156.9 Hz, ³*J*_{CP} = 7.7 Hz, N-CH₂-P); 51.94 (br s, OMe); 58.74 (t, ³*J*_{CP} = 7.0 Hz, CH₂-CH₂-N); 59.99 (s, Si-CH₂-O); 113.88 (s, C₂²); 129.65 (s, C₂³); 131.48 (s, C₂⁴); 160.02 (s, C₂¹); ²⁹Si NMR (CDCl₃; 79.5 MHz): *δ* = -0.30 (s, Si-CH₂-0); 1.07 (s, Si-CH₃); 3.98 (s, Si at the core) ppm.

### 2.2: Synthesis of the dendrimer with sodium salt of azabisphosphonic acid ends derived from tyramine

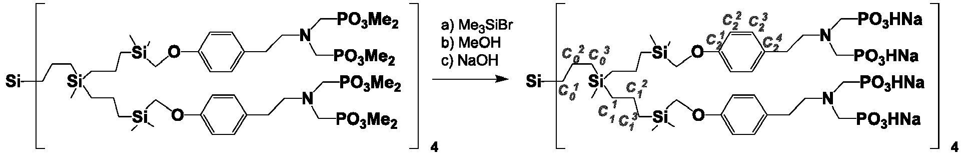

Trimethylsilyle bromide (120 µL, 8.56.10⁻¹ mmol) is added to a solution of dendrimer with dimethylphosphonate end obtained in **example 2.1** (92.9 mg, 2.14 10⁻² mmol) in acetonitrile (2,5 mL) at 0°C. The mixture is stirred at 25 °C during 12 h then evaporated to dryness under reduced pressure. The obtained residue is treated with methanol (2 x 15 mL), washed with ether (20 mL) and suspended in water (1 mL/100 mg) in the presence of one equivalent of NaOH for one phosphonic end. The solution is lyophilised to afford the dendrimer with sodium salt phosphonic acid ends as a white solid (yield: 85%).
³¹P-{¹H} NMR (D₂O/acetone-*d6* 7:3, 162.0 MHz): *δ* = 6.64 (s, PO₃HNa); ¹H NMR (D₂O/acetone-*d6* 7:3; 400.13 MHz): *δ* = -0.14 (br s, 60H, Si-CH₃ and Si-(CH₃)₂); 0.45 (br s, 48H, C₀¹-H, C₀³-H, C₁¹-H, C₁³-H; 1.23 (br s, 24H, C₀²-H, C₁²-H); 2.83 (AA' part of a AA'BB' system, br s, 16H, CH₂-CH₂-N); 3.11 (br s, 32H, N-CH₂-P); 3.31 (BB' part of a AA'BB' system, br s, 32H, CH₂-CH₂-N and Si-CH₂-O); 6.65 (m, 16H, C₂²-H); 7.05 (m, 16H, C₂³-H); ¹³C-{¹H} NMR (D₂O/acetone-*d6* 7:3, 100.6 MHz): *δ* = -5.04 (br s, Si-CH₃ and Si-(CH₃)₂); 17.5-19.2 (C₀¹, C₀², C₀³, C₁¹, C₁², C₁³); 29.30 (s, CH₂-CH₂-N); 53.74 (d, ¹*J*_{CP} = 122.7 Hz, N-CH₂-P); 57.69 (s, CH₂-CH₂-N); 60.08 (s, CH₂-CH₂-N); 114.14 (s, C₂²); 128.57 (s, C₂⁴); 130.14 (s, C₂³); 159.86 (s, C₂¹).

### Reference example 3: Synthesis of Salamonczyk-type of second generation with azabis phosphonic acid ends derived from tyramine

### 3.1. Synthesis of the diphenoxyamino phosphine derived from tyramine

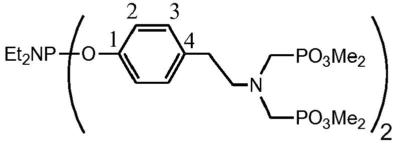

The experimental protocol used for preparing this molecule was inspired by that used by Salamonczyk in order to create his dendrimers (Tetrahedron Lett. 2000, 41, 1643). The aza bis phosphonate tyramine derivative obtained in **Example 1.1 is** weighed in a Schlenk tube under argon (2.3 g) and dissolved in 10 mL of distilled THF. The diethylaminodichlorophosphine is introduced into another Schlenk tube (0.5 mL) and placed in solution in 5 mL of distilled THF. The two Schlenk tubes are taken to -70°C. 1.4 mL of triethylamine are then added to the dichlorophosphine solution then the tyramine aza bis phosphonate solution is cannulated onto the mixture still at -70°C. The stirring is continued for half an hour at a low temperature then for 4 hours at ambient temperature. The mixture is then filtered on celite under argon then the solvent is eliminated under reduced pressure. The dry residue is kept under argon at a low temperature and used without other treatment in the rest of the synthesis.
³¹P{¹H} NMR (CDCl₃): δ = 30.4 (s, PO₃Me₂); 144.5 (s, Et₂NP) ppm.
¹H NMR (CDCl₃): δ = 1.00 (t, ³J_{HH} = 7.2 Hz, 6H, CH₃CH₂); 2.70 (m, 4H, N-CH₂CH₂); 3.00 (m, 4H, CH₂CH₂P); 3,11-3.23 (m, 12H, CH₂P, CH₃CH2); 3.69 (d, ³J_{HP} = 6.9 Hz, 24H, CH₃O); 6.90 (d, ³J_{HH} = 8.4 Hz, 4H, C²H); 6.97 (d, ³J_{HH} = 8.4 Hz, 4H, C³H) ppm.

### 3.2.: Synthesis of the dendrimer with azabisphosphonate ends derived from tyramine

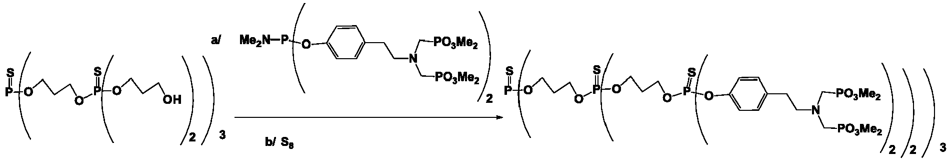

The phosphoramide derived from tyramine aza-bisphosphonate obtained as described in **example 3.1** (160 mg, 0.17 mmol solubilized in dichloromethane / acetonitrile (1 mL / 1 mL)) and tetrazole (180 mg, 2.60 mmol) are added to the dendrimer Salamonczyk with hydroxyle ends (1.64 g, 1.9 mmol) in solution in dichloromethane (4 mL). The mixture is stirred at 25 °C during 3 h 30 before addition of S₈ (512 mg, 2 mmol). The heterogeneous mixture is strired during 12 h at 25 °C then filtered. The filtrate is evaporated to dryness under reduced pressure and purified by chromatography on silica gel (elution gradient: dichloromethane / acetone (50:50 a 0:100) then acetone / methanol (100:0 a 0:100), Rf = 0.95 in methanol) to afford the dendrimer with dimethyphosphonate ends as a white solid (yield: 43%).
³¹P-{¹H} NMR (acetone-*d6*, 101.25 MHz): *δ* = 26.53 (s, PO₃Me₂), 58.72 (s, P²), 68.23 (s, P⁰ and P¹); ¹H NMR (acetone-*d6*, 300.13 MHz): *δ* = 2.15 (m, 18H, C₁²-H and C₀²-H); 2.85 (AA' part of a AA'BB' system, m, 24H, CH₂-CH₂-N); 3.17 (BB' part of a AA'BB' system, m, 24H, CH₂-CH₂-N); 3.25 (d, ²*J*_{HP} = 9.9 Hz, 48H, N-CH₂-P); 3.72 (d, ³*J*_{HP} = 10.5 Hz, 144H, OMe); 4.24 (m, 24H, C₀¹-H, C₀³-H and C₁¹-H); 4.44 (m, 12H, C₁³-H); 7.19 (m, 24H, C₂²-H); 7.36 (m, 24H, C₂³-H); ¹³C-{¹H} NMR (acetone-*d6*, 75,5 MHz): *δ* = 30.62 (m, C₀² and C₁²); 32.26 (s, CH₂-CH₂-N); 49.06 (dd, ¹*J*_{CP} = 157.6 Hz, ³*J*_{CP} = 8.2 Hz, N-CH₂-P); 52.13 (d, ²*J*_{CP} = 5.0 Hz, OMe); 58.13 (t, ³*J*_{CP} = 7.8 Hz, CH₂-CH₂-N); 64.49 (m, C₀¹, C₀³ and C₁³); 65.90 (m, ²*J*_{CP} = 6.0 Hz, C₁¹); 120.82 (d, ³*J*_{CP} = 4.5 Hz, C₂²); 130.28 (s, C₂³); 137.54 (s, C₂⁴); 148.99 (d, ²*J*_{CP} = 7.6 Hz, C₂¹).

### 3.3.: Synthesis of the dendrimer with sodium salt azabis phosphonic acid ends derived from tyramine

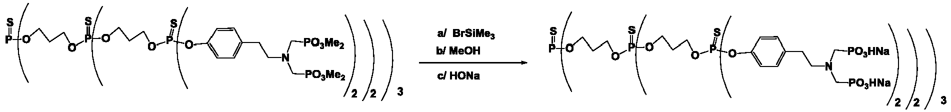

Trimethylsilyle bromide (190 µL, 1.44 mmol) is added to a solution of dendrimer with dimethylphosphonate ends obtained in **example 3.2** (140 mg, 2.39 10⁻² mmol) in acetonitrile (3,5 mL) at 0°C. The mixture is stirred at 25 °C during 12 h then evaporated to dryness under reduced pressure. The residue thus obtained is treated with methanol (2 x 15 mL), washed with ether (20 mL) and suspended in water (1 mL/100 mg) in presence of one equivalent of NaOH for one phosphonic end. The obtained solution is lyophilised to afford the dendrimer with sodium salt of phosphonic acid ends as a white solid (yield: 70%).
³¹P-{¹H} NMR (D₂O/CD₃CN 9:1, 81.0 MHz): *δ* = 10.19 (s, PO₃HNa), 14.63 (s, PO₃Na₂), 62.78 (s, P²), 70.58 (s, P⁰ and P¹); ¹H NMR (D₂O/CD₃CN 9:1, 300.13 MHz): *δ* = 2.03 (m, 18H, C₁²-H and C₀²-H); 3.07 (AA' part of a AA'BB' system, m, 24H, CH₂-CH₂-N); 3.40 (br s, 48H, N-CH₂-P); 3.63 (BB' part of a AA'BB' system, m, 24H, CH₂-CH₂-N); 4.13 (br s, 24H, C₀¹-H, C₀³-H and C₁¹-H); 4.38 (br s, 12H, C₁³-H); 7.12 (m, 24H, C₂²-H); 7.31 (m, 24H, C₂³-H); ¹³C-{¹H} RMN (CDCl₃; 75.5 MHz): *δ* = 29.12 (s, CH₂-CH₂-N); 32.26 (m, C₀² and C₁²); 52.64 (d, ¹*J*_{CP} = 131.7 Hz, N-CH₂-P); 58.03 (br s, CH₂-CH₂-N); 64.90 (m, C₀¹, C₀³, C₁¹ and C₁³); 121.45 (s, C₂²); 130.83 (s, C₂³); 134.51 (s, C₂⁴); 149.31 (d, ²*J*_{CP} = 7.2 Hz, C₂¹).

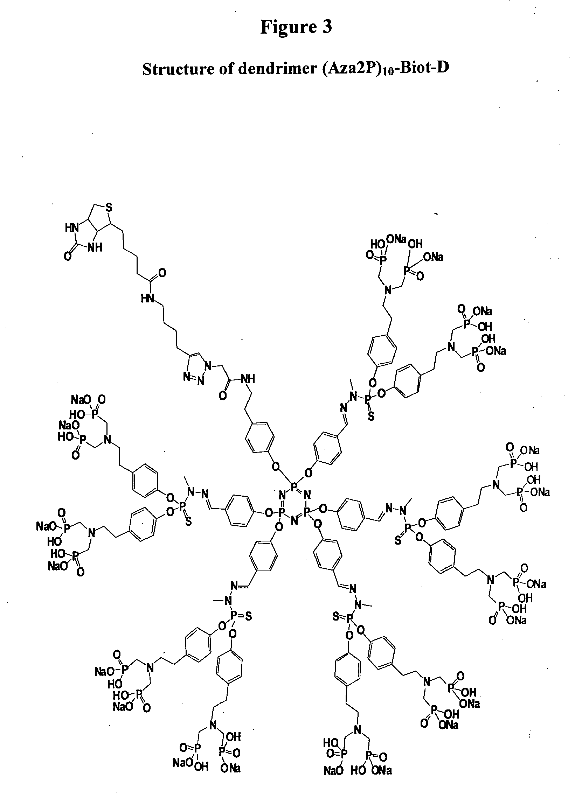

### Example 4: Synthesis of dimethylphosphonate terminated dendrimer with a biotinylated spacer (Aza 2P)₁₀-Biot-D (voir figure 3)

### 4.1. Synthesis of 2-chloro-N-[2-(4-hydroxy-phenyl)-ethyl]-acetamide

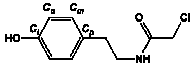

To a solution of tyramine (1.543 g, 11.25 mmol) in 25 mL of dichloromethane/saturated aqueous sodium carbonate mixture (1:1) was added chloroacetyl chloride (0.896 mL, 11.25 mmol) and the mixture was stirred at room temperature for 2 h. It was then diluted in water (50 mL) and extracted with dichloromethane (150 mL). The organic phase was dried over magnesium sulfate, filtered and evaporated to give a white solid which was purified by column chromatography (silica, dichloromethane/methanol, 97:3) to give 2-chloro-N-[2-(4-hydroxy-phenyl)-ethyl]-acetamide as a white solid (yield = 75 %).
¹H NMR (DMSO-*d6*, 300.1 MHz): δ = 2.61 (t, ³*J*_{HH} = 7.5 Hz, 2H, C₆H₄-CH₂-CH₂-NH), 3.24 (td, ³*J*_{HH} = 7.5 Hz, ³*J*_{HH} = 5.1 Hz, 2H, C₆H₄-CH₂-CH₂-NH), 4.03 (s, 2H, CO-CH₂), 6.68 (m, 2H, Cₒ-H), 6.99 (d, ³*J*_{HH} = 8.4 Hz, 2H, Cₘ-H), 8.23 (t, ³*J*_{HH} = 5.1 Hz, 1H, NH), 9.17 (s, 1H, OH); ¹³C{¹H} NMR (CDCl₃, 50.3 MHz): δ = 34.3 (s, C₆H₄-CH₂-CH₂-NH), 41.2 (s, CO-CH₂-Cl), 42.5 (s, C₆H₄-CH₂-CH₂-NH), 115.5 (s, Cₒ), 128.9 (s, Cₚ), 129.5 (s, Cₘ), 155.7 (s, Cᵢ), 165.9 (s, CO) ppm. DCI-MS (NH₃): *m*/*z* = 231 [M+NH₄]⁺, 214 [M+H]⁺.

### 4.2. Synthesis of 2-Azido-N-[2-(4-hydroxy-phenyl)-ethyl]-acetamide

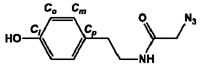

To a solution of 2-chloro-N-[2-(4-hydroxy-phenyl)-ethyl]-acetamide obtained in **example 4.1** (250 mg, 1.17 mmol) in DMSO (3 mL) was added sodium azide (152 mg, 2.34 mmol) and the mixture was stirred at room temperature for 12 h. The reaction mixture was then diluted in water (70 mL) and extracted with ethyl acetate (140 mL) The organic phase was dried over magnesium sulfate, filtered and evaporated to give 2-Azido-N-[2-(4-hydroxyphenyl)-ethyl]-acetamide as a viscous solid (yield = 90 %) that crystalises upon standing.
¹H NMR (CDCl₃, 300.1 MHz): δ = 2.75 (t, ³*J*_{HH} = 6.9 Hz, 2H, C₆H₄-CH₂-CH₂-NH), 3.51 (td, ³*J*_{HH} = 6.9 Hz, ³*J*_{HH} = 6.3 Hz, 2H, C₆H₄-CH₂-CH₂-NH), 3.95 (s, 2H, CO-CH₂), 6.51 (broad s, 1H, NH), 6.82 (m, 2H, Cₒ-H), 7.02 (m, 2H, Cₘ-H), 7.38 (broad s, 1H, OH); ¹³C{¹H} NMR (CDCl₃, 75.5 MHz): δ = 34.6 (s, C₆H₄-CH₂-CH₂-NH), 40.9 (s, C₆H₄-CH₂-CH₂-NH), 52.6 (s, CO-CH₂-N), 115.7 (s, Cₒ), 129.5 (s, Cₚ), 129.7 (s, Cₘ), 155.3 (s, Cᵢ), 167.1 (s, CO) ppm. DCI-MS (NH₃): *m*/*z* = 238 [M+NH₄]⁺.

### 4.3. Synthesis of 2-{4-[4-(1,3-Dioxo-1,3-dihydro-isoindol-2-yl)-butyl]-[1,2,3]triazol-1-yl}-N-[2-(4-hydroxy-phenyl)-ethyl]-acetamide

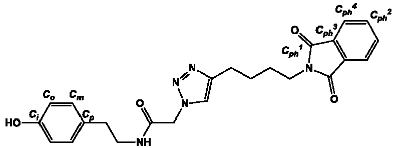

To 2.4 mL of tert-butanol/water mixture (1:1) were suspended 2-Azido-N-[2-(4-hydroxy-phenyl)-ethyl]-acetamide obtained in example 4.2 (385 mg, 1.750 mmol), *N*-(5-hexynyl)phthalimide (398 mg, 1.750 mmol), sodium ascorbate (35 mg, 0.175 mmol) and coppper sulfate (140 mg, 0.088 mmol). The reaction mixture was stirred at room temperature for 12 h and was then diluted in water, and filtered. The solid was washed with water and ether to give 2-{4-[4-(1,3-Dioxo-1,3-dihydro-isoindol-2-yl)-butyl]-[1,2,3]triazol-1-yl}-N-[2-(4-hydroxy-phenyl)-ethyl]-acetamide as a white solid (yield = 90 %). ¹H NMR (CD₃CN, 300.1 MHz): δ = 1.71 (m, 4H, CH₂-CH₂-CH₂-CH₂-N, CH₂-CH₂-CH₂-CH₂-N), 2.70 (m, 4H, C₆H₄-CH₂-CH₂-NH, CH₂-CH₂-CH₂-CH₂-N), 3.37 (q, ³*J*_{HH} = 6.9 Hz, ³*J*_{HH} = 6.3 Hz, 2H, C₆H₄-CH₂-CH₂-NH), 3.67 (t, ³*J*_{HH} = 6.3 Hz, 2H, CH₂-CH₂-CH₂-CH₂-N), 4.92 (s, 2H, CO-CH₂-N), 6.59 (broad s, 1H, NH), 6.73 (d, ³*J*_{HH} = 8.1 Hz, 2H, Cₒ-H), 7.02 (d, ³*J*_{HH} = 8.1 Hz, 2H, Cₘ-H), 7.50 (s, 1H, N-CH=C), 7.79 (m, 4H, Cₚₕ-H); ¹³C{¹H} NMR (CD₃CN, 75.5 MHz): δ = 24.7 (s, CH₂-CH₂-CH₂-CH₂-N), 26.5 (s, CH₂-CH₂-CH₂-CH₂-N), 27.7 (s, CH₂-CH₂-CH₂-CH₂-N), 34.1 (s, C₆H₄-CH₂-CH₂-NH), 37.4 (s, CH₂-CH₂-CH₂-CH₂-N), 40.8 (s, C₆H₄-CH₂-CH₂-NH), 52.1 (s, CO-CH₂-N), 115.1 (s, Cₒ), 122.7 (s, Cₚₕ⁴), 122.9 (s, N-CH=C), 129.8 (s, Cₘ), 130.2 (s, Cₚ), 132.3 (s, Cₚₕ³), 134.1 (s, Cₚₕ²), 147.5 (N-CH=C), 155.4 (s, Cᵢ), 165.6 (HN-CO-CH₂), 168.5 (s, Cₚₕ¹) ppm. DCI-MS (NH₃): *m*/*z* = 448 [M+NH₄]⁺.

### 4.4. Synthesis of tert-butyl 6-heptynoate ester

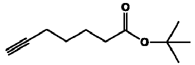

To a solution of 6-heptynoic acid (0.750 mL, 5.970 mmol), tert-butanol (1.7 mL, 17.91 mmol), and DMAP (73 mg, 0.597 mmol) in dichloromethane (12 mL) was added N,N'-dicyclohexylcarbodiimide (1.416 g, 6.864 mmol). The reaction mixture was stirred for 12 h at rt, and then the dicyclohexylurea was filtrated off and washed with dichloromethane (60 mL) and diethyl ether (30 mL). The organic phases were combined, dried over sodium sulfate and evaporated to dryness. The residue was purified by column chromatography (silica, pentane/ether, 98:2) to afford the tert-butyl 6-heptynoate ester as a colourless oil (yield = 30 %). ¹H NMR (CDCl₃, 300.1 MHz): δ = 1.45 (s, 9H, CH₃), 1.57 (m, 2H, CH₂-CH₂-CO), 1.70 (m, 2H, CH₂-CH₂-CH₂-CO), 1.96 (t, ⁴*J*_{HH} = 2.6 Hz, 1H, CH), 2.23 (m, 2H, CH₂-CO, C-CH₂); ¹³C{¹H} NMR (CDCl₃, 62.9 MHz): δ = 18.2 (C-CH₂), 24.1 (s, CH₂-CH₂-CO), 27.8 (s, CH₂-CH₂-CH₂-CO), 28.1 (s, CH₃), 35.0 (s, CH₂-CO), 68.5 (s, CH), 84.1 (s, C-CH₂), 172.8 (s, CO) ppm.

### 4.5. Synthesis of 5-(1-{[2-(4-Hydroxy-phenyl)-ethylcarbamoyl]-methyl}-1H-[1,2,3]triazol-4-yl)-pentanoic acid tert-butyl ester

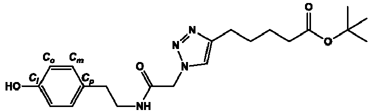

To 1.5 mL of tert-butanol/water mixture (1:1) were suspended 2-Azido-N-[2-(4-hydroxy-phenyl)-ethyl]-acetamide obtained in **example 4.2** (254 mg, 1.152 mmol), tert-butyl 6-heptynoate ester obtained in **example 4.4** (210 mg, 1.152 mmol), sodium ascorbate (23 mg, 0.015 mmol) and coppper sulfate (9 mg, 0.057 mmol). The reaction mixture was stirred at room temperature for 12 h. It was then diluted in 0.6 mL of THF and 10 mL of water, stirred for 10 min, and extracted with ethyl acetate (60 mL). The organic phase was washed with brine (30 mL), dried over sodium sulfate, filtered and evaporated to give a sticky solid which was purified by column chromatography (silica, ether/acetone, 1:0 to 8:2) to give 5-(1-{[2-(4-Hydroxy-phenyl)-ethylcarbamoyl]-methyl}-1H-[1,2,3]triazol-4-yl)-pentanoic acid tert-butyl ester as a sticky solid (yield = 85 %). ¹H NMR (CDCl₃, 300.1 MHz): δ = 1.47 (s, 9H, CH₃), 1.69 (m, 4H, CH₂-CH₂-CH₂-CH₂-CO, CH₂-CH₂-CH₂-CH₂-CO), 2.30 (t, ³*J*_{HH} = 6.4 Hz, 2H, CH₂-CH₂-CH₂-CH₂-CO), 2.71 (m, 4H, C₆H₄-CH₂-CH₂-NH, CH₂-CH₂-CH₂-CH₂-CO), 3.47 (q, ³*J*_{HH} = 6.3 Hz, 2H, C₆H₄-CH₂-CH₂-NH), 4.95 (s, 2H, CO-CH₂-N), 6.13 (t, ³*J*_{HH} = 5.5 Hz, 1H, NH), 6.76 (d, ³*J*_{HH} = 8.4 Hz, 2H, Cₒ-H), 6.89 (d, ³*J*_{HH} = 8.4 Hz, 2H, Cₘ-H), 7.31 (s, 1H, OH), 7.61 (s, 1H, N-CH=C); ¹³C{¹H} NMR (CDCl₃, 75.5 MHz): δ = 24.6(s, CH₂-CH₂-CH₂-CH₂-CO), 25.6 (s, CH₂-CH₂-CH₂-CH₂-CO), 28.1 (s, CH₃), 28.4 (s, CH₂-CH₂-CH₂-CH₂-CO), 34.0 (s, C₆H₄-CH₂-CH₂-NH), 35.2 (s, CH₂-CH₂-CH₂-CH₂-CO), 40.8 (s, C₆H₄-CH₂-CH₂-NH), 53.0 (s, CO-CH₂-N), 80.8 (s, C(CH₃)), 115.6 (s, Cₒ), 122.3 (s, N-CH=C), 129.3 (s, Cₚ), 129.7 (s, Cₘ), 148.5 (N-CH=C), 155.4 (s, Cᵢ), 165.4 (HN-CO-CH₂), 173.8 (s, CO₂) ppm. FAB-MS (>0): *m*/*z* = 403 [M+H]⁺, 347 [M-C₄H₉+2H]⁺.

### 4.6. Synthesis of penta(4-formylphenoxy)-chlorocyclotriphosphazene

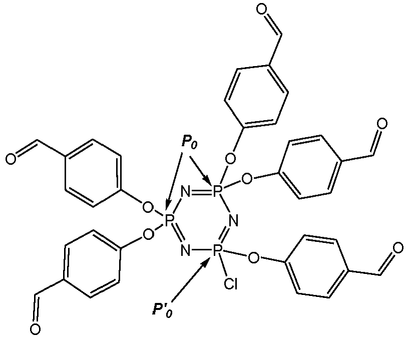

2591 mg of 4-hydroxybenzaldehyde sodium salt (18 mmol) are added at 0°C and under an inert atmosphere to a solution containing 1.2 g of hexachlorocyclotriphosphazene (3.45 mmol) in THF (300 mL). The reaction medium is stirred for 12 hours while the temperature is allowed to gradually return to ambient temperature. The crude reaction product is evaporated to dryness then purified by "flash" chromatography on a silica column. The product is isolated in the form of translucent oil with a yield of 70 %.
³¹P{¹H} NMR (CDCl₃, 81 MHz): δ = 9.2 (d, ²J_{PP} = 86.6 Hz, P₀); 24.3 (t, ²J_{PP} = 86.6 Hz, P'₀) ppm.

### 4.7. Synthesis of persubstituted cyclotriphosphazene derivative with a protected amine terminated spacer

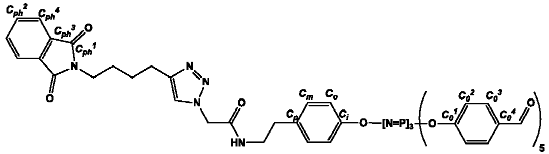

To a mixture of 2-{4-[4-(1,3-Dioxo-1,3-dihydro-isoindol-2-yl)-butyl]-[1,2,3]triazol-1-yl}-N-[2-(4-hydroxy-phenyl)-ethyl]-acetamide obtained in **example 4.3** (173 mg, 0.386 mmol) and of compound of **example 4.6** (272 mg, 0.351 mmol) in THF (15 mL) was added caesium carbonate (126 mg, 0.386 mmol), and the mixture was stirred at rt for 12 h. The reaction mixture was centrifugated, filtered and evaporated. The residue was purified by column flash chromatography (silica, pentane/ethyl acetate, 1:1) to give the title compound as a white oil (yield = 85 %). ³¹P{¹H} NMR (CDCl₃, 121.5 MHz): δ = 7.4 (s, N₃P₃); ¹H NMR (CDCl₃, 300.1 MHz): δ = 1.74 (m, 4H, CH₂-CH₂-CH₂-CH₂-N, CH₂-CH₂-CH₂-CH₂-N), 2.76 (m, 4H, C₆H₄-CH₂-CH₂-NH, CH₂-CH₂-CH₂-CH₂-N), 3.46 (q, ³*J*_{HH} = 6.7 Hz, 2H, C₆H₄-CH₂-CH₂-NH), 3.71 (t, ³*J*_{HH} = 6.5 Hz, 2H, CH₂-CH₂-CH₂-CH₂-N), 4.99 (s, 2H, CO-CH₂-N), 6.32 (t, ³*J*_{HH} = 5.7 Hz, 1H, NH), 6.91 (d, ³*J*_{HH} = 8.6 Hz, 2H, Cₒ-H), 6.98 (d, ³*J*_{HH} = 8.6 Hz, 2H, Cₘ-H), 7.14 (m, 10H, C₀²-H), 7.44 (s, 1H, N-CH=C), 7.73 (m, 12H, C₀³-H, Cₚₕ-H), 7.82 (m, 2H, Cₚₕ-H), 9.94 (s, 5H, CHO); ¹³C{¹H} NMR (CDCl₃, 75.5 MHz): δ = 25.0 (s, CH₂-CH₂-CH₂-CH₂-N), 26.4 (s, CH₂-CH₂-CH₂-CH₂-N), 27.9 (s, CH₂-CH₂-CH₂-CH₂-N), 34.7 (s, C₆H₄-CH₂-CH₂-NH), 37.5 (s, CH₂-CH₂-CH₂-CH₂-N), 40.8 (s, C₆H₄-CH₂-CH₂-NH), 53.0 (s, CO-CH₂-N), 120.7 (s, Cₒ), 121.3 (2 s, C₀²), 122.6 (s, N-CH=C), 123.2 (s, Cₚₕ⁴), 129.9 (s, Cₘ), 131.4 (s, C₀³), 132.1 (s, Cₚₕ³), 133.6 (2 s, C₀⁴), 133.7 (s, C₀⁴), 134.0 (s, Cₚₕ²), 136.0 (s, Cₚ), 148.3 (s, N-CH=C), 148.7 (m, Cᵢ), 154.6 (s, C₀¹), 154.7 (s, C₀¹), 165.4 (s, HN-CO-CH₂-N), 168.4 (s, Cₚₕ¹), 190.4 (s, CHO), 190.5 (s, CHO), 190.6 (s, CHO) ppm. FAB-MS: *m*/*z* = 1187 [M+H]⁺.

### 4.8. Synthesis of chlorinated dendrimer with a protected amine terminated spacer

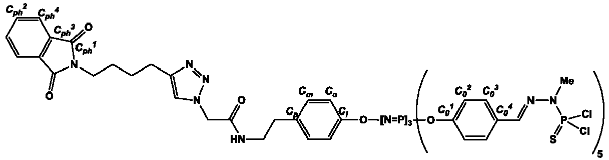

To an ice-cooled solution of N-methyldichlorothiophosphorhydrazide **2** (0.969 mmol) in chloroform (6.4 mL) was added the compound obtained in **example 4.7** (200 mg, 0.169 mmol) and the mixture was stirred at rt for 2 h. After the evaporation of the solvent, the residue was diluted in the minimum of chloroform and precipitated by the addition of a large amount of pentane. This purification step was repeated twice to give dendrimer the title compound as a white solid (yield = 80 %). ³¹P{¹H} NMR (CDCl₃, 121.5 MHz): δ = 8.3 (broad s, N₃P₃), 62.4 (2 s, P=S), 62.5 (s, P=S); ¹H NMR (CDCl₃, 300.1 MHz): δ = 1.76 (broad s, 4H, CH₂-CH₂-CH₂-CH₂-N, CH₂-CH₂-CH₂-CH₂-N), 2.77 (m, 4H, C₆H₄-CH₂-CH₂-NH, CH₂-CH₂-CH₂-CH₂-N), 3.43 (m, 2H, C₆H₄-CH₂-CH₂-NH), 3.49 (d, ³*J*_{HP} = 14.0 Hz, 9H, N-CH₃), 3.50 (d, ³*J*_{HP} = 14.0 Hz, 6H, N-CH₃), 3.71 (t, ³*J*_{HH} = 6.3 Hz, 2H, CH₂-CH₂-CH₂-CH₂-N), 5.00 (s, 2H, CO-CH₂-N), 6.41 (broad s, 1H, NH), 6.90 (d, ³*J*_{HH} = 8.5 Hz, 2H, Cₒ-H), 6.98 (m, 7H, Cₘ-H, C₀²-H), 7.04 (m, 5H, C₀²-H), 7.49 (s, 1H, N-CH=C), 7.61 (m, 13H, C₀³-H, CH=N), 7.70 (m, 4H, CH=N, Cₚₕ-H), 7.82 (m, 2H, Cₚₕ-H); ¹³C{¹H} NMR (CDCl₃, 75.5 MHz): δ = 24.7 (s, CH₂-CH₂-CH₂-CH₂-N), 26.3 (s, CH₂-CH₂-CH₂-CH₂-N), 27.9 (s, CH₂-CH₂-CH₂-CH₂-N), 32.0 (2 d, ²*J*_{CP} = 12.9 Hz, N-CH₃), 34.8 (s, C₆H₄-CH₂-CH₂-NH), 37.5 (s, CH₂-CH₂-CH₂-CH₂-N), 41.0 (s, C₆H₄-CH₂-CH₂-NH), 53.2 (s, CO-CH₂-N), 121.1 (s, Cₒ), 121.4 (s, C₀²), 123.1 (s, N-CH=C), 123.2 (s, Cₚₕ⁴), 128.6 (s, C₀³), 129.8 (s, Cₘ), 131.3 (s, Cₚₕ³), 132.0 (s, C₀⁴), 134.0 (s, Cₚₕ²), 135.3 (s, Cₚ), 140.7 (m, CH=N), 147.8 (s, N-CH=C), 148.9 (m, Cᵢ), 151.7 (broad s, C₀¹), 165.0 (s, HN-CO-CH₂-N), 168.4 (s, Cₚₕ¹) ppm.

### 4.9. Synthesis of dimethylphosphonate terminated dendrimer with a protected amine terminated spacer

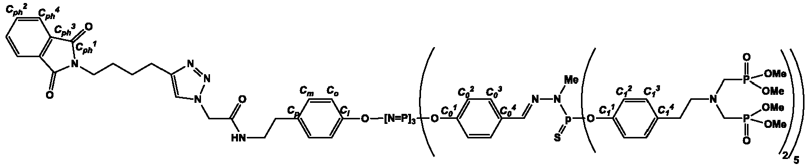

To a solution of dendrimer obtained in **example 4.7** (316 mg, 0.159 mmol) in acetone (17 mL) were added phenol obtained in **example 1.1** (666 mg, 1.747 mmol) and caesium carbonate (569 mg, 1.747 mmol) and the mixture was stirred at rt for 12 h. The reaction mixture was centrifugated, filtered and evaporated. The resulting crude oil was eluted on a plug of silica with acetone to remove the unreacted phenol then with acetone/methanol/water mixture (7:2:1). The resulting dendrimer solution was concentrated to dryness under reduced pressure, dissolved in 10 mL of dichloromethane, dried over sodium sulfate, filtered (micropore, 0.2 µm) and finally evaporated to dryness under reduced pressure to afford the title compound as a colourless oil (yield = 85 %) ³¹P{¹H} NMR (CDCl₃, 121.5 MHz): δ = 8.4 (s, N₃P₃), 26.7 (2 s, PO₃Me₂), 26.8 (2 s, PO₃Me₂), 63.1 (2 s, P=S), 63.2 (s, P=S); ¹H NMR (CDCl₃, 300.1 MHz): δ = 1.70 (broad s, 4H, CH₂-CH₂-CH₂-CH₂-N, CH₂-CH₂-CH₂-CH₂-N), 2.69 (m, 24H, C₆H₄-CH₂-CH₂-N, C₆H₄-CH₂-CH₂-NH, CH₂-CH₂-CH₂-CH₂-N), 3.03 (m, 20H, C₆H₄-CH₂-CH₂-N), 3.16 (d, ²*J*_{HP} = 9.3 Hz, 40H, N-CH₂-P), 3.26 (m, 17H, N-CH₃, C₆H₄-CH₂-CH₂-NH), 3.71 (broad d, ³*J*_{HP} = 10.4 Hz, 122H, P(O)(OCH₃), CH₂-CH₂-CH₂-CH₂-N), 4.87 (s, 2H, CO-CH₂-N), 6.84 (d, ³*J*_{HH} = 8.3 Hz, 2H, Cₒ-H), 6.97 (m, 7H, Cₘ-H, C₀²-H), 7.06 (m, 15H, C₀²-H, C₁²-H), 7.15 (d, ³*J*_{HH} = 8.3 Hz, 20H, C₁³-H), 7.41 (s, 1H, N-CH=C), 7.28 (m, 17H, C₀³-H, CH=N, Cₚₕ-H), 7.79 (m, 2H, Cₚₕ-H); ¹³C{¹H} NMR (CDCl₃, 75.5 MHz): δ = 25.1 (s, CH₂-CH₂-CH₂-CH₂-N), 26.6 (s, CH₂-CH₂-CH₂-CH₂-N), 28.1 (s, CH₂-CH₂-CH₂-CH₂-N), 32.9 (m, C₆H₄-CH₂-CH₂-N, N-CH₃), 34.7 (s, C₆H₄-CH₂-CH₂-NH), 37.6 (s, CH₂-CH₂-CH₂-CH₂-N), 40.9 (s, C₆H₄-CH₂-CH₂-NH), 49.5 (dd, ¹*J*_{CP} = 157.5 Hz, ³*J*_{CP} = 7.3 Hz, N-CH₂-P), 52.4 (s, CO-CH₂-N), 52.6 (m, P(O)(OCH₃)), 58.1 (t, ³*J*_{CP} = 7.6 Hz, C₆H₄-CH₂-CH₂-N), 121.0 (s, Cₒ), 121.2 (broad d, ³*J*_{CP} = 4.2 Hz, C₁², C₀²), 122.4 (s, N-CH=C), 123.2 (s, Cₚₕ⁴), 128.2 (s, C₀³), 128.3 (s, C₀³), 129.6 (s, Cₘ), 129.9 (s, C₁³), 132.1 (s, C₀⁴ or Cₚₕ³), 132.2 (s, C₀⁴ or Cₚₕ³), 133.9 (s, Cₚₕ²), 135.6 (s, Cₚ), 136.6 (s, C₁⁴), 138.7 (m, CH=N), 147.8 (s, N-CH=C), 148.9 (broad d, ²*J*_{CP} = 7.1 Hz, C₁¹, Cᵢ), 151.2 (m, C₀¹), 165.4 (s, HN-CO-CH₂-N), 168.3 (s, Cₚₕ¹) ppm.

### 4.10. Synthesis of persubstituted cyclotriphosphazene derivative with a protected acid terminated spacer

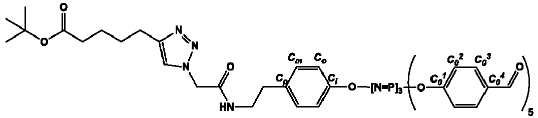

To a mixture of the compound obtained in **example 4.5** (140 mg, 0.348 mmol) and compound of **example 4.6** (270 mg, 0.348 mmol) in THF (5 mL) was added caesium carbonate (113 mg, 0.348 mmol), and the mixture was stirred at room temperature for 12 h. The reaction mixture was centrifugated, filtered and evaporated. The residue was purified by column flash chromatography (silica, pentane/ethyl acetate, 1:1) to give the title compound as a colourless viscous oil (yield = 87 %). ³¹P{¹H} NMR (CDCl₃, 101.3 MHz): δ = 7.4 (s, N₃P₃); ¹H NMR (CDCl₃, 200.1 MHz): δ = 1.41 (s, 9H, CH₃), 1.65 (m, 4H, CH₂-CH₂-CH₂-CH₂-CO, CH₂-CH₂-CH₂-CH₂-CO), 2.22 (t, ³*J*_{HH} = 6.8 Hz, 2H, CH₂-CH₂-CH₂-CH₂-CO), 2.72 (broad t, ³*J*_{HH} = 7.0 Hz, 4H, C₆H₄-CH₂-CH₂-NH, CH₂-CH₂-CH₂-CH₂-CO), 3.47 (q, ³*J*_{HH} = 6.8 Hz, 2H, C₆H₄-CH₂-CH₂-NH), 4.97 (s, 2H, CO-CH₂-N), 6.37 (t, ³*J*_{HH} = 5.4 Hz, 1H, NH), 6.91 (m, 4H, Cₒ-H, Cₘ-H), 7.11 (m, 10H, C₀²-H), 7.42 (s, 1H, N-CH=C), 7.70 (m, 10H, C₀³-H), 9.92 (s, 5H, CHO); ¹³C{¹H} NMR (CDCl₃, 62.9 MHz): δ = 24.5(s, CH₂-CH₂-CH₂-CH₂-CO), 25.3 (s, CH₂-CH₂-CH₂-CH₂-CO), 28.1 (s, CH₃), 28.6 (s, CH₂-CH₂-CH₂-CH₂-CO), 34.7 (s, C₆H₄-CH₂-CH₂-NH), 35.1 (s, CH₂-CH₂-CH₂-CH₂-CO), 40.8 (s, C₆H₄-CH₂-CH₂-NH), 53.0 (s, CO-CH₂-N), 80.2 (s, C(CH₃)), 120.7 (s, Cₒ), 121.3 (broad s, C₀²), 122.5 (s, N-CH=C), 129.9 (s, Cₘ), 130.9 (s, C₀³), 133.7 (2 s, C₀⁴), 135.9 (s, Cₚ), 148.7 (broad s, N-CH=C, Cᵢ), 154.6 (s, C₀¹), 165.4 (s, HN-CO-CH₂), 172.9 (s, CO₂), 190.4 (s, CHO), 190.5 (s, CHO), 190.6 (s, CHO) ppm.

### 4.11. Synthesis of fluorescent dendrimer with a PSCl₂ termination

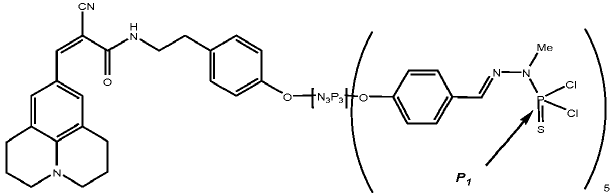

100 mg of the compound obtained in Stage 5 (0.05 mmol) is added at 0°C to a solution of dichlorothiophospho-(N-methyl)-hydrazide (0.3 mmol) in chloroform (1.5 mL). The reaction mixture is stirred for 12 hours. After evaporation of the reaction solvent, the product is diluted in a minimum amount of dichloromethane and precipitated by addition of a large volume of pentane. This treatment is carried out three times. The product is isolated with a yield of 90 %.
NMR ³¹P{¹H} (CDCl₃, 81.02 MHz): δ = 11.8 (bs, N₃P₃); 65.9 (s, P₁); 66.0 (s, P₁); 66.1 (s, P₁) ppm.

### 4.12. Synthesis of chlorinated dendrimer with a protected acid terminated spacer

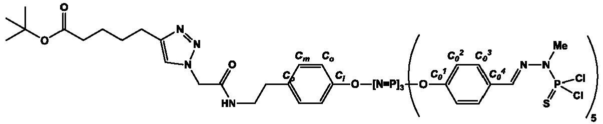

To an ice-cooled solution of N-methyldichlorothiophosphorhydrazide **of example 4.11** (0.655 mmol) in chloroform (4.3 mL) was added the compound of **example 4.10** (130 mg, 0.114 mmol) and the mixture was stirred at room temperature for 2h. After the evaporation of the solvent, the residue was diluted in the minimum of chloroform and precipitated by the addition of a large amount of pentane. This purification step was repeated twice to give the title compound as a white solid (yield = 85 %). ³¹P{¹H} NMR (CDCl₃, 121.5 MHz): δ = 8.3 (broad s, N₃P₃), 62.4 (2 s, P=S), 62.5 (s, P=S); ¹H NMR (CDCl₃, 300.1 MHz): δ = 1.45 (s, 9H, C(CH₃)), 1.71 (m, 4H, CH₂-CH₂-CH₂-CH₂-CO, CH₂-CH₂-CH₂-CH₂-CO), 2.27 (t, ³*J*_{HH} = 7.1 Hz, 2H, CH₂-CH₂-CH₂-CH₂-CO), 2.79 (m, 4H, C₆H₄-CH₂-CH₂-NH, CH₂-CH₂-CH₂-CH₂-CO), 3.45 (broad s, 2H, C₆H₄-CH₂-CH₂-NH), 3.50 (d, ³*J*_{HP} = 14.0 Hz, 9H, N-CH₃), 3.51 (d, ³*J*_{HP} = 14.0 Hz, 6H, N-CH₃), 5.07 (broad s, 2H, CO-CH₂-N), 6.68 (broad s, 1H, NH), 6.90 (d, ³*J*_{HH} = 8.0 Hz, 2H, Cₒ-H), 6.99 (broad d, ³*J*_{HH} = 8.6 Hz, 6H, Cₘ-H, C₀²-H), 7.05 (d, ³*J*_{HH} = 8.6 Hz, 6H, C₀²-H), 7.47 (s, 1H, N-CH=C), 7.60 (d, 10H, C₀³-H), 7.64 (broad s, 3H, CH=N), 7.68 (broad s, 2H, CH=N); ¹³C{¹H} NMR (CDCl₃, 75.5 MHz): δ = 24.4 (s, CH₂-CH₂-CH₂-CH₂-CO), 24.9 (s, CH₂-CH₂-CH₂-CH₂-CO), 28.1 (s, C(CH₃)), 28.4 (s, CH₂-CH₂-CH₂-CH₂-CO), 32.0 (d, ³*J*_{CP} = 12.9 Hz, N-CH₃), 32.1 (d, ³*J*_{CP} = 12.9 Hz, N-CH₃), 34.8 (s, C₆H₄-CH₂-CH₂-NH), 35.1 (s, CH₂-CH₂-CH₂-CH₂-CO), 41.1 (s, C₆H₄-CH₂-CH₂-NH), 53.4 (s, CO-CH₂-N), 80.3 (s, C(CH₃)), 121.1 (s, Cₒ), 121.4 (broad s, C₀²), 123.5 (s, N-CH=C), 128.6 (s, C₀³), 129.7 (s, Cₘ), 131.3 (broad s, C₀⁴), 135.3 (s, Cₚ), 140.7 (m, CH=N), 147.7 (s, N-CH=C), 148.9 (m, Cᵢ), 151.7 (m, C₀¹), 164.7 (s, HN-CO-CH₂), 172.8 (s, CO₂) ppm.

### 4.13 Synthesis of dimethylphosphonate terminated dendrimer with a protected acid terminated spacer

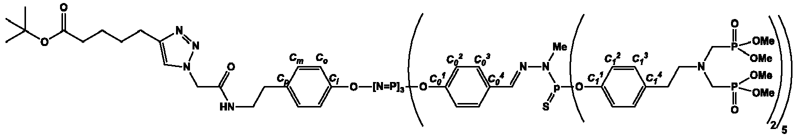

To a solution of compound obtained in **example 4.12** (315 mg, 0.162 mmol) in acetone (17 mL) were added phenol **of example 1.1** (680 mg, 1.783 mmol) and caesium carbonate (581 mg, 1.783 mmol) and the mixture was stirred at room temperature for 12 h. The reaction mixture was centrifugated, filtered and evaporated. The resulting crude oil was eluted on a plug of silica with acetone to remove the unreacted phenol then with acetone/methanol/water mixture (7:2:1). The resulting dendrimer solution was concentrated to dryness under reduced pressure, dissolved in 10 mL of dichloromethane, dried over sodium sulfate, filtered (micropore, 0.2 µm) and finally evaporated to dryness under reduced pressure to afford the tilte compound as a sticky solid (yield = 90 %) ³¹P{¹H} NMR (CDCl₃, 121.5 MHz): δ = 8.4 (broad s, N₃P₃), 26.7 (2 s, PO₃Me₂), 26.8 (s, PO₃Me₂), 63.0 (s, P=S), 63.1 (s, P=S); ¹H NMR (CDCl₃, 300.1 MHz): δ = 1.38 (s, 9H, C(CH₃)), 1.62 (m, 4H, CH₂-CH₂-CH₂-CH₂-CO, CH₂-CH₂-CH₂-CH₂-CO), 2.19 (t, ³*J*_{HH} = 7.1 Hz, 2H, CH₂-CH₂-CH₂-CH₂-CO), 2.70 (m, 24H, C₆H₄-CH₂-CH₂-NH, CH₂-CH₂-CH₂-CH₂-CO, C₆H₄-CH₂-CH₂-N), 2.99 (m, 20H, C₆H₄-CH₂-CH₂-N), 3.13 (d, ²*J*_{HP} = 9.3 Hz, 40H, N-CH₂-P), 3.23 (m, 17H, N-CH₃, C₆H₄-CH₂-CH₂-NH), 3.67 (d, ³*J*_{HP} = 10.5 Hz, 120H, P(O)(OCH₃)), 4.85 (broad s, 2H, CO-CH₂-N), 6.81 (d, ³*J*_{HH} = 8.3 Hz, 2H, Cₒ-H), 6.99 (m, 52H, Cₘ-H, C₀²-H, C₁²-H, C₁³-H), 7.39 (s, 1H, N-CH=C), 7.58 (m, 15H, C₀³-H, CH=N); ¹³C{¹H} NMR (CDCl₃, 75.5 MHz): δ = 24.5 (s, CH₂-CH₂-CH₂-CH₂-CO), 25.3 (s, CH₂-CH₂-CH₂-CH₂-CO), 28.1 (s, C(CH₃)), 28.6 (s, CH₂-CH₂-CH₂-CH₂-CO), 32.9 (broad s, C₆H₄-CH₂-CH₂-N, N-CH₃), 34.6 (s, C₆H₄-CH₂-CH₂-NH), 35.1 (s, CH₂-CH₂-CH₂-CH₂-CO), 40.9 (s, C₆H₄-CH₂-CH₂-NH), 49.4 (dd, ¹*J*_{CP} = 157.6 Hz, ³*J*_{CP} = 7.2 Hz, N-CH₂-P), 52.7 (m, P(O)(OCH₃)), 53.4 (s, CO-CH₂-N), 58.1 (t, ³*J*_{CP} = 7.5 Hz, C₆H₄-CH₂-CH₂-N), 80.1 (s, C(CH₃)), 121.0 (s, Cₒ), 121.2 (broad d, ³*J*_{CP} = 4.1 Hz, C₁², C₀²), 122.4 (s, N-CH=C), 128.3 (s, C₀³), 129.6 (s, Cₘ), 129.9 (s, C₁³), 132.1 (d, ⁴*J*_{CP} = 5.3 Hz, C₀⁴), 135.6 (s, Cₚ), 136.5 (s, C₁⁴), 138.7 (d, ³*J*_{CP} = 13.9 Hz, CH=N), 147.9 (s, N-CH=C), 148.9 (broad d, ²*J*_{CP} = 6.9 Hz, C₁¹, Cᵢ), 151.2 (m, C₀¹), 165.4 (s, HN-CO-CH₂), 172.9 (s, CO₂) ppm.

### 4.14. Synthesis of dimethylphosphonate terminated dendrimer with a protected acid terminated spacer

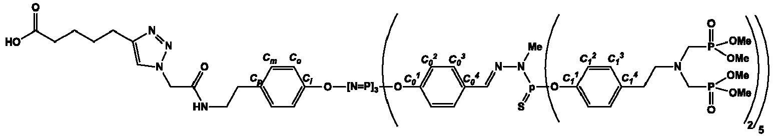

Compound of **example 4.13** (107 mg, 0.020 mmol) was dissolved in a solution of 30 % of TFA in dichloromethane, and the reaction mixture was allowed to stir at rt for 1.5 h and evaporated to dryness. This sequence was repeated 6 times and the residue was suspended into ethyl acetate so that remaining traces of TFA were removed upon evaporation to dryness. The residue was purified by column chromatography (silica, dichloromethane/methanol, 85:15) to give title compound as a sticky solid (yield = 85 %). ³¹P{¹H} NMR (CDCl₃, 121.5 MHz): δ = 8.4 (broad s, N₃P₃), 26.8 (s, PO₃Me₂), 63.1 (s, P=S), 63.2 (s, P=S); ¹H NMR (CDCl₃, 300.1 MHz): δ = 1.63 (m, 4H, CH₂-CH₂-CH₂-CH₂-CO, CH₂-CH₂-CH₂-CH₂-CO), 2.25 (m, 2H, CH₂-CH₂-CH₂-CH₂-CO), 2.73 (m, 24H, C₆H₄-CH₂-CH₂-NH, CH₂-CH₂-CH₂-CH₂-CO, C₆H₄-CH₂-CH₂-N), 3.03 (m, 20H, C₆H₄-CH₂-CH₂-N), 3.17 (d, ²*J*_{HP} = 9.3 Hz, 40H, N-CH₂-P), 3.26 (m, 17H, N-CH₃, C₆H₄-CH₂-CH₂-NH), 3.70 (d, ³*J*_{HP} = 10.5 Hz, 120H, P(O)(OCH₃)), 4.82 (broad s, 2H, CO-CH₂-N), 6.88 (d, ³*J*_{HH} = 8.3 Hz, 2H, Cₒ-H), 7.08 (m, 52H, Cₘ-H, C₀²-H, C₁²-H, C₁³-H), 7.30 (s, 1H, N-CH=C), 7.61 (m, 15H, C₀³-H, CH=N); ¹³C{¹H} NMR (CDCl₃, 75.5 MHz): δ = 24.3 (s, CH₂-CH₂-CH₂-CH₂-CO), 25.2 (s, CH₂-CH₂-CH₂-CH₂-CO), 28.5 (s, CH₂-CH₂-CH₂-CH₂-CO), 32.9 (broad s, C₆H₄-CH₂-CH₂-N, N-CH₃), 33.6 (s, CH₂-CH₂-CH₂-CH₂-CO), 34.5 (s, C₆H₄-CH₂-CH₂-NH), 40.7 (s, C₆H₄-CH₂-CH₂-NH), 49.4 (dd, ¹*J*_{CP} = 157.9 Hz, ³*J*_{CP} = 7.1 Hz, N-CH₂-P), 52.7 (broad s, P(O)(OCH₃)), 53.4 (s, CO-CH₂-N), 58.1 (t, ³*J*_{CP} = 7.4 Hz, C₆H₄-CH₂-CH₂-N), 120.4 (s, Cₒ), 121.2 (broad d, ³*J*_{CP} = 4.1 Hz, C₁², C₀²), 122.5 (s, N-CH=C), 128.3 (s, C₀³), 129.7 (s, Cₘ), 129.9 (s, C₁³), 132.2 (d, ⁴*J*_{CP} = 5.5 Hz, C₀⁴), 135.6 (s, Cₚ), 136.5 (s, C₁⁴), 138.8 (m, CH=N), 147.9 (s, N-CH=C), 148.9 (broad d, ²*J*_{CP} = 7.4 Hz, C₁¹, Cᵢ), 151.4 (broad s, C₀¹), 165.3 (s, HN-CO-CH₂), 175.1 (s, CO₂) ppm.

### 4.15. Synthesis of amine terminated spacer

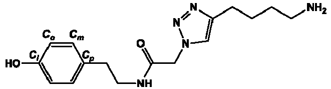

To a solution of 2-{4-[4-(1,3-Dioxo-1,3-dihydro-isoindol-2-yl)-butyl]-[1,2,3]triazol-1-yl}-N-[2-(4-hydroxy-phenyl)-ethyl]-acetamide (200 mg, 0.047 mmol) in ethanol (5 mL) was added hydrazine hydrate (0.070 mL, 2.23 mmol) and the reaction mixture was refluxed for 3 h. The mixture was evaporated to dryness to remove solvent and excess of hydrazine, resulting in a residue made of the title compound and by-product phthalhydrazide. The crude product was used without further purification. ¹H NMR (CD₃OD, 200.3 MHz): δ = 1.70 (m, 4H, CH₂-CH₂-CH₂-CH₂-N, CH₂-CH₂-CH₂-CH₂-N), 2.72 (m, 4H, C₆H₄-CH₂-CH₂-NH, CH₂-CH₂-CH₂-CH₂-N), 2.86 (m, 2H, C₆H₄-CH₂-CH₂-NH), 3.41 (m, 2H, CH₂-CH₂-CH₂-CH₂-N), 5.07 (s, 2H, CO-CH₂-N), 6.72 (d, ³*J*_{HH} = 8.5 Hz, 2H, Cₒ-H), 7.02 (d, ³*J*_{HH} = 8.5 Hz, 2H, Cₘ-H), 7.68 (s, 1H, N-CH=C) ppm.

### 4.16 Synthesis of the biotinylated spacer

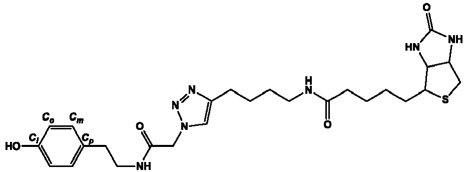

To a solution of biotin (65 mg, 0.265 mmol) and DIPEA (0.046 mL, 0.265 mmol) in DMF (2 mL) were added a solution of TBTU (89 mg, 0.277 mmol) in DMF (3 mL) and a solution of the compound of **example 4.15** (80 mg, 0.252 mmol) in DMF (5 mL). The reaction mixture was stirred at room temperature for 24 h. The solvent was evaporated to dryness and the residue was purified by column chromatography (silica, dichloromethane/methanol, 9:1) to give title compound as a colourless oil (yield = 70 %). ¹H NMR (CD₃OD, 500.3 MHz): δ = 1.45 (m, 2H, CO-CH₂-CH₂-CH₂-CH₂), 1.67 (m, 8H, CO-CH₂-CH₂-CH₂-CH₂, CO-CH₂-CH₂-CH₂-CH₂, CH₂-CH₂-CH₂-CH₂-NH, CH₂-CH₂-CH₂-CH₂-NH), 2.21 (t, ³*J*_{HH} = 7.3 Hz, 2H, CO-CH₂-CH₂-CH₂-CH₂), 2.74 (m, 5H, C₆H₄-CH₂-CH₂-NH, CH₂-CH₂-CH₂-CH₂-NH, CH₂-S), 2.93 (dd, ²*J*_{HH} = 5.0 Hz, ³*J*_{HH} = 12.7 Hz, 1H, CH₂-S), 3.22 (m, 3H, CH₂-CH₂-CH₂-CH₂-NH, CH-S), 3.43 (t, ³*J*_{HH} = 7.2 Hz, 2H, C₆H₄-CH₂-CH₂-NH), 4.30 (m, 1H, CH-CH-NH), 4.50 (m, 1H, CH₂-CH-NH), 5.06 (s, 2H, CO-CH₂-N), 6.72 (m, 2H, Cₒ-H), 7.03 (broad d, ³*J*_{HH} = 8.4 Hz, 2H, Cₘ-H), 7.69 (s, 1H, N-CH=C); ¹³C{¹H} NMR (CD₃OD, 125.8 MHz): δ = 24.5 (s, CH₂-CH₂-CH₂-CH₂-NH), 25.5 (s, CO-CH₂-CH₂-CH₂-CH₂), 26.3 (s, CH₂-CH₂-CH₂-CH₂-NH), 28.1 (s, CO-CH₂-CH₂-CH₂-CH₂), 28.4 (2 s, CO-CH₂-CH₂-CH₂-CH₂, CH₂-CH₂-CH₂-CH₂-NH), 34.1 (s, C₆H₄-CH₂-CH₂-NH), 35.4 (s, CO-CH₂-CH₂-CH₂-CH₂), 38.6 (s, CH₂-CH₂-CH₂-CH₂-NH), 39.6 (s, CH₂-S), 41.0 (s, C₆H₄-CH₂-CH₂-NH), 51.8 (s, CO-CH₂-N), 55.6 (s, CH-S), 60.2 (s, CH₂-CH-NH), 61.9 (s, CH-CH-NH), 114.9 (s, Cₒ), 123.3 (s, N-CH=C), 129.4 (s, Cₘ), 129.5 (s, Cₚ), 147.5 (N-CH=C), 155.6 (s, Cᵢ), 164.7 (s, HN-CO-NH), 166.4 (s, HN-CO-CH₂-N), 174.6 (s, (CH₂)₄-NH-CO) ppm.

### 4.17. Synthesis of persubstituted cyclotriphosphazene derivative with a biotinylated spacer

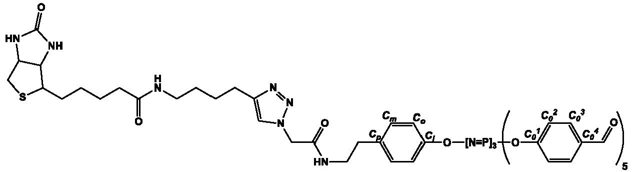

To a mixture of the compound of **example 4.16** (65 mg, 0.120 mmol) and compound of **example 4.6** (93 mg, 0.120 mmol) in DMF (5 mL) was added caesium carbonate (39 mg, 0.120 mmol), and the mixture was stirred at room temperature for 12 h. The reaction mixture was centrifugated, filtered and evaporated. The residue was purified by column flash chromatography (silica, dichloromethane/methanol, 9:1) to give title compound as a white oil (yield = 85 %). ³¹P{¹H} NMR (CDCl₃, 81.0 MHz): δ = 8.4 (s, N₃P₃); ¹H NMR (CDCl₃, 300.1 MHz): δ = 1.42 (broad s, 2H, CO-CH₂-CH₂-CH₂-CH₂), 1.72 (m, 8H, CO-CH₂-CH₂-CH₂-CH₂, CO-CH₂-CH₂-CH₂-CH₂, CH₂-CH₂-CH₂-CH₂-NH, CH₂-CH₂-CH₂-CH₂-NH), 2.24 (broad s, 2H, CO-CH₂-CH₂-CH₂-CH₂), 2.72 (m, 5H, C₆H₄-CH₂-CH₂-NH, CH₂-CH₂-CH₂-CH₂-NH, CH₂-S), 2.88 (m, 1H, CH₂-S), 3.18 (m, 3H, CH₂-CH₂-CH₂-CH₂-NH, CH-S), 3.43 (broad s, 2H, C₆H₄-CH₂-CH₂-NH), 4.32 (broad s, 1H, CH-CH-NH), 4.49 (broad s, 1H, CH₂-CH-NH), 5.03 (broad s, 2H, CO-CH₂-N), 6.53 (broad s, 1H, CH-CH-NH-CO or CH₂-CH-NH-CO), 6.73 (broad s, 1H, CH₂-CH-NH-CO), 6.88 (broad s, 2H, Cₒ-H), 7.00 (broad s, 2H, Cₘ-H), 7.12 (m, 10H, C₀²-H), 7.29 (m, 1H, (CH₂)₄-NH-CO), 7.51 (broad s, 1H, NH-CO-CH₂-N), 7.55 (broad s, 1H, N-CH=C), 7.72 (m, 10H, C₀³-H), 9.93 (2 s, 5H, CHO); ¹³C{¹H} NMR (CDCl₃, 75.5 MHz): δ = 24.9 (s, CH₂-CH₂-CH₂-CH₂-NH), 25.7 (s, CO-CH₂-CH₂-CH₂-CH₂), 26.4 (s, CH₂-CH₂-CH₂-CH₂-NH), 28.1 (s, CO-CH₂-CH₂-CH₂-CH₂), 28.5 (2 s, CO-CH₂-CH₂-CH₂-CH₂, CH₂-CH₂-CH₂-CH₂-NH), 34.6 (s, C₆H₄-CH₂-CH₂-NH), 35.9 (s, CO-CH₂-CH₂-CH₂-CH₂), 39.0 (s, CH₂-CH₂-CH₂-CH₂-NH), 40.6 (s, CH₂-S), 41.0 (s, C₆H₄-CH₂-CH₂-NH), 52.8 (s, CO-CH₂-N), 55.7 (s, CH-S), 60.2 (s, CH₂-CH-NH), 61.9 (s, CH-CH-NH), 120.7 (s, Cₒ), 121.3 (s, C₀²), 123.0 (s, N-CH=C), 129.9 (s, Cₘ), 133.4 (s, C₀³),133.6 (s, C₀⁴), 133.7 (s, C₀⁴), 136.3 (s, Cₚ), 148.0 (s, N-CH=C)), 148.5 (s, Cᵢ), 154.6 (broad s, C₀¹), 164.0 (s, HN-CO-NH), 165.7 (s, HN-CO-CH₂-N), 173.4 (s, (CH₂)₄-NH-CO), 190.5 (s, CHO), 190.6 (s, CHO), 190.8 (s, CHO) ppm.

### 4.18. Synthesis of chlorinated dendrimer with a biotinylated spacer

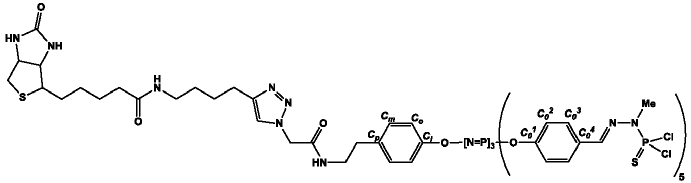

To an ice-cooled solution of N-methyldichlorothiophosphorhydrazide **of example 4.11** (0.355 mmol) in chloroform (2.3 mL) was added the compound of **example 4.17** (70 mg, 0.055 mmol) and the mixture was stirred at room temperature for 2 h. After the evaporation of the solvent, the residue was diluted in the minimum of chloroform and precipitated by the addition of a large amount of pentane. This purification step was repeated twice to give the title compound as a white solid (yield = 90 %). ³¹P{¹H} NMR (CDCl₃, 101.3 MHz): δ = 8.3 (broad s, N₃P₃), 62.3 (s, P=S), 62.6 (s, P=S); ¹H NMR (CDCl₃, 300.1 MHz): δ = 1.42 (m, 2H, CO-CH₂-CH₂-CH₂-CH₂), 1.70 (m, 8H, CO-CH₂-CH₂-CH₂-CH₂, CO-CH₂-CH₂-CH₂-CH₂, CH₂-CH₂-CH₂-CH₂-NH, CH₂-CH₂-CH₂-CH₂-NH), 2.28 (m, 2H, CO-CH₂-CH₂-CH₂-CH₂), 2.83 (broad s, 6H, C₆H₄-CH₂-CH₂-NH, CH₂-CH₂-CH₂-CH₂-NH, CH₂-S), 3.12 (m, 3H, CH₂-CH₂-CH₂-CH₂-NH, CH-S), 3.48 (broad d, ³*J*_{HP} = 13.8 Hz, 17H, N-CH₃, C₆H₄-CH₂-CH₂-NH), 4.34 (broad s, 1H, CH-CH-NH), 4.52 (broad s, 1H, CH₂-CH-NH), 5.31 (broad s, 2H, CO-CH₂-N), 6.95 (m, 16H, Cₒ-H, Cₘ-H, C₀²-H, CH-CH-NH-CO, CH₂-CH-NH-CO), 7.63 (m, 17H, N-CH=C, C₀³-H, CH=N), 7.96 (s, 1H, (CH₂)₄-NH-CO), 8.28 (s, 1H, NH-CO-CH₂-N); ¹³C{¹H} NMR (CDCl₃, 75.5 MHz): δ = 24.1 (s, CH₂-CH₂-CH₂-CH₂-NH), 25.9 (broad s, CO-CH₂-CH₂-CH₂-CH₂, CH₂-CH₂-CH₂-CH₂-NH), 28.0 (s, CO-CH₂-CH₂-CH₂-CH₂), 28.3 (broad s, CO-CH₂-CH₂-CH₂-CH₂, CH₂-CH₂-CH₂-CH₂-NH), 32.0 (d, ²*J*_{CP} = 12.8 Hz, N-CH₃), 32.1 (d, ²*J*_{CP} = 12.8 Hz, N-CH₃), 34.7 (s, C₆H₄-CH₂-CH₂-NH), 35.4 (s, CO-CH₂-CH₂-CH₂-CH₂), 39.1 (s, CH₂-CH₂-CH₂-CH₂-NH), 40.7 (s, CH₂-S), 41.2 (s, C₆H₄-CH₂-CH₂-NH), 53.3 (s, CO-CH₂-N), 55.7 (s, CH-S), 60.5 (s, CH₂-CH-NH), 61.9 (s, CH-CH-NH), 121.0 (s, Cₒ), 121.3 (s, C₀²), 123.0 (s, N-CH=C), 128.9 (s, C₀³), 129.8 (s, Cₘ), 131.3 (s, C₀⁴), 135.8 (s, Cₚ), 140.9 (m, CH=N), 145.7 (N-CH=C), 148.7 (broad s, Cᵢ), 151.7 (s, C₀¹), 164.2 (s, HN-CO-NH), 164.6 (HN-CO-CH₂-N), 174.6 (s, (CH₂)₄-NH-CO) ppm.

### 4. 19. Synthesis of dimethylphosphonate terminated dendrimer with a biotinylated spacer

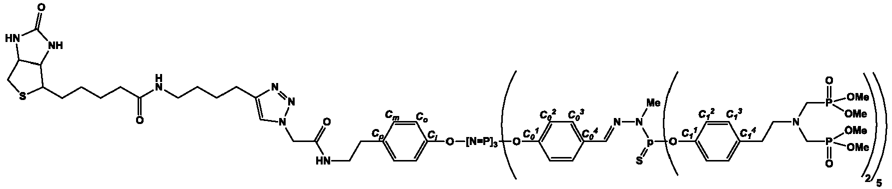

To a solution of the compound of **example 4.18** (48 mg, 0.023 mmol) in acetone (5 mL) were added phenol **of example 1.1** (92 mg, 0.241 mmol) and caesium carbonate (79 mg, 0.241 mmol) and the mixture was stirred at room temperature for 12 h. The reaction mixture was centrifugated, filtered and evaporated. The resulting crude oil was eluted on a plug of silica with acetone to remove the unreacted phenol then with acetone/methanol/water mixture (7:2:1). The resulting dendrimer solution was concentrated to dryness under reduced pressure, dissolved in 10 mL of dichloromethane, dried over sodium sulfate, filtered (micropore, 0.2 µm) and finally evaporated to dryness under reduced pressure to afford title compound as a colourless oil (yield = 90 %). ³¹P{¹H} NMR (acetone-*d6*, 202.5 MHz): δ = 8.9 (s, N₃P₃), 26.3 (2 s, PO₃Me₂), 26.4 (2 s, PO₃Me₂), 62.8 (2 s, P=S); ¹H NMR (acetone-*d6*, 500.3 MHz): δ = 1.42 (m, 2H, CO-CH₂-CH₂-CH₂-CH₂), 1.53 (m, 2H, CH₂-CH₂-CH₂-CH₂-NH), 1.62 (m, 2H, CO-CH₂-CH₂-CH₂-CH₂), 1.67 (m, 2H, CH₂-CH₂-CH₂-CH₂-NH), 1.74 (m, 2H, CO-CH₂-CH₂-CH₂-CH₂), 2.16 (broad t, ³*J*_{HH} = 7.2 Hz, 2H, CO-CH₂-CH₂-CH₂-CH₂), 2.67 (m, 3H, CH₂-CH₂-CH₂-CH₂-NH, CH₂-S), 2.75 (m, 2H, C₆H₄-CH₂-CH₂-NH), 2.80 (m, 20H, C₆H₄-CH₂-CH₂-N), 2.89 (m, 1H, CH₂-S), 3.06 (m, 20H, C₆H₄-CH₂-CH₂-N), 3.20 (2 broad d, ²*J*_{HP} = 12.7 Hz, 43H, N-CH₂-P, CH₂-CH₂-CH₂-CH₂-NH, CH-S), 3.48 (m, 17H, N-CH₃, C₆H₄-CH₂-CH₂-NH), 3.69 (2 d, 120H, ³*J*_{HP} = 10.5 Hz, P(O)(OCH₃)), 4.27 (m, 1H, CH-CH-NH), 4.45 (m, 1H, CH₂-CH-NH), 5.07 (s, 2H, CO-CH₂-N), 5.90 (broad s, 1H, CH-CH-NH-CO), 6.10 (s, 1H, CH₂-CH-NH-CO), 6.90 (d, ³*J*_{HH} = 8.3 Hz, 2H, Cₒ-H), 7.04 - 7.17 (m, 32H, Cₘ-H, C₀²-H, C₁²-H), 7.27 (m, 20H, C₁³-H), 7.32 (t, ³*J*_{HH} = 5.6 Hz, 1H, (CH₂)₄-NH-CO), 7.65 (s, 1H, N-CH=C), 7.73 (m, 10H, C₀³-H), 7.88 (broad s, 3H, CH=N), 7.94 (broad s, 2H, CH=N), 8.00 (t, ³*J*_{HH} = 5.5 Hz, 1H, NH-CO-CH₂-N); ¹³C{¹H} NMR (acetone-*d6*, 125.8 MHz): δ = 25.0 (s, CH₂-CH₂-CH₂-CH₂-NH), 25.6 (s, CO-CH₂-CH₂-CH₂-CH₂), 26.6 (s, CH₂-CH₂-CH₂-CH₂-NH), 28.2 (broad s, CO-CH₂-CH₂-CH₂-CH₂, CO-CH₂-CH₂-CH₂-CH₂), 28.2 (s, CH₂-CH₂-CH₂-CH₂-NH), 32.2 (s, C₆H₄-CH₂-CH₂-N), 32.8 (2 d, ²*J*_{CP} = 12.2 Hz, N-CH₃), 34.5 (s, C₆H₄-CH₂-CH₂-NH), 35.5 (s, CO-CH₂-CH₂-CH₂-CH₂), 38.5 (s, CH₂-CH₂-CH₂-CH₂-NH), 40.2 (s, CH₂-S), 40.7 (s, C₆H₄-CH₂-CH₂-NH), 49.1 (dd, ¹*J*_{CP} = 157.3 Hz, ³*J*_{CP} = 8.2 Hz, N-CH₂-P), 52.0 (m, P(O)(OCH₃), CO-CH₂-N), 55.7 (s, CH-S), 58.1 (t, ³*J*_{CP} = 7.7 Hz, C₆H₄-CH₂-CH₂-N), 59.9 (s, CH₂-CH-NH), 61.5 (s, CH-CH-NH), 120.8 (s, Cₒ), 121.0 (d, ³*J*_{CP} = 4.0 Hz, C₁²), 121.3 (m, C₀²), 122.8 (s, N-CH=C), 128.4 (broad s, C₀³), 129.9 (s, Cₘ), 130.1 (s, C₁³), 132.6 (m, C₀⁴), 136.6 (s, Cₚ), 137.3 (s, C₁⁴), 139.5 (broad t, ³*J*_{CP} = 4.9 Hz, CH=N), 147.2 (s, N-CH=C), 148.9 (m, C₁¹), 151.2 (m, Cᵢ, C₀¹), 162.8 (s, HN-CO-NH), 165.7 (HN-CO-CH₂-N), 172.1 (s, (CH₂)₄-NH-CO) ppm.

### 4. 20. Synthesis of dimethylphosphonate terminated dendrimer with a biotinylated spacer [(Aza2P)₁₀-Biot-D]

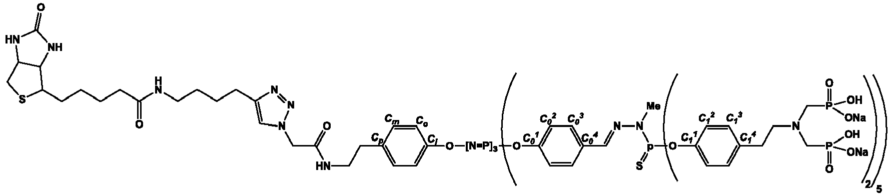

To a vigorously stirred solution of the compound of **example 4.19** (36 mg, 0.007 mmol) in dry dichloromethane (2 mL) was added at 0 °C, under a dry argon atmosphere, bromotrimethylsilane (40 µL, 0.299 mmol). The reaction mixture was stirred at room temperature overnight and then evaporated to dryness under reduced pressure. The crude residue was washed twice with methanol (2 mL) for 1 h at rt and evaporated to dryness under reduced pressure. The resulting white solid was washed once with diethylether (4 mL) and then transformed into its sodium salt as follows: the dendrimer was suspended in water (1 mL/100 mg) and one equivalent of sodium hydroxide per terminal phosphonic acid was added. The resulting solution was lyophilised to afford title compound as a white powder (yield = 85 %). ³¹P{¹H} NMR (D₂O/CD₃CN 7:1, 202.5 MHz): δ = 7.1 (s, PO₃HNa), 9.5 (s, N₃P₃), 64.5 (s, P=S), 64.6 (s, P=S); ¹H NMR (D₂O/CD₃CN 7:1, 500.3 MHz): δ = 1.64 (m, 2H, CO-CH₂-CH₂-CH₂-CH₂)), 1.82 (m, 4H, CO-CH₂-CH₂-CH₂-CH₂, CH₂-CH₂-CH₂-CH₂-NH), 1.93 (m, 4H, CH₂-CH₂-CH₂-CH₂-NH, CO-CH₂-CH₂-CH₂-CH₂), 2.47 (s, 2H, CO-CH₂-CH₂-CH₂-CH₂), 3.10 (m, 7H, CH₂-CH₂-CH₂-CH₂-NH, CH₂-S, C₆H₄-CH₂-CH₂-NH, CH-S), 3.54 (broad s, 22H, C₆H₄-CH₂-CH₂-N, CH₂-CH₂-CH₂-CH₂-NH), 3.75 (broad d, ²*J*_{HP} = 10.1 Hz, 42H, N-CH₂-P, C₆H₄-CH₂-CH₂-NH), 3.81 (m, 15H, N-CH₃), 4.13 (m, 20H, C₆H₄-CH₂-CH₂-N), 4.54 (broad s, 1H, CH-CH-NH), 4.70 (broad s, 1H, CH₂-CH-NH), 5.33 (broad s, 2H, CO-CH₂-N), 7.26 (broad s, 2H, Cₒ-H), 7.42 (m, 12H, Cₘ-H, C₀²-H), 7.60 (m, 20H, C₁²-H), 7.69 (s, 1H, N-CH=C), 7.82 (m, 20H, C₁³-H), 8.06 (m, 10H, C₀³-H), 8.29 (broad s, 3H, CH=N), 8.33 (broad s, 2H, CH=N) ppm. ¹³C{¹H} NMR (D₂O/CD₃CN 7:1, 125.8 MHz): δ = 24.8 (s, CH₂-CH₂-CH₂-CH₂-NH), 25.8 (s, CO-CH₂-CH₂-CH₂-CH₂), 26.4 (s, CH₂-CH₂-CH₂-CH₂-NH), 28.3 (broad s, CO-CH₂-CH₂-CH₂-CH₂), 28.5 (s, CO-CH₂-CH₂-CH₂-CH₂, CH₂-CH₂-CH₂-CH₂-NH), 29.3 (s, C₆H₄-CH₂-CH₂-N), 33.1 (d, ²*J*_{CP} = 8.9 Hz, N-CH₃), 34.3 (s, C₆H₄-CH₂-CH₂-NH), 36.1 (s, CO-CH₂-CH₂-CH₂-CH₂), 39.3 (s, CH₂-CH₂-CH₂-CH₂-NH), 40.3 (s, CH₂-S), 40.6 (s, C₆H₄-CH₂-CH₂-NH), 52.2 (s, CO-CH₂-N), 55.4 (broad d, ¹*J*_{CP} = 125.6 Hz, N-CH₂-P), 55.8 (s, CH-S), 57.9 (broad s, C₆H₄-CH₂-CH₂-N), 60.6 (s, CH₂-CH-NH), 62.3 (s, CH-CH-NH), 121.0 (s, Cₒ), 121.9 (broad s, ³*J*_{CP} = 4.0 Hz, C₀², C₁²), 122.5 (s, N-CH=C), 128.9 (s, C₀³), 130.7 (s, Cₘ), 131.1 (s, C₁³), 133.0 (m, C₀⁴), 135.1 (broad s, Cₚ, C₁⁴), 141.1 (broad m, CH=N), 148.7 (s, N-CH=C), 149.7 (broad s, C₁¹), 151.1 (m, Cᵢ, C₀¹), 164.2 (s, HN-CO-NH), 167.2 (HN-CO-CH₂-N), 175.9 (s, (CH₂)₄-NH-CO) ppm.

### Example 5: Inhibition of the proliferation of CD4 T lymphocytes by dendrimer Gc1. 5.1. Methods

Peripheral blood samples are obtained from adult healthy volunteers through the Etablissement Français du Sang (Toulouse, France). Peripheral Blood Mononuclear Cells (PBMC) are isolated by Ficoll density gradient (Amersham Biosciences AB).

PBMC are cultured at a final concentration of 1.5 x 10⁶ cells/mL in complete RPMI 1640 medium, *i.e.* supplemented with 10% of heat inactivated fetal calf serum (FCS) (Invitrogen), 1 mM sodium pyruvate (Invitrogen), 2 mM L-glutamine, 100 U/mL penicillin and 100 µg/mL streptomycin (Cambrex Bioscience). Proliferation is activated by 400 U/mL of interleukin 2 (IL2) (Sanofi-Aventis) (a "growth factor" for lymphocytes). Dendrimer Gc1 or (Aza2P)₁₂ is made soluble in sterile water at 2 mM and filtered through a 0.2 µm membrane before use at 20 µM in culture.

Proliferation of CD4 T lymphocytes is evaluated by flow cytometry by analyzing the intracellular dilution of a fluorescent probe: the 5,6-carboxyfluorescein diacetate N-succinimidylester (CFSE). Briefly, PBMC are labelled with 1 µM CFSE in serum free PBS for 8 minutes at 37°C and then labelling is stopped by adding an equal volume of FCS. The unconjugated CFSE is eliminated by 2 washes in PBS, and the CFSE-labelled PBMC are cultured for 6 days. Then, CFSE dilution is analyzed by flow cytometry to measure the proliferation CD4 T cells. After gating for CD4 positive cells (mouse anti-human CD4-PE-Cy5 clone 13B8.2, Beckman-Coulter), CFSE fluorescence intensities are analyzed, and the percentage of CD4 T cells which have proliferated during the culture is taken into account. Each condition is done in triplicate. The overall differences are evaluated by analysis of variance test (ANOVA) with Sigma Stat software (Systat Software).

### 5.2. Results

They are shown in Figure 4.

T lymphocytes, especially CD4 T lymphocytes, proliferate in presence of IL2. We observe that, after a few days with IL2 alone, 70.2 % of CD4 T lymphocytes have divided (left peak in Figure 4A, the right peak corresponds to non-divided CD4 T lymphocytes).

In the same conditions but with 20 µM of Gc1, only 17.2% of CD4 T lymphocytes have divided (left signal Figure 4B, the right peak corresponds to non-divided CD4 T lymphocytes).

### Example 6: Inhibition of the proliferation of purified CD4 T lymphocytes by bisphosphonic dendrimers.

### 6.1. Methods

PBMC from adult healthy volunteers are prepared as described in example 1.

CD4 T lymphocytes are then purified from PBMC by positive selection using microbeads conjugated with anti-CD4 mAb, according to the manufacter's instructions (Miltenyi Biotec). The cells obtained after the purification are > 98% pure (as verified by flow cytometry).

Purified CD4 T lymphocytes are cultured at a final concentration of 1.5 x 10⁶ cells/mL in complete RPMI 1640 medium (as described in example 1.1). Then, their proliferation is triggered by microbeads coated with anti-CD3 and anti-CD28 mAbs (Dynabeads®, Dynal Biotech ASA). Dendrimer Gc1 or (Aza2P)₁₂ and its analogs are made soluble in sterile water at 2 mM and filtered through a 0.2 µm membrane before use at 20 µM in culture.

Proliferation of purified CD4 T lymphocytes is evaluated by flow cytometry by analyzing the intracellular dilution of a fluorescent probe: the CFSE.

### 6.2. Results

They are shown in Figures 5 and 6.

CD4 T lymphocytes strongly proliferate after triggering by microbeads coated with anti-CD3 and anti-CD28 mAbs in presence of IL2. We observe that, after a few days with microbeads and IL2, 88 % of purified CD4 T lymphocytes have divided (Figure 5A).

In the same conditions but with 20 µM of Gc1, only 10.2% of purified CD4 T lymphocytes have divided (Figure 5B).

We observe the same inhibitory effect with the julolidin ((Aza2P)₁₀-Julo-D) and the biotinylated ((Aza2P)₁₀-Biot-D) analogs of Gc1 (Figure 6).

These results show a direct inhibitory effect of azabisphosphonic dendrimers on the proliferation of CD4 T lymphocytes.

### Example 7: Transcriptomic study of monocytes activated by dendrimer Gc1.

### 7.1. Methods

### 7.1.1. Cell preparation and purification

Peripheral Blood Mononuclear Cells (PBMC) from adult healthy volunteers are prepared as described in example 1.

Monocytes are then purified by positive selection using microbeads conjugated with anti-CD14 mAb, according to the manufacter's instructions (Miltenyi Biotec). The cells obtained after the purification are > 95% pure. The CD14 negative fraction is collected as Peripheral Blood Lymphocytes (PBL).

### 7.1.2. Monocyte culture and activation

Monocytes are cultured at a final concentration of 1 x 10⁶ cells/mL in complete RPMI 1640 medium (as described in example 1.1).

For genechip analysis, 10 x 10⁶ monocytes from 3 donors (and from 3 more donors for quantitative PCR) are cultured in 25 cm² dishes. They are stimulated with 20 µM of dendrimer for 6 hours or remain untreated (control cells).

For the functional experiments, freshly purified monocytes are cultured in 6-well plates. The cells are stimulated for 24 hours (immunophenotyping by flow cytometry analyses, MLR) or 96 hours (CD206 detection) with different stimuli or remain untreated (control). Dendrimer-activated monocytes (*da*-monocytes) are obtained with 20 µM of the dendrimer Gc1 or (Aza2P)₁₂, alternatively-activated monocytes (*alt*-monocytes) with 10 U/mL recombinant human IL4 (Peprotech). The classical activation of monocytes is obtained by priming the cells over night (18 hours) with 100 U/mL of human recombinant IFN-γ (R&D Systems) and, after a washing step in PBS, by stimulation with 10 ng/mL LPS from *E. coli* 011/B4 strain (InvivoGen) for the remaining 6 hours or 78 hours.

### 7.1.3. RNA extraction and complementary RNA (cRNA) synthesis

Total RNA are extracted from 10 x 10⁶ monocytes using TRIzol™ Reagent (Invitrogen), according to the manufacter's instructions. The quality and integrity of the RNA obtained are assessed by using an Agilent 2100 Bioanalyser (Agilent Technologies) after a denaturating step at 70°C for 2 minutes. cRNA are prepared according to one-Cycle Target Labelling protocol (Affymetrix) starting from 1 µg of total RNA.

### 7.1.4. Affymetrix genechip analysis

cRNA are fragmented and hybridized to Affymetrix HG-U133 plus 2.0 arrays. The chips are then washed and scanned, according to the manufacter's instructions. HG-U133 plus 2.0 arrays contain 54,675 sets of oligonucleotide probes that correspond to ≈39,000 unique human genes or predicted genes. GeneChip operating Software, Version 1.1 (Affymetrix), is used for the primary image analysis of the array, for the normalization (global scaling method, target value of 100), and for the comparisons between control and dendrimer-treated samples. Monocytes from 3 donors are analyzed after 6 hour incubation in the presence or absence of the dendrimer Gc1 or (Aza2P)₁₂. Genes are considered to be differentially regulated in *da-*monocytes compared to control cells if they have a fold change of ≥ 2.0 or ≤ -2.0 for at least two donors of the three. The annotation tool, which is an automated method for the functional annotation of gene lists, is performed with the Affymetrix NetAffx data base (http://www.affymetrix.com/analysis/netaffx/index.affx). Gene Ontology data mining for biological process at level 4 and 5 is conducted on line via the DAVID website (http://david.abcc.ncifcrf.gov/).

### 7.1.5. Quantitative RT-PCR

Total RNA are purified as described above, and 5 µg are used to synthesize single-strand cDNA using M-MLV reverse transcriptase RNase H Minus (Promega), according to the manufacter's instructions. Quantitative RT-PCR is then performed using the Platinium SYBR Green qPCR SuperMix UDG (Invitrogen) with forward and reverse primers at a final concentration of 300 nM. The primers are designed using Primer Express software from mRNA sequences submitted to Ensembl data base, and are listed in Figure 7. Quantification of the different mRNA is performed on a 7000 Sequence Detection System (Applied Biosystems). PCR amplification begins with 1 cycle of 50°C for 2 minutes and 95°C for 10 minutes, followed with 40 cycles of denaturation at 95°C for 15 seconds and annealing/extension at 60°C for 60 seconds. All experiments are performed in duplicate. For each donor, the relative expression is calculated as 2^{-ΔΔCt} where ΔΔCt is the difference between ΔCt of the sample and the ΔCt of the reference consisting of the control monocytes. ΔCt is the difference between the Ct of the target gene and the GAPDH gene. For statistical analysis, a one-tailed paired *t* test is performed comparing the ΔCt values for the *da-*monocytes and the corresponding control monocytes with Sigma Stat software (Systat Software).

### 7.1.6. Flow cytometry

Flow cytometry is performed on a LSR-II cytometer (BD Biosciences). The data are analyzed with FACSDiva (BD Biosciences) or WinMDI softwares. The expression of surface markers is performed using mouse anti-human fluorochrome-conjugated mAbs specific for MHC class II-FITC (clone Tü36), CD86-PE (clone 2331), MHC class I-PE-Cy5 (clone G46-2.6) and CD13-PE (clone WM15) from BD Biosciences and specific for CD64-FITC (clone 22) and CD206-PE (clone 3.29B1.10) from Beckman-Coulter. Appropriate isotype-matched antibodies are used as negative control.

### 7.1.7. Allogenic Mixed Leucocyte Reactions (MLR)

MLR are performed in 96-well round bottom plates in a total volume of 200 µL in complete RPMI 1640 medium. Stimulation is assayed by incubating responder PBL (10⁵ cells) with different numbers of allogenic stimulating monocytes (activated with the different stimuli for 24 hours) (PBL:monocyte ratios ranging from 4:1 to 100:1). The proliferation of the CD4 T cells is analyzed by measuring the cytoplasmic dilution of CFSE (as described in **5.1. Methods**). The CFSE-labelled PBL and the differently activated monocytes are then co-cultured for 6 days. Then, CFSE dilution is analyzed by flow cytometry to measure the proliferation of alloantigen-induced CD4 T cells. After gating for CD4 positive cells CFSE fluorescence intensities are analyzed, and the percentage of CD4 T cells which have proliferated during the MLR is taken into account. Each condition is done in triplicate. The overall differences are evaluated by analysis of variance test (ANOVA) with Sigma Stat software (Systat Software).

### 7.1.8. Intracellular detection of IL10

Six days following the primary stimulation with one of the differently activated monocytes at a ratio of PBL:monocytes of 4:1, cells were restimulated with 10000 anti-CD3/anti-CD28 beads for 5 hours (according to the technical description by J. P. Edwards et al., (2006) J Leukoc Biol 80, 1298-1307) and with 10 µg/mL Brefeldin A (Sigma-Aldrich) to inhibit cytokine secretion. Cells are then harvested, washed with PBS and incubated 15 minutes at 4°C with mouse mAb anti-human CD4-PE (clone 13B8.2, Beckman-Coulter) in PBS with 5% FCS. Cells washed twice, are then fixed by 2% paraformaldehyde in PBS and permeabilized with 1% saponin in PBS. Intracellular staining is performed with AlloPhycoCyanin-conjugated rat anti-human IL10 (clone JES3-19F1, BD Bioscience) for 30 minutes at 4°C in PBS with 1% saponin and analyzed by flow cytometry. Each condition is done in triplicate and results are expressed as the mean ± SD. Intracellular IL10 mean fluorescence intensity (mfi) means across the differently activated monocytes are compared by one-way analysis of variance, the comparisons between CD4⁺/CFSE⁻ and CD4⁺/CFSE⁺ cells are done with Student's t-test using Sigma Stat software (Systat Software).

### 7.2. Results

### 7.2.1. Human monocyte transcriptional profile after activation by dendrimer Gc1 or (Aza2P)₁₂

Results are shown in Figures 8 and 9.

78 genes were found over-expressed and 62 genes were found under-expressed by *da-*monocytes. On the one hand, up-regulation of genes coding for proteins characterizing the alternative activation of monocytes/macrophages such as the mannose receptor (MRC), the IL1 receptor antagonist (IL1 RN), the immuno-suppressive cytokine IL10, and the chemokine CCL18 (also called AMAC-1 for Alternative Macrophage Activation-associated CC-chemokine-1) is detected. C1QR1 (important for the anti-inflammatory phagocytosis of apoptotic cells), CHI3L1, matrix metalloproteinases (of which MMP1) and SLAMF1 are also found over-expressed. On the other hand, down-regulation of genes coding for CXCR4 (receptor for the pro-inflammatory CXL12 chemokine), metallothioneins and IFITs (gene clusters induced by IFN-γ) is detected. These genes are likely to be expressed after IFN-γ and/or LPS treatment. Moreover, we detect a down-regulated expression of adhesion molecules such as CD9, CD11a and CD18. CD11a/CD18 also know as lymphocyte function antigen 1 (LFA-1) are implicated in mechanisms of leukocyte recruitment on inflamed tissue (42).

### 7.2.2. Validation of the alternative-like activation of human monocytes by quantitative RT-PCR and analysis of da-monocyte phenotype

Results are shown in Figure 10.

9 gene transcripts of high relevance for the classical activation pathway (one pro-inflammatory chemokine: CCL5 and 3 pro-inflammatory cytokines: IL1β, IL6, IL12) or for the alternative activation pathway (MRC1, IL1 RN, IL10, CCL18 and CD23) are quantified. Results of quantitative RT-PCR are compared in *da*-monocytes and untreated monocytes of the 3 donors enlisted for the transcriptional study plus 3 supplementary donors. 5 genes whose products indicate an alternative activation (MRC1, IL1 RN, IL10, CCL18 and CD23) are significantly up-regulated in *da*-monocytes, whereas the expression levels of 3 genes out of 4 selected to indicate a classical activation (IL1β, IL6 and IL12) are not significantly modified and the fourth gene (coding for CCL5) is significantly under-expressed. These results confirm those of example 3.2.1.

### 7.2.3. Phenotype of da-monocytes

Results are shown in Figure 11.

Expression of the mannose receptor MRC1 (CD206) is strongly enhanced by *alt-*monocytes and by *da*-monocytes, whereas this marker is weakly expressed by *class-*monocytes. On the contrary, CD64 (a FcyRI) is overexpressed in *class*-monocytes, but not in *alt-* or *da*-monocytes. Finally, Figure 11 shows that CD13, a cell membrane-associated aminopeptidase N, also discriminates between the different types of monocyte activation. *Class*-monocytes express a higher level of CD13 than *alt-* and *da*-monocytes. Taken together, the quantitative study of gene transcripts and the analysis of the phenotype of differently activated monocytes strengthen the hypothesis of an alternative activation of human monocytes by bisphosphonic dendrimers.

### 7.2.4. The activation of monocytes by the dendrimer Gc1 or (Aza2P)12 is anti-inflammatory and immuno-suppressive

Results are shown in Figures 12 (1) and (2).

After 24 hours stimulations, the phenotypes of *alt-, class-* and *da*-monocytes are compared for the expression of HLA-DR, HLA-A,B,C (antigen presenting molecules) and CD86 (co-stimulatory molecule) markers by flow cytometry (Figure 12(1)A). As expected, *class*-monocytes express high levels of antigen-presenting molecules and CD86, whereas *alt-*monocytes express lower levels of these molecules and appear as poor antigen-presenting cells (APC). In this respect, *da*-monocytes are closely related to *alt*-monocytes and also appear as poor APC. To compare the stimulatory capacity of differently activated monocytes, MLR with PBL and allogeneic activated-monocytes are performed. MLR activation is conducted for 4 different PBL/monocyte ratios from 4:1 to 100:1. MLR are measured as the percentage of divided CD4⁺ T lymphocytes among the PBL by using the dilution of the fluorescent dye CFSE. The results for 3 representative donors are compiled in Figure 12(1) B with *alt-, class-* and *da*-monocytes as activators of MLR. As awaited, MLR activation by *class*-monocytes is always significantly higher than activation by *alt*-monocytes, except for the 4:1 ratio of donor 1. Statistical analysis is given in Figure 12(2). It is noteworthy that *da-*monocytes systematically give the lowest activating effect on MLR. In detail, MLR activations by *da*-monocytes are always lower than activations by class-monocytes. When compared to activations by *alt*-monocytes, activations by *da*-monocytes are also significantly lower, except for donor 2, 25:1 ratio of donor 1 and 10:1 and 25:1 ratios of donor 3.

### 7.2.5. Intracellular detection of IL10

Results are shown in Figure 13.

Intracellular labelling of IL10 is compared in divided (CFSE⁻) and in non-divided (CFSE⁺) CD4⁺ T cells (Figure 13 (A)) resulting from the different MLR contexts after re-stimulation for 5 hours with anti-CD3/anti-CD28 mAbs coated on microbeads. The results from a representative donor out of 3 show an increase of the percentage of IL10⁺ CD4⁺ dividing cells in all MLR (Figure 13 (B)). Figure 13 (C) shows a comparison of the production of IL10 in divided and non-divided CD4⁺ T cells for the three MLR contexts independently. Statistical analysis of the mfi detected after labelling of IL10 gained in experiments with 3 donors indicates a significantly increased production of IL10 in divided (CFSE⁻) CD4⁺ T lymphocytes in MLR with *da-* and *alt*-monocytes. Conversely, the divided and non-divided CD4⁺ T cells in MLR with *class*-monocytes do not show significantly different levels of IL10 production. Figure 13 (D) shows the comparison of the production of IL10 in divided cells aroused from the three different MLR. The production of IL10 by CD4⁺ T cells is significantly enhanced in MLR with *da-* and *alt*-monocytes when compared to MLR with class-monocytes.

Thus, *alt-* and *da*-monocytes are poor inducers of CD4 T cell proliferation. What is more, *da*-monocytes induced IL10-producing CD4 T cells.

### Example 8: Inhibition of the differentiation of monocytes in osteoclasts by 20 µM of dendrimer Gc1 or (Aza2P)₁₂.

### 8.1. Introduction

The combination of cytokines such as RANK-Ligand and M-CSF induces the differentiation of human monocytes in osteoclasts. In bone biology, osteoclasts are responsible for bone resorption whereas osteoblasts rebuilt it. In some pathological contexts, the balance between the activities of osteoclasts and osteoblasts is compromised in the direction of the osteoclastic activity resulting in bone resorption.

### 8.2. Methods

PBMC from adult healthy volunteers are prepared as described in example 1.

Monocytes are then purified by negative selection using a kit containing a mixture of mouse anti-human mAbs and superparamagnetic polystyrene beads coated with a human antimouse IgG mAbs, according to the manufacter's instructions (Dynal). The cells obtained after the purification are > 90% pure.

Pure monocytes are activated or not during 6 hours with 20µM of dendrimer Gc1 or (Aza2P)₁₂. After three washings, activated and not activated monocytes are cultured for 21 days in α-MEM complete medium, *i.e.* supplemented with 10% of heat inactivated fetal calf serum (FCS) (Invitrogen), 2 mM L-glutamine, 100 U/mL penicillin and 100 µg/mL streptomycin (Cambrex Bioscience) in presence or not of 20 µM of dendrimer Gc1 or (Aza2P)₁₂. To generate osteoclasts, 50 ng/mL of M-CSF (PeproTech) and 30 ng/mL of sRANK-Ligand (PeproTech) are added to the culture medium. Each three days, half medium is changed and 25 ng/mL of M-CSF and 100 ng/mL of sRANK-Ligand are added. At day 21, the cells are fixed and stained with the leucocyte phosphatase acid kit according to the manufacter's instructions (Sigma-Aldrich).

### 8.3. Results

They are shown in Figure 14.

When added at 20 µM in the culture medium, dendrimer Gc1 or (Aza2P)₁₂ inhibits the differentiation of human monocytes in osteoclasts (picture 1 of Figure 14 shows osteoclasts as giant multinuclear cells whereas picture 2 shows nucleus of undifferenciated monocytes).

When monocytes are pre-incubated with 20 µM of dendrimer Gc1 or (Aza2P)₁₂ during 6 hours, then rinsed before stimulation with RANK-Ligand and M-CSF, we also observe an inhibition of their differentiation in osteoclasts (picture 3 of Figure 14 shows only a few osteoclasts among undifferenciated monocytes).

### Example 9: Inhibition of bone resorption in vitro

### 9.1. Methods

PBMC from adult healthy volunteers are prepared as described in example 1.

Monocytes are then purified as described in section 4.2.

Monocytes were cultured in bone matrix 96-well plates (OsteoAssay Human bone plate, Lonza, USA) and differentiated into osteoclasts in the presence of M-CSF and sRANK-Ligand as described in section 4.2. Supernatants were collected and bone resorption was evaluated using a Crosslaps assay (Nordicbioscience, Danemark) which measures collagen degradation fragments.

### 9.2. Results

They are shown in Figure 15.

Results show the release of fragments of bone matrix collagen (CTX, in nM) measured at days (D) 12 and 16. Dendrimer Gc1 or (Aza2P)₁₂ inhibits the *in vitro* resorption of bone when added directly in the differanciating medium (Figure 15 (A)) or when monocytes have been pre-incubated for 6 hours and rinsed before stimulation by RANK-Ligand et M-CSF (Figure 15 B). Dendrimer Gc1 or (Aza2P)₁₂ at 200 nM inhibits approximatively 60% of bone resorption at day 12, and 80% at day 15 (Figure 15 (A)).

When monocytes have been pre-incubated with the dendrimer Gc1 or (Aza2P)₁₂ before induction of the differentiation, inhibition of bone resorption is around 80% at day 12 and 50% at day 15 (Figure 15 (B)).

Taken together, these results show that bisphosphonic dendrimers activate human monocytes toward an « alternative-like » response and thus can be used as drugs for the treatment of uncontrolled inflammatory disorders in chronic or acute diseases such as psoriasis, rheumatoid arthritis or auto-immune disorders.

## Claims

1. Dendrimers with monophosphonic or bisphosphonic terminations for use in the treatment of chronic inflammatory diseases and chronic inflammatory diseases of auto-immune origin, corresponding to the following general formula (1a): 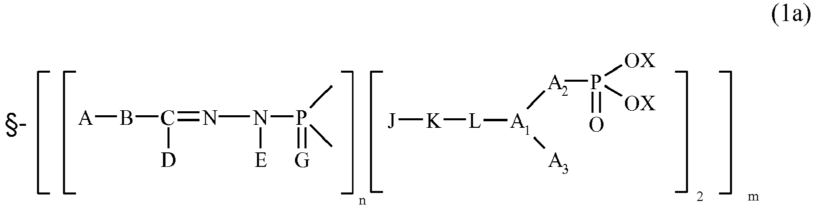 where n represents an integer from 0 to 3, selected from the group comprising the compounds where
- when n = 0, formula (1a) corresponds to the following formula (2a), 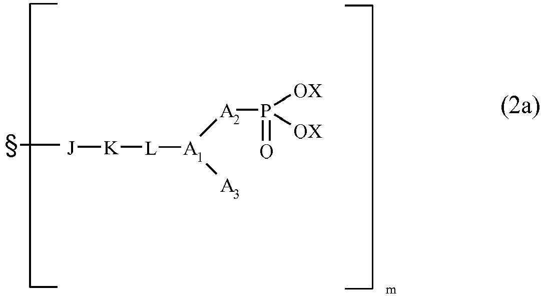
- when n = 1, formula (1a) corresponds to the following formula (3a), 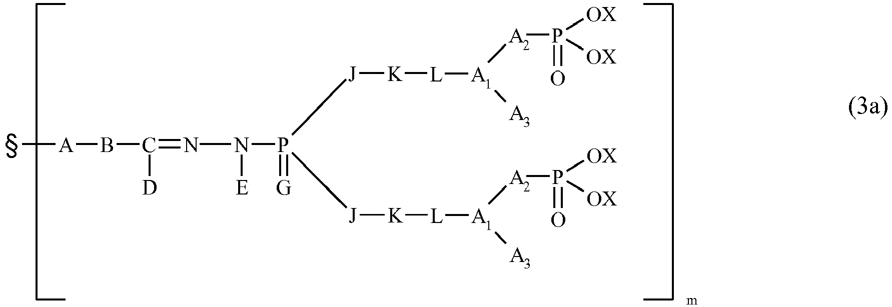
- when n = 2, formula (1a) corresponds to the following formula (4a), 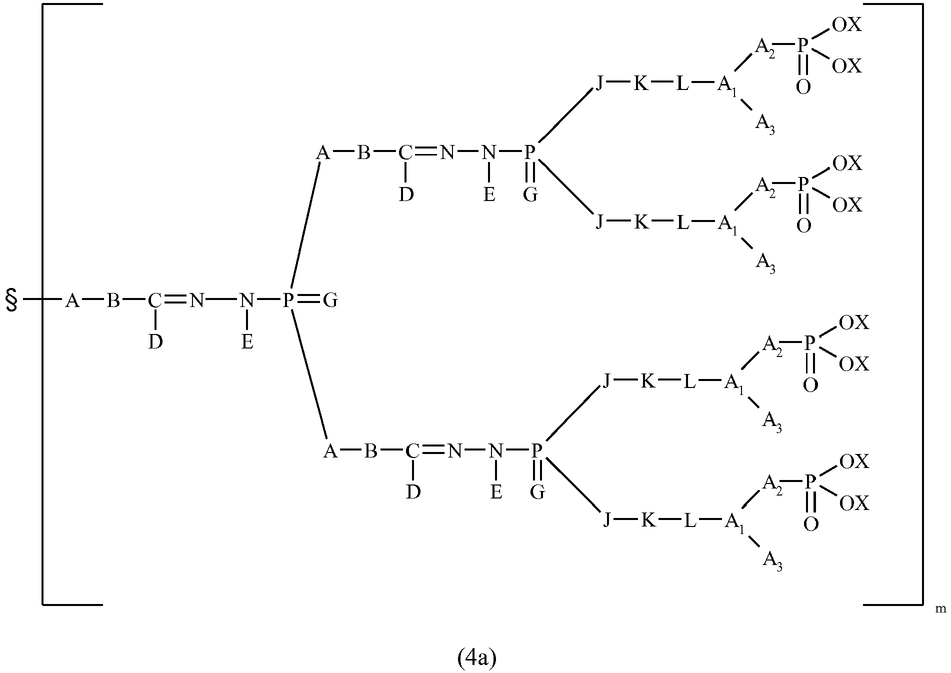
- and when n = 3, formula (1a) corresponds to the following formula (5a), 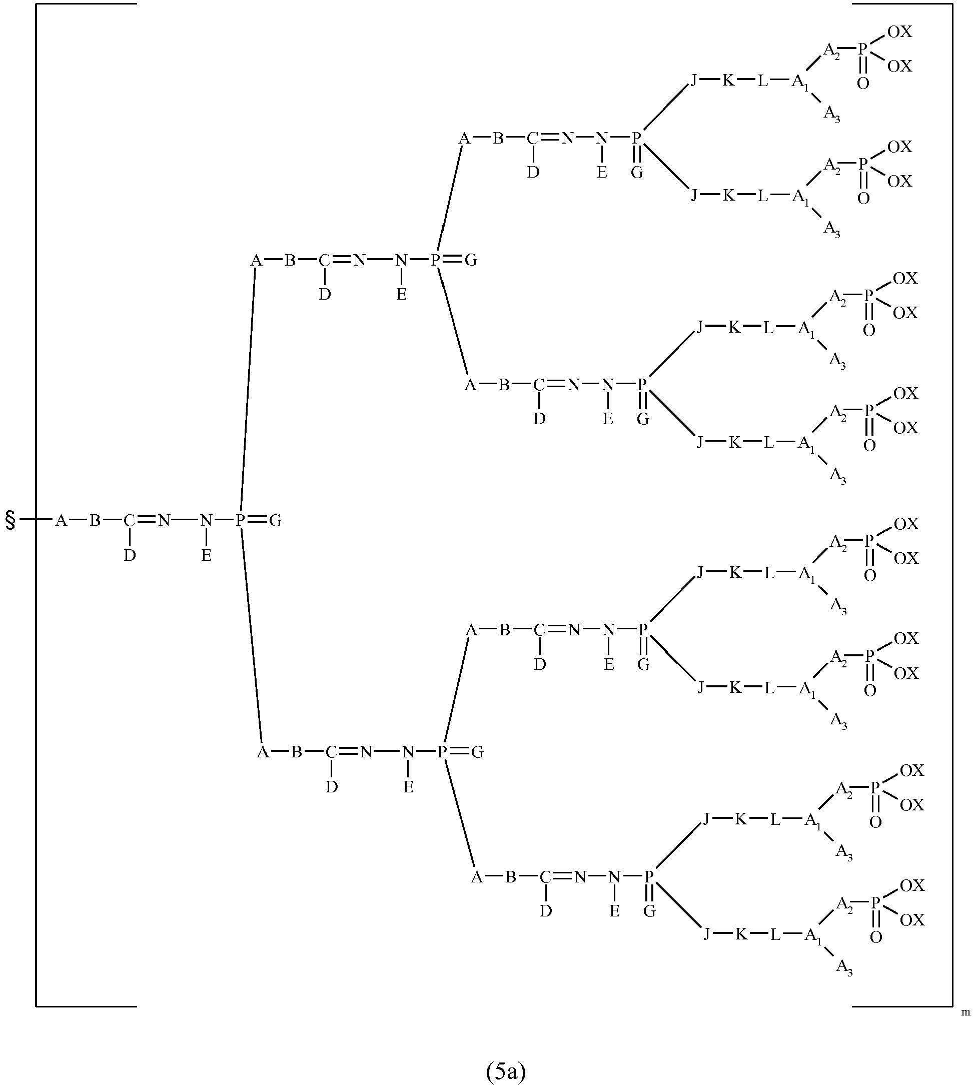 or of formula (7) 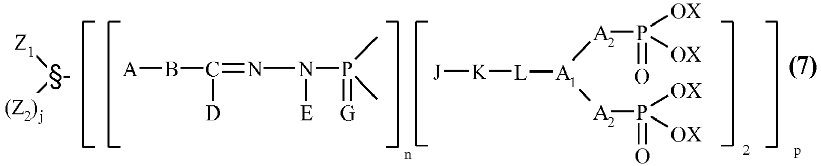 wherein
n represents an integer from 0 to 3, m represents 6, p represents m - 1 or m - 2, and j represents 0 when p represents m - 1 and 1 when p represents m - 2, selected from the group comprising the compounds wherein
- when p = m - 1, formula (7) corresponds to the following formula (8): 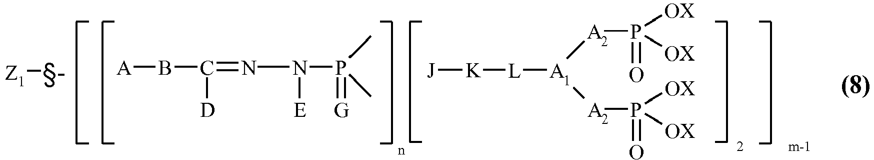
- when p = m - 2, formula (7) corresponds to the following formula (9): 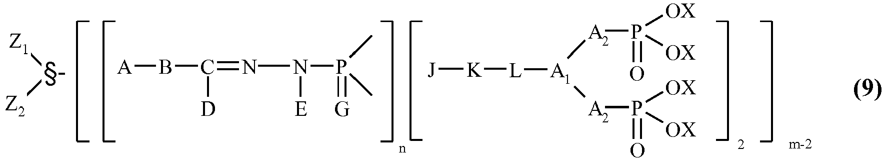 wherein the
• the central core § is : 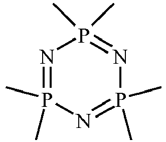
• m represents 6;
• the generation chain corresponds to the formula: where 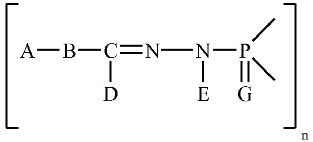
- A represents an oxygen, sulphur, phosphorus atom or an -NR- group;
- B represents an aryl group of 6 to 24 carbon atoms, a heteroaryl group of 1 to 24 carbon atoms, an alkyl group of 1 to 16 carbon atoms, an oligoethyleneglycol group, a polyethyleneglycol group, two aryl groups of 6 to 24 carbon atoms linked by an oxygen, a nitrogen, a sulphur, an alkyl group of 1 to 16 carbon atoms, an heteroalkyl group of 1 to 24 carbon atoms, an oligoethyleneglycol group, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
- D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a -NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
- E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, -OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
- G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
• the intermediate chain corresponds to the formula:
-J-K-L-
where
- J represents an oxygen atom, a sulphur atom, or a -NR- group;
- K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, each being able to be optionally substituted by a halogen atom or a NO₂, -NRR', -CN, -CF₃, - OH group, an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
- L represents a linear, branched or cyclic hydrocarbon chain with 0 to 10 members, optionally containing one or more double or triple bonds, each of said members being able to be optionally a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, -NO₂, -NRR', -CN, -CF₃, -OH, an alkoxy group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
R and R' representing independently of each other H or an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
• the terminal group corresponds to the formula: 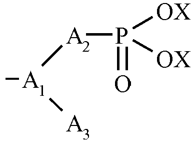 where
A₁ represents N; a P=Y group, where Y represents O, S, or no atom; an N-R group; or a C-R group; R representing H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, a -NR'R" group, an alkoxy group of 1 to 16 carbon atoms, an alkyl group of 1 to 16 carbon atoms, a halogen atom, a -NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R' and R" representing independently of each other H or an alkyl group of 1 to 16 carbon atoms, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
A₂ represents a single bond or a linear, branched or cyclic hydrocarbon chain with 1 to 6 members, each of said members optionally containing one or more heteroatoms, said heteroatoms being preferably chosen from a sulphur, oxygen, phosphorus or nitrogen atom, more preferably nitrogen, and being optionally substituted by at least one substituent chosen from H, an alkyl group of 1 to 16 carbon atoms, a halogen, a NO₂ group, a NRR' group, a -CN group, a -CF₃ group, a hydroxyl group, an alkoxy group of 1 to 16 carbon atoms, an aryl or heteroaryl group of 1 to 24 carbon atoms, the heteroelement being preferably chosen from oxygen, nitrogen or sulphur, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R and R' representing independently of each other H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, a NR"R''' group, an alkoxy group of 1 to 16 carbon atoms, an alkyl group of 1 to 16 carbon atoms, a halogen atom, a NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R" and R''' representing independently of each other H or an alkyl group of 1 to 16 carbon atoms, an aryl group of 6 to 24 carbon atoms or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹;
A₃ represents H, or a linear, branched or cyclic hydrocarbon chain with 1 to 6 members, each of said members being optionally chosen from a heteroatom, said heteroatom being preferably chosen from sulphur, nitrogen, phosphorus or silicon, more preferably nitrogen, each member being able to be optionally substituted by at least one group chosen from a hydroxyl group, a NR"R''' group, an alkoxy group of 1 to 16 carbon atoms, an alkyl group of 1 to 16 carbon atoms, a halogen atom, a NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R" and R''' representing independently of each other H or an alkyl group of 1 to 16 carbon atoms, an aryl group of 6 to 24 carbon atoms, or an aralkyl group of 7 to 16 carbon atoms, an oligoethyleneglycol group comprising preferentially 1 to 12 ethyleneglycol moieties, a polyethyleneglycol group 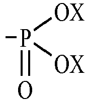 having a molecular weight ranging preferentially from 300 g.mol⁻¹ to 3000 g.mol⁻¹ or in particular A₃ can represent 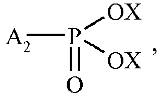 each A₂ being identical or different;
each OX, identical or different for each phosphonic group, represents OH, Oalkyl, where the alkyl group comprises from 1 to 16 carbon atoms, Oaryl, where the aryl group comprises from 6 to 24 carbon atoms, Oaralkyl, where the aralkyl group comprises from 7 to 24 carbon atoms, Oalkylaryl, where the alkylaryl group comprises from 7 to 24 carbon atoms, OSiR'₁R'₂R'₃, where R'₁, R'₂ and R'₃, identical or different, represent an alkyl group of 1 to 16 carbon atoms, or O⁻M⁺, where M⁺ is a cation of elements of group IA, IB, IIA, IIB or IIIA, IIIB of the periodic table of the elements, preferably M⁺ is chosen from the cations of the sodium, potassium, copper, calcium, barium, zinc, magnesium, lithium and aluminium atoms, or hydrocarbon groups of 1 to 100 carbon atoms, or nitrogenous groups of 0 to 100 carbon atoms, such as NR₁R₂R₃R₄, where, independently of each other R₁, R₂, R₃ and R₄ represent H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, a NR"R''' group, an alkoxy group of 1 to 16 carbon atoms, an alkyl group of 1 to 16 carbon atoms, a halogen atom, a NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R" and R''' representing independently of each other H or an alkyl group of 1 to 16 carbon atoms, an aryl group of 6 to 24 carbon atoms or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹,
Z₁ and Z₂ being identical or different, optionally linked together, in particular by means of a covalent bond, and representing H or a linear, branched or cyclic hydrocarbon chain with 1 to 16 members, optionally containing one or more double or triple bonds, each of said members being optionally chosen from a heteroatom, said heteroatom being preferably chosen from a nitrogen, oxygen, phosphorus, silicon or sulphur atom, an aryl group of 6 to 24 carbon atoms, a heteroaryl group of 1 to 24 carbon atoms, a carboxyl group, a >C=NR group, each member being able to be optionally substituted by at least one substituent chosen from a hydroxyl group, a NR"R''' group, an alkoxy group of 1 to 16 carbon atoms, an alkyl group of 1 to 16 carbon atoms, a halogen atom, a NO₂ group, a -CN group, a -CF₃ group, an aryl group of 6 to 24 carbon atoms, an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, R" and R''' representing independently of each other H or an alkyl group of 1 to 16 carbon atoms, an aryl group of 6 to 24 carbon atoms or an aralkyl group of 7 to 16 carbon atoms, an oligoethyleneglycol group comprising preferentially 1 to 12 ethyleneglycol moieties, a polyethyleneglycol group having a molecular weight ranging preferentially from 300 g.mol⁻¹ to 3000 g.mol⁻¹, the first member of said hydrocarbon chain preferably being oxygen or nitrogen.

2. Dendrimers for use according to claim 1 corresponding to general formula: 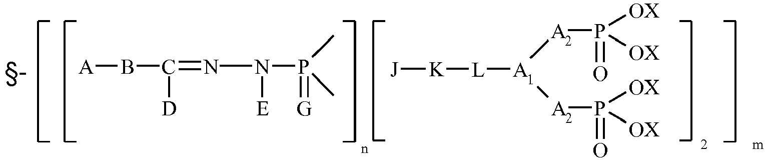 or 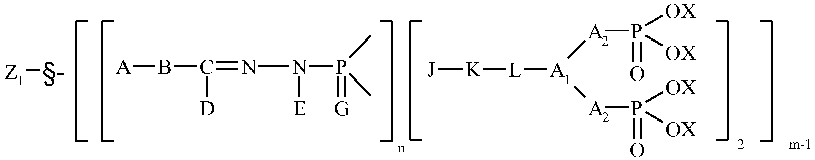 wherein:
§ represents 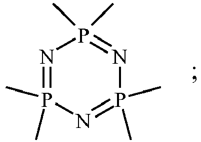
m represents 6;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non-substituted linear saturated hydrocarbon chain with two carbon atoms;
A₁ represents a nitrogen atom;
A₂ represents a CH₂ group;
X represents a methyl group, or a hydrogen or sodium atom, and
Z₁ represents a phenyloxy group.

3. Dendrimers according to any of claims 1 or 2 selected from the group comprising: 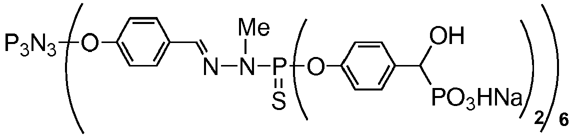 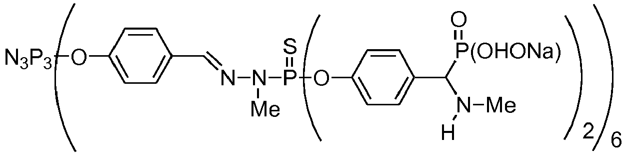 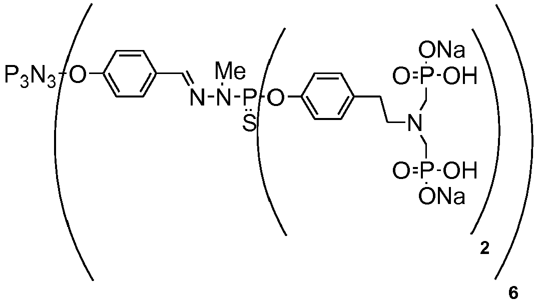 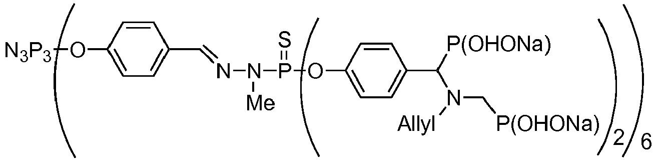 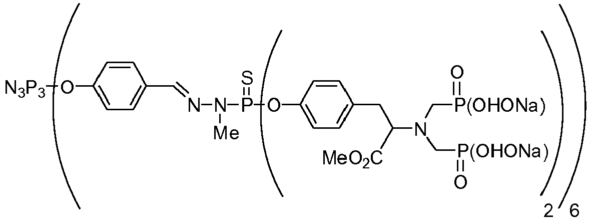 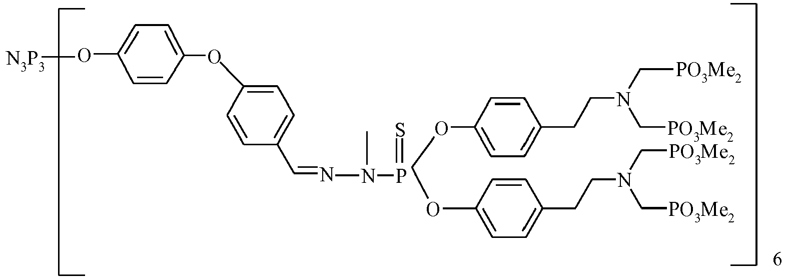 or of GC1 compounds of the following formulae: 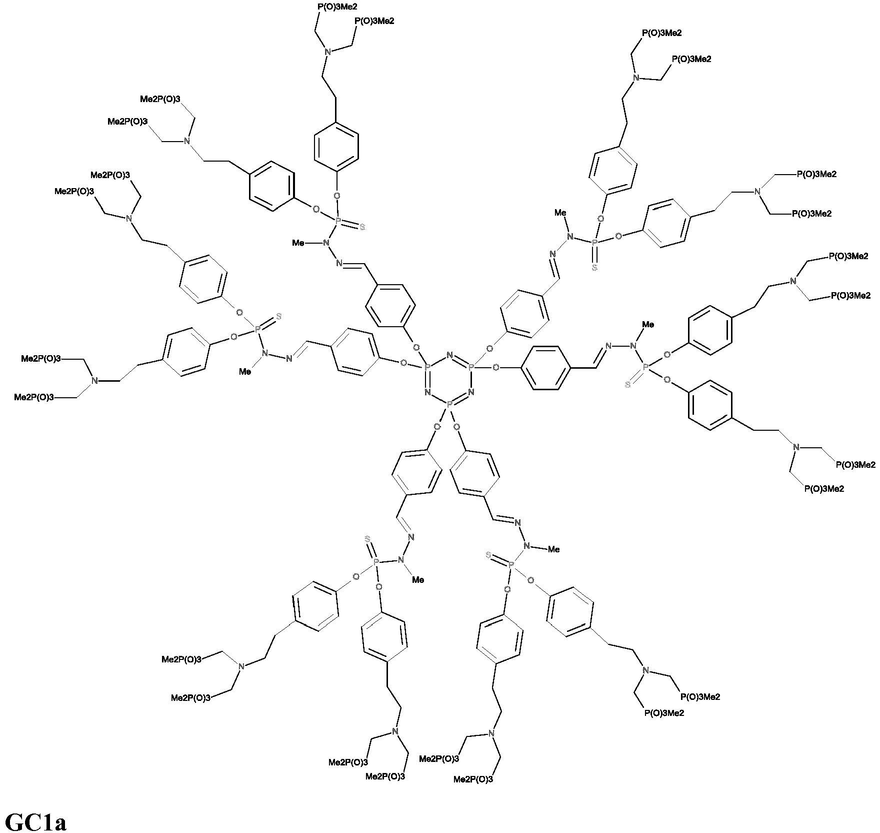 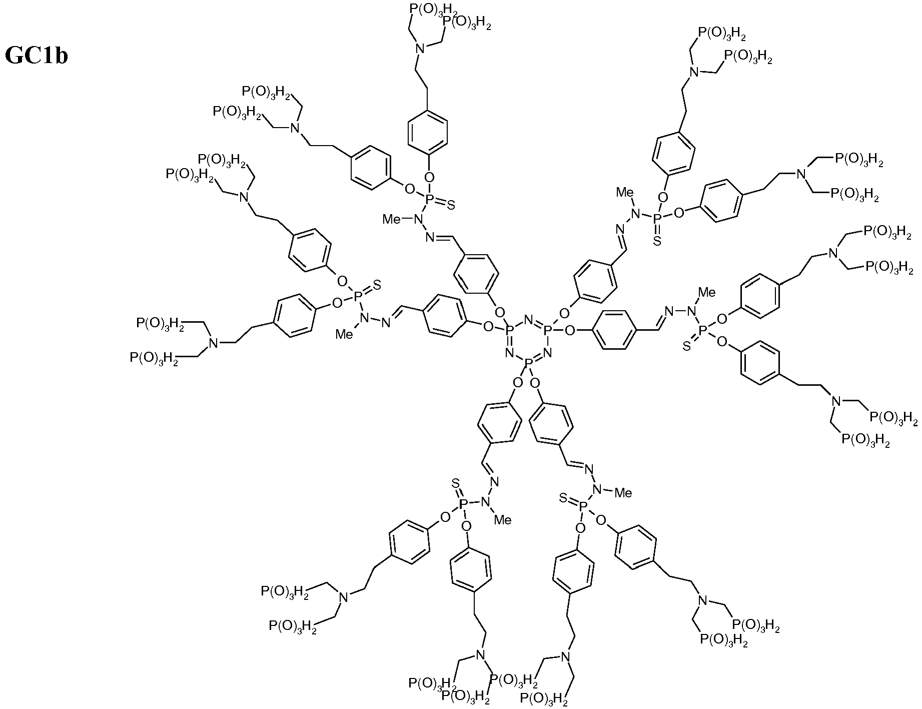 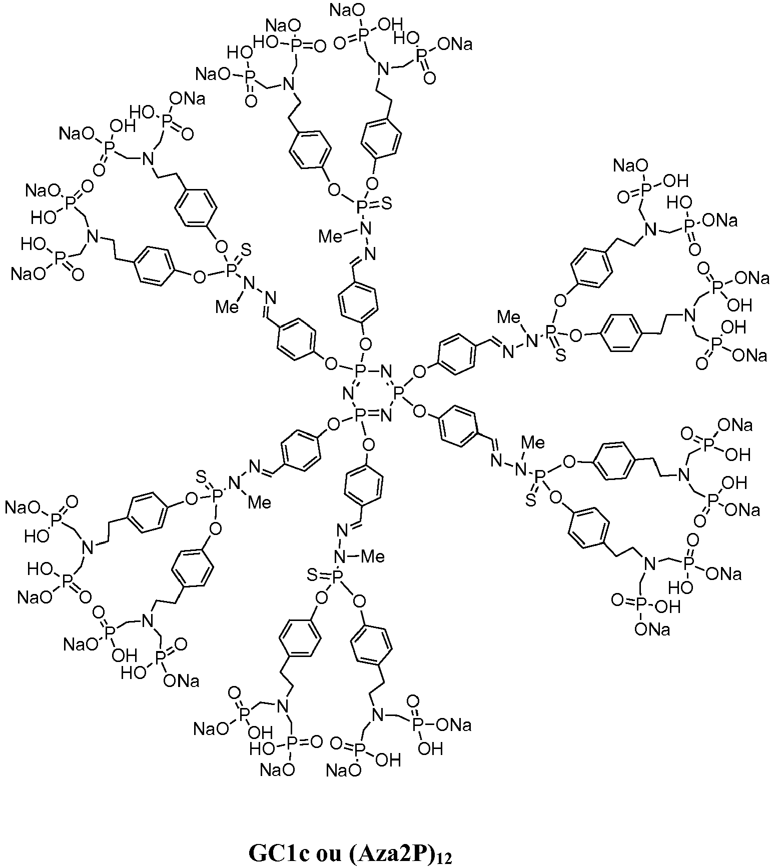 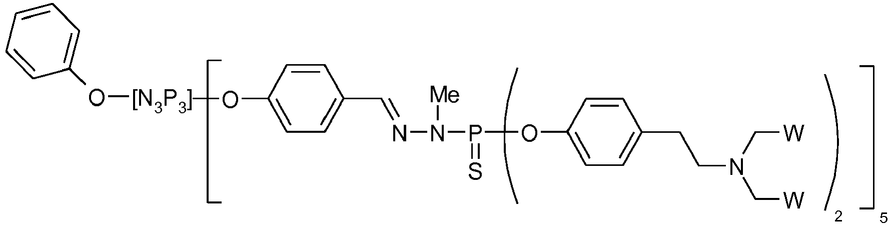 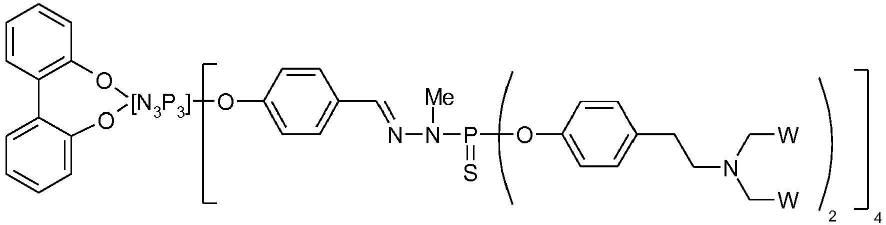 in which W represents PO₃Me₂, PO₃HNa, PO₃H₂, said compounds corresponding, in particular, to compound GC1' of the following formula (10): 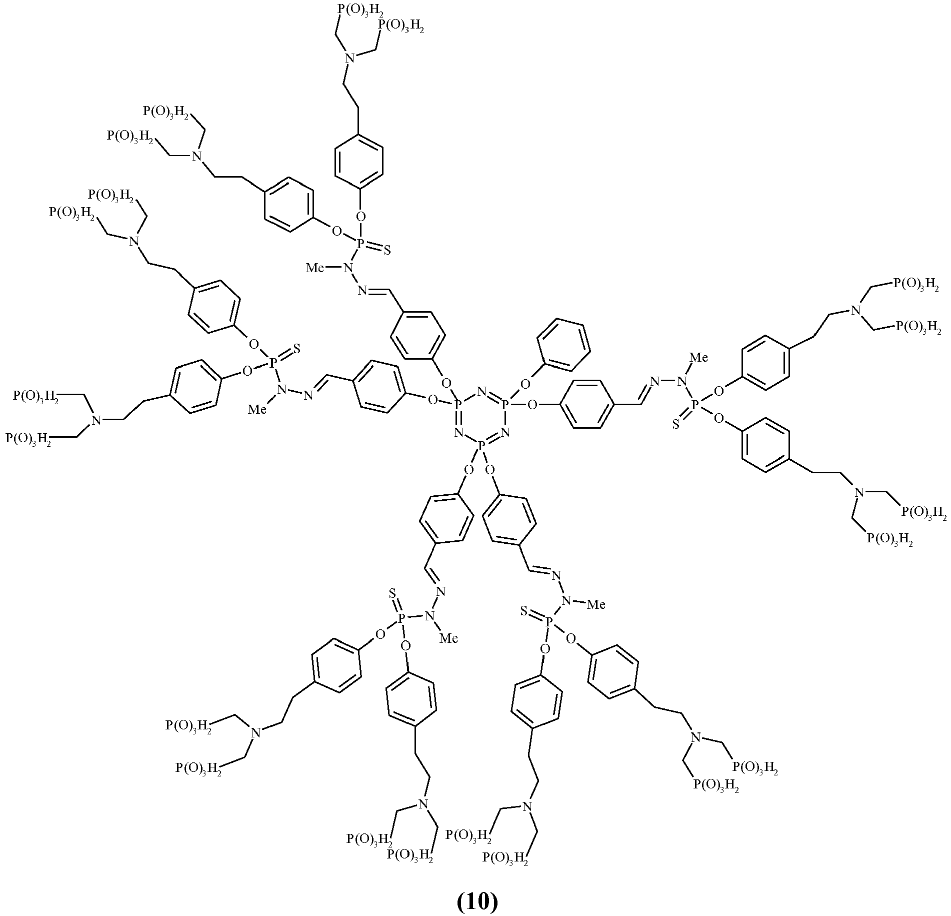 or of the formula (**10a**) 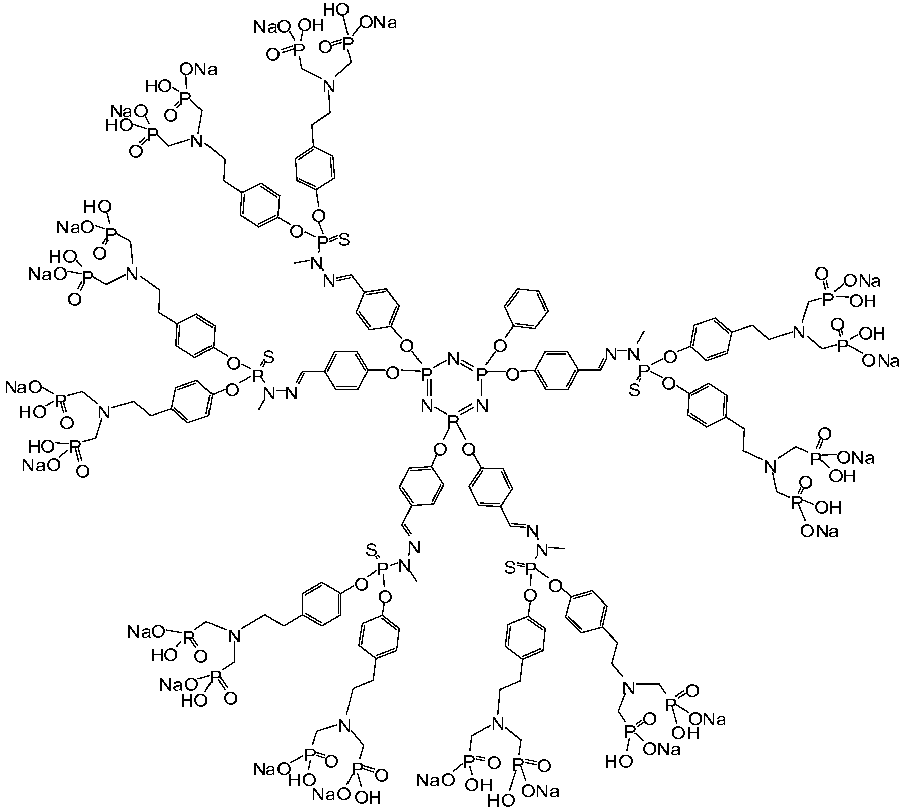
- or compounds of the following formula: 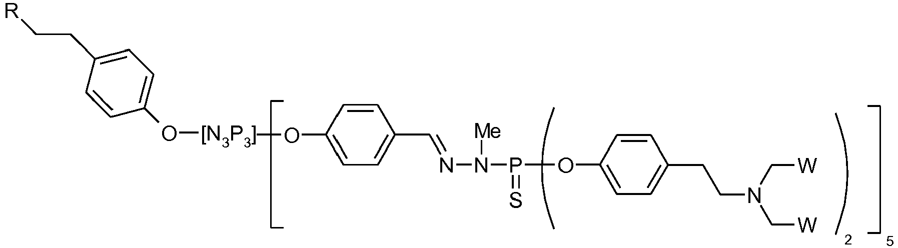 in which W represents PO₃Me₂, PO₃HNa, or PO₃H₂ and R is selected from the group comprising (a) fluorescent groups chosen from: 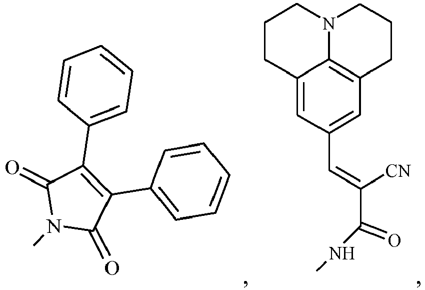 and (b) a biotinyl group of formula 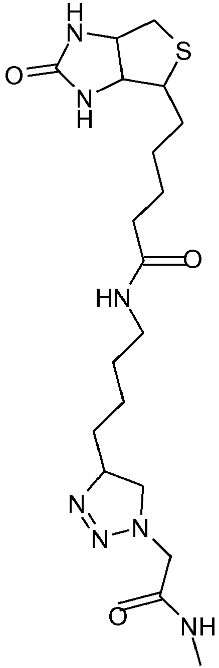 said compounds corresponding in particular to the compounds of the following formulae :

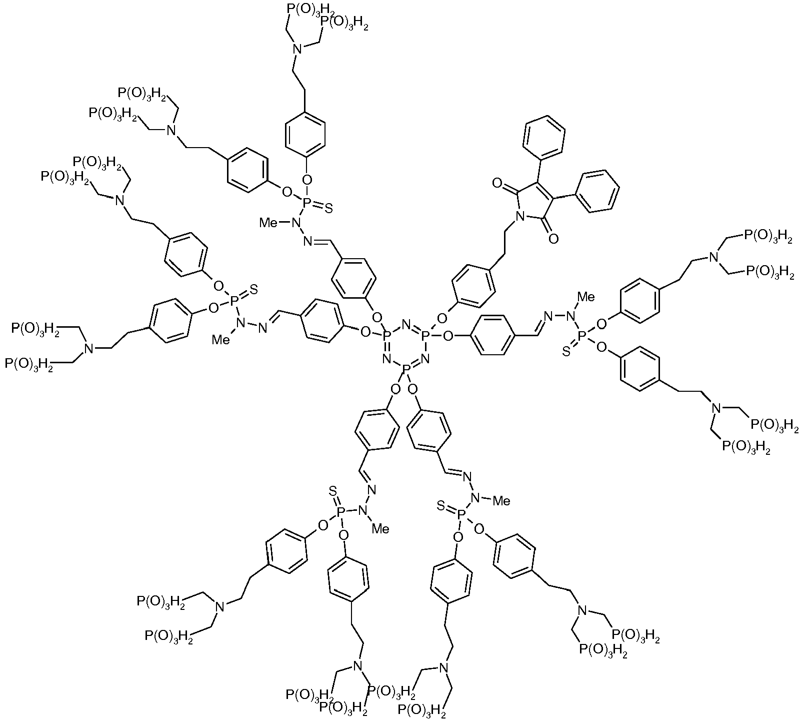

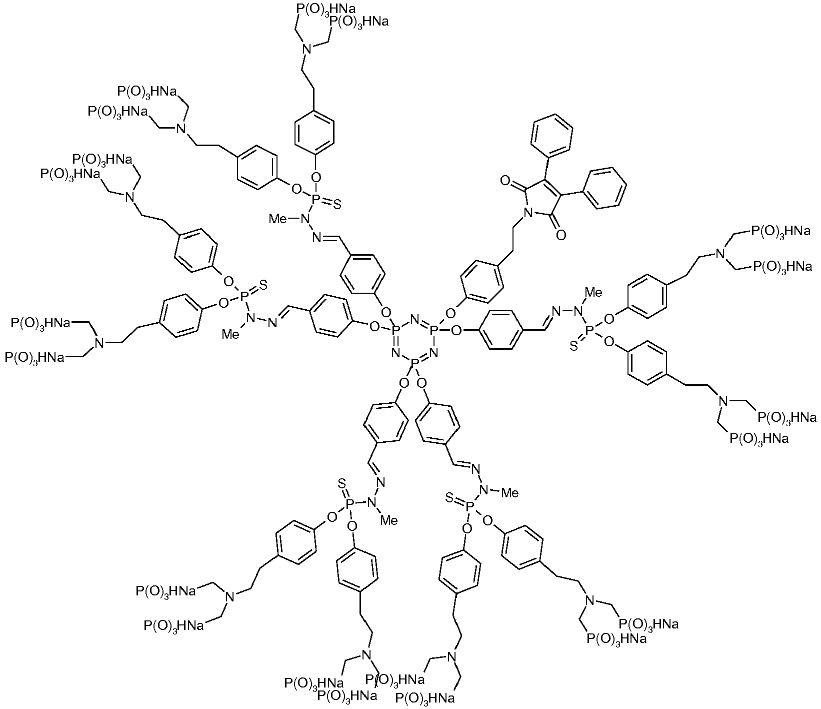

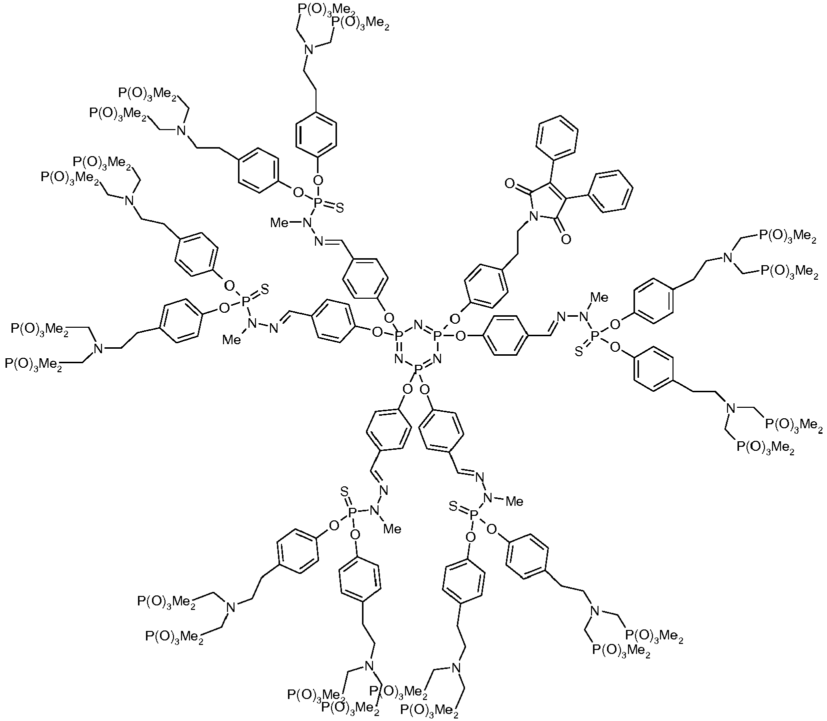

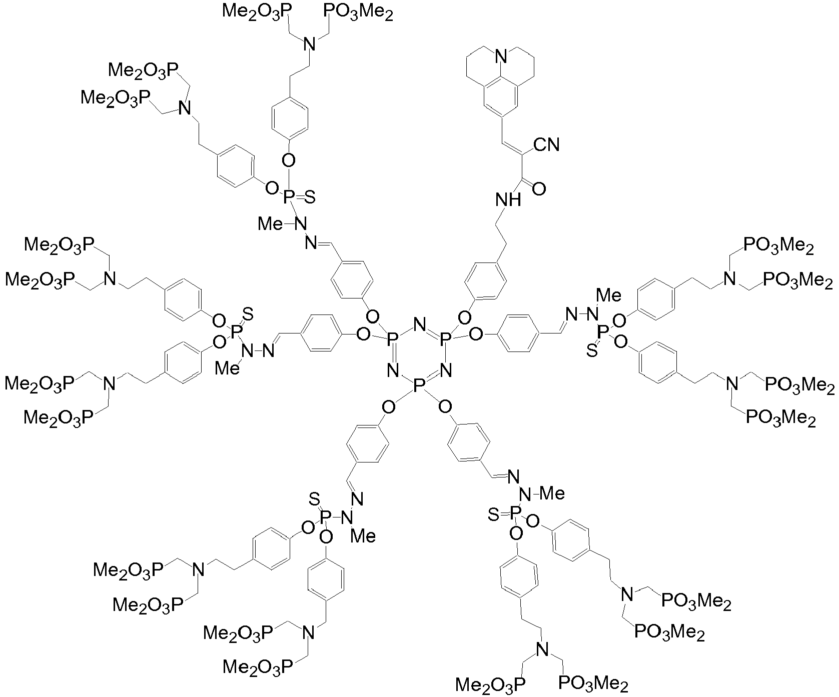

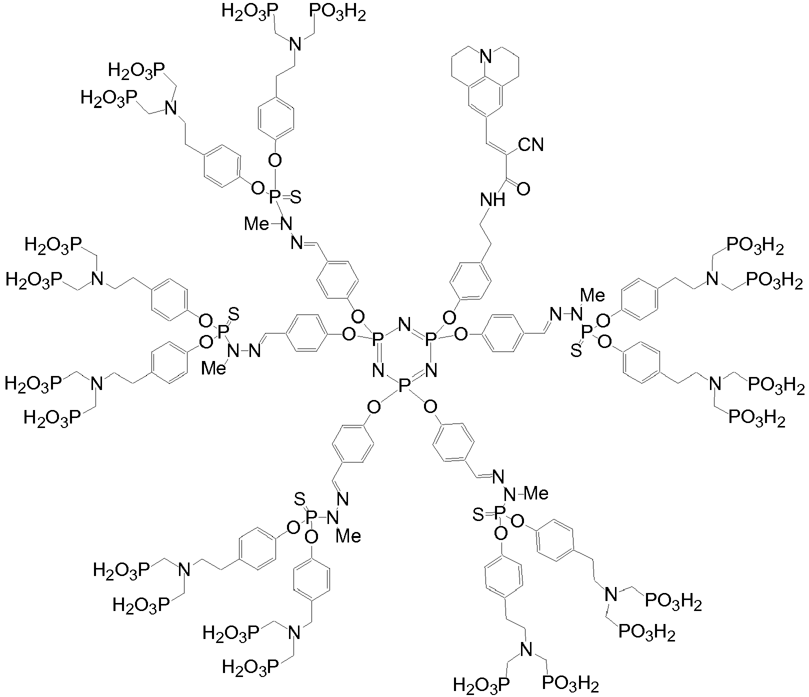

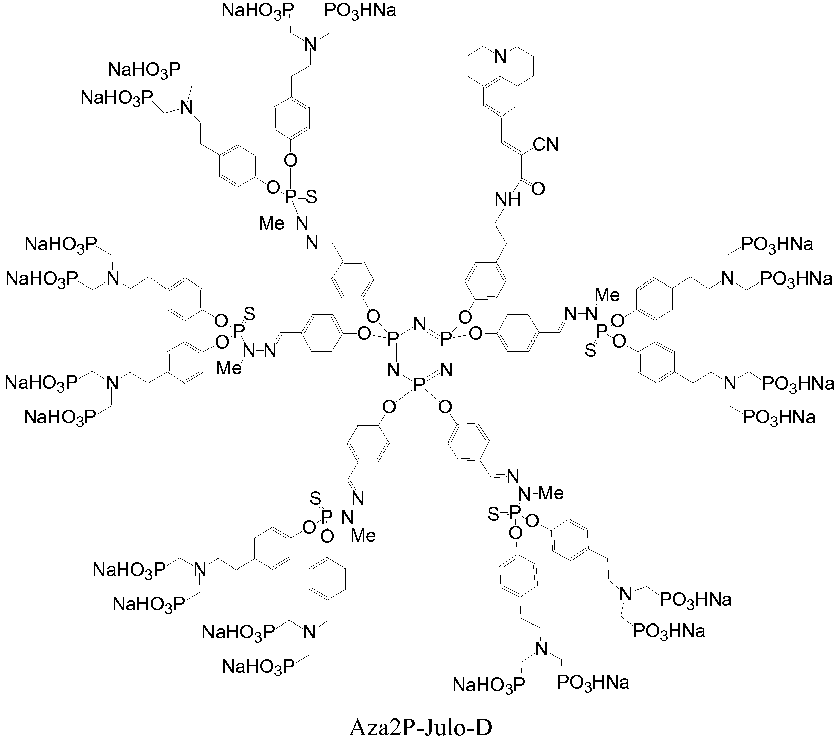

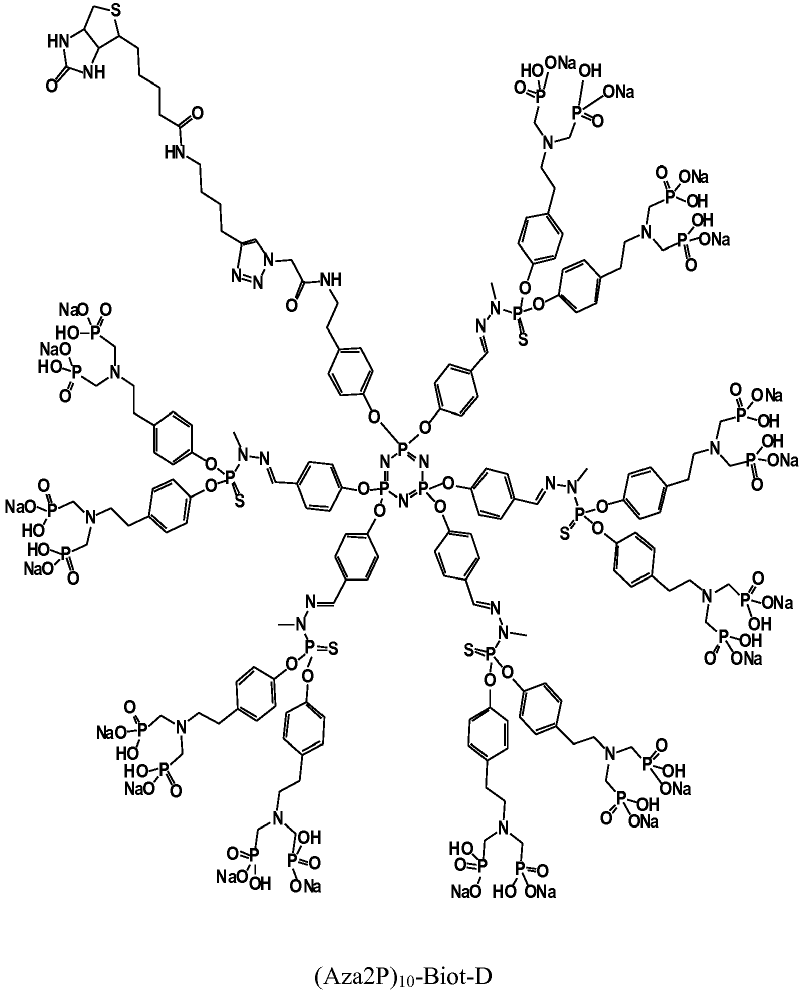

4. Dendrimers for use according to claim 1 selected from the group comprising:
A) 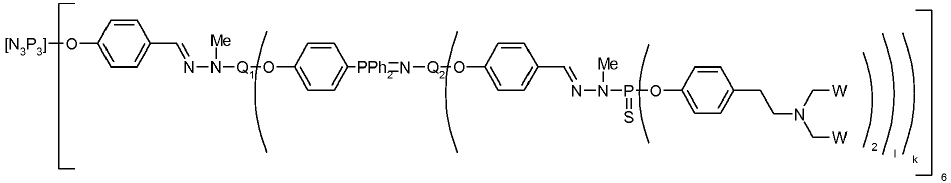 in which W represents PO₃Me₂, PO₃HNa, or PO₃H₂, Q₁ and Q₂, identical or different, represent P=S or cyclotriphosphazene (N₃P₃), 1 represents 2 when Q₂ represents P=S or 5 when Q₂ represents N₃P₃ and k represents 2 when Q₁ represents P=S or 5 when Q₁ represents N₃P₃, said dendrimers being in particular represented by the following formulae: 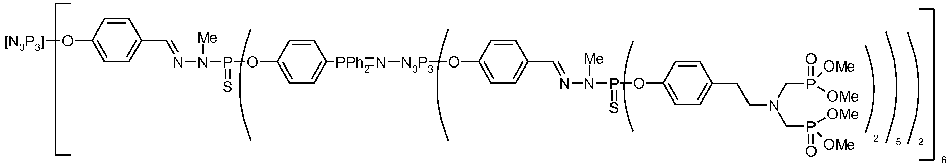 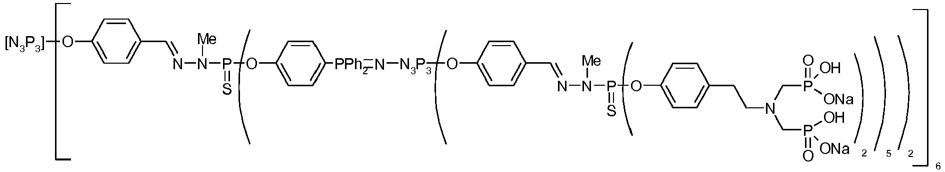 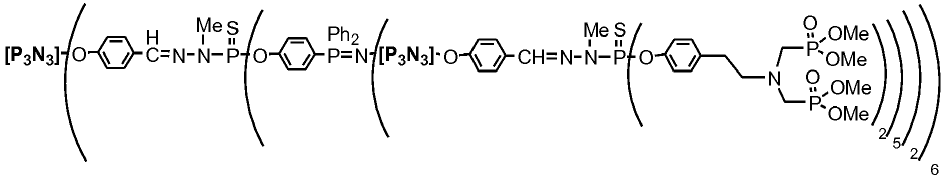 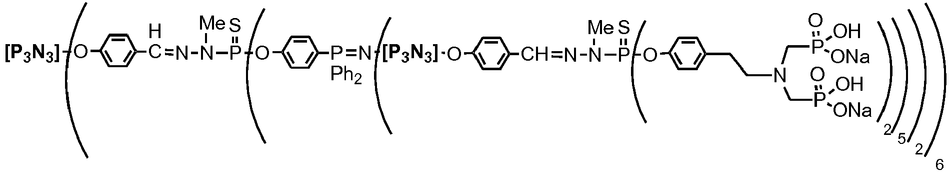 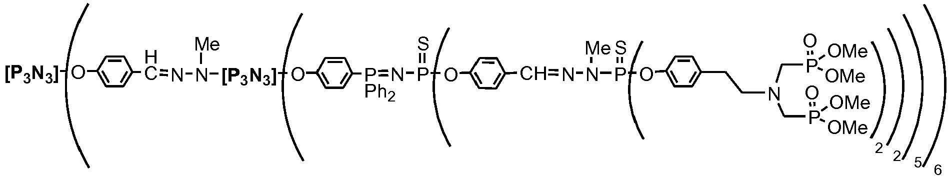 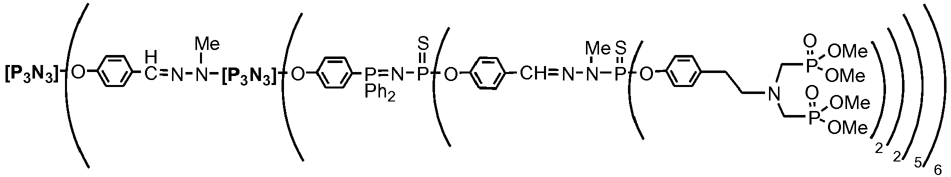 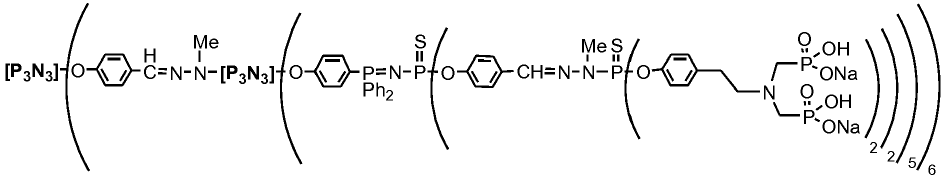 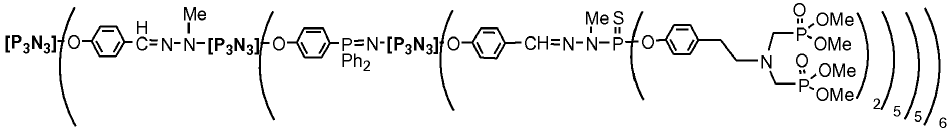 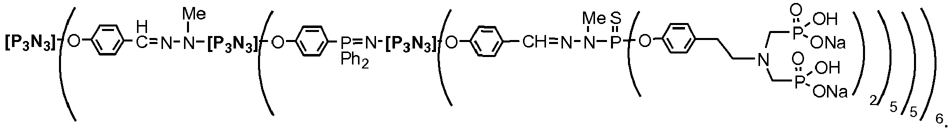
B) 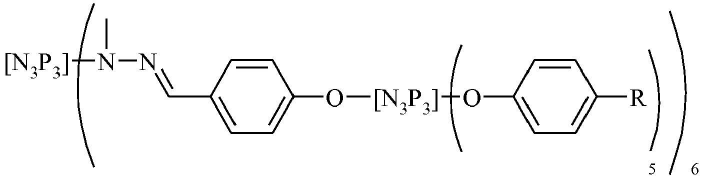 in which R represents a group chosen from 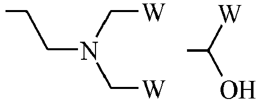 where W represents PO₃Me₂, PO₃HNa, or PO₃H₂.
C) 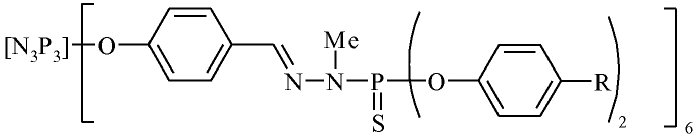 in which R represents a group chosen from: 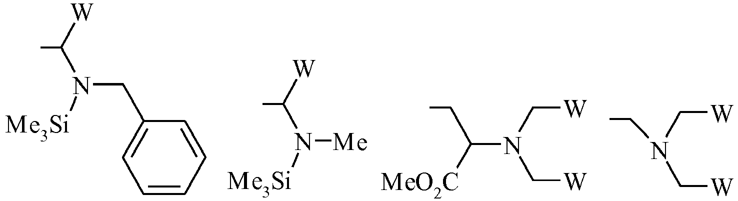 where W represents PO₃Si₂Me₆, PO₃Me₂, PO₃HNa, or PO₃H₂.
D) 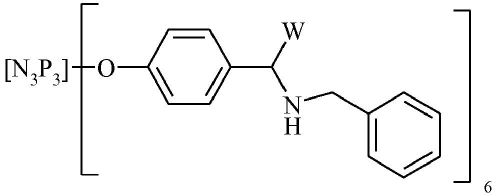 in which W represents PO₃Me₂, PO₃HNa, or PO₃H₂.H)

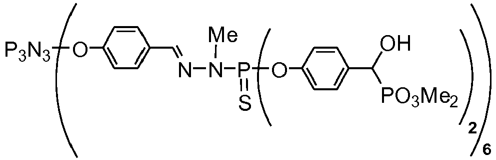

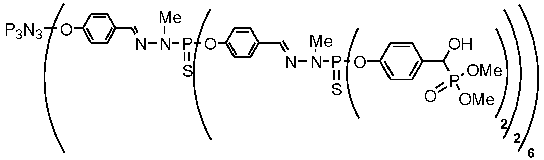

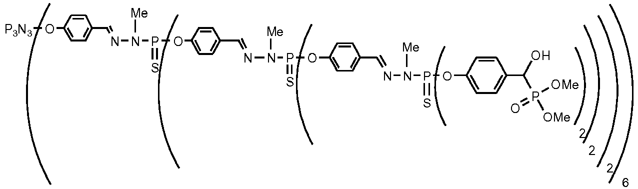

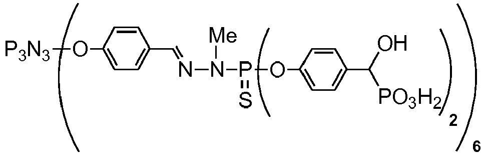

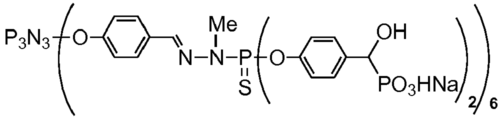

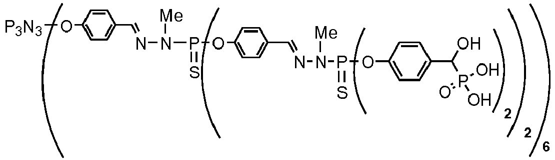

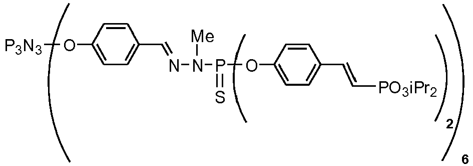

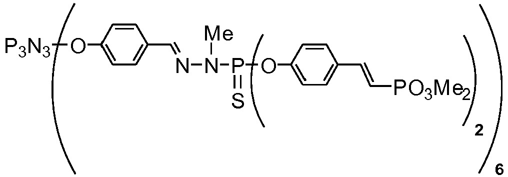

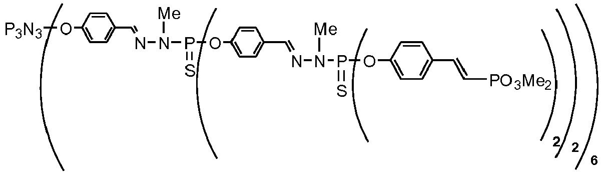

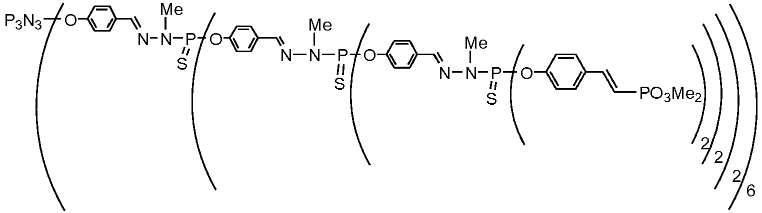

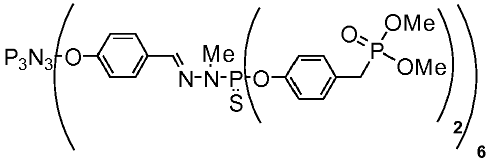

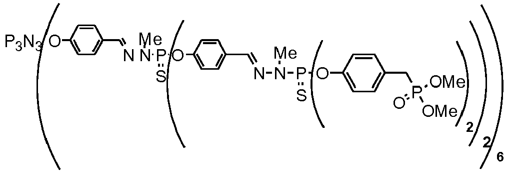

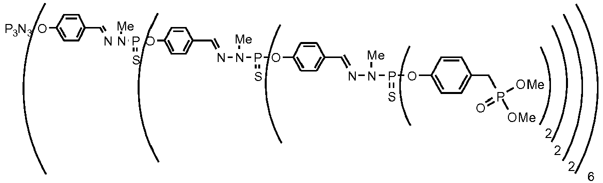

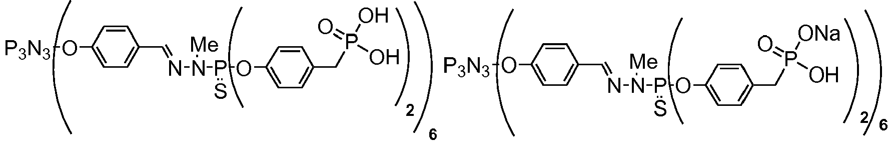

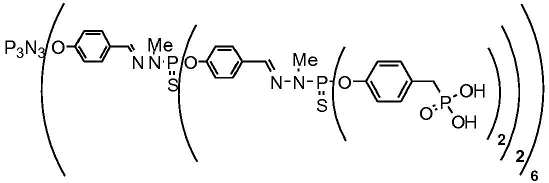

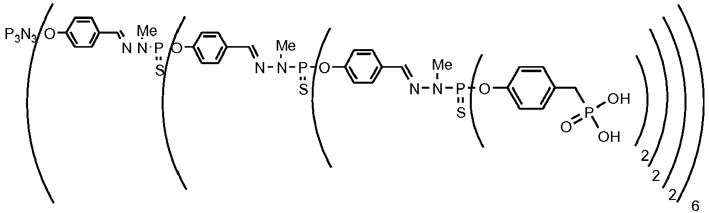

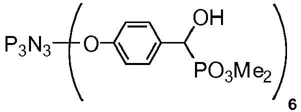

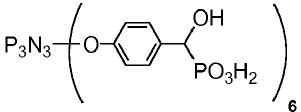

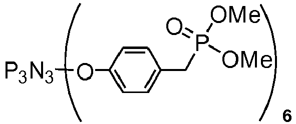

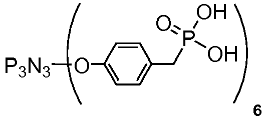

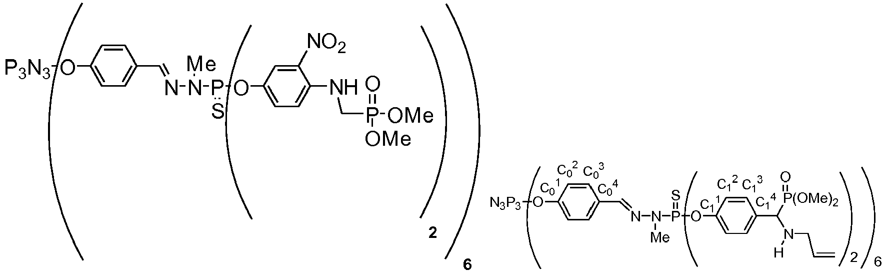

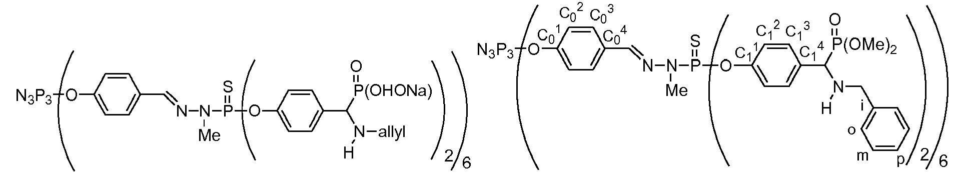

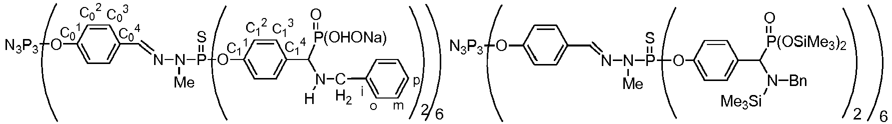

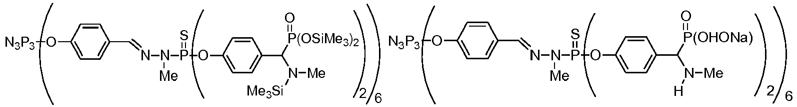

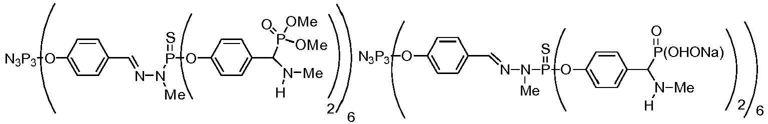

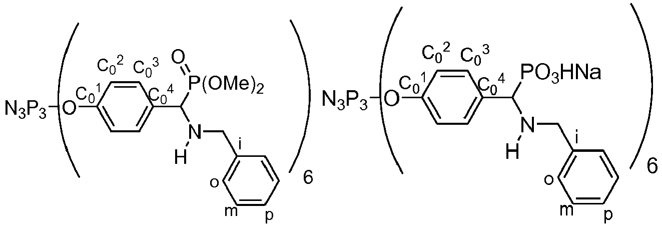

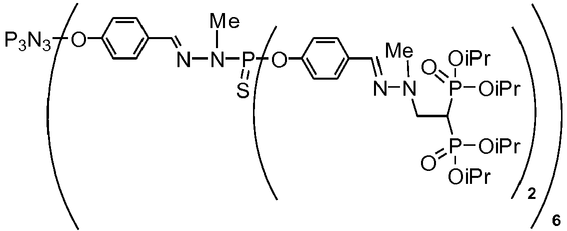

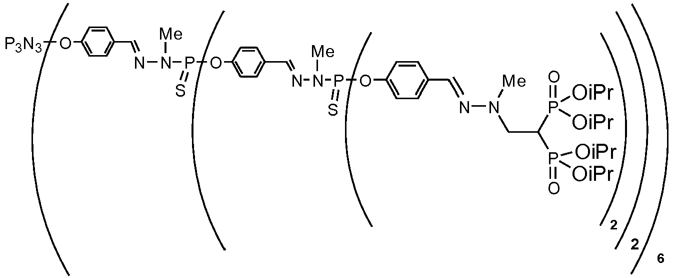

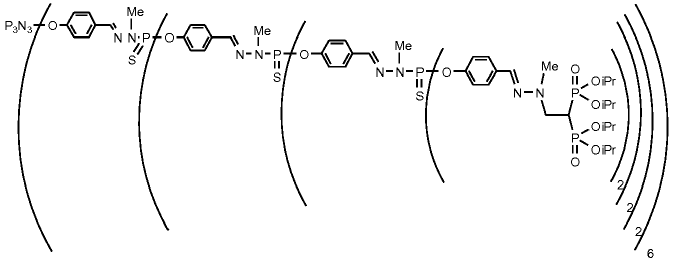

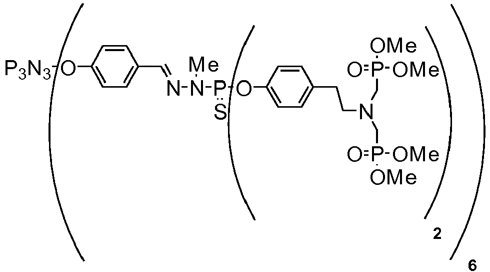

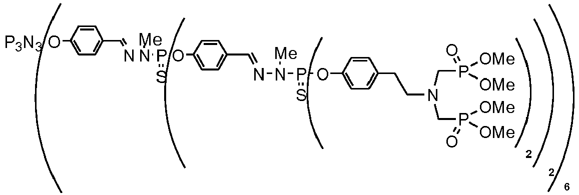

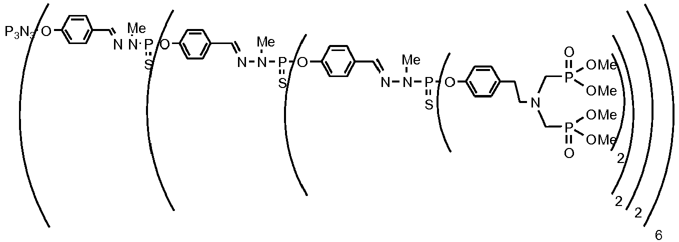

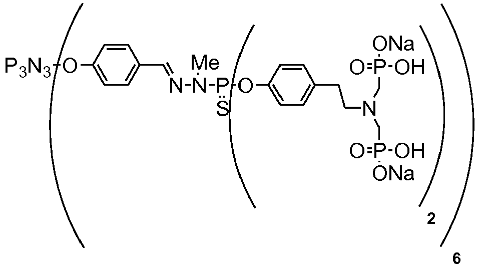

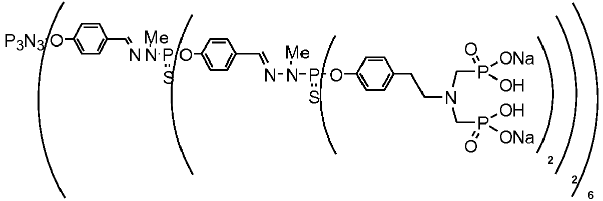

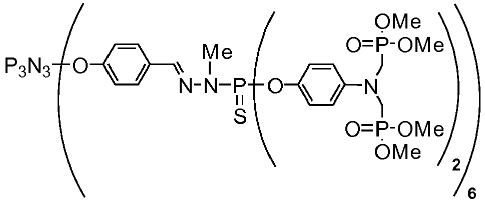

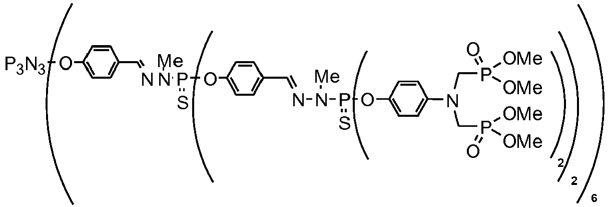

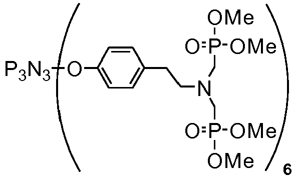

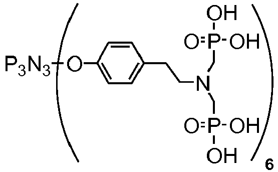

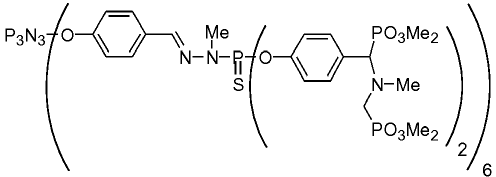

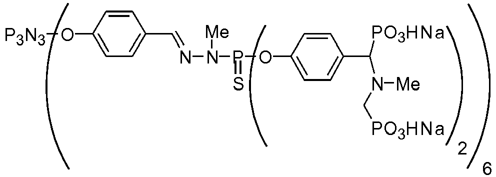

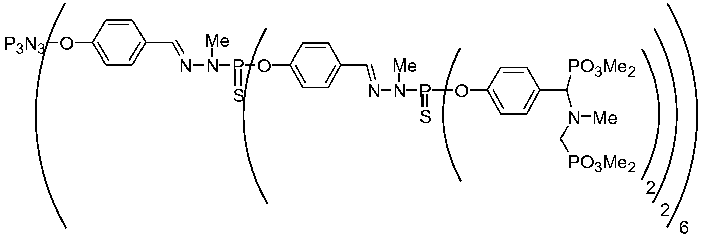

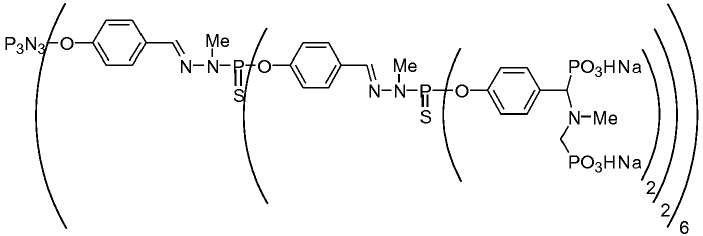

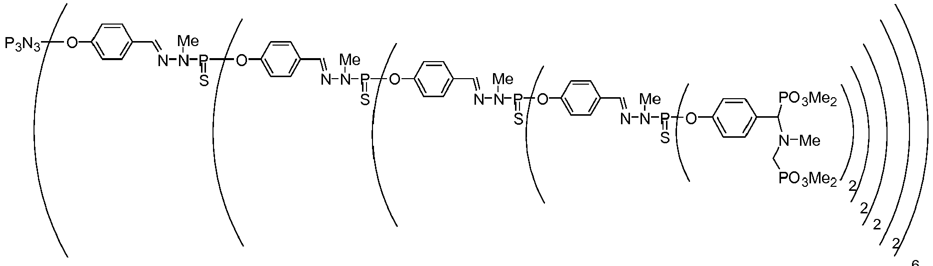

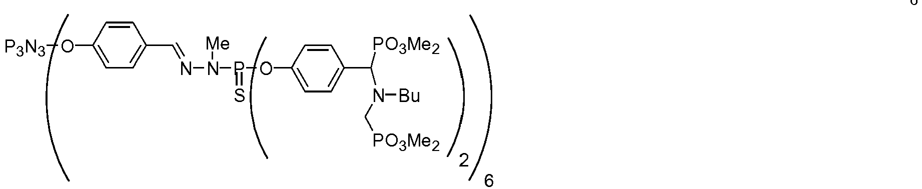

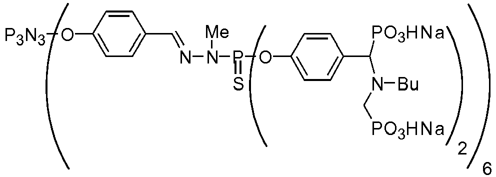

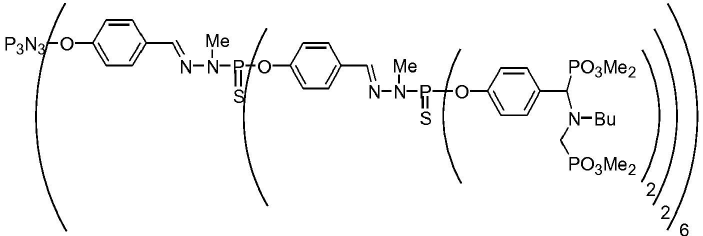

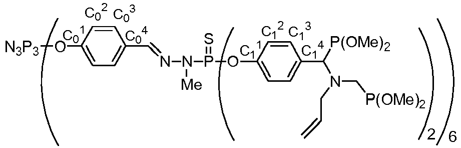

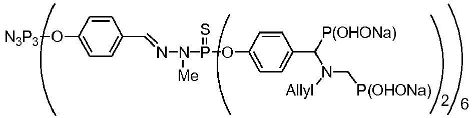

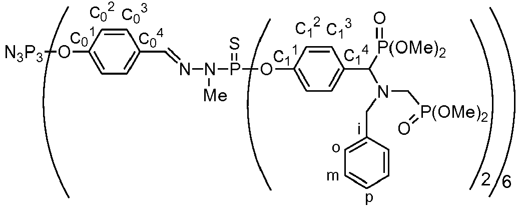

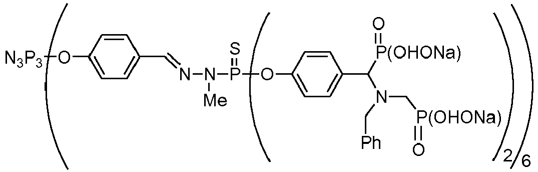

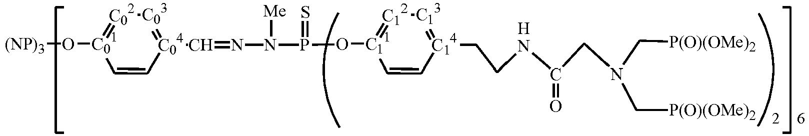

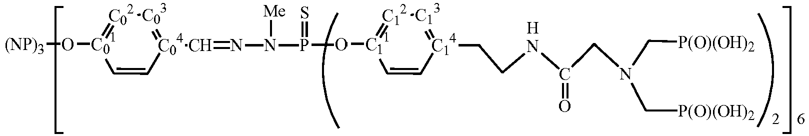

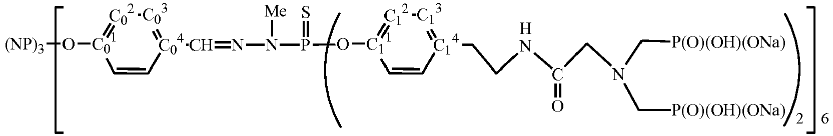

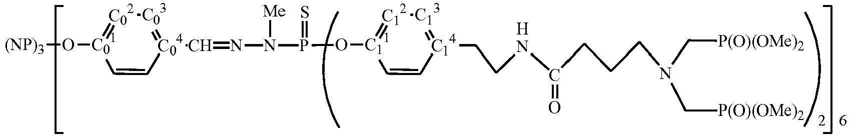

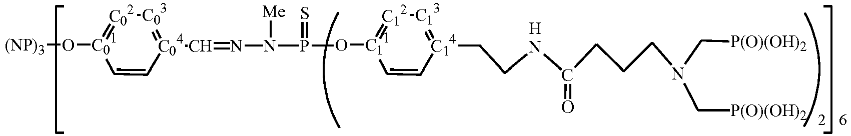

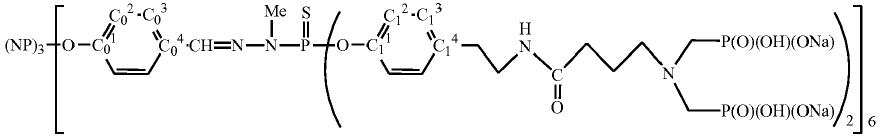

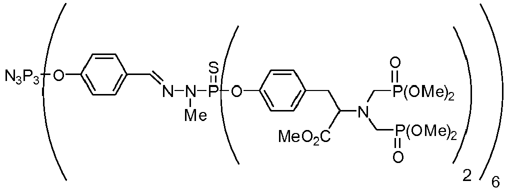

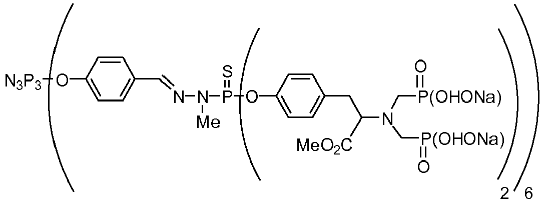

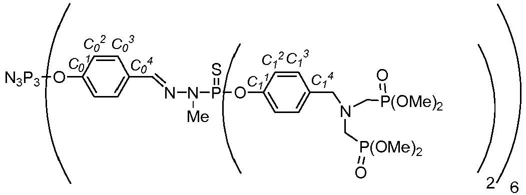

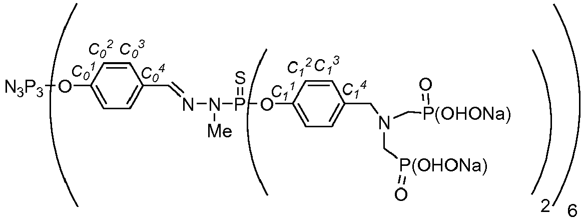

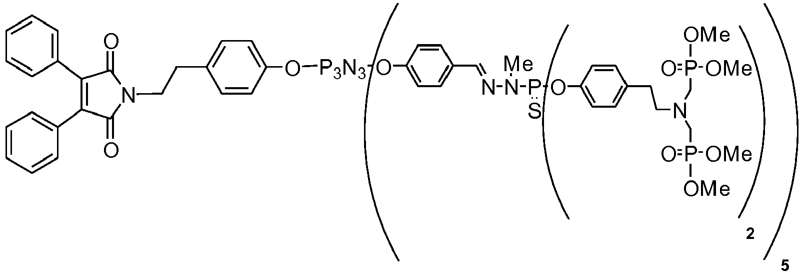

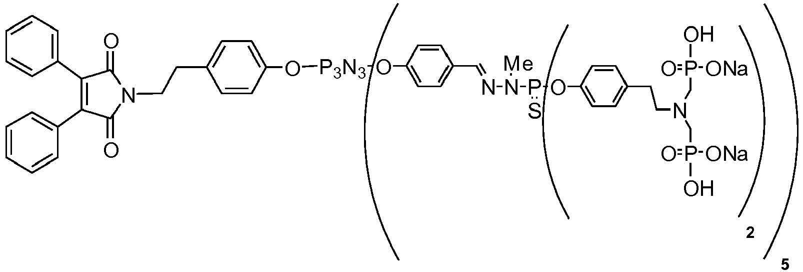

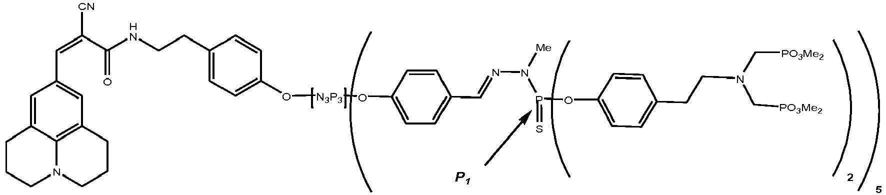

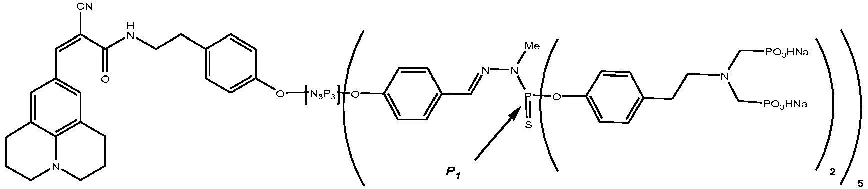

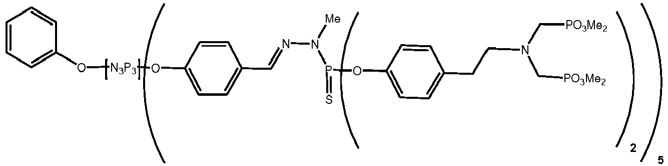

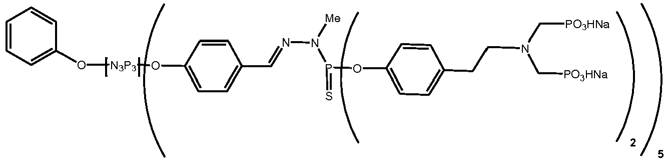

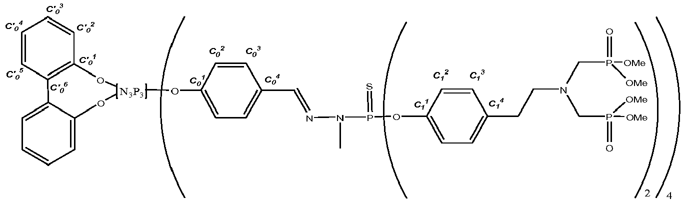

5. Use of dendrimers with biphosphonic terminations corresponding to the following general formula (1a) as defined in claim 1, for the preparation of drugs useful for the treatment of chronic inflammatory diseases.

6. Dendrimers for use according to claim 1 wherein the chronic inflammatory disease is selected from the group comprising rheumatoid arthritis, psoriasis and juvenile idiotypical arthritis.

7. Pharmaceutical compositions containing as active substance at least one dendrimer with monophosphonic or bisphosphonic terminations according to any of claims 1 to 4 associated with a pharmaceutically acceptable carrier for use in the treatment of chronic inflammatory diseases.

## Patentansprüche

1. Dendrimere mit monophosphonischen oder bisphosphonischen Terminierungen zur Verwendung bei der Behandlung chronischer entzündlicher Erkrankungen und chronischer entzündlicher Erkrankungen autoimmunen Ursprungs, entsprechend der folgenden allgemeinen Formel (la): 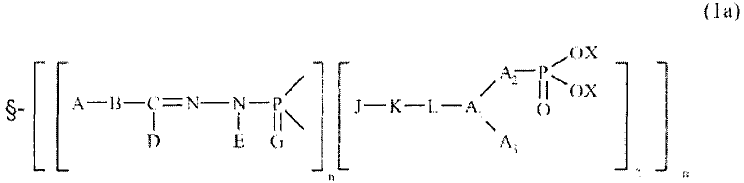 wobei n eine ganze Zahl von 0 bis 3 darstellt, ausgewählt aus der Gruppe umfassend die Verbindungen, wo
- wenn n = 0, die Formel (1a) der folgenden Formel (2a) entspricht, 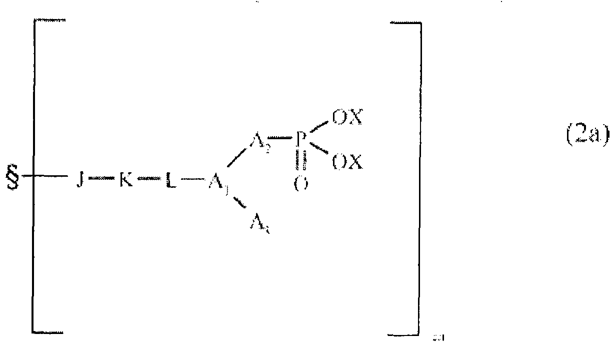
- wenn n = 1, die Formel (la) der folgenden Formel (3a) entspricht, 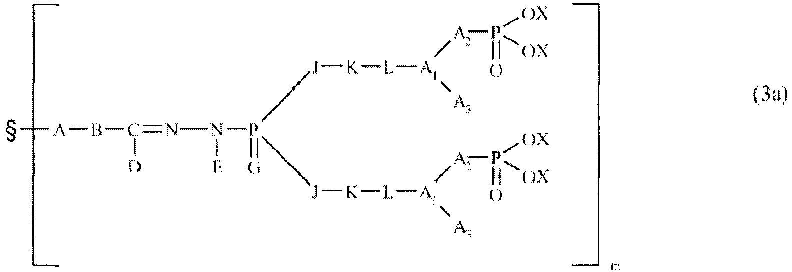
- wenn n = 2, die Formel (la) der folgenden Formel (4a) entspricht, 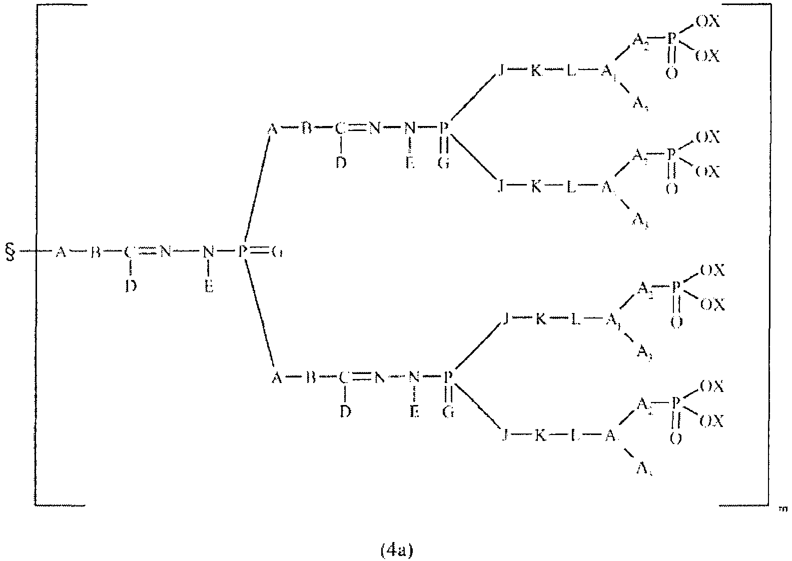
- und wenn n = 3, die Formel (la) der folgenden Formel (5a), 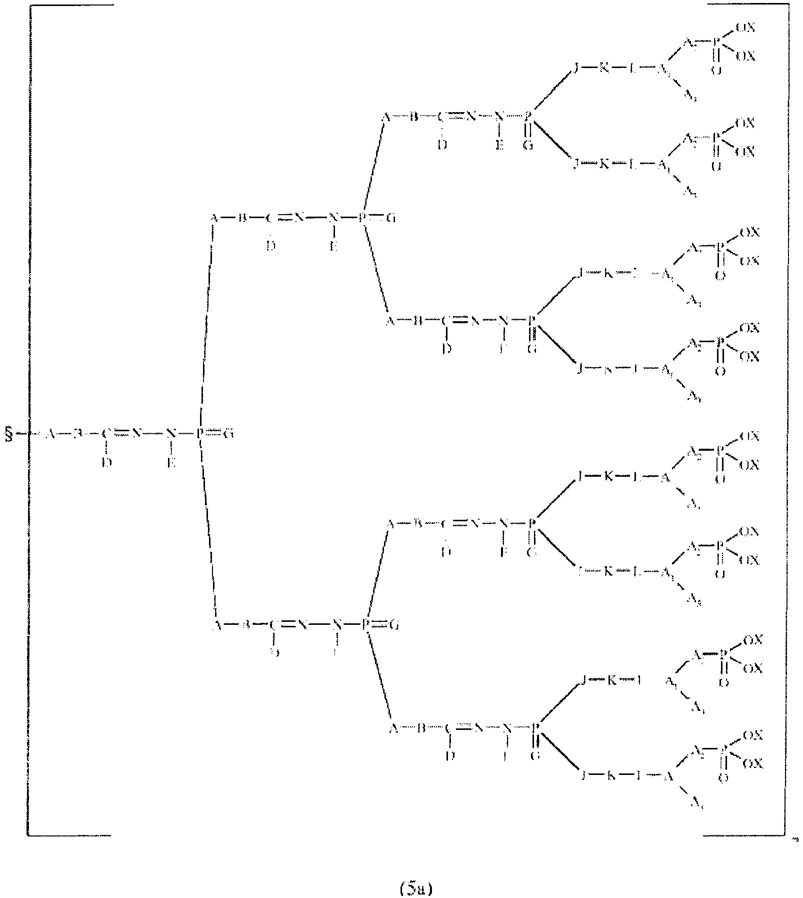 oder der Formel (7) entspricht 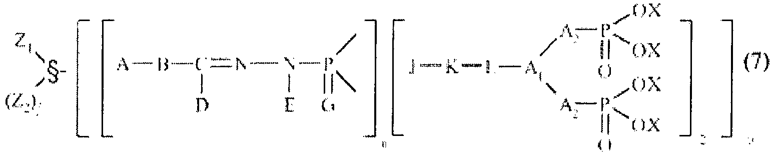 wobei
n eine ganze Zahl von 0 bis 3 darstellt, m 6 darstellt, p m - 1 oder m - 2 darstellt und j 0, wenn p m - 1 darstellt, und 1 darstellt, wenn p m - 2 darstellt, ausgewählt aus der Gruppe umfassend die Verbindungen, wobei
- wenn p = m - 1, die Formel (7) der folgenden Formel (8) entspricht: 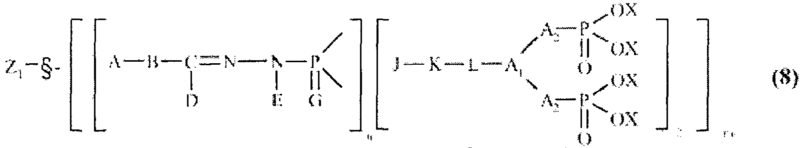
- wenn p = m 2, die Formel (7) der folgenden Formel (9) entspricht: 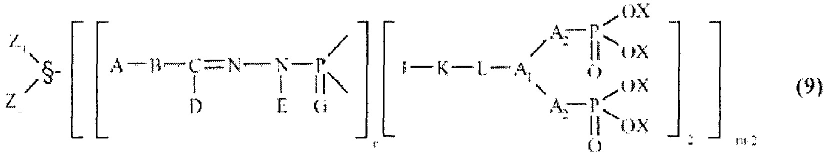 wobei der
• mittige Kern § 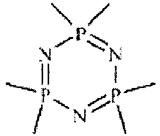 ist,
• m 6 darstellt;
• die Generationskette der Formel entspricht: 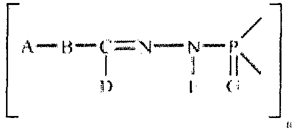 wobei
- A ein Sauerstoff-, Schwefel-, Phosphoratom oder eine - NR-Gruppe darstellt;
- B Folgendes darstellt: eine Arylgruppe von 6 bis 24 Kohlenstoffatomen, eine Heteroarylgruppe von 1 bis 24 Kohlenstoffatomen, eine Alkylgruppe von 1 bis 16 Kohlenstoffatomen, eine Oligoethylenglycolgruppe, eine Polyethylenglycolgruppe, zwei Arylgruppen von 6 bis 24 Kohlenstoffatomen, die durch einen Sauerstoff, einen Stickstoff, einen Schwefel, eine Alkylgruppe von 1 bis 16 Kohlenstoffatomen, eine Heteroalkylgruppe von 1 bis 24 Kohlenstoffatomen, eine Oligoethylenglycolgruppe verknüpft sind, wobei jede in der Lage ist, wahlweise durch ein Halogenatom oder eine NO₂-, -NRR'-, -CN-, - CF₃-, -OH-Gruppe, eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, substituiert zu sein;
- D Folgendes darstellt: ein Wasserstoffatom, eine Alkylgruppe von 1 bis 16 Kohlenstoffatomen, eine Alkoxygruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis zwölf Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, wobei jede in der Lage ist, wahlweise durch ein Halogenatom oder eine NO₂-, -NRR'-, -CN-, -CF₃-, - OH-Gruppe, eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, substituiert zu sein;
- E Folgendes darstellt: ein Wasserstoffatom, eine Alkylgruppe von 1 bis 16 Kohlenstoffatomen, eine Alkoxygruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, wobei jede in der Lage ist, wahlweise durch ein Halogenatom oder eine NO₂-, -NRR'-, -CN-, -CF₃-, - OH-Gruppe, eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, substituiert zu sein;
- G Folgendes darstellt: ein Sauerstoff-, Stickstoff-, Schwefel-, Selen-, Telluratom oder eine =NR-Gruppe;
R und R' unabhängig voneinander H oder eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycoleinheiten umfasst, eine Polyethylenglycolgruppe, die ein Molekulargewicht bevorzugt im Bereich von 300 g.Mol⁻¹ bis 3000 g.Mol⁻¹ aufweist, darstellen;
• die Zwischenproduktkette der Formel:
-J-K-L-
entspricht,
wobei
- J ein Sauerstoffatom, ein Schwefelatom oder eine -NR-Gruppe darstellt;
- K Folgendes darstellt: eine Arylgruppe von 6 bis 24 Kohlenstoffatomen, eine Heteroarylgruppe von 1 bis 24 Kohlenstoffatomen, wobei das Heteroelement bevorzugt unter Sauerstoff, Stickstoff oder Schwefel ausgewählt ist, eine Alkylgruppe von 1 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, wobei jede in der Lage ist, wahlweise durch ein Halogenatom oder eine NO₂-, -NRR'-, -CN-, -CF₃-,-OH-Gruppe, eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, substituiert zu sein;
- L Folgendes darstellt: eine lineare, verzweigte oder cyclische Kohlenwasserstoffkette mit 0 bis 10 Teilnehmersubstanzen, die wahlweise eine oder mehrere Doppel- oder Dreifachbindungen enthalten, wobei jede der Teilnehmersubstanzen wahlweise ein Heteroatom sein kann, wobei das Heteroatom bevorzugt unter Sauerstoff, Schwefel, Stickstoff, Phosphor, Silicium ausgewählt ist, wobei jede Teilnehmersubstanz in der Lage ist, wahlweise durch mindestens einen Substituenten substituiert zu sein, der unter einer Alkylgruppe von 1 bis 16 Kohlenstoffatomen, einem Halogen, einem Sauerstoffatom, NO₂-, -NRR'-, -CN-, -CF₃-, -OH, einer Alkoxygruppe von 1 bis 16 Kohlenstoffatomen, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, ausgewählt ist;
R und R' unabhängig voneinander H oder eine Alkylgruppe von 1 bis 16 Kohlenstoffatomen, eine Arylgruppe von 6 bis 24 Kohlenstoffatome, eine Aralkylgruppe von 7 bis 16 Kohlenstoffatomen, eine Oligoethylenglycolgruppe, die bevorzugt 1 bis 12 Ethylenglycoleinheiten umfasst, eine Polyethylenglycolgruppe, die ein Molekulargewicht bevorzugt im Bereich von 300 g.Mol⁻¹ bis 3000 g.Mol⁻¹ aufweist, darstellen;
• die endständige Gruppe der Formel: 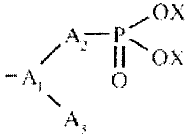 entspricht, wobei
A₁ Folgendes darstellt: N; eine P=Y-Gruppe, wobei Y O, S oder kein Atom darstellt; eine N-R-Gruppe; oder eine C-R-Gruppe; R Folgendes darstellt: H oder eine lineare, verzweigte oder cyclische Kohlenwasserstoffkette mit 1 bis 16 Teilnehmersubstanzen, die wahlweise ein oder mehrere Heteroatome enthalten, wobei die Heteroatome bevorzugt unter Sauerstoff, Schwefel, Stickstoff, Phosphor, Silicium und/oder einer oder mehreren Doppel- oder Dreifachbindungen ausgewählt werden, wobei jede der Teilnehmersubstanzen wahlweise durch mindestens einen Substituenten substituiert ist, der unter einer Hydroxylgruppe, einer -NR'R"-Gruppe, einer Alkoxygruppe von 1 bis 16 Kohlenstoffatomen, einer Alkylgruppe von 1 bis 16 Kohlenstoffatomen, einem Halogenatom, einer - NO₂-Gruppe, einer -CN-Gruppe, einer -CF₃-Gruppe, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, ausgewählt ist, R' und R" unabhängig voneinander Folgendes darstellen: H oder eine Alkylgruppe von 1 bis 16 Kohlenstoffatomen, eine Arylgruppe von 6 bis 24 Kohlenstoffatomen, eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist;
A₂ Folgendes darstellt: eine Einfachbindung oder eine lineare, verzweigte oder cyclische Kohlenwasserstoffkette mit 1 bis 6 Teilnehmersubstanzen darstellt, wobei jede der Teilnehmersubstanzen wahlweise ein oder mehrere Heteroatome enthält, wobei die Heteroatome bevorzugt unter einem Schwefel-, Sauerstoff-, Phosphor- oder Stickstoffatom, noch bevorzugter Stickstoff ausgewählt werden und wahlweise durch mindestens einen Substituenten substituiert sind ausgewählt unter H, einer Alkylgruppe von 1 bis 16 Kohlenstoffatomen, einem Halogen, einer NO₂-Gruppe, einer NRR'-Gruppe, einer -CN-Gruppe, einer -CF₃-Gruppe, einer Hydroxylgruppe, einer Alkoxygruppe von 1 bis 16 Kohlenstoffatomen, einer Aryl- oder Heteroarylgruppe von 1 bis 24 Kohlenstoffatomen, wobei das Heteroelement bevorzugt unter Sauerstoff, Stickstoff oder Schwefel ausgewählt ist, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, R und R' unabhängig voneinander H oder eine lineare, verzweigte oder cyclische Kohlenwasserstoffkette mit 1 bis 16 Teilnehmersubstanzen darstellen, die wahlweise ein oder mehrere Heteroatome enthalten, wobei die Heteroatome bevorzugt unter Sauerstoff, Schwefel, Stickstoff, Phosphor, Silicium und/oder einer oder mehreren Doppel- oder Dreifachbindungen ausgewählt werden, wobei jede der Teilnehmersubstanzen wahlweise durch mindestens einen Substituenten substituiert ist, ausgewählt unter einer Hydroxylgruppe, einer NR"R'''-Gruppe, einer Alkoxygruppe von 1 bis 16 Kohlenstoffatomen, einer Alkylgruppe von 1 bis 16 Kohlenstoffatomen, einem Halogenatom, einer NO₂-Gruppe, einer -CN-Gruppe, einer -CF₃-Gruppe, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, R" und R''' unabhängig voneinander H oder eine Alkylgruppe von 1 bis 16 Kohlenstoffatomen, eine Arylgruppe von 6 bis 24 Kohlenstoffatomen oder eine Aralkylgruppe von 7 bis 16 Kohlenstoffatomen, eine Oligoethylenglycolgruppe, die bevorzugt 1 bis 12 Ethylenglycoleinheiten umfasst, eine Polyethylenglycolgruppe, die ein Molekulargewicht bevorzugt im Bereich von 300 g.Mol⁻¹ bis 3000 g.Mol⁻¹ aufweist, darstellen.
A₃ Folgendes darstellt: H oder eine lineare, verzweigte oder cyclische Kohlenwasserstoffkette mit 1 bis 16 Teilnehmersubstanzen, wobei jede der Teilnehmersubstanzen wahlweise von einem Heteroatom ausgewählt ist, wobei das Heteroatom bevorzugt unter Schwefel, Stickstoff, Phosphor oder Silicium, noch bevorzugter Stickstoff, ausgewählt ist, wobei jede Teilnehmersubstanz in der Lage ist, wahlweise durch mindestens eine Gruppe substituiert zu werden ausgewählt unter einer Hydroxylgruppe, einer NR"R'''-Gruppe, einer Alkoxygruppe von 1 bis 16 Kohlenstoffatomen, einer Alkylgruppe von 1 bis 16 Kohlenstoffatomen, einem Halogenatom, einer NO₂-Gruppe, einer -CN-Gruppe, einer -CF₃-Gruppe, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, R" und R''' unabhängig voneinander H oder eine Alkylgruppe von 1 bis 16 Kohlenstoffatomen, eine Arylgruppe von 6 bis 24 Kohlenstoffatomen oder eine Aralkylgruppe von 7 bis 16 Kohlenstoffatomen, eine Oligoethylenglycolgruppe, die bevorzugt 1 bis 12 Ethylenglycoleinheiten umfasst, eine Polyethylenglycolgruppe, 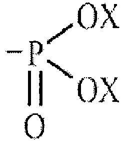 die ein Molekulargewicht bevorzugt im Bereich von 300 g.Mol⁻¹ bis 3000 g.Mol⁻¹ aufweist, darstellen oder
A₃ insbesondere 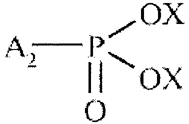 darstellen kann, wobei A₂ identisch oder verschieden sind;
jedes OX, die identisch oder bezüglich jeder Phosphongruppe verschieden sind, Folgendes darstellt: OH, Oalkyl, wobei die Alkylgruppe 1 bis 16 Kohlenstoffatome umfasst, Oaryl, wobei die Arylgruppe 6 bis 24 Kohlenstoffatome umfasst, Oaralkyl, wobei die Aralkylgruppe 7 bis 24 Kohlenstoffatome umfasst, Oalkylaryl, wobei die Alkylarylgruppe 7 bis 24 Kohlenstoffatome umfasst, OSiR'₁R'₂R'₃, wobei R'₁, R'₂ und R'₃, die identisch oder verschieden sind, Folgendes darstellen: eine Alkylgruppe von 1 bis 16 Kohlenstoffatomen oder O⁻M⁺, wobei M⁺ ein Kation von Elementen der Gruppe IA, IB, IIA, IIB oder IIIA, IIIB der Periodentabelle der Elemente ist, M⁺ bevorzugt unter Kationen der Natrium-, Kalium-, Kupfer-, Calcium-, Barium-, Zink-, Magnesium-, Lithium- und Aluminiumatome ausgewählt ist, oder Kohlenwasserstoffgruppen von 1 bis 100 Kohlenstoffatomen oder stickstoffhaltigen Gruppen von 0 bis 100 Kohlenstoffatomen, wie NR₁R₂R₃R₄⁺, wobei, unabhängig voneinander, R₁, R₂, R₃ und R₄ H oder eine lineare, verzweigte oder cyclische Kohlenwasserstoffkette mit 1 bis 16 Teilnehmersubstanzen, die wahlweise ein oder mehrere Heteroatome enthalten, darstellen, wobei die Heteroatome bevorzugt unter Sauerstoff, Schwefel, Stickstoff, Phosphor, Silicium und/oder einer oder mehreren Doppel- oder Dreifachbindungen ausgewählt werden, wobei jede der Teilnehmersubstanzen wahlweise durch mindestens einen Substituenten substituiert ist, der unter einer Hydroxylgruppe, einer -NR"R'''-Gruppe, einer Alkoxygruppe von 1 bis 16 Kohlenstoffatomen, einer Alkylgruppe von 1 bis 16 Kohlenstoffatomen, einem Halogenatom, einer -NO₂-Gruppe, einer -CN-Gruppe, einer -CF₃-Gruppe, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, ausgewählt ist, R" und R''' unabhängig voneinander Folgendes darstellen: H oder eine Alkylgruppe von 1 bis 16 Kohlenstoffatomen, eine Arylgruppe von 6 bis 24 Kohlenstoffatomen oder eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist,
wobei Z₁ und Z₂, die identisch oder verschieden sind, wahlweise, insbesondere durch eine kovalente Bindung, verknüpft sind und Folgendes darstellen: H oder eine lineare, verzweigte oder cyclische Kohlenwasserstoffkette mit 1 bis 16 Teilnehmersubstanzen, die wahlweise eine oder mehrere Doppel- oder Dreifachbindungen enthalten, wobei jede der Teilnehmersubstanzen wahlweise von einem Heteroatom ausgewählt ist, wobei das Heteroatom bevorzugt unter einem Stickstoff-, Sauerstoff-, Phosphor-, Silicium- oder Schwefelatom, einer Arylgruppe von 6 bis 24 Kohlenstoffatomen, einer Heteroarylgruppe von 1 bis 24 Kohlenstoffatomen, einer Carboxylgruppe, einer >C=NR-gruppe, wobei jede Teilnehmersubstanz in der Lage ist, wahlweise durch mindestens einen Substituenten substituiert zu werden, der unter einer Hydroxylgruppe, einer NR"R'''-Gruppe, einer Alkoxygruppe von 1 bis 16 Kohlenstoffatomen, einer Alkylgruppe von 1 bis 16 Kohlenstoffatomen, einem Halogenatom, einer -NO₂-Gruppe, einer -CN-Gruppe, einer -CF₃-Gruppe, einer Arylgruppe von 6 bis 24 Kohlenstoffatomen, einer Aralkylgruppe von 7 bis 16 Kohlenstoffatomen, einer Oligoethylenglycolgruppe, die bevorzugt 1 bis 12 Ethylenglycolanteile umfasst, einer Polyethylenglycolgruppe, die ein Molekulargewicht bevorzugt im Bereich von 300 g.Mol⁻¹ bis 3000 g.Mol⁻¹ aufweist, ausgewählt ist, R" und R''' unabhängig voneinander Folgendes darstellen: H oder eine Alkylgruppe von 1 bis 16 Kohlenstoffatomen, eine Arylgruppe von 6 bis 24 Kohlenstoffatomen oder eine Aralkylgruppe von 7 bis 16 Kohlenstoffatomen, eine Oligoethylenglycolgruppe, die bevorzugt 1 bis 12 Ethylenglycolanteile umfasst, eine Polyethylenglycolgruppe, die ein Molekulargewicht bevorzugt im Bereich von 300 g.Mol⁻¹ bis 3000 g.Mol⁻¹ aufweist, wobei die erste Mitgliedersubstanz der Kohlenwasserstoffkette bevorzugt Sauerstoff oder Stickstoff ist.

2. Dendrimere zur Verwendung nach Anspruch 1 entsprechend der allgemeinen Formel: 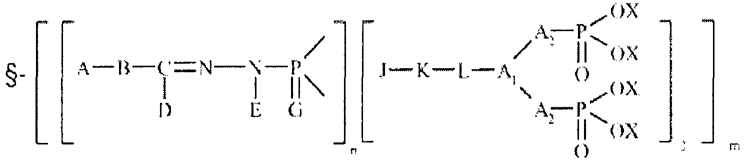 oder 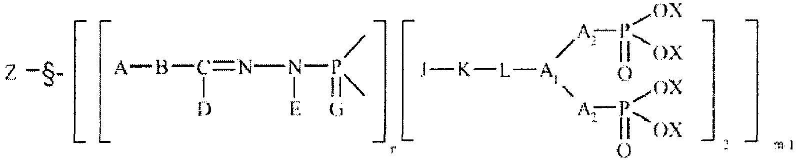 wobei
§ 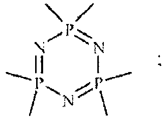 darstellt;
m 6 darstellt;
n 0, 1 oder 2 darstellt;
A ein Sauerstoffatom darstellt;
B eine Benzolgruppe darstellt;
D Wasserstoff darstellt;
E eine Methylgruppe darstellt;
G ein Schwefelatom darstellt;
J ein Sauerstoffatom darstellt;
K eine Benzolgruppe darstellt;
L eine nichtsubstituierte, lineare, gesättigte Kohlenwasserstoffkette mit zwei Kohlenstoffatomen darstellt;
A₁ ein Stickstoffatom darstellt;
A₂ eine CH₂-Gruppe darstellt;
X eine Methylgruppe oder ein Wasserst
off- oder Natriumatom darstellt und
Z₁ eine Phenyloxygruppe darstellt.

3. Dendrimere nach einem der Ansprüche 1 oder 2, ausgewählt aus der Gruppe umfassend: 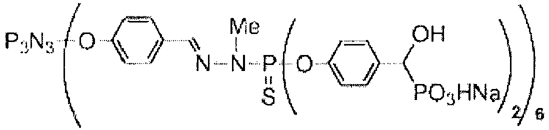 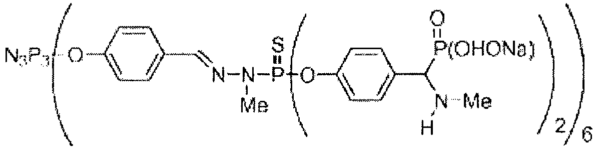 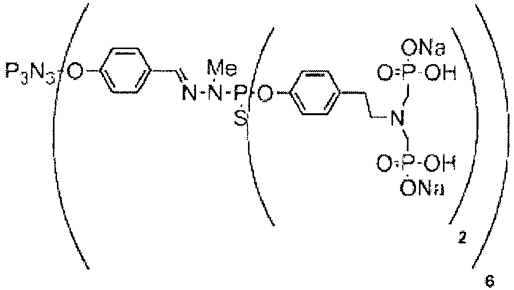 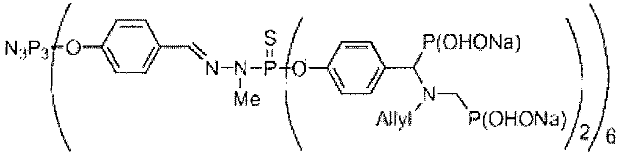 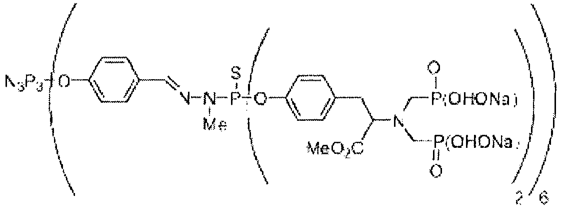 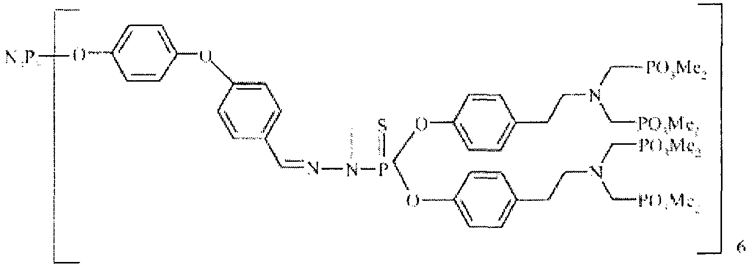 oder von GC1-Verbindungen der folgenden Formeln: 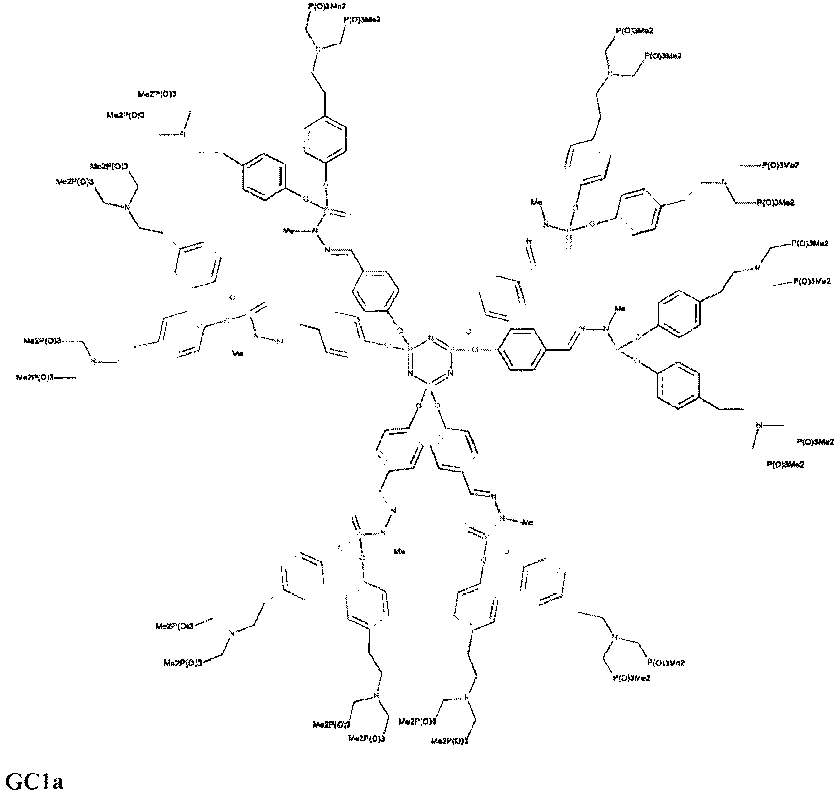 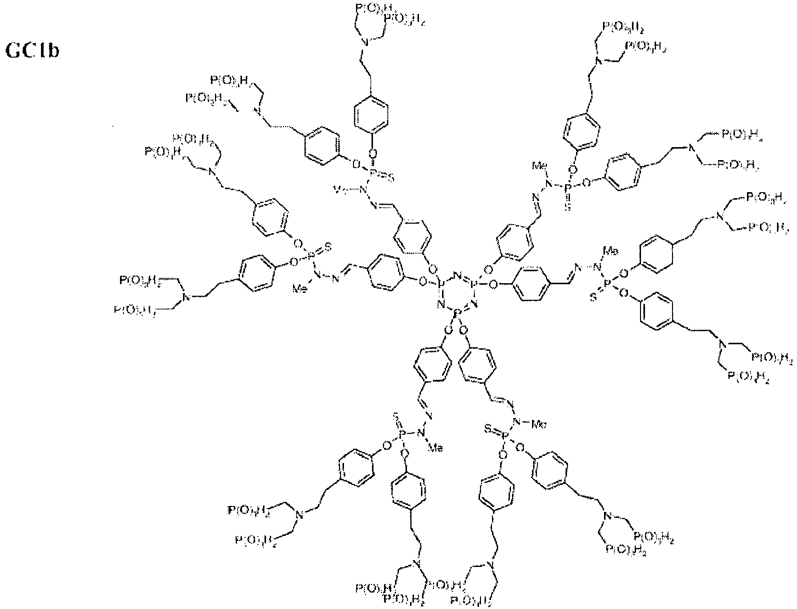 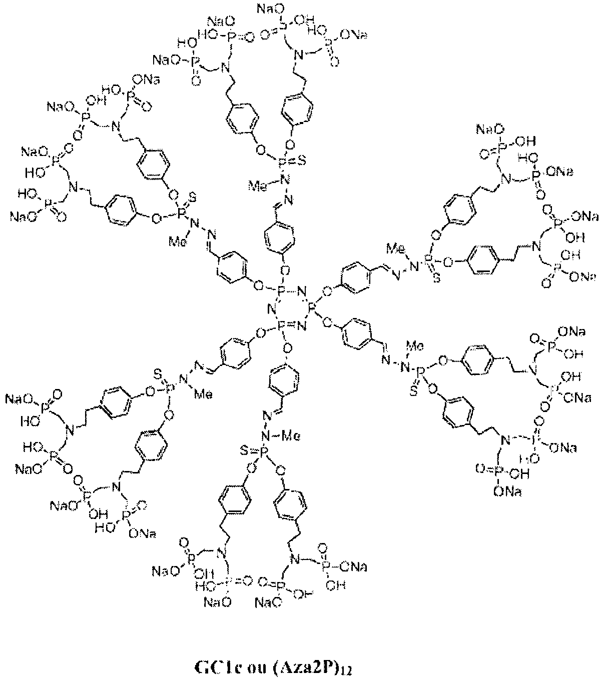 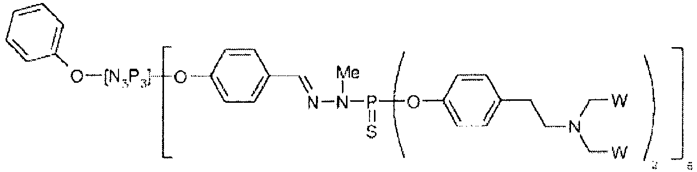 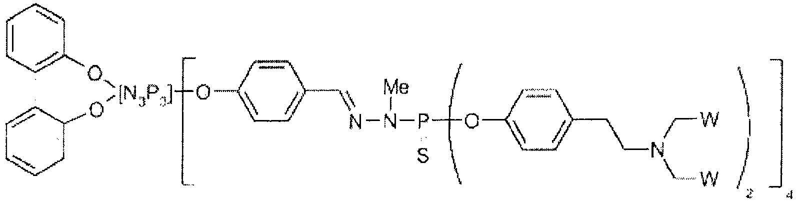 wobei W PO₃Me₂, PO₃HNa, PO₃H₂ darstellt, wobei die Verbindungen insbesondere der Verbindung GC1' der folgenden Formel (10): 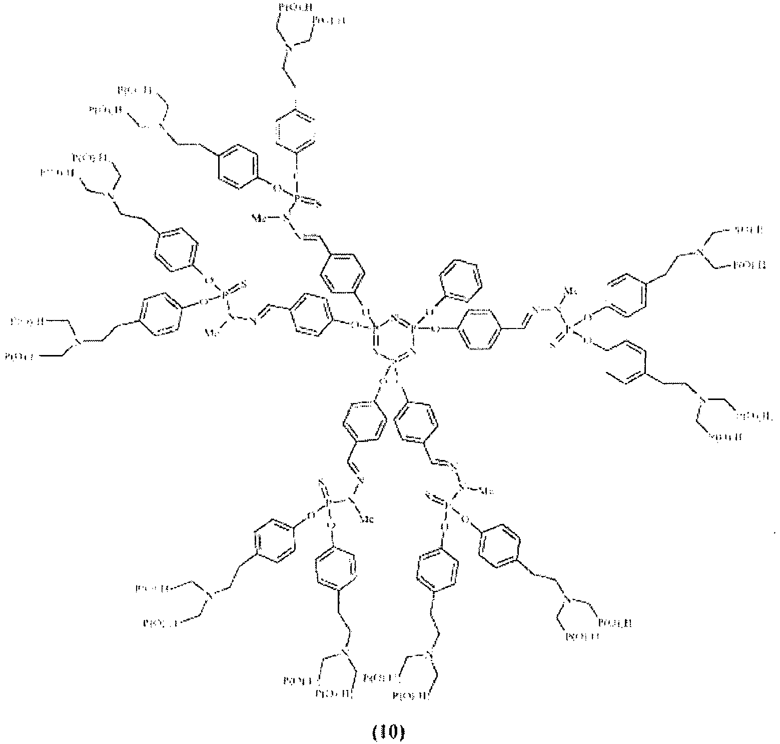 oder der Formel(10a) 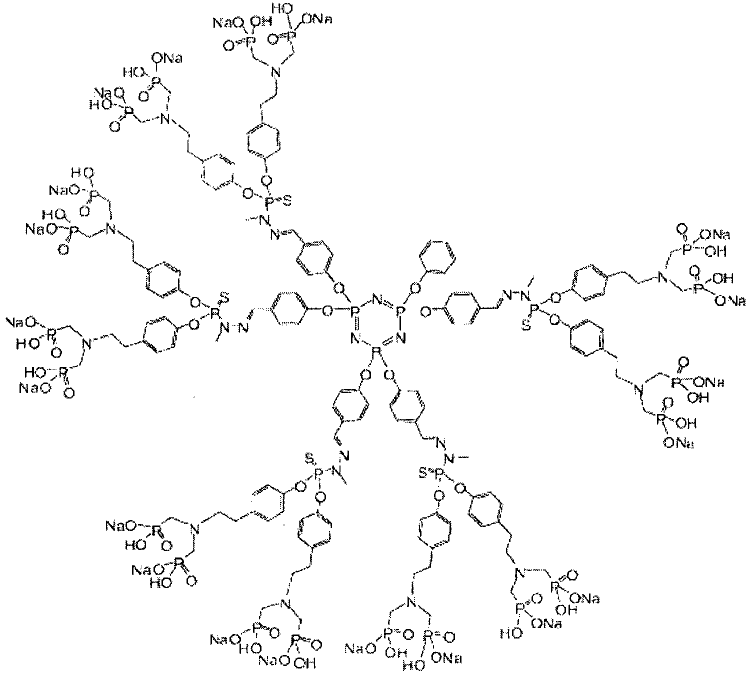 oder Verbindungen der folgenden Formel entsprechen: 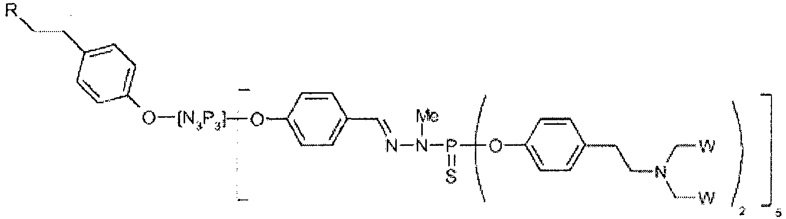 wobei W PO₃Me₂, PO₃HNa oder PO₃H₂ darstellt und R aus der Gruppe ausgewählt ist umfassend (a) fluoreszierende Gruppen ausgewählt von: 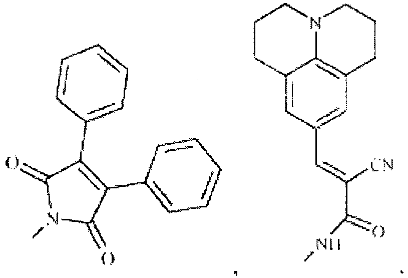 und (b) eine Biotinylgruppe der Formel 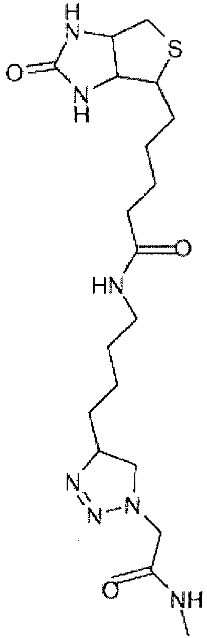 wobei die Verbindungen insbesondere den Verbindungen der folgenden Formeln entsprechen:

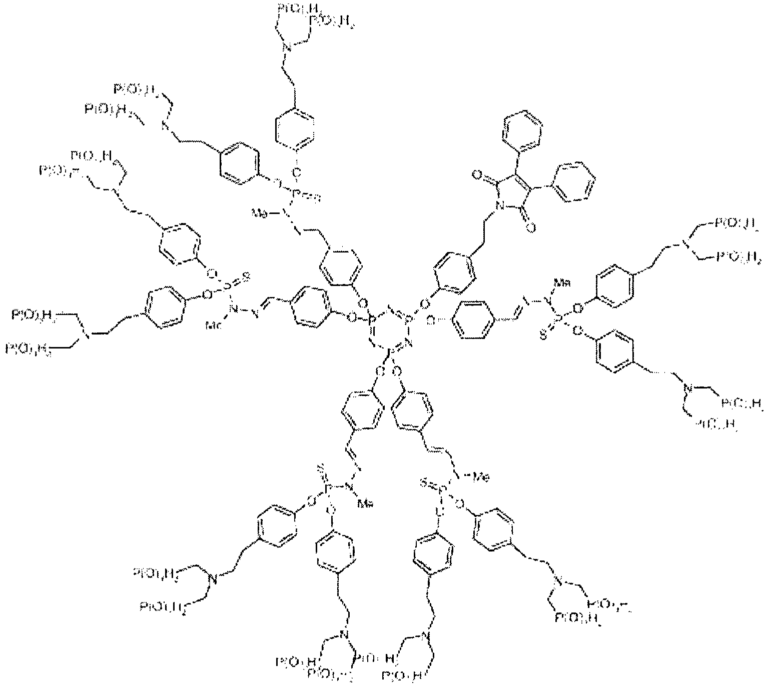

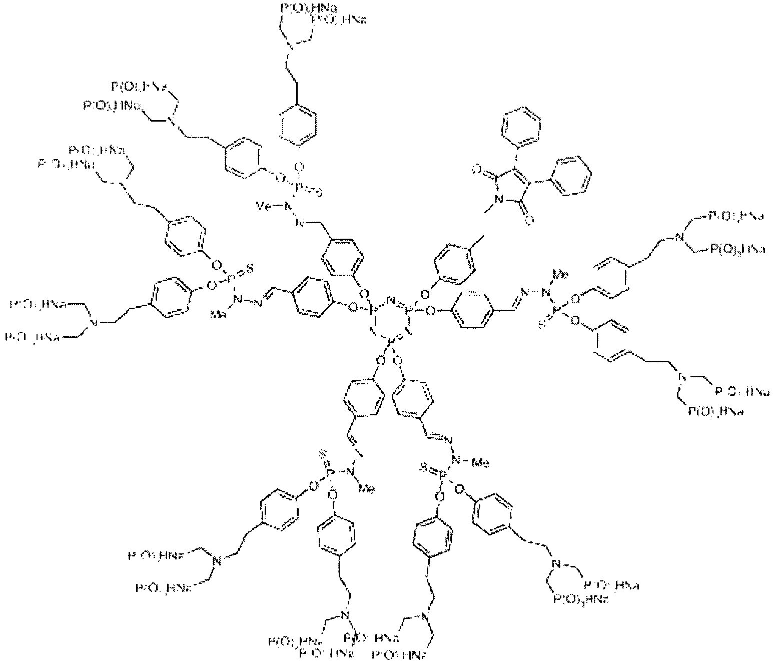

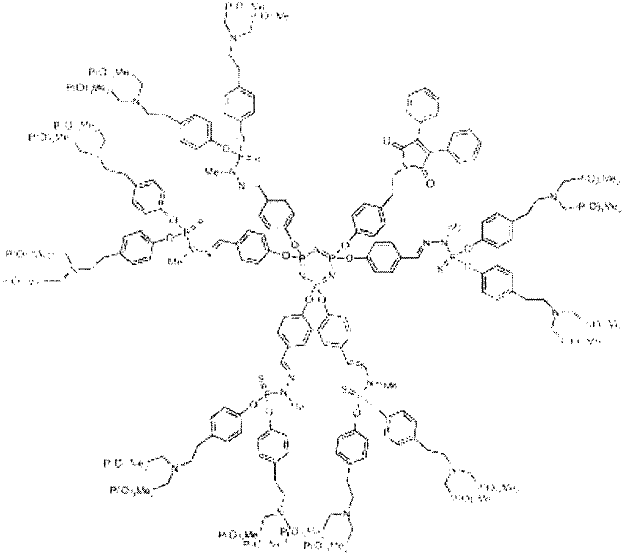

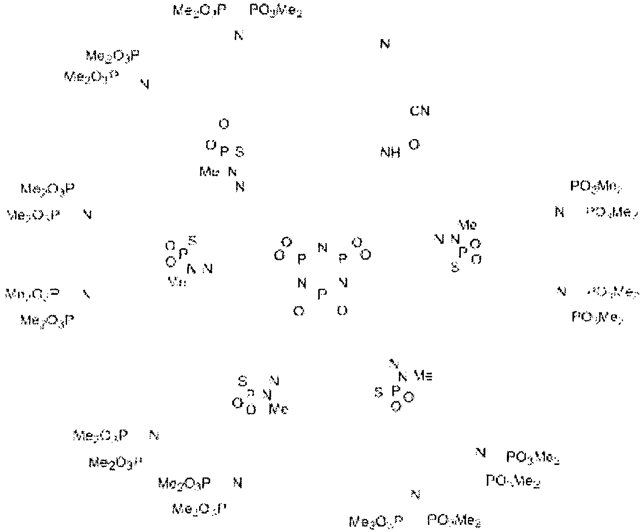

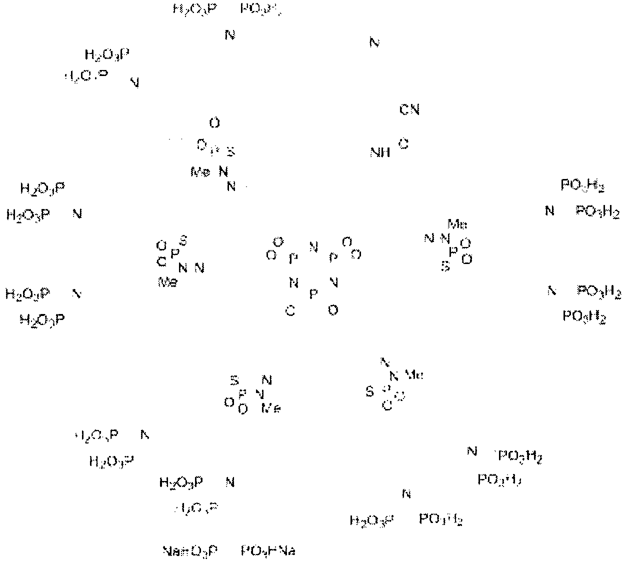

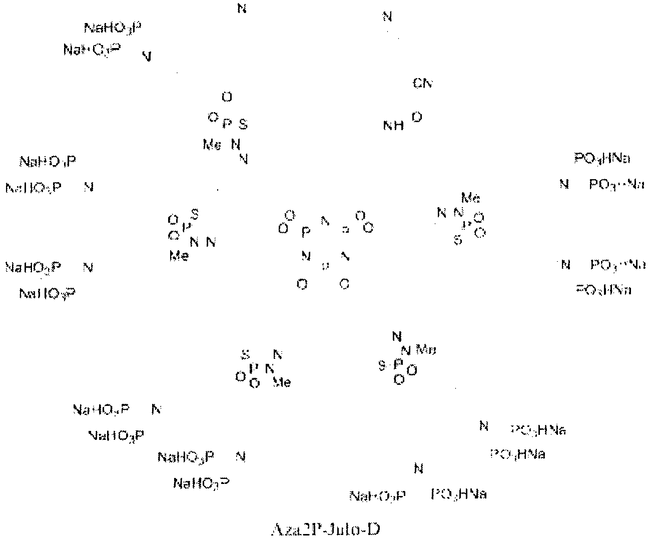

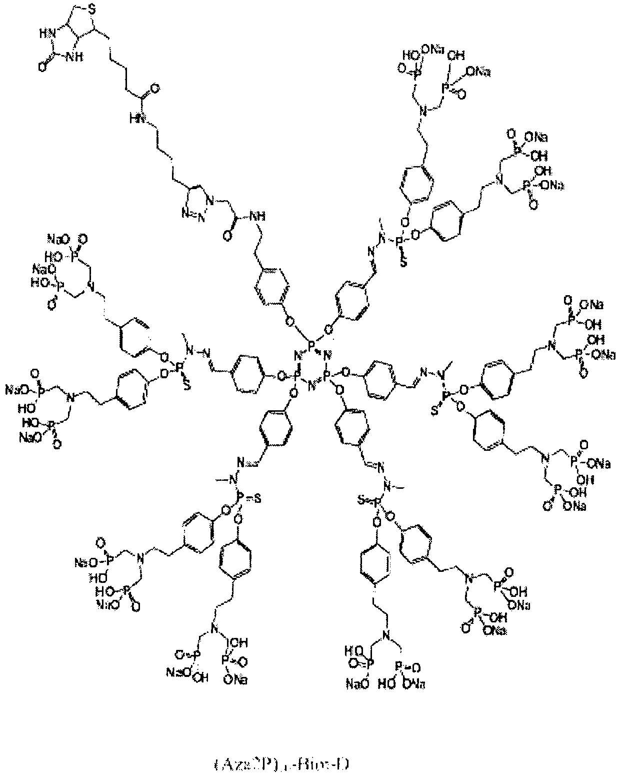

4. Dendrimere zur Verwendung nach Anspruch 1, ausgewählt aus der Gruppe umfassend:
A) 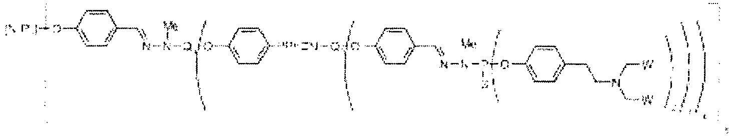 wobei W PO₃Me₂, PO₃HNa oder PO₃H₂ darstellt, Q₁ und Q₂, die identisch oder verschieden sind, P=S oder Cyclotriphosphazen (N₃P₃) darstellen, 1 2 darstellt, wenn Q₂ P=S oder 5 darstellt, wenn Q₂ N₃P₃ darstellt, und k 2 darstellt, wenn Q₁ P=S oder 5 darstellt, wenn Q₁ N₃P₃ darstellt, wobei die Dendrimere insbesondere durch die folgenden Formeln dargestellt sind: 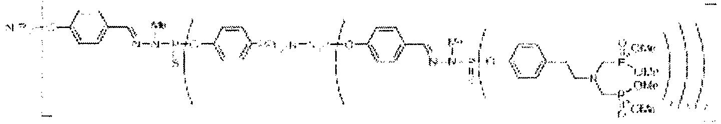 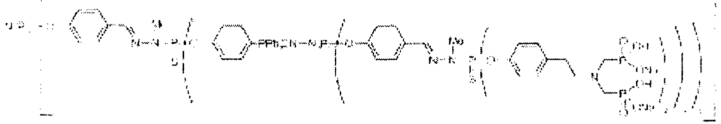 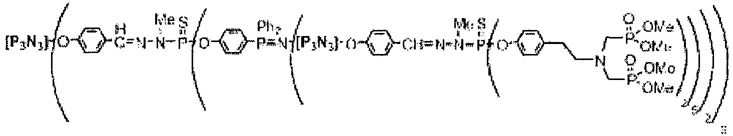 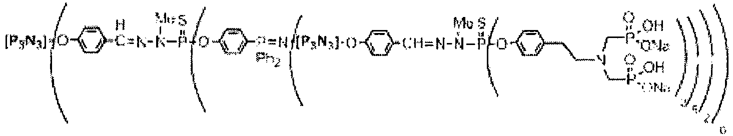 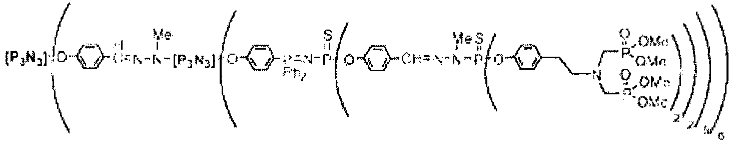 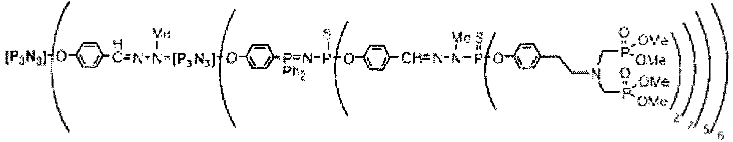 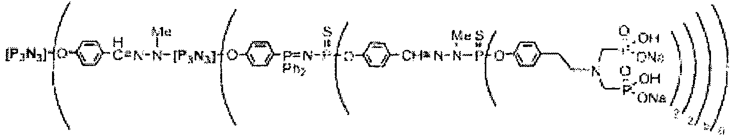 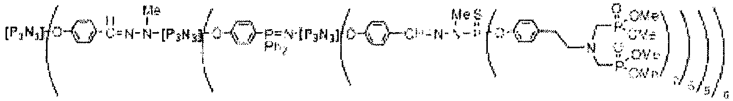 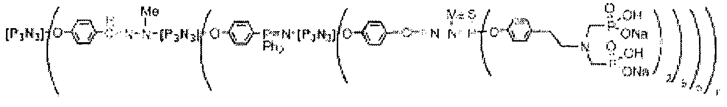
B) 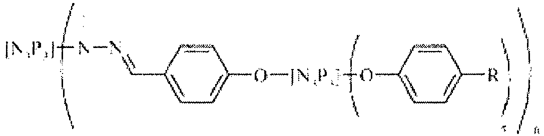 wobei R eine Gruppe darstellt ausgewählt von 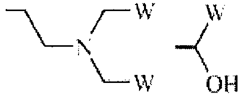 wobei W PO₃Me₂, PO₃HNa oder PO₃H₂ darstellt,
C) 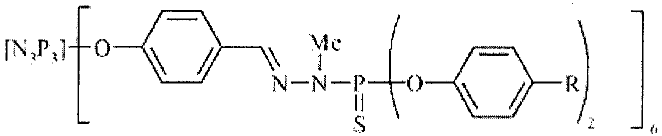 wobei R eine Gruppe darstellt ausgewählt unter: 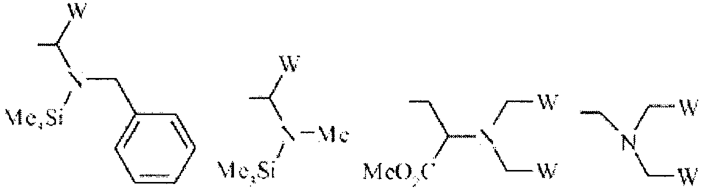 wobei W PO₃Si₂Me₆, PO₃Me₂, PO₃HNa oder PO₃H₂ darstellt, 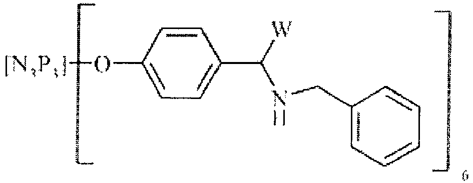 wobei W PO₃Me₂, PO₃HNa oder PO₃H₂.H darstellt,

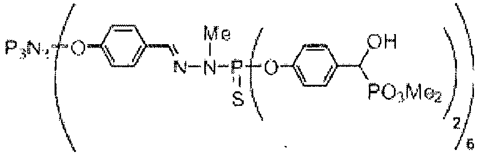

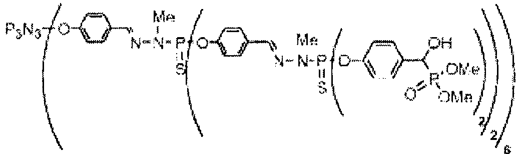

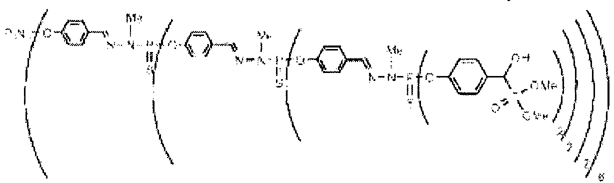

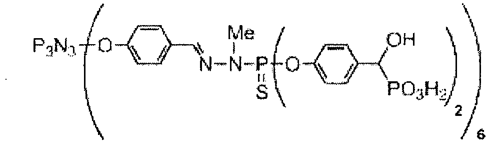

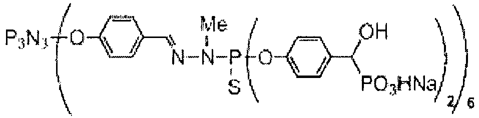

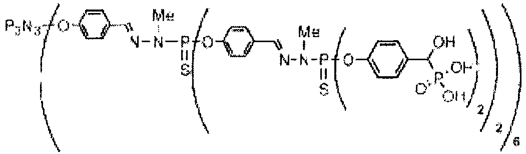

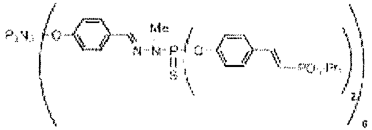

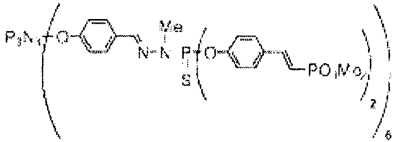

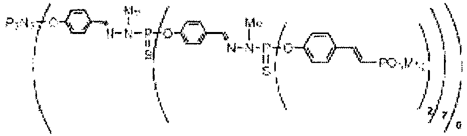

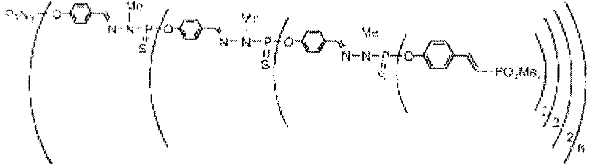

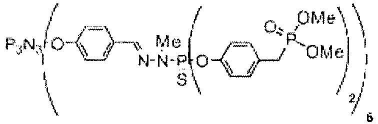

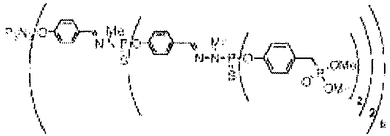

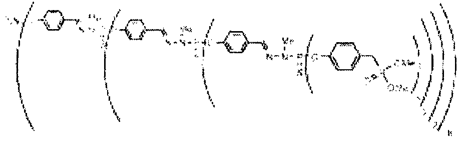

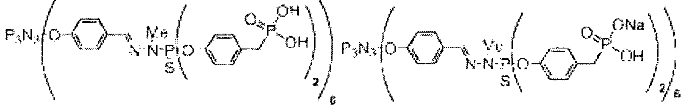

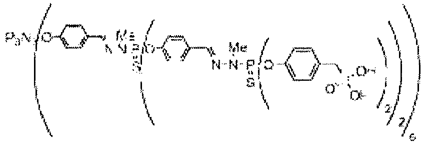

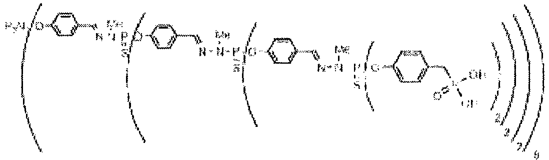

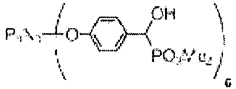

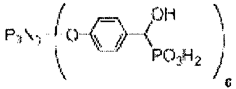

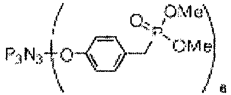

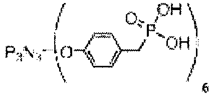

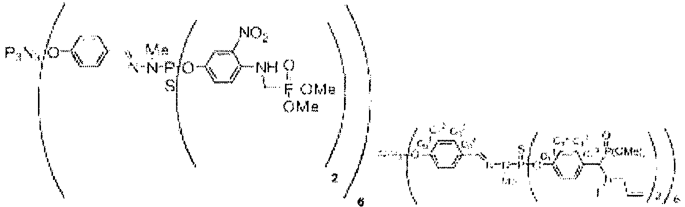

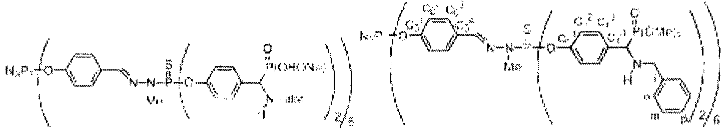

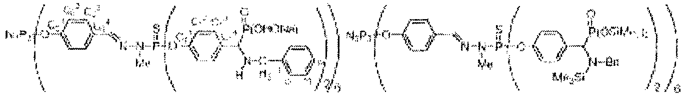

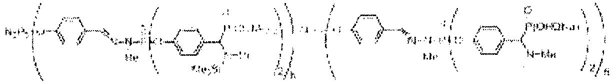

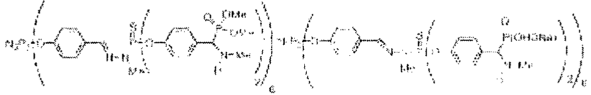

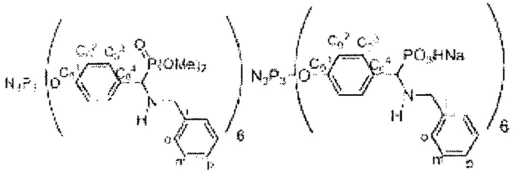

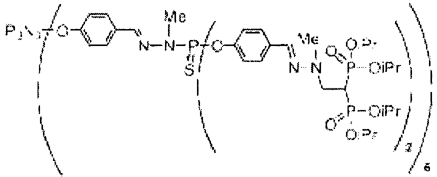

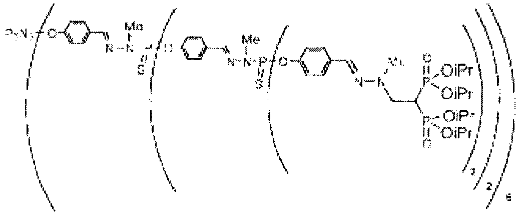

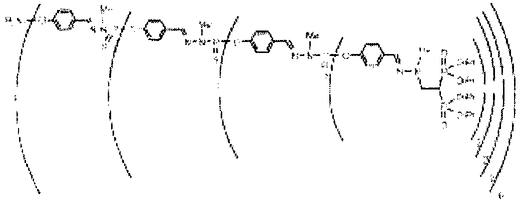

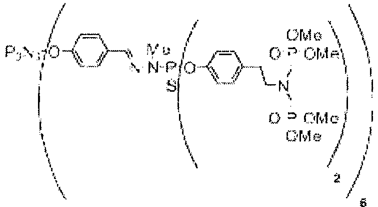

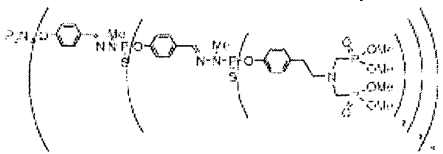

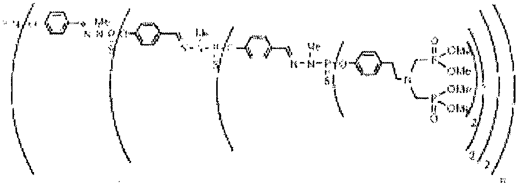

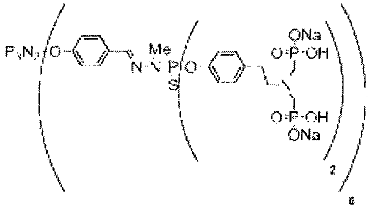

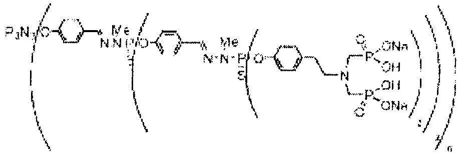

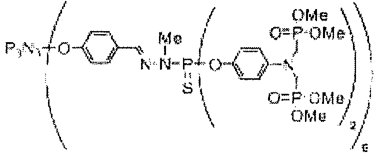

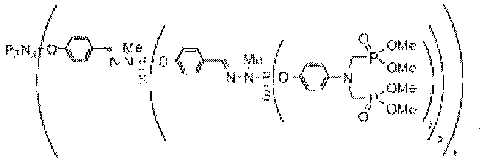

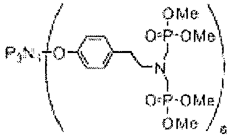

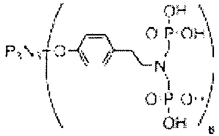

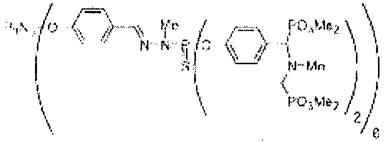

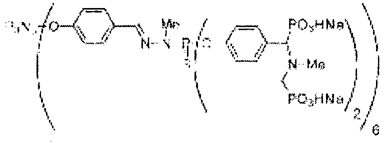

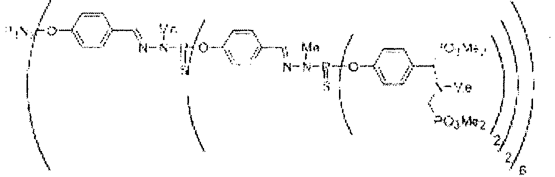

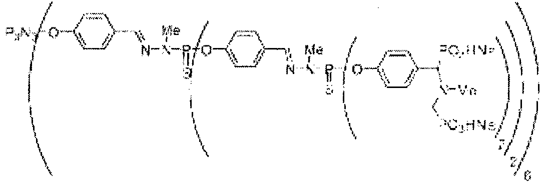

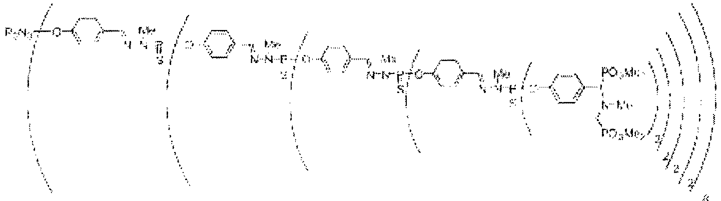

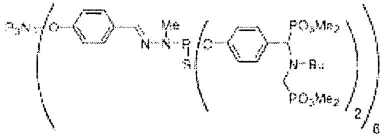

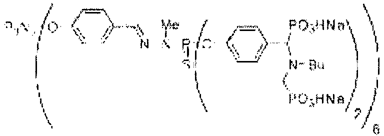

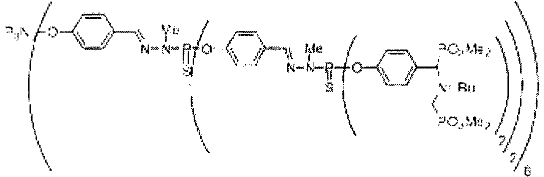

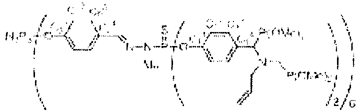

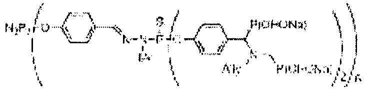

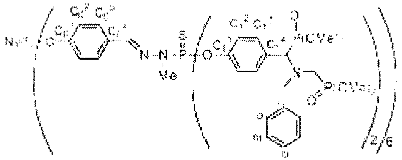

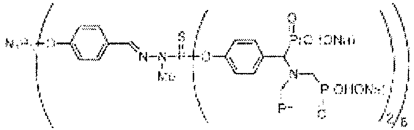

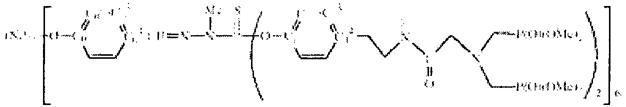

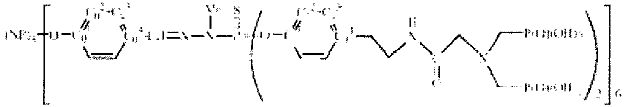

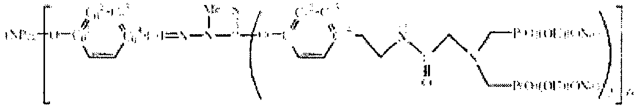

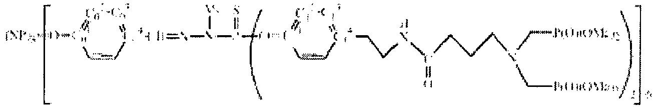

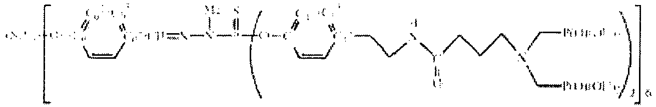

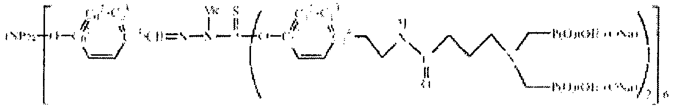

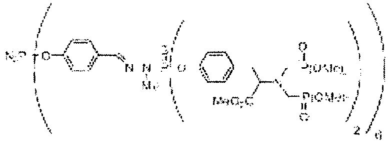

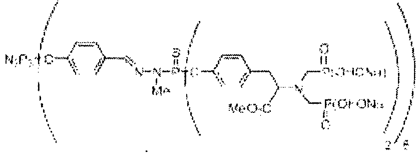

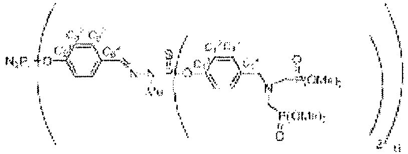

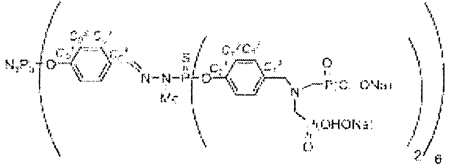

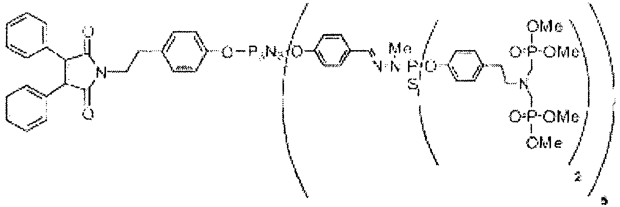

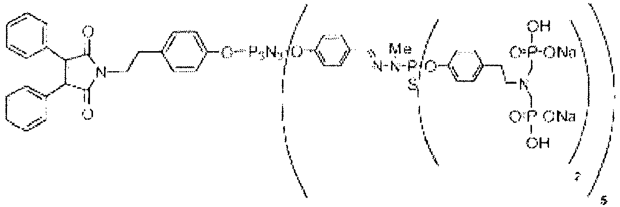

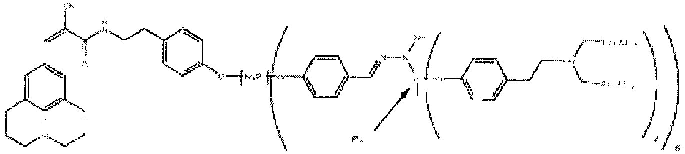

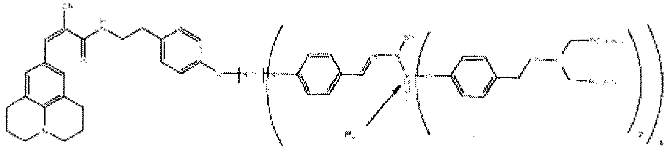

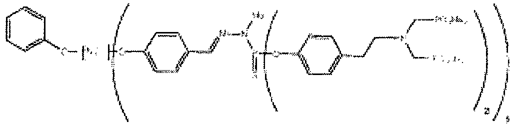

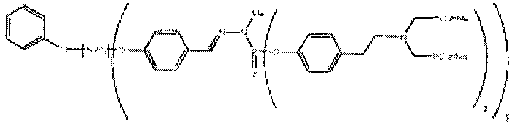

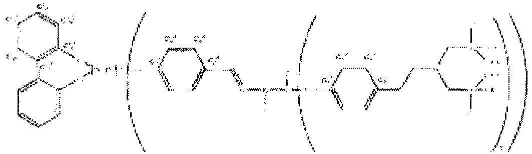

5. Verwendung von Dendrimeren mit biphosphonischen Terminierungen entsprechend der allgemeinen Formel (la) wie in Anspruch 1 definiert, zur Herstellung von Arzneimitteln, die zur Behandlung chronischer entzündlicher Erkrankungen nützlich sind.

6. Dendrimere zur Verwendung nach Anspruch 1, wobei die chronische entzündliche Erkrankung aus der Gruppe ausgewählt ist bestehend aus rheumatoider Arthritis, Schuppenflechte und juveniler idiopathischer Arthritis.

7. Pharmazeutische Zusammensetzungen, die als Wirkstoff mindestens ein Dendrimer mit monophosphonischen oder biphosphonischen Terminierungen nach einem der Ansprüche 1 bis 4 enthalten, das mit einem pharmazeutisch akzeptablen Träger verbunden ist, zur Verwendung bei der Behandlung chronischer entzündlicher Erkrankungen.

## Revendications

1. Dendrimères avec terminaisons monophosphoniques ou bisphosphoniques pour une utilisation dans le traitement de maladies inflammatoires chroniques, et de maladies inflammatoires chroniques d'origine auto-immune, correspondant à la formule générale (la) suivante : 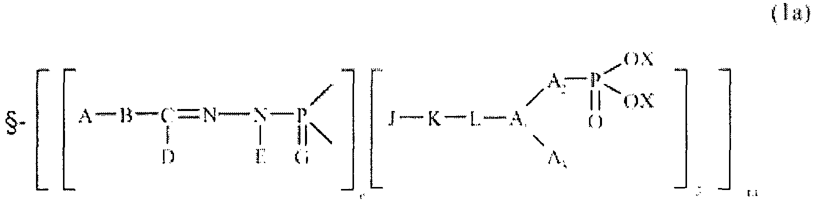 où n représente un nombre entier de 0 à 3, choisi dans le groupe comprenant les composés où, lorsque n = 0, la formule (la) correspond à la formule suivante (2a), 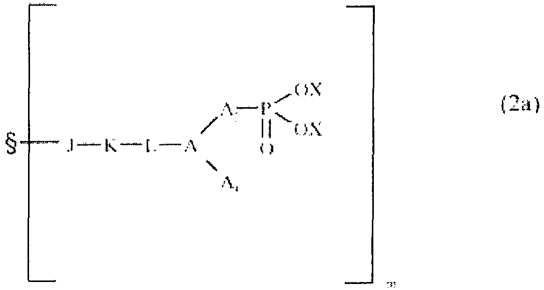
- lorsque n = 1, la formule (la) correspond à la formule suivante (3a), 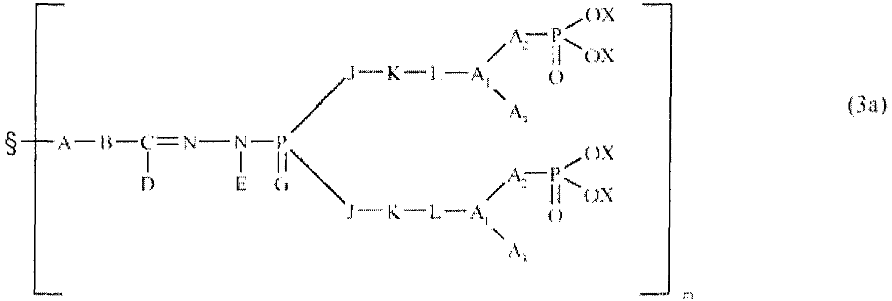
- lorsque n = 2, la formule (la) correspond à la suivante formule (4a), 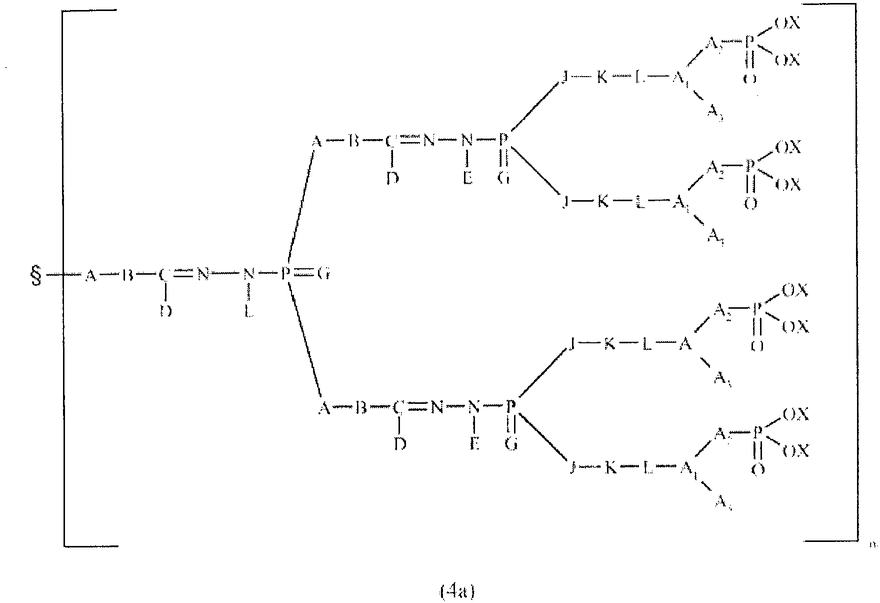
- et quand n = 3, la formule (la) correspond à la formule suivante (5a), 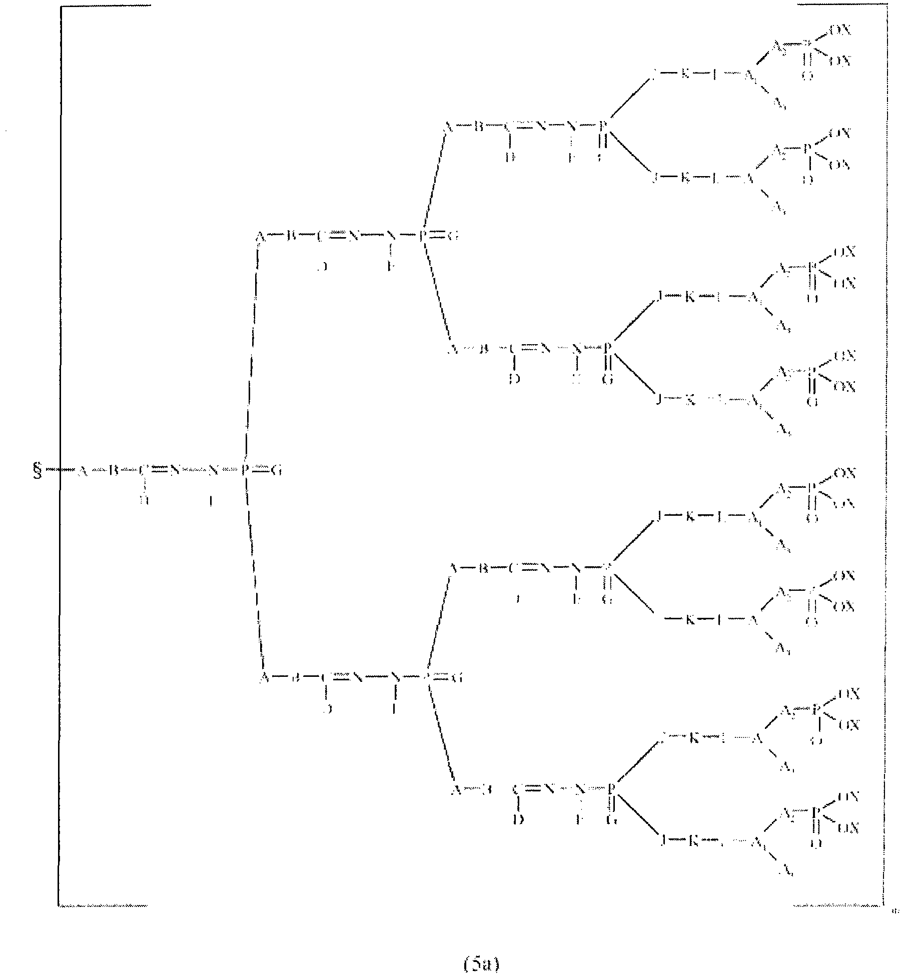 ou la formule (7) 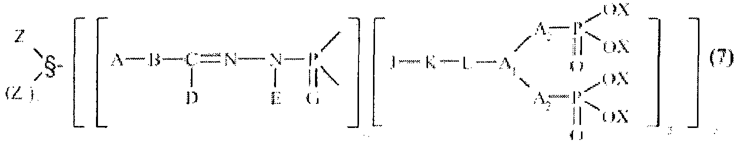 dans laquelle
n représente un nombre entier de 0 à 3, m représente 6, p représente m-1 ou m-2, et j représente O lorsque p représente m-1 et 1 lorsque p représente m-2, sélectionné parmi groupe comprenant les composés dans lesquels
- lorsque p = m-1, la formule (7) correspond à la formule (8) suivante: 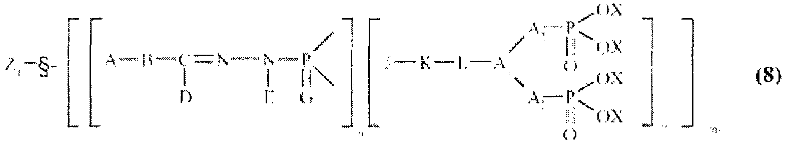
- quand p = m - 2, la formule (7) correspond à la formule suivante (9): 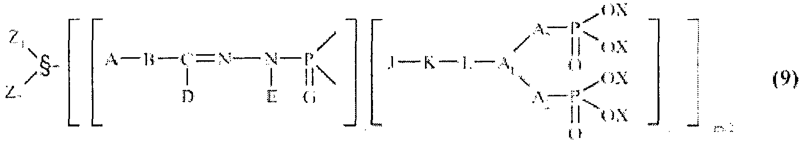 dans lesquelles:
• le noyau central § est : 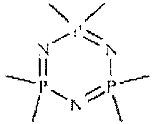
• m représente 6 ;
• la chaîne de génération correspond à la formule: 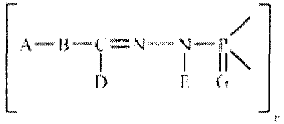 où
- A représente un atome d'oxygène, de soufre, de phosphore ou un groupe -NR-;
- B représente un groupe aryle de 6 à 24 atomes de carbone, un groupe hétéroaryle de 1 à 24 atomes de carbone, un groupe alkyle de 1 à 16 atomes de carbone, un groupe oligoéthylèneglycol, un groupe polyéthylèneglycol, deux groupes aryle de 6 à 24 atomes de carbone lié par un oxygène, un azote, un soufre, un groupe alkyle de 1 à 16 atomes de carbone, un groupe hétéroalkyle de 1 à 24 atomes de carbone, un groupe oligoéthylèneglycol, chacun pouvant être éventuellement substitué par un atome d'halogène ou un NO₂, -NRR', -CN, -CF₃, -OH, un groupe alkyle de 1 à 16 atomes de carbone, un groupe aryle de 6 à 24 atomes de carbone, un groupe aralkyle de 7 à 16 atomes de carbone, un groupe oligoéthylèneglycol comprenant préférentiellement 1 à 12 fragments d'éthylèneglycol, un groupe polyéthylèneglycol ayant une masse moléculaire allant préférentiellement de 300 g.mol⁻¹ à 3000 g.mol⁻¹;
- D représente un atome d'hydrogène, un groupe alkyle de 1 à 16 atomes de carbone, un groupe alcoxy de 1 à 16 atomes de carbone, un groupe aryle de 6 à 24 atomes de carbone, un groupe aralkyle de 7 à 16 atomes de carbone, un groupe oligoéthylèneglycol comprenant de préférence 1 à 12 fragments d'éthylèneglycol, un groupe polyéthylèneglycol ayant une masse moléculaire allant préférentiellement de 300 g.mol⁻¹ à 3000 g.mol⁻¹, chacun pouvant être éventuellement substitué par un atome d'halogène ou un- NO₂, -NRR', -CN, -CF₃, -OH, un groupe alkyle de 1 à 16 atomes de carbone, un groupe aryle de 6 à 24 atomes de carbone, un groupe aralkyle de 7 à 16 atomes de carbone, un groupe oligoéthylèneglycol comprenant préférentiellement de 1 à 12 groupements d'éthylèneglycol, un groupe polyéthylèneglycol ayant une masse moléculaire préférentiellement de 300 g.mol⁻¹ à 3000 g.mol⁻¹;
- E représente un atome d'hydrogène, un groupe alkyle de 1 à 16 atomes de carbone, un groupe alcoxy de 1 à 16 atomes de carbone, un groupe aryle de 6 à 24 atomes de carbone, un groupe aralkyle de 7 à 16 atomes de carbone, un groupe oligoéthylèneglycol comprenant de préférence 1 à 12 fragments d'éthylèneglycol, un groupe polyéthylèneglycol ayant une masse moléculaire allant préférentiellement de 300 g.mol⁻¹ à 3000 g.mol⁻¹, chacun pouvant être éventuellement substitué par un atome d'halogène ou un NO₂, -NRR', -CN, -CF₃, -OH, un groupe alkyle de 1 à 16 atomes de carbone, un groupe aryle de 6 à 24 atomes de carbone, un groupe aralkyle de 7 à 16 atomes de carbone, un groupe oligoéthylèneglycol comprenant préférentiellement 1 à 12 fragments d'éthylèneglycol, un groupe polyéthylèneglycol ayant une masse moléculaire préférentiellement de 300 g.mol⁻¹ à 3000 g.mol⁻¹;
- G représente un atome d'oxygène, d'azote, de soufre, de sélénium, de tellure ou un groupe = NR; R et R' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un groupe oligoéthylèneglycol comprenant de préférence 1 à 12 des groupements d'éthylèneglycol, un groupe polyéthylèneglycol ayant une masse moléculaire allant préférentiellement de 300 g.mol⁻¹ à 3000 g.mol⁻¹;
• la chaîne intermédiaire correspond à la formule:
-J-K-L-
où - J représente un atome d'oxygène, un atome de soufre, ou un groupe -NR-;
- K représente un groupe aryle de 6 à 24 atomes de carbone, un groupe hétéroaryle de 1 à 24 atomes de carbone, l'hétéro-élément étant de préférence choisi parmi l'oxygène, l'azote ou le soufre, un groupe alkyle de 1 à 16 atomes de carbone, un groupe oligoéthylèneglycol comprenant préférentiellement 1 à 12 fragments d'éthylèneglycol, un groupe polyéthylèneglycol ayant une masse moléculaire allant préférentiellement de 300 g.mol⁻¹ à 3000 g.mol⁻¹, chacun pouvant être éventuellement substitué par un atome d'halogène ou un NO₂, NRR', -CN, -CF₃, -OH, un groupe alkyle de 1 à 16 atomes de carbone, un groupe aryle de 6 à 24 atomes de carbone, un groupe aralkyle de 7 à 16 atomes de carbone, un groupe oligoéthylèneglycol comprenant préférentiellement 1 à 12 fragments d'éthylèneglycol, un groupe polyéthylèneglycol ayant une masse moléculaire allant préférentiellement de 300 g.mol⁻¹ à 3000 g.mol⁻¹;
- L représente une chaîne hydrocarbonée linéaire, ramifiée ou cyclique de 0 à 10 éléments, contenant éventuellement une ou plusieurs liaisons doubles ou triples, chacun desdits éléments pouvant être éventuellement un hétéroatome, ledit hétéroatome étant de préférence choisi parmi l'oxygène, le soufre, l'azote , le phosphore, le silicium, chaque élément pouvant être éventuellement substitué par au moins un substituant choisi parmi un groupe alkyle de 1 à 16 atomes de carbone, un halogène, un atome d'oxygène, -NO₂, -NRR', -CN, -CF₃, -OH, un groupe alcoxy de 1 à 16 atomes de carbone, un groupe aryle de 6 à 24 atomes de carbone, un groupe aralkyle de 7 à 16 atomes de carbone, un groupe oligoéthylèneglycol comprenant de préférence 1 à 12 groupements éthylèneglycol, un groupe polyéthylèneglycol ayant une masse moléculaire poids compris préférentiellement de 300 g.mol⁻¹ à 3000 g.mol⁻¹;
R et R' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un groupe oligoéthylèneglycol comprenant de préférence 1 à 12 des groupements d'éthylèneglycol, un groupe polyéthylèneglycol ayant une masse moléculaire allant préférentiellement de 300 g.mol⁻¹ à 3000 g.mol⁻¹;
• le groupe terminal étant représenté par la formule: 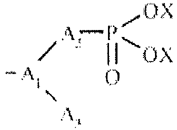 où
A1 représente N; un groupe P = Y, où Y représente O, S ou aucun atome; un groupe N-R; ou un groupe C-R; R représentant H ou une chaîne hydrocarbonée linéaire, ramifiée ou cyclique de 1 à 16 éléments, contenant éventuellement un ou plusieurs hétéroatomes, lesdits hétéroatomes étant de préférence choisis parmi l'oxygène, le soufre, l'azote, le phosphore, le silicium et / ou une ou plusieurs double ou triple liaisons , chacun desdits éléments étant éventuellement substitué par au moins un substituant choisi parmi un groupe hydroxyle, un groupe -NR'R", un groupe alcoxy de 1 à 16 atomes de carbone, un groupe alkyle de 1 à 16 atomes de carbone, un atome d'halogène, un groupe -NO₂, un groupe -CN, un groupe -CF₃, un groupe aryle de 6 à 24 atomes de carbone, un groupe aralkyle de 7 à 16 atomes de carbone, un groupe oligoéthylèneglycol comprenant préférentiellement 1 à 12 groupements éthylèneglycol, un groupe polyéthylèneglycol ayant une masse moléculaire allant préférentiellement de 300 g.mol⁻¹ à 3000 g.mol⁻¹, R 'et R "représentant indépendamment l'un de l'autre un H ou un groupe alkyle de 1 à 16 atomes de carbone, un groupe aryle de 6 à 24 atomes de carbone, un groupe aralkyle de 7 à 16 atomes de carbone, un groupe oligoéthylèneglycol comprenant préférentiellement 1 à 12 fragments d'éthylèneglycol, un groupe polyéthylèneglycol ayant une masse moléculaire allant préférentiellement de 300 g.mol⁻¹ à 3000 g.mol⁻¹;
A2 représente une liaison simple ou une chaîne hydrocarbonée linéaire, ramifiée ou cyclique de 1 à 6 éléments, chacun desdits éléments contenant éventuellement un ou plusieurs hétéroatomes, lesdits hétéroatomes étant de préférence choisis parmi un atome de soufre, d'oxygène, de phosphore ou d'azote, de préférence l'azote et étant éventuellement substitué par au moins un substituant choisi parmi H, un groupe alkyle de 1 à 16 atomes de carbone, un halogène, un groupe NO₂, un groupe NRR', un groupe -CN, un groupe -CF₃, un groupe hydroxyle, un groupe alcoxy de 1 à 16 atomes de carbone, un groupe aryle ou hétéroaryle de 1 à 24 atomes de carbone, l'élément hétéro-élément étant de préférence choisi parmi l'oxygène, l'azote ou le soufre, un groupe aralkyle de 7 à 16 atomes de carbone, un groupe oligoéthylèneglycol comprenant préférentiellement 1 à 12 fragments d'éthylèneglycol, un groupe polyéthylèneglycol ayant un poids moléculaire allant préférentiellement de 300 g.mol⁻¹ à 3000 g.mol⁻¹, R et R' représentant indépendamment l'un de l'autre un H ou une chaîne hydrocarbonée linéaire, ramifiée ou cyclique de 1 à 16 éléments, contenant éventuellement un ou plusieurs hétéroatomes, lesdits hétéroatomes étant de préférence choisis parmi l'oxygène, le soufre, l'azote, le phosphore, le silicium et / ou une ou plusieurs double ou triple liaisons, chacun desdits éléments étant éventuellement substitué par au moins un substituant choisi parmi un groupe hydroxyle, un groupe NR"R''', un groupe alcoxy de 1 à 16 atomes de carbone, un groupe alkyle de 1 à 16 atomes de carbone, un atome d'halogène, un groupe NO₂, un groupe -CN, un groupe -CF₃, un groupe aryle de 6 à 24 atomes de carbone, un groupe aralkyle de 7 à 16 atomes de carbone, un groupe oligoéthylèneglycol comprenant préférentiellement 1 à 12 fractions éthylèneglycol, un groupe polyéthylèneglycol ayant une masse moléculaire préférentielle de 300 g.mol⁻¹ à 3000 g.mol⁻¹, R" et R''' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un groupe oligoéthylèneglycol comprenant préférentiellement 1 à 12 groupes d'éthylèneglycol, un groupe polyéthylèneglycol ayant un poids moléculaire variant préférentiellement de 300 g.mol⁻¹ à 3000 g.mol⁻¹
A3 représente un H ou une chaîne hydrocarbonée linéaire, ramifiée ou cyclique de 1 à 6 éléments, chacun desdits éléments contenant éventuellement un hétéroatome, ledit hétéroatome étant de préférence choisis parmi un atome de soufre, d'oxygène, de phosphore ou de silicium, de préférence l'azote et étant éventuellement substitué par au moins un substituant choisi parmi un groupe hydroxyl, un groupe NR"R''', un groupe alcoxy de 1 à 16 atomes de carbone, un groupe alkyle de 1 à 16 atomes de carbone, un atome d'halogène, un groupe NO₂, un groupe -CN, un groupe -CF₃, un groupe aryle de 6 à 24 atomes de carbone, un groupe aralkyle de 7 à 16 atomes de carbone, un groupe oligoéthylèneglycol comprenant préférentiellement 1 à 12 fractions éthylèneglycol, un groupe polyéthylèneglycol ayant une masse moléculaire préférentielle de 300 g.mol⁻¹ à 3000 g.mol⁻¹, R" et R''' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un groupe oligoéthylèneglycol comprenant préférentiellement 1 à 12 groupes d'éthylèneglycol, un groupe polyéthylèneglycol 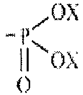 ayant un poids moléculaire variant préférentiellement de 300 g.mol⁻¹ à 3000 g.mol⁻¹ ou
en particulier A₃ peut représenter 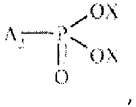 chaque A₂ étant identique ou différent;
chaque OX, identique ou différent pour chaque groupe phosphonique, représente OH, Oalkyle, où le groupe alkyle comprend de 1 à 16 atomes de carbone, Oaryl, où le groupe aryle comprend de 6 à 24 atomes de carbone, Oaralkyle, dans lequel le groupe aralkyle comprend de 7 à 24 atomes de carbone, Oalkylaryle, où le groupe alkylaryle comprend de 7 à 24 atomes de carbone, OSiR'iR'₂R'₃, où R'₁, R'₂ et R'₃, identiques ou différents, représentent un groupe alkyle de 1 à 16 atomes de carbone, ou 0⁻M⁺, où M⁺ est un cation d'éléments du groupe IA, IB, IIA, IIB ou IIIA, IIIB du tableau périodique des éléments, de préférence M⁺ est choisi parmi les cations des atomes de sodium, de potassium, de cuivre, de calcium, de baryum, de zinc, de magnésium, de lithium et d'aluminium, ou des groupes hydrocarbonés de 1 à 100 atomes de carbone, ou des groupes azotés de 0 à 100 atomes de carbone, tels que NR₁R₂R₃R₄⁺, où, indépendamment l'un de l'autre, R₁, R₂, R₃ et R₄ représentent H ou une chaîne hydrocarbonée linéaire, ramifiée ou cyclique de 1 à 16 éléments, éventuellement introduire un ou plusieurs hétéroatomes, lesdits hétéroatomes étant de préférence choisis parmi l'oxygène, le soufre, l'azote, le phosphore, le silicium et / ou une ou plusieurs liaisons doubles ou triples, chacun desdits éléments étant éventuellement substitué par au moins un substituant choisi parmi un groupe hydroxyle, un groupe NR"R''', un groupe alcoxy de 1 à 16 atomes de carbone, un groupe alkyle de 1 à 16 atomes de carbone, un atome d'halogène, un groupe NO₂, un groupe -CN, un groupe -CF₃, un groupe aryle de 6 à 24 atomes de carbone, un groupe aralkyle de 7 à 16 atomes de carbone, un groupe oligoéthylèneglycol comprenant préférentiellement 1 à 12 groupements éthylèneglycol, un groupe polyéthylèneglycol ayant une masse moléculaire préférentielle allant de 300 g.mol⁻¹ à 3000 g.mol⁻¹, R" et R''' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un oligoéthylèneglycol groupe comprenant de préférence 1 à 12 groupements d'éthylèneglycol, un polyéthylèneglycol groupe ayant une masse moléculaire allant préférentiellement de 300 g.mol⁻¹ à 3000 g.mol⁻¹,
Z₁ et Z₂ étant identiques ou différents, optionnellement liés ensemble, en particulier au moyen d'une liaison covalente, et représentant un H ou une chaîne hydrocarbonée linéaire, ramifiée ou cyclique de 1 à 6 éléments, optionnellement contenant une ou plusieurs double ou triple liaisons, chacun desdits éléments contenant éventuellement un hétéroatome, ledit hétéroatome étant de préférence choisis parmi un atome d'azote, d'oxygène, de phosphore, de silicium ou de soufre, un groupe aryle de 6 à 24 atomes de carbone, un groupe hétéroaryle de 1 à 24 atomes de carbone, un groupe carboxyle, un groupe -C=NR, chaque élément étant éventuellement substitué par au moins un substituant choisi parmi un groupe hydroxyl, un groupe NR"R''', un groupe alcoxy de 1 à 16 atomes de carbone, un groupe alkyle de 1 à 16 atomes de carbone, un atome d'halogène, un groupe NO₂, un groupe -CN, un groupe -CF₃, un groupe aryle de 6 à 24 atomes de carbone, un groupe aralkyle de 7 à 16 atomes de carbone, un groupe oligoéthylèneglycol comprenant préférentiellement 1 à 12 fractions éthylèneglycol, un groupe polyéthylèneglycol ayant une masse moléculaire préférentielle de 300 g.mol⁻¹ à 3000 g.mol⁻¹, R" et R''' représentant indépendamment l'un de l'autre un H ou un groupe alkyle de 1 à 16 atomes de carbone, un groupe aryle de 6 à 24 atomes de carbone ou un groupe aralkyle de 7 à 16 atomes de carbone, un groupe oligoéthylèneglycol comprenant préférentiellement 1 à 12 groupes d'éthylèneglycol, un groupe polyéthylèneglycol ayant une masse moléculaire préférentielle de 300 g.mol⁻¹ à 3000 g.mol⁻¹, le premier élément de ladite chaîne hydrocarbonée étant de préférence l'oxygène ou l'azote.

2. Dendrimères pour une utilisation selon la revendication 1 correspondant à la formule générale : 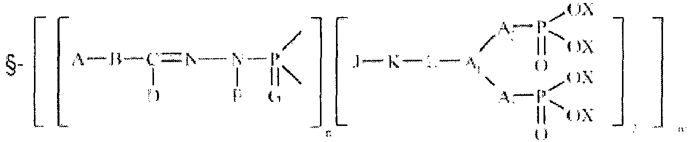 ou 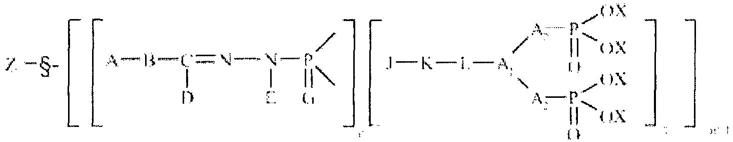 dans lesquelles
§ représente 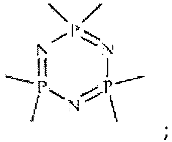 m représente 6;
n représente 0, 1 ou 2;
A représente un atome d'oxygène;
B représente un groupe benzène;
D représente l'hydrogène;
E représente un groupe méthyle;
G représente un atome de soufre;
J représente un atome d'oxygène;
K représente un groupe benzène;
L représente une chaîne hydrocarbonée saturée linéaire non substituée avec deux atomes de carbone;
A₁ représente un atome d'azote;
A₂ représente un groupe CH₂;
X représente un groupe méthyle, ou un atome d'hydrogène ou de sodium; et
Z₁ représente un groupe phényloxy.

3. Dendrimères selon l'une quelconque des revendications 1 ou 2, choisis dans le groupe comprenant: 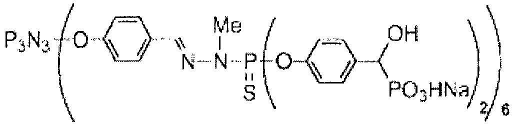 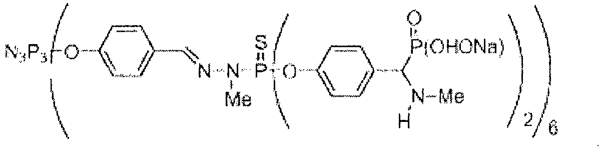 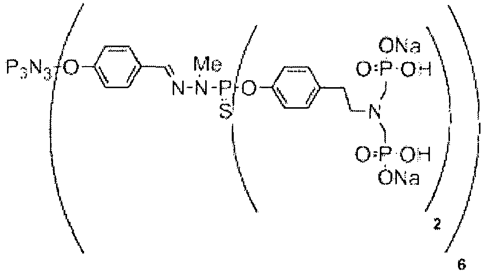 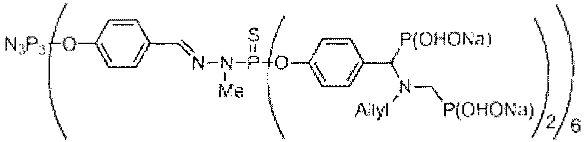 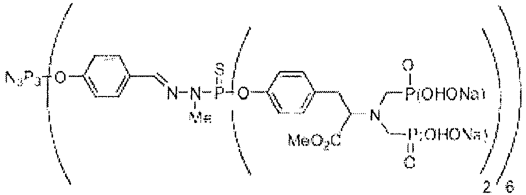 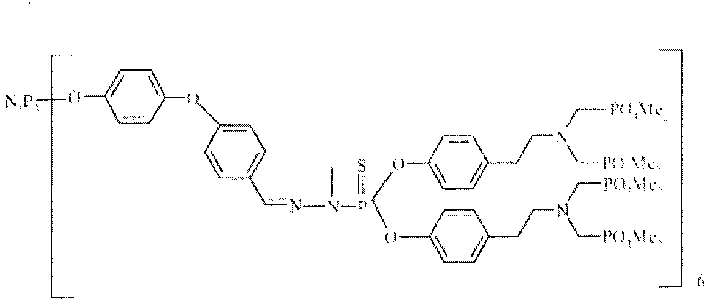 ou de composés GC1 de formules suivantes: 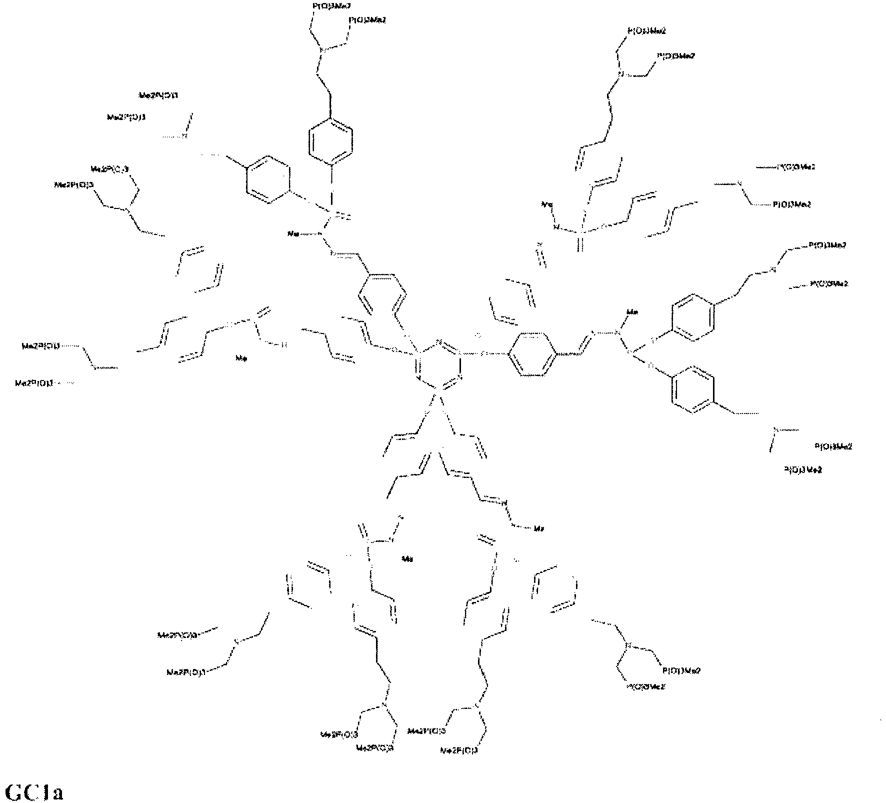 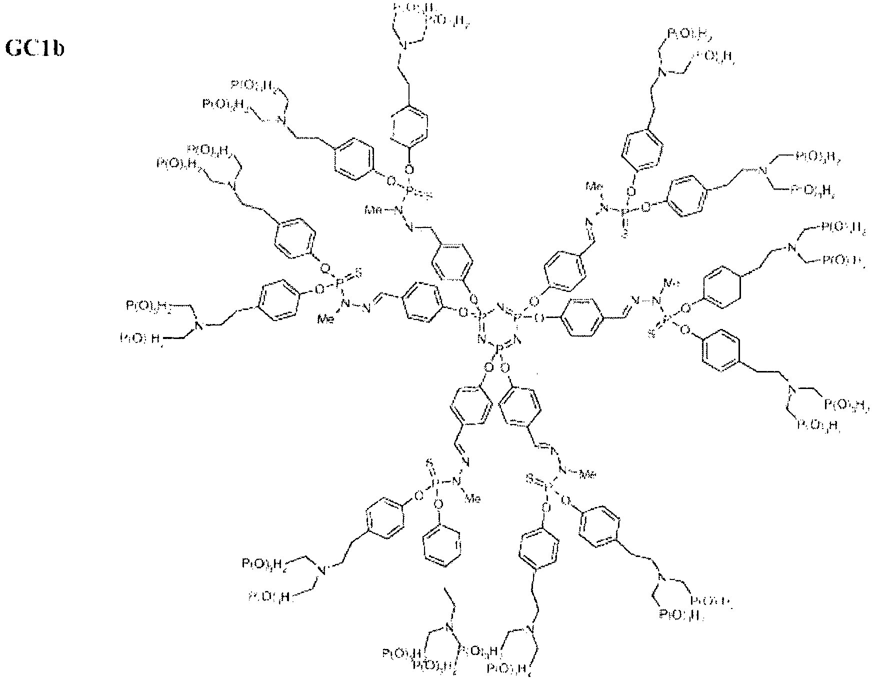 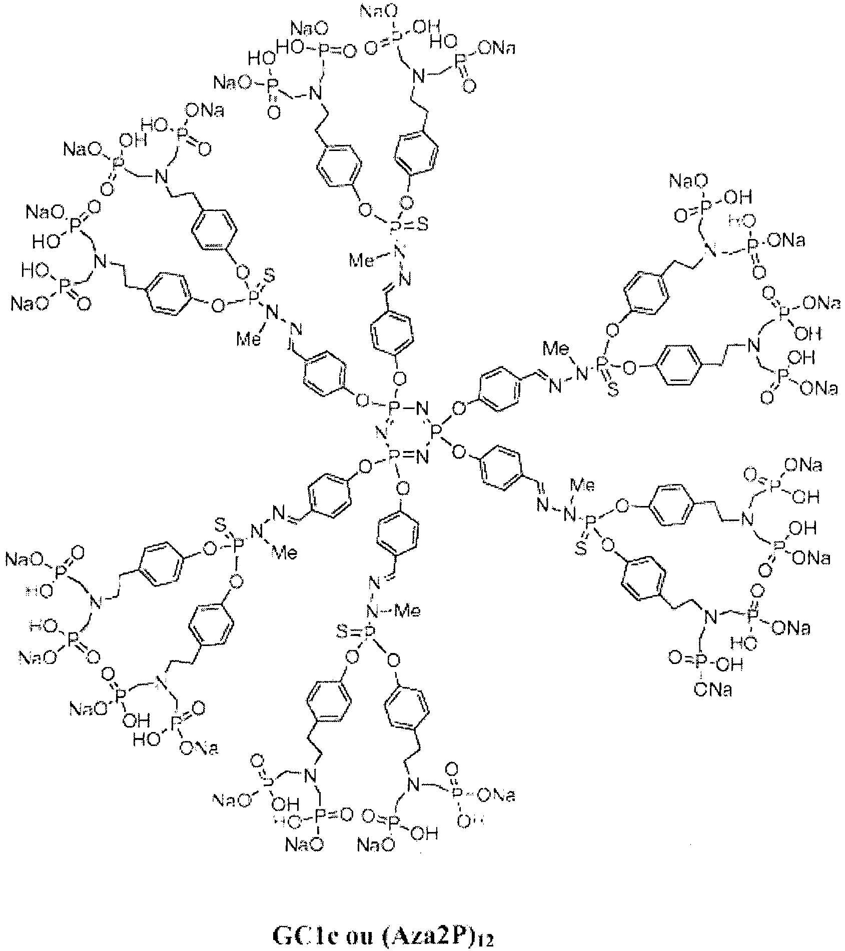 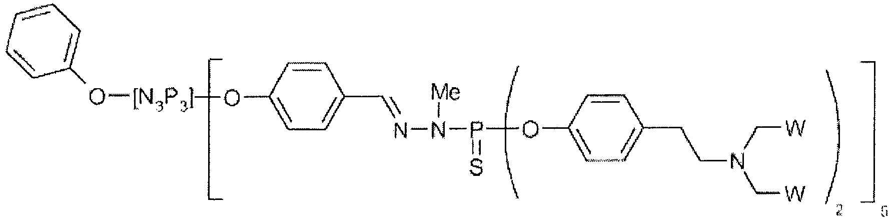 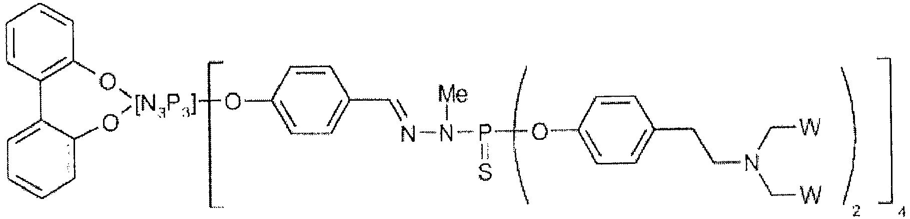 dans laquelle W représente PO₃Me₂, ou PO₃HNa, PO₃H₂, lesdits composés correspondant notamment au composé GC1' de la formule suivante (10): 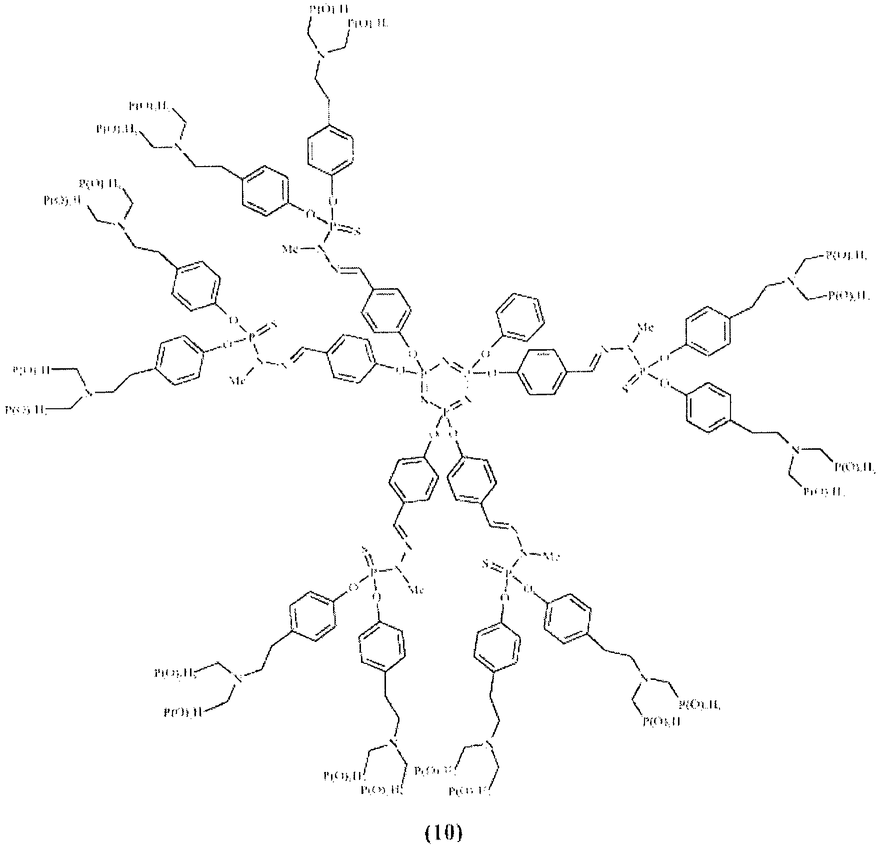 ou de formule 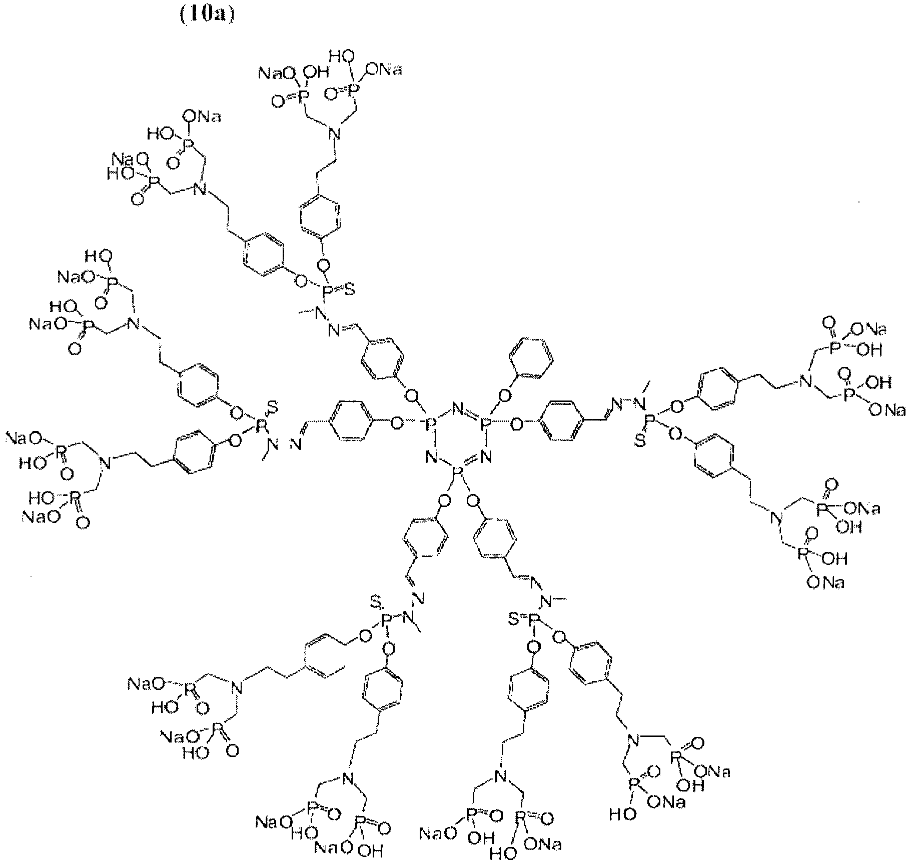
- ou des composés de formule suivante: 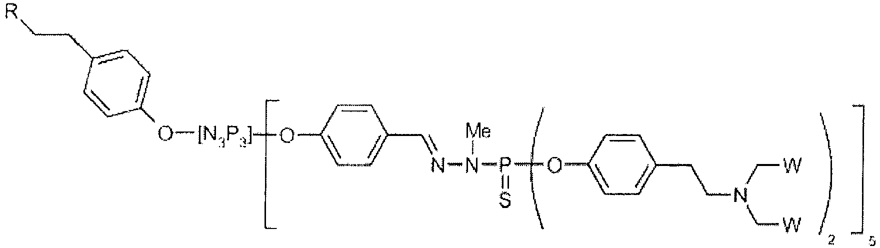 dans laquelle W représente PO₃Me₂, PO₃HNa, ou PO₃H₂, et R est choisi dans le groupe comprenant (a) des groupes fluorescents choisis parmi: 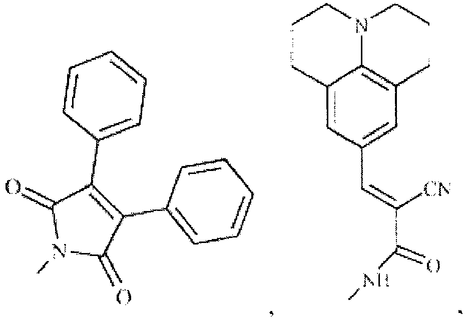 et (b) un groupe biotinyle de formule 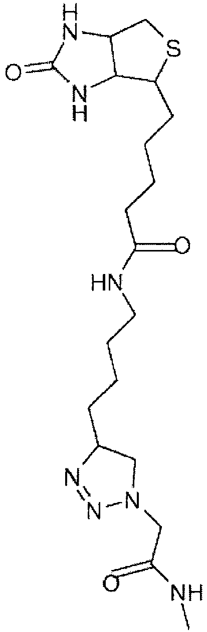 lesdits composés correspondant notamment aux composés des formules suivantes :

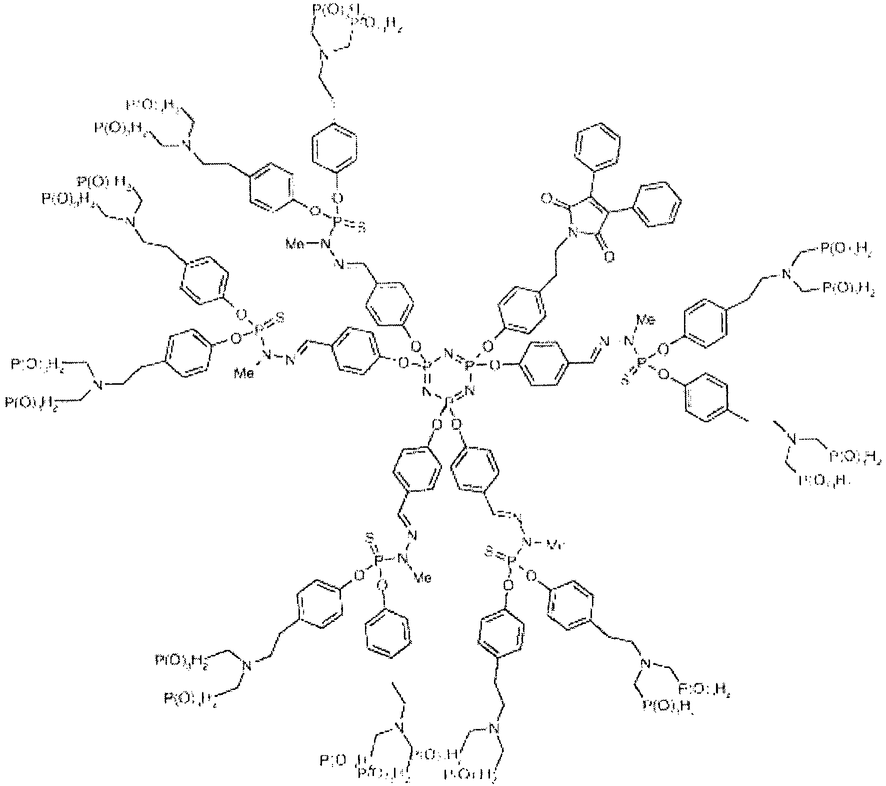

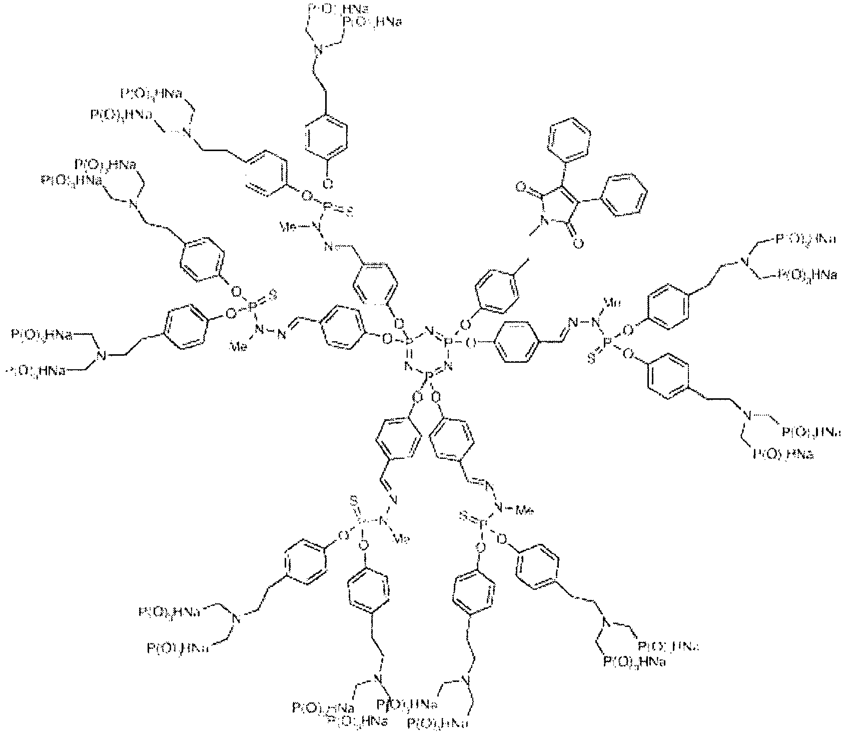

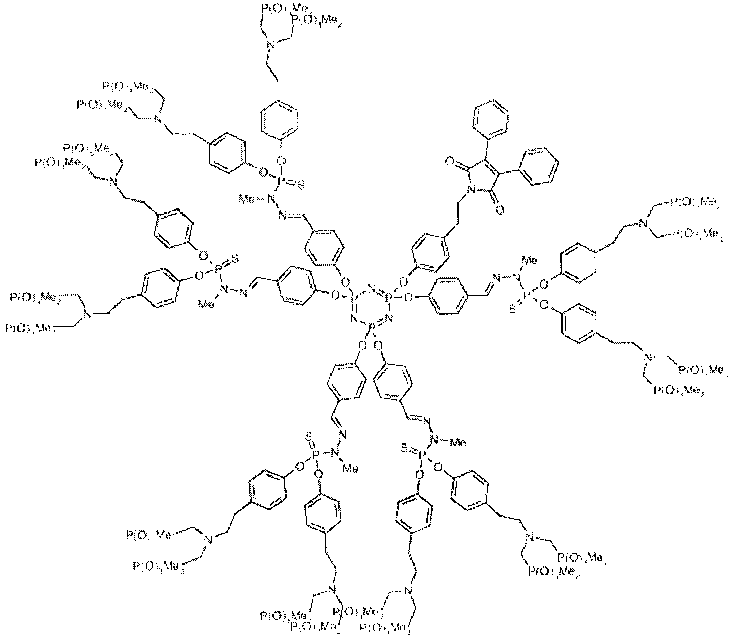

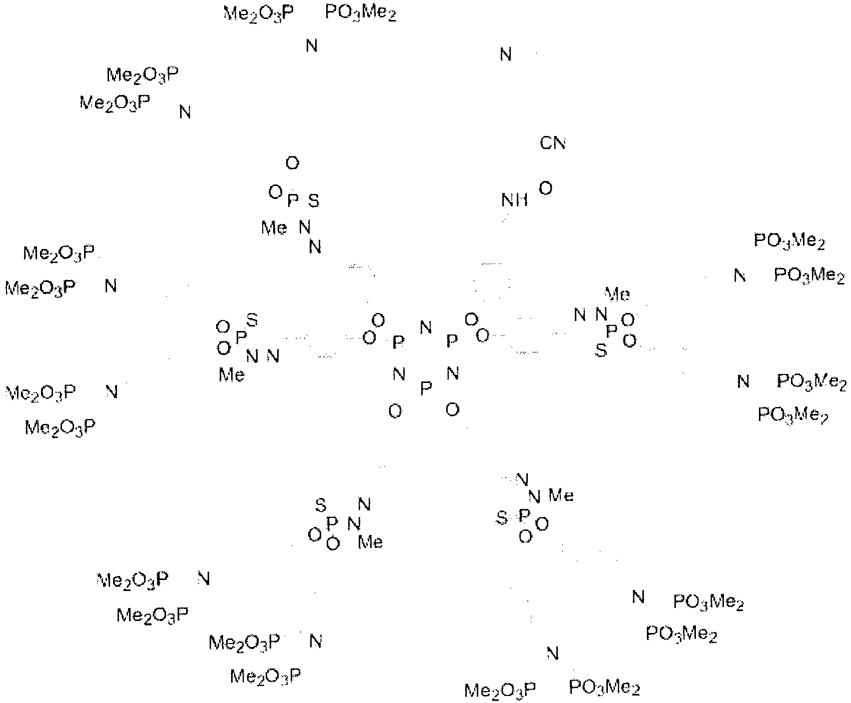

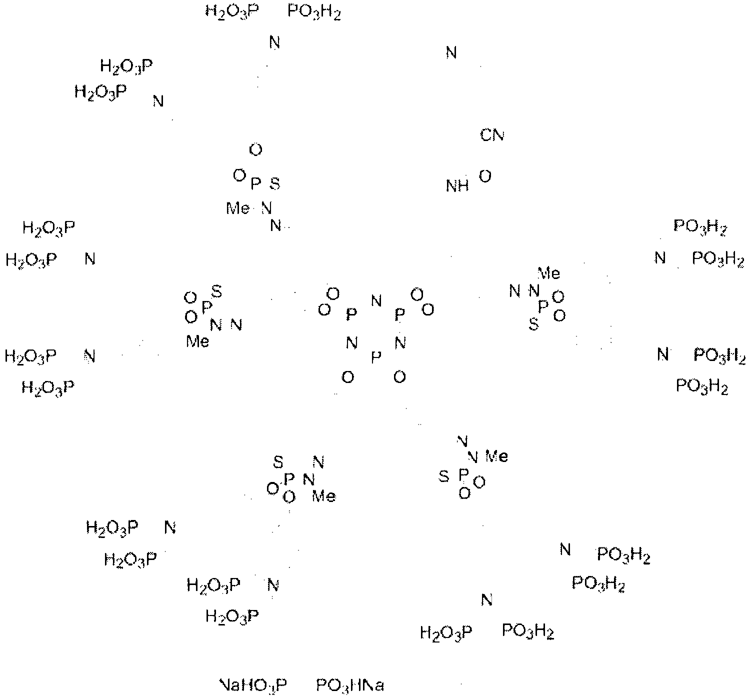

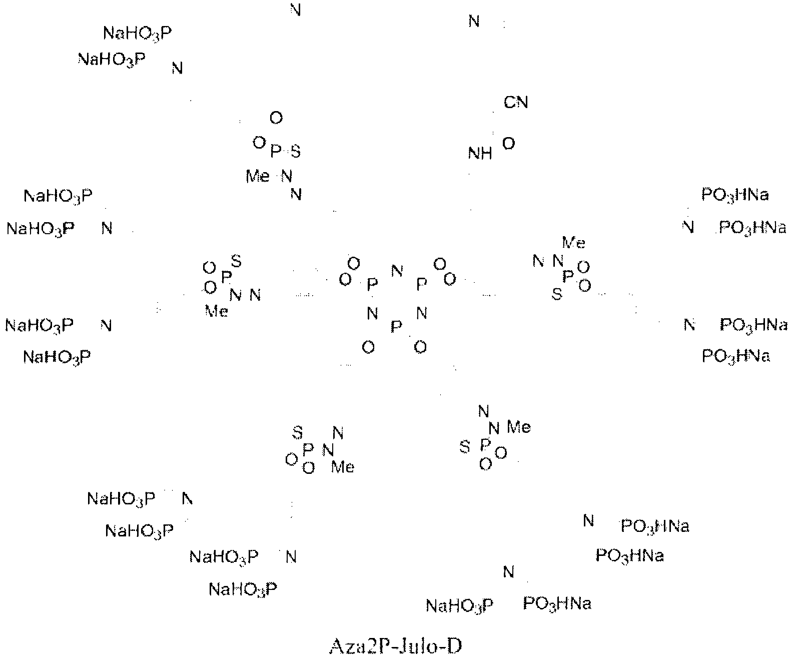

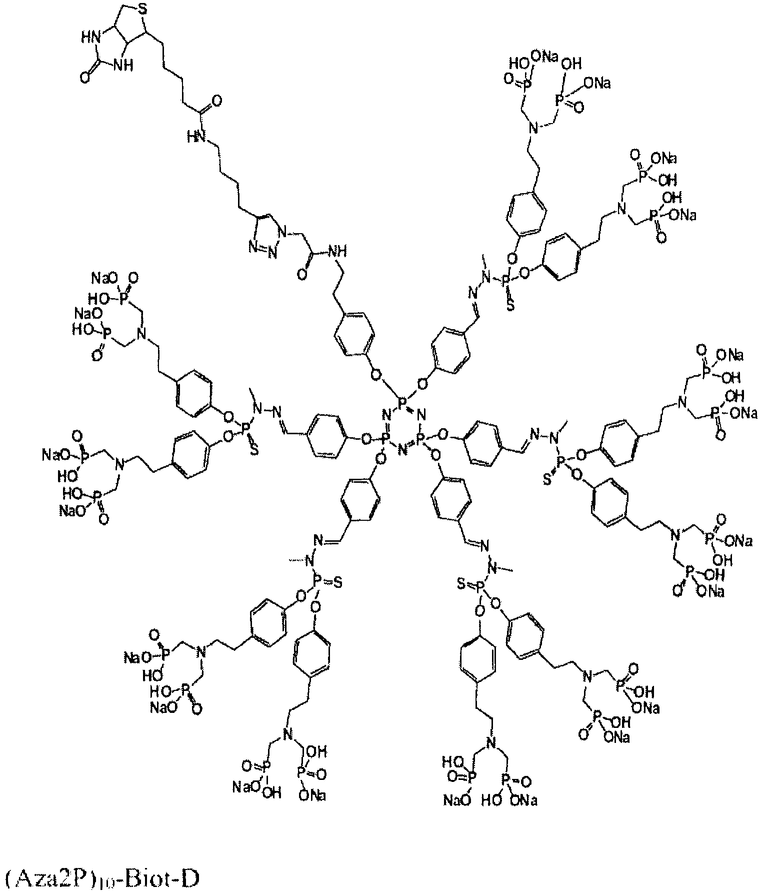

4. Dendrimères pour une utilisation selon la revendication 1, choisis dans le groupe comprenant:
A) 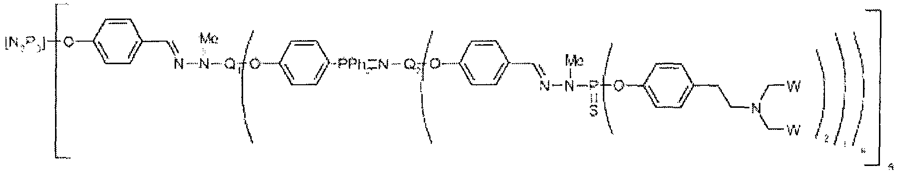 dans laquelle W représente PO₃Me₂, PO₃HNa, ou PO₃H₂, Q₁ et Q₂, identiques ou différents, représentent P = S ou cyclotriphosphazène (N₃P₃), 1 représente 2 lorsque Q₂ représente P=S ou 5 lorsque Q₂ représente N₃P₃ et k représente 2 lorsque Q₁ représente P=S ou 5 lorsque Q₁ représente N₃P₃, les dendrimères étant notamment représentés par les formules suivantes: 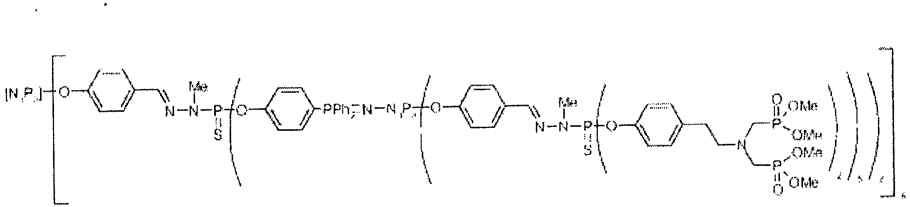 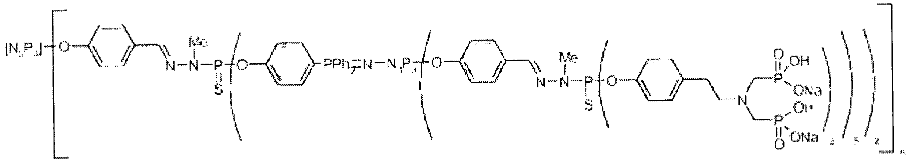 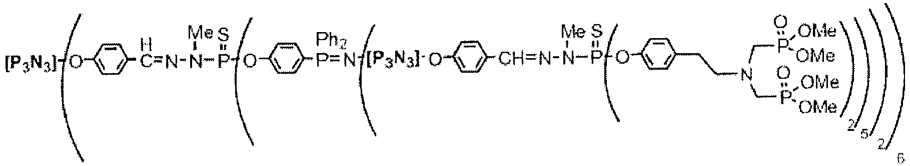 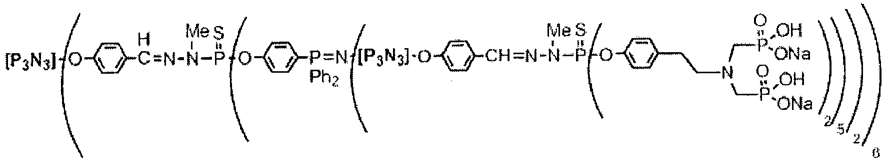 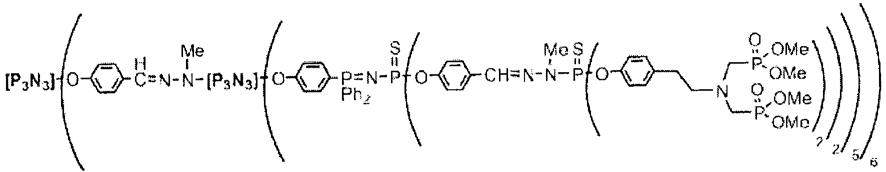 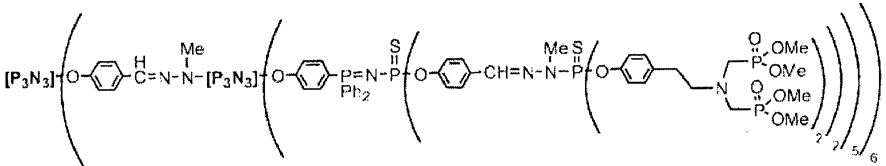 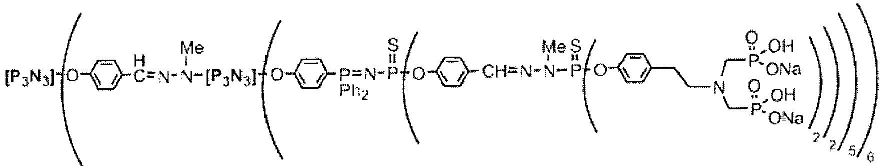 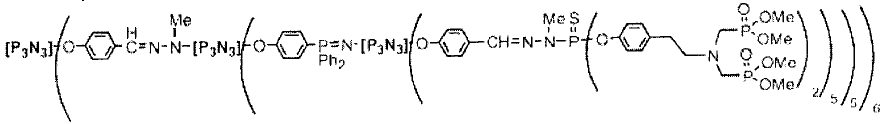 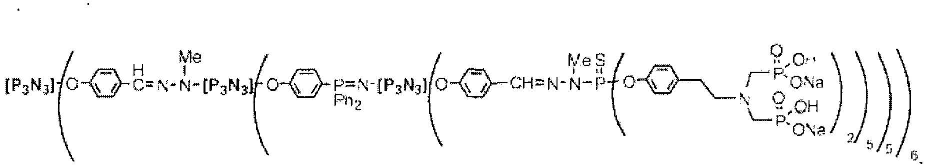
B) 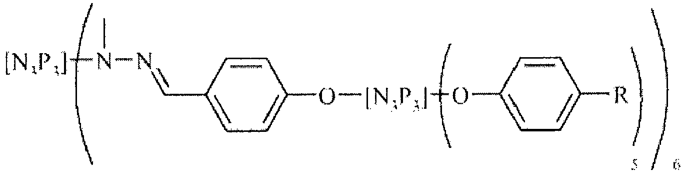 dans laquelle R représente un groupe choisi parmi 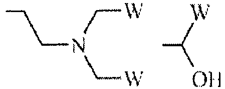 où W représente PO₃Me₂, PO₃HNa, ou PO₃H₂. 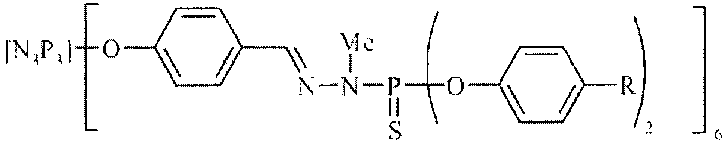 dans laquelle R représente un groupe choisi parmi 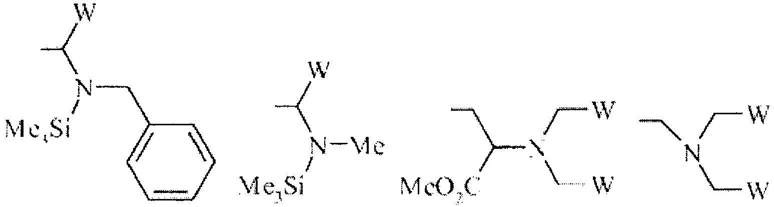 où W représente PO₃Me₂, PO₃HNa, ou PO₃H₂.
D) 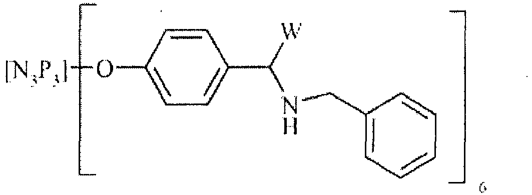 où W représente PO₃Me₂, PO₃HNa, ou PO₃H₂.

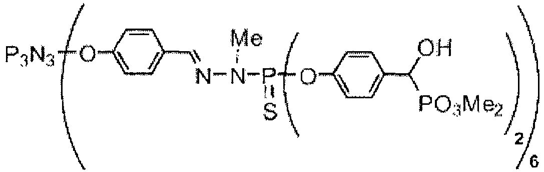

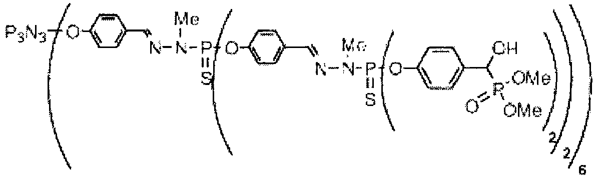

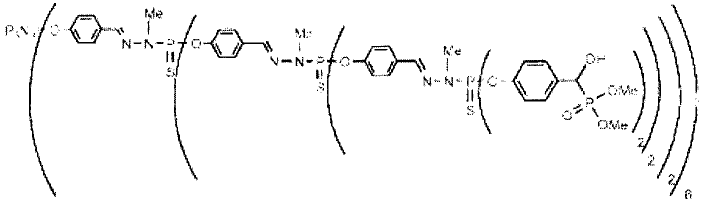

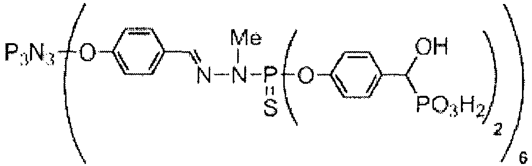

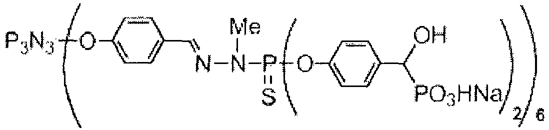

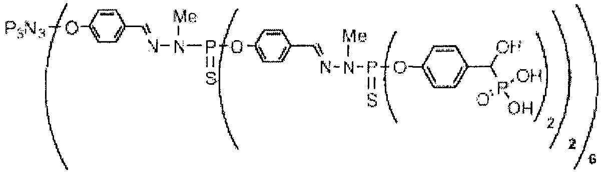

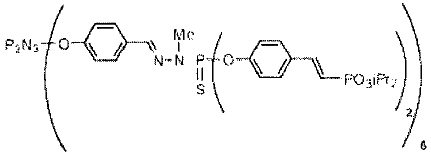

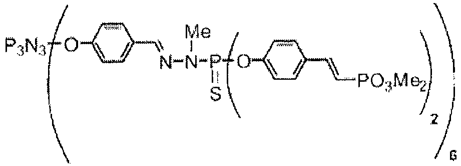

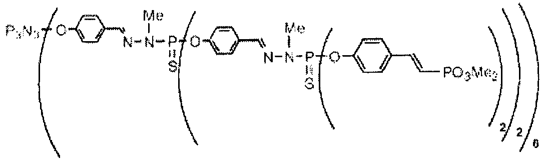

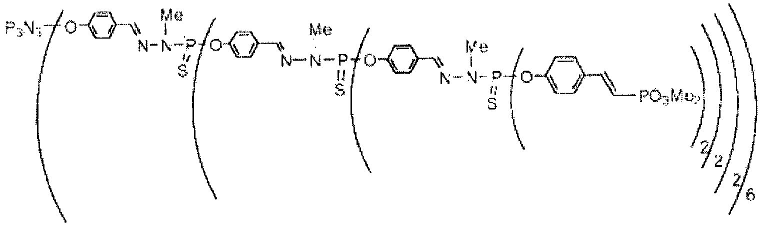

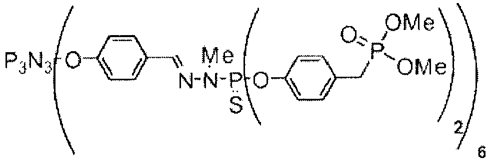

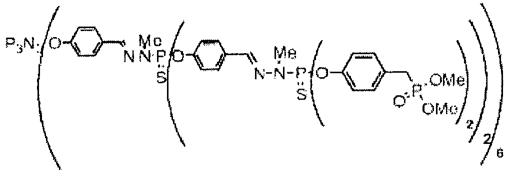

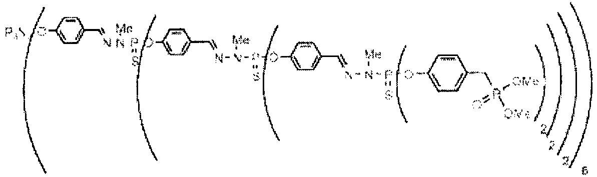

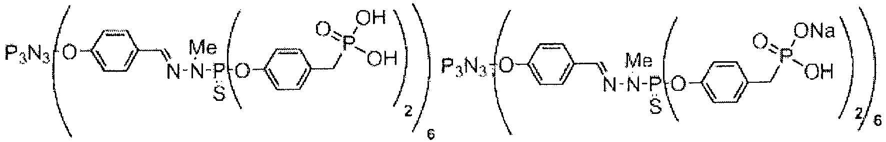

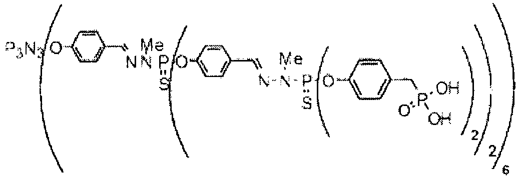

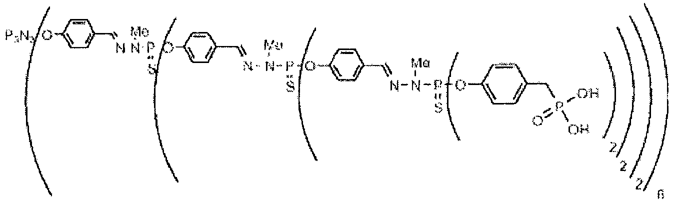

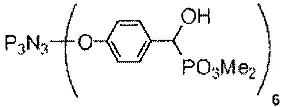

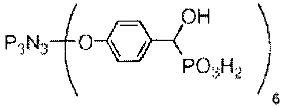

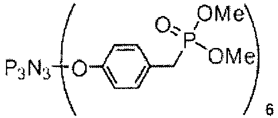

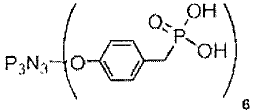

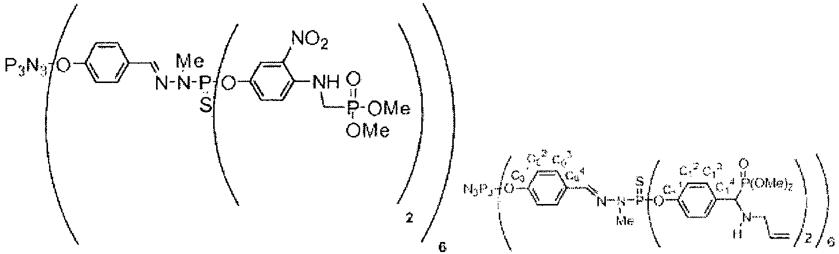

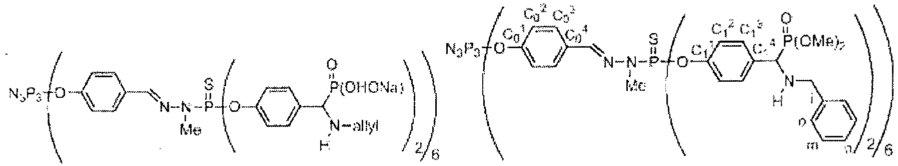

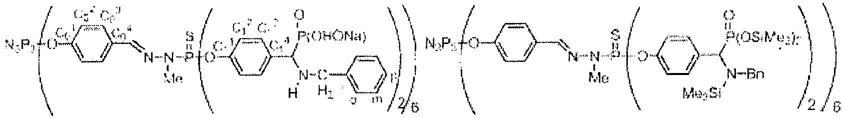

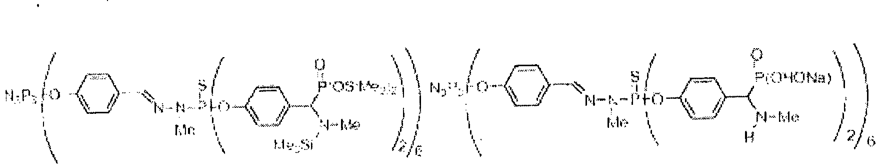

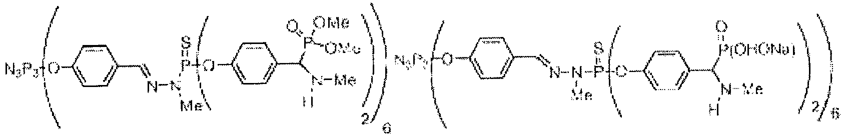

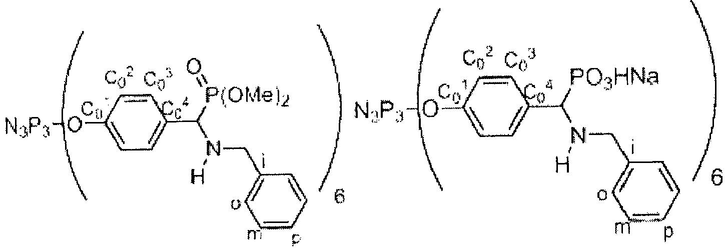

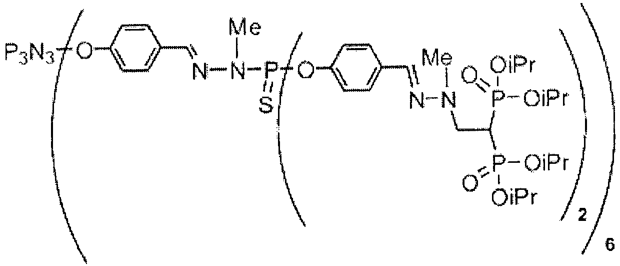

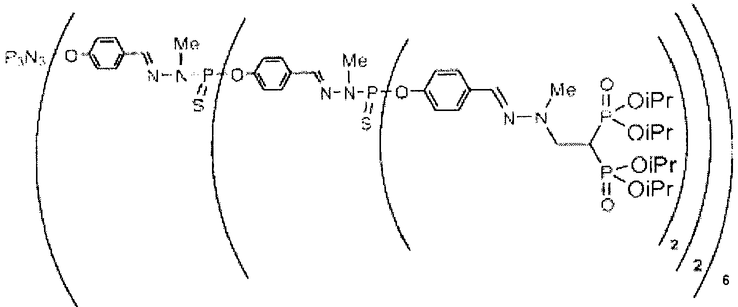

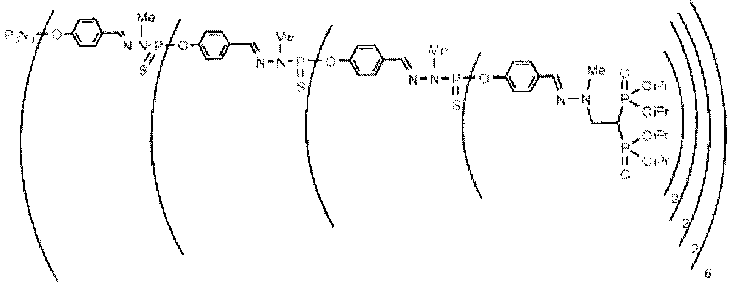

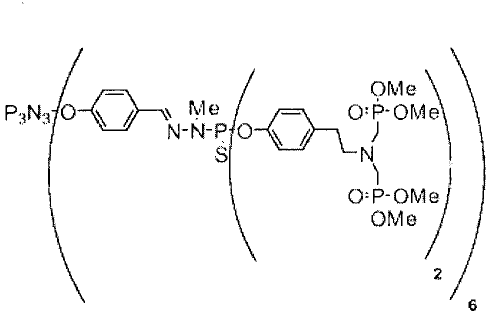

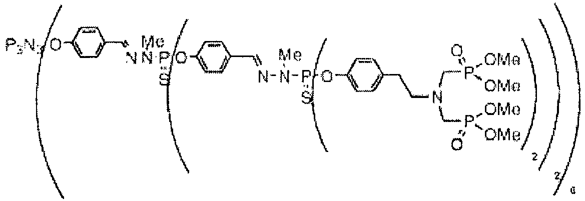

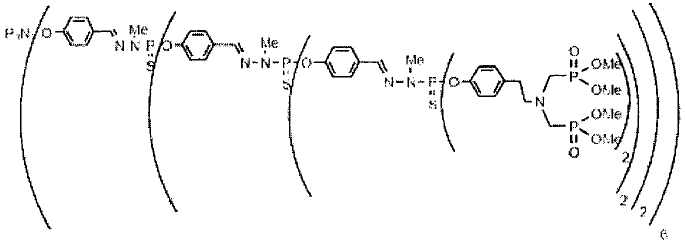

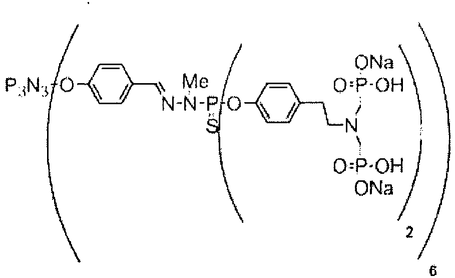

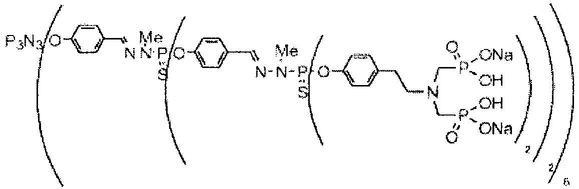

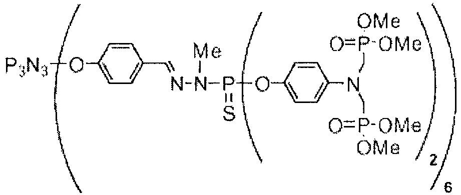

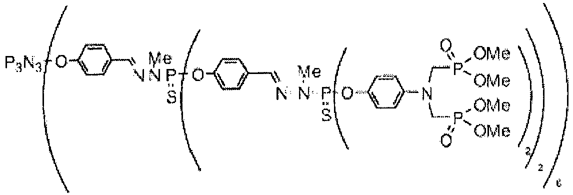

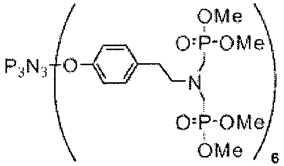

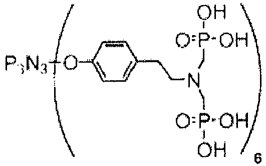

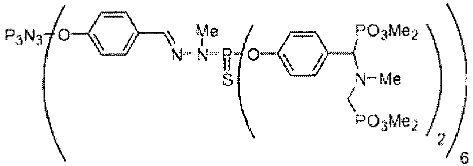

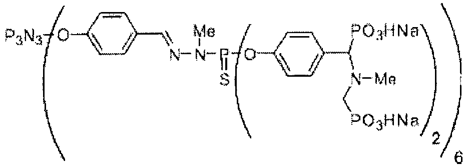

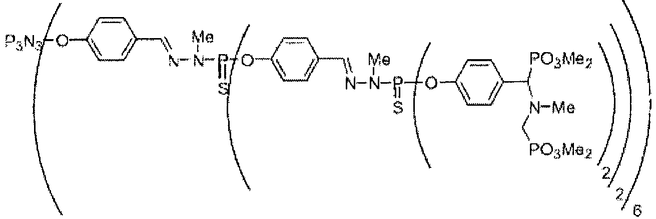

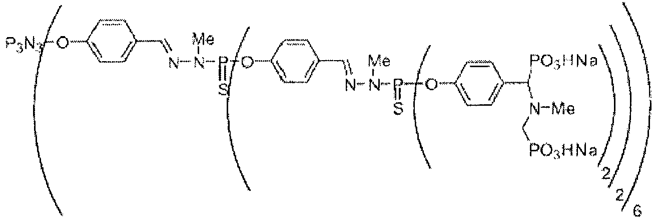

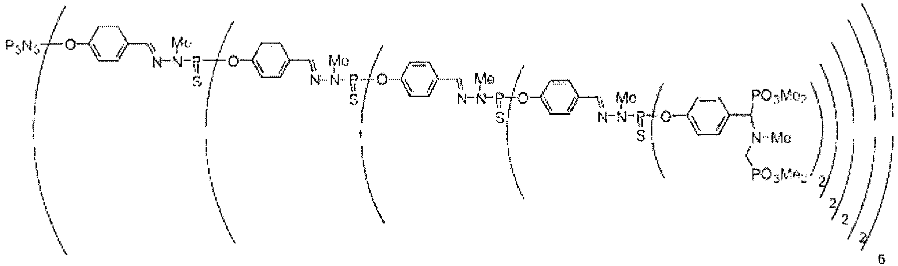

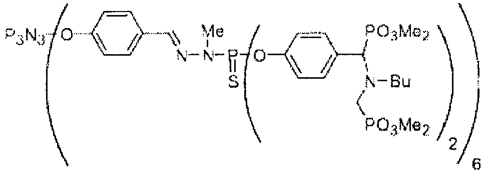

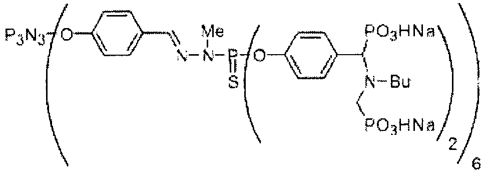

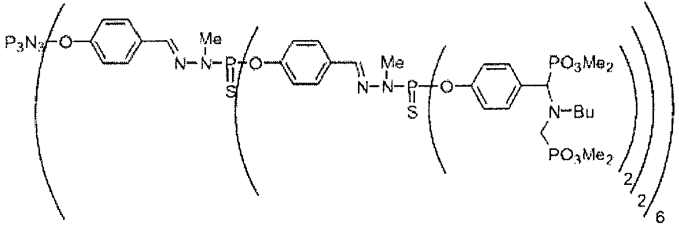

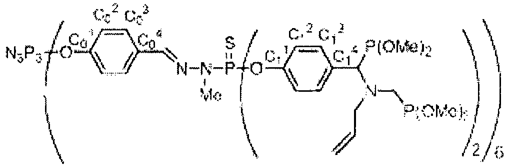

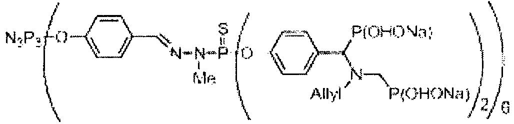

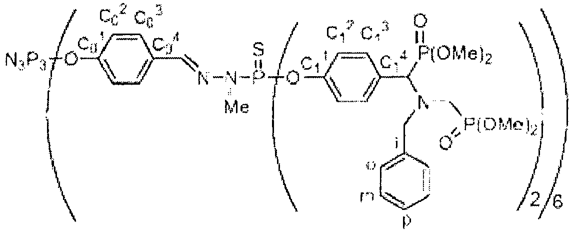

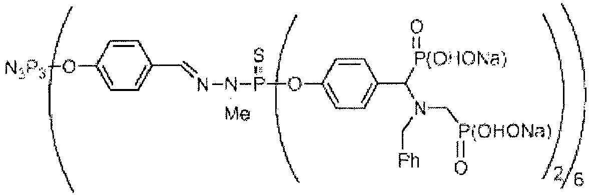

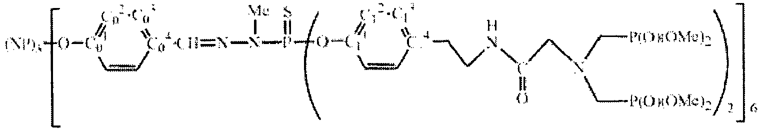

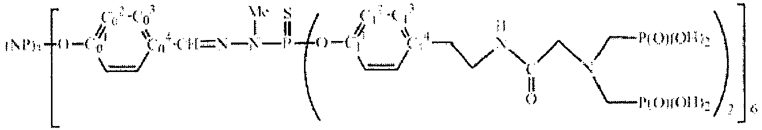

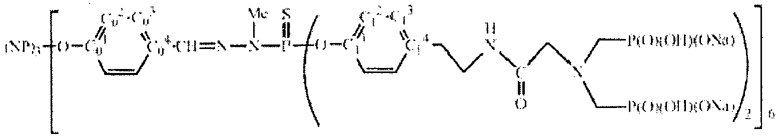

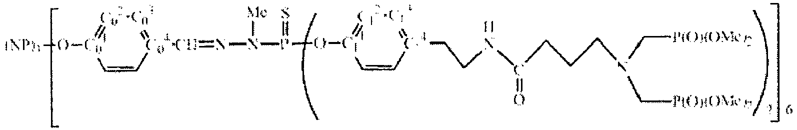

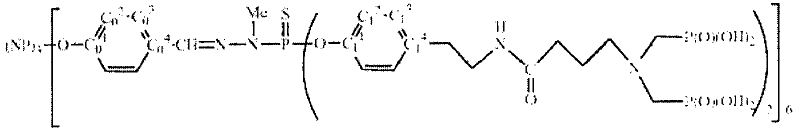

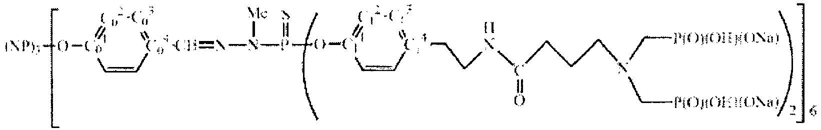

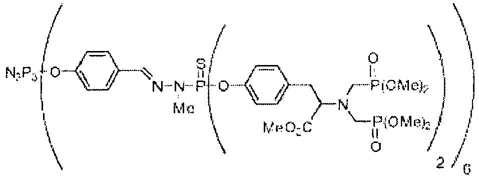

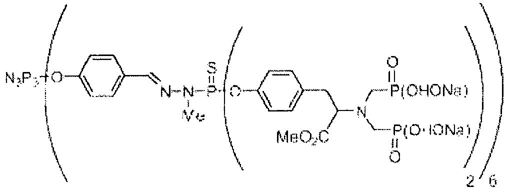

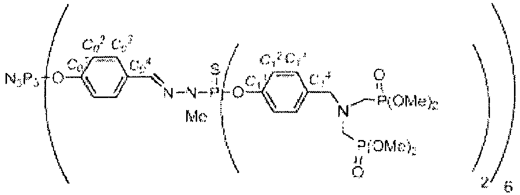

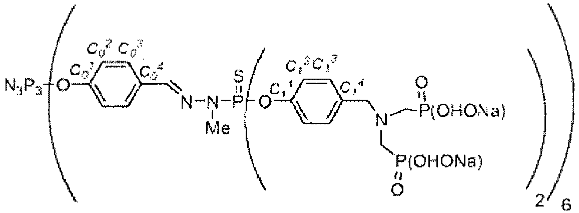

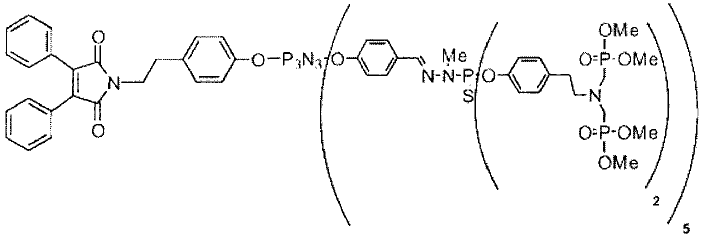

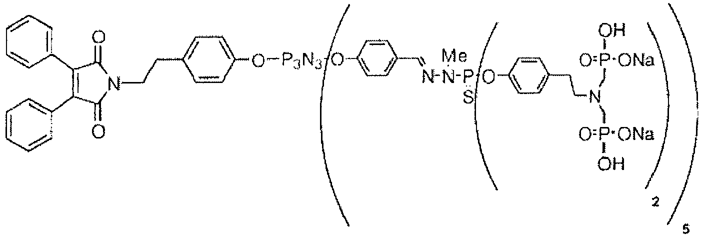

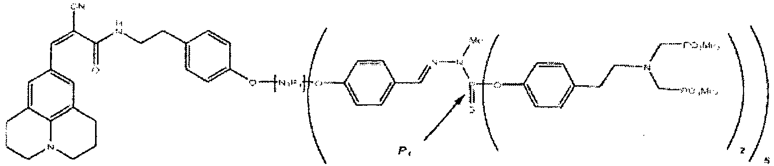

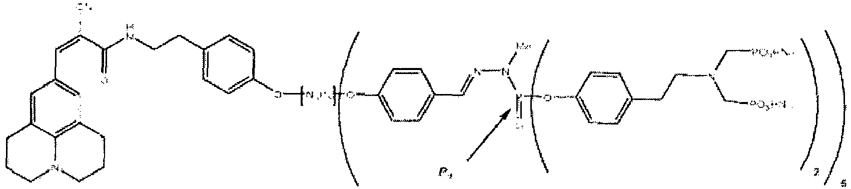

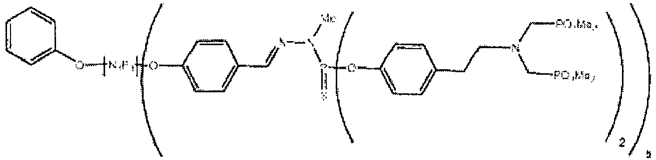

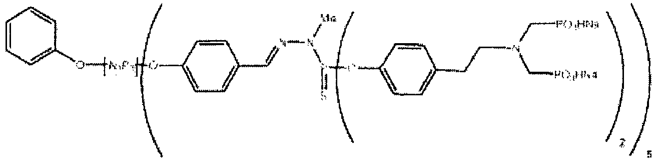

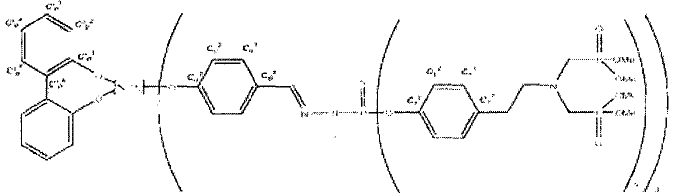

5. Utilisation de dendrimères avec des terminaisons biphosphoniques correspondant à la formule générale (la) suivante définie selon la revendication 1, pour la préparation de médicaments utiles pour le traitement de maladies inflammatoires chroniques.

6. Dendrimères pour une utilisation selon la revendication 1 dans laquelle la maladie inflammatoire chronique est choisie dans le groupe comprenant la polyarthrite rhumatoïde, le psoriasis et l'arthrite idiotypique juvénile.

7. Compositions pharmaceutiques contenant comme substance active au moins un dendrimère avec des terminaisons monophosphoniques ou bisphosphoniques selon l'une quelconque des revendications 1 à 4, associées à un support pharmaceutiquement acceptable pour le traitement de maladies inflammatoires chroniques.
